(19) Europäisches Patentamt
European Patent Office
Office européen des brevets

(11) EP 3 489 262 A1

(12) EUROPEAN PATENT APPLICATION
published in accordance with Art. 153(4) EPC

(43) Date of publication:
**29.05.2019 Bulletin 2019/22**

(21) Application number: **17831354.0**

(22) Date of filing: **19.07.2017**

(51) Int Cl.:
***C07K 16/46*** *(2006.01)*

(86) International application number:
**PCT/KR2017/007791**

(87) International publication number:
**WO 2018/016881 (25.01.2018 Gazette 2018/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **19.07.2016 KR 20160091157**

(71) Applicant: **Ibentrus, Inc.**
**Daejeon 34054 (KR)**

(72) Inventors:
- **KIM, Hoeon**
**Seongnam-si**
**Gyeonggi-do 13597 (KR)**
- **BAE, Sohyun**
**Daejeon 34140 (KR)**

(74) Representative: **van Kooij, Adriaan et al**
**Arnold & Siedsma**
**Bezuidenhoutseweg 57**
**2594 AC The Hague (NL)**

# (54) BISPECIFIC PROTEINS AND METHODS FOR PREPARING SAME

(57) The present invention relates to a bispecific protein and a method preparing the same, wherein mutation is introduced into heavy chains and/or light chains to enhance heterodimerization between a heavy chain (CH3 domain or Fc) and a heavy chain (CH3 domain or Fc) and dimerization between a heavy chain (CH1 domain) and a light chain, both targeting the same material, thereby constructing heterodimeric bispecific proteins of high purity. A bispecific protein according to the present invention can find applications in a variety of fields comprising cancer therapy, singling regulation, diagnosis, etc.

FIG. 1

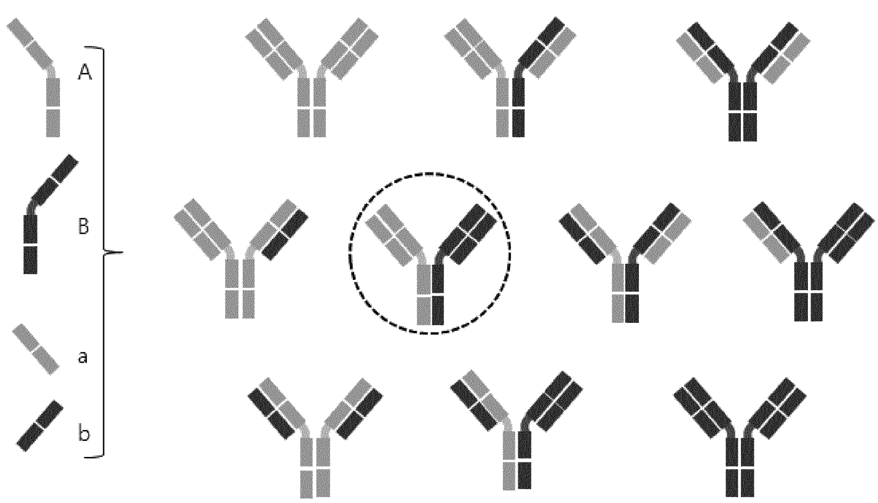




Printed by Jouve, 75001 PARIS (FR)

<110> Ibentrus, Inc.

<120> Bispecific protein and methods of preparation thereof

<130> OPP20173983KR

<150> KR10-2016-0091157
<151> 2016-07-19

<160> 54

<170> KopatentIn 2.0

<210> 1
<211> 108
<212> PRT
<213> Homo sapiens The CH1 domain of human IgG1

<400> 1
Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr
100 105

<210> 2
<211> 105
<212> PRT
<213> Homo sapiens The CH1 domain of human IgG2

<400> 2
Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser

35 40 45

Gly Val His Thr Phe Pro Ala Val Leu Gln Ser Ser Gly Leu Tyr Ser
50 55 60

Leu Ser Ser Val Val Thr Val Pro Ser Ser Asn Phe Gly Thr Gln Thr
65 70 75 80

Tyr Thr Cys Asn Val Asp His Lys Pro Ser Asn Thr Lys Val Asp Lys
85 90 95

Thr Val Glu Arg Lys Cys Cys Val Glu
100 105

<210> 3
<211> 110
<212> PRT
<213> Homo sapiens The CH1 domain of human IgG3

<400> 3
Ala Ser Thr Lys Gly Pro Ser Val Phe Pro Leu Ala Pro Cys Ser Arg
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Thr Cys Asn Val Asn His Lys Pro Ser Asn Thr Lys Val Asp Lys
85 90 95

Arg Val Glu Leu Lys Thr Pro Leu Gly Asp Thr Thr His Thr
100 105 110

<210> 4
<211> 110
<212> PRT
<213> Homo sapiens The CH1 domain of human IgG4

<400> 4
Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Lys Thr
65 70 75 80

Tyr Thr Cys Asn Val Asp His Lys Pro Ser Asn Thr Lys Val Asp Lys
85 90 95

Arg Val Ser Pro Asn Met Val Pro His Ala His His Ala Gln
100 105 110

<210> 5
<211> 108
<212> PRT
<213> Homo sapiens The CH1 domain of human IgA1

<400> 5
Ala Ser Pro Thr Ser Pro Lys Val Phe Pro Leu Ser Leu Cys Ser Thr
1 5 10 15

Gln Pro Asp Gly Asn Val Val Ile Ala Cys Leu Val Gln Gly Phe Phe
20 25 30

Pro Gln Glu Pro Leu Ser Val Thr Trp Ser Glu Ser Gly Gln Gly Val
35 40 45

Thr Ala Arg Asn Phe Pro Pro Ser Gln Asp Ala Ser Gly Asp Leu Tyr
50 55 60

Thr Thr Ser Ser Gln Leu Thr Leu Pro Ala Thr Gln Cys Leu Ala Gly
65 70 75 80

Lys Ser Val Thr Cys His Val Lys His Tyr Thr Asn Pro Ser Gln Asp
85 90 95

Val Thr Val Pro Cys Pro Val Pro Ser Thr Pro Pro
100 105

<210> 6
<211> 108
<212> PRT
<213> Homo sapiens The CH1 domain of human IgA2

<400> 6
Ala Ser Pro Thr Ser Pro Lys Val Phe Pro Leu Ser Leu Asp Ser Thr
1 5 10 15

Pro Gln Asp Gly Asn Val Val Val Ala Cys Leu Val Gln Gly Phe Phe
20 25 30

Pro Gln Glu Pro Leu Ser Val Thr Trp Ser Glu Ser Gly Gln Asn Val
35 40 45

Thr Ala Arg Asn Phe Pro Pro Ser Gln Asp Ala Ser Gly Asp Leu Tyr
50 55 60

Thr Thr Ser Ser Gln Leu Thr Leu Pro Ala Thr Gln Cys Pro Asp Gly
65 70 75 80

Lys Ser Val Thr Cys His Val Lys His Tyr Thr Asn Pro Ser Gln Asp
85 90 95

Val Thr Val Pro Cys Pro Val Pro Pro Pro Pro Pro
100 105

<210> 7
<211> 110
<212> PRT
<213> Homo sapiens The CH1 domain of human IgD

<400> 7
Ala Pro Thr Lys Ala Pro Asp Val Phe Pro Ile Ile Ser Gly Cys Arg
1 5 10 15

His Pro Lys Asp Asn Ser Pro Val Val Leu Ala Cys Leu Ile Thr Gly
20 25 30

Tyr His Pro Thr Ser Val Thr Val Thr Trp Tyr Met Gly Thr Gln Ser
35 40 45

Gln Pro Gln Arg Thr Phe Pro Glu Ile Gln Arg Arg Asp Ser Tyr Tyr
50 55 60

Met Thr Ser Ser Gln Leu Ser Thr Pro Leu Gln Gln Trp Arg Gln Gly
65 70 75 80

Glu Tyr Lys Cys Val Val Gln His Thr Ala Ser Lys Ser Lys Lys Glu
85 90 95

Ile Phe Arg Trp Pro Glu Ser Pro Lys Ala Gln Ala Ser Ser
100 105 110

<210> 8
<211> 110
<212> PRT
<213> Homo sapiens The CH1 domain of human IgE

<400> 8
Ala Ser Thr Gln Ser Pro Ser Val Phe Pro Leu Thr Arg Cys Cys Lys

1 5 10 15

Asn Ile Pro Ser Asn Ala Thr Ser Val Thr Leu Gly Cys Leu Ala Thr
20 25 30

Gly Tyr Phe Pro Glu Pro Val Met Val Thr Cys Asp Thr Gly Ser Leu
35 40 45

Asn Gly Thr Thr Met Thr Leu Pro Ala Thr Thr Leu Thr Leu Ser Gly
50 55 60

His Tyr Ala Thr Ile Ser Leu Leu Thr Val Ser Gly Ala Trp Ala Lys
65 70 75 80

Gln Met Phe Thr Cys Arg Val Ala His Thr Pro Ser Ser Thr Asp Trp
85 90 95

Val Asp Asn Lys Thr Phe Ser Val Cys Ser Arg Asp Phe Thr
100 105 110

<210> 9
<211> 110
<212> PRT
<213> Homo sapiens The CH1 domain of human IgM

<400> 9
Gly Ser Ala Ser Ala Pro Thr Leu Phe Pro Leu Val Ser Cys Glu Asn
1 5 10 15

Ser Pro Ser Asp Thr Ser Ser Val Ala Val Gly Cys Leu Ala Gln Asp
20 25 30

Phe Leu Pro Asp Ser Ile Thr Leu Ser Trp Lys Tyr Lys Asn Asn Ser
35 40 45

Asp Ile Ser Ser Thr Arg Gly Phe Pro Ser Val Leu Arg Gly Gly Lys
50 55 60

Tyr Ala Ala Thr Ser Gln Val Leu Leu Pro Ser Lys Asp Val Met Gln
65 70 75 80

Gly Thr Asp Glu His Val Val Cys Lys Val Gln His Pro Asn Gly Asn
85 90 95

Lys Glu Lys Asn Val Pro Leu Pro Val Ile Ala Glu Leu Pro
100 105 110

<210> 10
<211> 105
<212> PRT
<213> Homo sapiens The CL domain of human kappa chain

<400> 10
Val Ala Ala Pro Ser Val Phe Ile Phe Pro Pro Ser Asp Glu Gln Leu
1 5 10 15

Lys Ser Gly Thr Ala Ser Val Val Cys Leu Leu Asn Asn Phe Tyr Pro
20 25 30

Arg Glu Ala Lys Val Gln Trp Lys Val Asp Asn Ala Leu Gln Ser Gly
35 40 45

Asn Ser Gln Glu Ser Val Thr Glu Gln Asp Ser Lys Asp Ser Thr Tyr
50 55 60

Ser Leu Ser Ser Thr Leu Thr Leu Ser Lys Ala Asp Tyr Glu Lys His
65 70 75 80

Lys Val Tyr Ala Cys Glu Val Thr His Gln Gly Leu Ser Ser Pro Val
85 90 95

Thr Lys Ser Phe Asn Arg Gly Glu Cys
100 105

<210> 11
<211> 103
<212> PRT
<213> Homo sapiens The CL domain of human lambda 1 chain

<400> 11
Lys Ala Asn Pro Thr Val Thr Leu Phe Pro Pro Ser Ser Glu Glu Leu
1 5 10 15

Gln Ala Asn Lys Ala Thr Leu Val Cys Leu Ile Ser Asp Phe Tyr Pro
20 25 30

Gly Ala Val Thr Val Ala Trp Lys Ala Asp Gly Ser Pro Val Lys Ala
35 40 45

Gly Val Glu Thr Thr Lys Pro Ser Lys Gln Ser Asn Asn Lys Tyr Ala
50 55 60

Ala Ser Ser Tyr Leu Ser Leu Thr Pro Glu Gln Trp Lys Ser His Arg
65 70 75 80

Ser Tyr Ser Cys Gln Val Thr His Glu Gly Ser Thr Val Glu Lys Thr
85 90 95

Val Ala Pro Thr Glu Cys Ser
100

<210> 12
<211> 103
<212> PRT

<213> Homo sapiens The CL domain of human lambda 2 chain

<400> 12
Lys Ala Ala Pro Ser Val Thr Leu Phe Pro Pro Ser Ser Glu Glu Leu
1 5 10 15

Gln Ala Asn Lys Ala Thr Leu Val Cys Leu Ile Ser Asp Phe Tyr Pro
20 25 30

Gly Ala Val Thr Val Ala Trp Lys Ala Asp Ser Ser Pro Val Lys Ala
35 40 45

Gly Val Glu Thr Thr Thr Pro Ser Lys Gln Ser Asn Asn Lys Tyr Ala
50 55 60

Ala Ser Ser Tyr Leu Ser Leu Thr Pro Glu Gln Trp Lys Ser His Arg
65 70 75 80

Ser Tyr Ser Cys Gln Val Thr His Glu Gly Ser Thr Val Glu Lys Thr
85 90 95

Val Ala Pro Thr Glu Cys Ser
100

<210> 13
<211> 103
<212> PRT
<213> Homo sapiens The CL domain of human lambda 3 chain

<400> 13
Lys Ala Ala Pro Ser Val Thr Leu Phe Pro Pro Ser Ser Glu Glu Leu
1 5 10 15

Gln Ala Asn Lys Ala Thr Leu Val Cys Leu Ile Ser Asp Phe Tyr Pro
20 25 30

Gly Ala Val Thr Val Ala Trp Lys Ala Asp Ser Ser Pro Val Lys Ala
35 40 45

Gly Val Glu Thr Thr Thr Pro Ser Lys Gln Ser Asn Asn Lys Tyr Ala
50 55 60

Ala Ser Ser Tyr Leu Ser Leu Thr Pro Glu Gln Trp Lys Ser His Lys
65 70 75 80

Ser Tyr Ser Cys Gln Val Thr His Glu Gly Ser Thr Val Glu Lys Thr
85 90 95

Val Ala Pro Thr Glu Cys Ser
100

<210> 14

<211> 103
<212> PRT
<213> Homo sapiens The CL domain of human lambda 7 chain

<400> 14
Lys Ala Ala Pro Ser Val Thr Leu Phe Pro Pro Ser Ser Glu Glu Leu
1 5 10 15

Gln Ala Asn Lys Ala Thr Leu Val Cys Leu Val Ser Asp Phe Tyr Pro
20 25 30

Gly Ala Val Thr Val Ala Trp Lys Ala Asp Gly Ser Pro Val Lys Val
35 40 45

Gly Val Glu Thr Thr Lys Pro Ser Lys Gln Ser Asn Asn Lys Tyr Ala
50 55 60

Ala Ser Ser Tyr Leu Ser Leu Thr Pro Glu Gln Trp Lys Ser His Arg
65 70 75 80

Ser Tyr Ser Cys Arg Val Thr His Glu Gly Ser Thr Val Glu Lys Thr
85 90 95

Val Ala Pro Thr Glu Cys Ser
100

<210> 15
<211> 108
<212> PRT
<213> Homo sapiens The CH3 domain of human IgG1

<400> 15
Lys Gly Gln Pro Arg Glu Pro Gln Val Tyr Thr Leu Pro Pro Ser Arg
1 5 10 15

Glu Glu Met Thr Lys Asn Gln Val Ser Leu Thr Cys Leu Val Lys Gly
20 25 30

Phe Tyr Pro Ser Asp Ile Ala Val Glu Trp Glu Ser Asn Gly Gln Pro
35 40 45

Glu Asn Asn Tyr Lys Thr Thr Pro Pro Val Leu Asp Ser Asp Gly Ser
50 55 60

Phe Phe Leu Tyr Ser Lys Leu Thr Val Asp Lys Ser Arg Trp Gln Gln
65 70 75 80

Gly Asn Val Phe Ser Cys Ser Val Met His Glu Ala Leu His Asn His
85 90 95

Tyr Thr Gln Lys Ser Leu Ser Leu Ser Pro Gly Lys
100 105

<210> 16
<211> 108
<212> PRT
<213> Homo sapiens The CH3 domain of human IgG2

<400> 16
Lys Gly Gln Pro Arg Glu Pro Gln Val Tyr Thr Leu Pro Pro Ser Arg
1 5 10 15

Glu Glu Met Thr Lys Asn Gln Val Ser Leu Thr Cys Leu Val Lys Gly
20 25 30

Phe Tyr Pro Ser Asp Ile Ala Val Glu Trp Glu Ser Asn Gly Gln Pro
35 40 45

Glu Asn Asn Tyr Lys Thr Thr Pro Pro Met Leu Asp Ser Asp Gly Ser
50 55 60

Phe Phe Leu Tyr Ser Lys Leu Thr Val Asp Lys Ser Arg Trp Gln Gln
65 70 75 80

Gly Asn Val Phe Ser Cys Ser Val Met His Glu Ala Leu His Asn His
85 90 95

Tyr Thr Gln Lys Ser Leu Ser Leu Ser Pro Gly Lys
100 105

<210> 17
<211> 108
<212> PRT
<213> Homo sapiens The CH3 domain of human IgG3

<400> 17
Lys Gly Gln Pro Arg Glu Pro Gln Val Tyr Thr Leu Pro Pro Ser Arg
1 5 10 15

Glu Glu Met Thr Lys Asn Gln Val Ser Leu Thr Cys Leu Val Lys Gly
20 25 30

Phe Tyr Pro Ser Asp Ile Ala Val Glu Trp Glu Ser Ser Gly Gln Pro
35 40 45

Glu Asn Asn Tyr Asn Thr Thr Pro Pro Met Leu Asp Ser Asp Gly Ser
50 55 60

Phe Phe Leu Tyr Ser Lys Leu Thr Val Asp Lys Ser Arg Trp Gln Gln
65 70 75 80

Gly Asn Ile Phe Ser Cys Ser Val Met His Glu Ala Leu His Asn Arg
85 90 95

Phe Thr Gln Lys Ser Leu Ser Leu Ser Pro Gly Lys

100 105

<210> 18
<211> 108
<212> PRT
<213> Homo sapiens The CH3 domain of human IgG4

<400> 18
Lys Gly Gln Pro Arg Glu Pro Gln Val Tyr Thr Leu Pro Pro Ser Gln
1 5 10 15

Glu Glu Met Thr Lys Asn Gln Val Ser Leu Thr Cys Leu Val Lys Gly
20 25 30

Phe Tyr Pro Ser Asp Ile Ala Val Glu Trp Glu Ser Asn Gly Gln Pro
35 40 45

Glu Asn Asn Tyr Lys Thr Thr Pro Pro Val Leu Asp Ser Asp Gly Ser
50 55 60

Phe Phe Leu Tyr Ser Arg Leu Thr Val Asp Lys Ser Arg Trp Gln Glu
65 70 75 80

Gly Asn Val Phe Ser Cys Ser Val Met His Glu Ala Leu His Asn His
85 90 95

Tyr Thr Gln Lys Ser Leu Ser Leu Ser Leu Gly Lys
100 105

<210> 19
<211> 120
<212> PRT
<213> Homo sapiens The CH3 domain of human IgA1

<400> 19
Gly Asn Thr Phe Arg Pro Glu Val His Leu Leu Pro Pro Pro Ser Glu
1 5 10 15

Glu Leu Ala Leu Asn Glu Leu Val Thr Leu Thr Cys Leu Ala Arg Gly
20 25 30

Phe Ser Pro Lys Asp Val Leu Val Arg Trp Leu Gln Gly Ser Gln Glu
35 40 45

Leu Pro Arg Glu Lys Tyr Leu Thr Trp Ala Ser Arg Gln Glu Pro Ser
50 55 60

Gln Gly Thr Thr Thr Phe Ala Val Thr Ser Ile Leu Arg Val Ala Ala
65 70 75 80

Glu Asp Trp Lys Lys Gly Asp Thr Phe Ser Cys Met Val Gly His Glu
85 90 95

Ala Leu Pro Leu Ala Phe Thr Gln Lys Thr Ile Asp Arg Leu Ala Gly
100 105 110

Lys Pro Thr His Val Asn Val Ser
115 120

<210> 20
<211> 120
<212> PRT
<213> Homo sapiens The CH3 domain of human IgA2

<400> 20
Gly Asn Thr Phe Arg Pro Glu Val His Leu Leu Pro Pro Pro Ser Glu
1 5 10 15

Glu Leu Ala Leu Asn Glu Leu Val Thr Leu Thr Cys Leu Ala Arg Gly
20 25 30

Phe Ser Pro Lys Asp Val Leu Val Arg Trp Leu Gln Gly Ser Gln Glu
35 40 45

Leu Pro Gln Glu Pro Ser Gln Gly Thr Thr Thr Phe Ala Val Thr Ser
50 55 60

Ile Leu Arg Val Ala Ala Glu Asp Trp Lys Lys Gly Asp Thr Phe Ser
65 70 75 80

Cys Met Val Gly His Glu Ala Leu Pro Leu Ala Phe Thr Gln Lys Thr
85 90 95

Ile Asp Arg Leu Ala Phe Thr Gln Lys Thr Ile Asp Arg Leu Ala Gly
100 105 110

Lys Pro Thr His Val Asn Val Ser
115 120

<210> 21
<211> 119
<212> PRT
<213> Homo sapiens The CH3 domain of human IgD

<400> 21
Ala Ala Gln Ala Pro Val Lys Leu Ser Leu Asn Leu Leu Ala Ser Ser
1 5 10 15

Asp Pro Pro Glu Ala Ala Ser Trp Leu Leu Cys Glu Val Ser Gly Phe
20 25 30

Ser Pro Pro Asn Ile Leu Leu Met Trp Leu Glu Asp Gln Arg Glu Val
35 40 45

Asn Thr Ser Gly Phe Ala Pro Ala Arg Pro Pro Pro Gln Pro Arg Ser
50 55 60

Thr Thr Phe Trp Ala Trp Ser Val Leu Arg Val Pro Ala Pro Pro Ser
65 70 75 80

Pro Gln Pro Ala Thr Tyr Thr Cys Val Val Ser His Glu Asp Ser Arg
85 90 95

Thr Leu Leu Asn Ala Ser Arg Ser Leu Glu Val Ser Tyr Val Thr Asp
100 105 110

His Gly Pro Met Lys Val Lys
115

<210> 22
<211> 120
<212> PRT
<213> Homo sapiens The CH4 domain of human IgE

<400> 22
Ala Ala Pro Glu Val Tyr Ala Phe Ala Thr Pro Glu Trp Pro Gly Ser
1 5 10 15

Arg Asp Lys Arg Thr Leu Ala Cys Leu Ile Gln Asn Phe Met Pro Glu
20 25 30

Asp Ile Ser Val Gln Trp Leu His Asn Glu Val Gln Leu Pro Asp Ala
35 40 45

Arg His Ser Thr Thr Gln Pro Arg Lys Thr Lys Gly Ser Gly Phe Phe
50 55 60

Val Phe Ser Arg Leu Glu Val Thr Arg Ala Glu Trp Glu Gln Lys Asp
65 70 75 80

Glu Phe Ile Cys Arg Ala Val His Glu Ala Ala Ser Pro Ser Gln Thr
85 90 95

Val Gln Arg Ala Val Ser Val Asn Pro Gly Lys Glu Leu Asp Val Cys
100 105 110

Val Glu Glu Ala Glu Gly Glu Ala
115 120

<210> 23
<211> 120
<212> PRT
<213> Homo sapiens The CH3 domain of human IgM

<400> 23
Lys Gly Val Ala Leu His Arg Pro Asp Val Tyr Leu Leu Pro Pro Ala

1 5 10 15

Arg Glu Gln Leu Asn Leu Arg Glu Ser Ala Thr Ile Thr Cys Leu Val
20 25 30

Thr Gly Phe Ser Pro Ala Asp Val Phe Val Gln Trp Met Gln Arg Gly
35 40 45

Gln Pro Leu Ser Pro Glu Lys Tyr Val Thr Ser Ala Pro Met Pro Glu
50 55 60

Pro Gln Ala Pro Gly Arg Tyr Phe Ala His Ser Ile Leu Thr Val Ser
65 70 75 80

Glu Glu Glu Trp Asn Thr Gly Glu Thr Tyr Thr Cys Val Ala His Glu
85 90 95

Ala Leu Pro Asn Arg Val Thr Glu Arg Thr Val Asp Lys Ser Thr Gly
100 105 110

Lys Pro Thr Leu Tyr Asn Val Ser
115 120

<210> 24
<211> 1386
<212> DNA
<213> Artificial Sequence

<220>
<223> CDS of TNFRSF1B (Enbrel : NM_001066), wherein 1-771 region encodes ectodomain

<400> 24
atggcgcccg tcgccgtctg ggccgcgctg gccgtcggac tggagctctg ggctgcggcg 60

cacgccttgc ccgcccaggt ggcatttaca ccctacgccc cggagcccgg gagcacatgc 120

cggctcagag aatactatga ccagacagct cagatgtgct gcagcaaatg ctcgccgggc 180

caacatgcaa aagtcttctg taccaagacc tcggacaccg tgtgtgactc ctgtgaggac 240

agcacataca cccagctctg gaactgggtt cccgagtgct tgagctgtgg ctcccgctgt 300

agctctgacc aggtggaaac tcaagcctgc actcgggaac agaaccgcat ctgcacctgc 360

aggcccggct ggtactgcgc gctgagcaag caggaggggt gccggctgtg cgcgccgctg 420

cgcaagtgcc gcccgggctt cggcgtggcc agaccaggaa ctgaaacatc agacgtggtg 480

tgcaagccct gtgccccggg gacgttctcc aacacgactt catccacgga tatttgcagg 540

ccccaccaga tctgtaacgt ggtggccatc cctgggaatg caagcatgga tgcagtctgc 600

acgtccacgt cccccacccg gagtatggcc ccaggggcag tacacttacc ccagccagtg 660

tccacacgat cccaacacac gcagccaact ccagaaccca gcactgctcc aagcacctcc 720

ttcctgctcc caatgggccc cagcccccca gctgaaggga gcactggcga cttcgctctt 780

ccagttggac tgattgtggg tgtgacagcc ttgggtctac taataatagg agtggtgaac 840

tgtgtcatca tgacccaggt gaaaaagaag cccttgtgcc tgcagagaga agccaaggtg 900

cctcacttgc ctgccgataa ggcccggggt acacagggcc ccgagcagca gcacctgctg 960

atcacagcgc cgagctccag cagcagctcc ctggagagct cggccagtgc gttggacaga 1020

agggcgccca ctcggaacca gccacaggca ccaggcgtgg aggccagtgg ggccggggag 1080

gcccgggcca gcaccgggag ctcagattct tcccctggtg gccatgggac ccaggtcaat 1140

gtcacctgca tcgtgaacgt ctgtagcagc tctgaccaca gctcacagtg ctcctcccaa 1200

gccagctcca caatgggaga cacagattcc agcccctcgg agtccccgaa ggacgagcag 1260

gtccccttct ccaaggagga atgtgccttt cggtcacagc tggagacgcc agagaccctg 1320

ctggggagca ccgaagagaa gcccctgccc cttggagtgc ctgatgctgg gatgaagccc 1380

agttaa 1386

<210> 25
<211> 1008
<212> DNA
<213> Artificial Sequence

<220>
<223> CDS of Fas Receptor (NM_000043), wherein 1-519 region encodes ectodomain

<400> 25
atgctgggca tctggaccct cctacctctg gttcttacgt ctgttgctag attatcgtcc 60

aaaagtgtta atgcccaagt gactgacatc aactccaagg gattggaatt gaggaagact 120

gttactacag ttgagactca gaacttggaa ggcctgcatc atgatggcca attctgccat 180

aagccctgtc ctccaggtga aaggaaagct agggactgca cagtcaatgg ggatgaacca 240

gactgcgtgc cctgccaaga agggaaggag tacacagaca aagcccattt ttcttccaaa 300

tgcagaagat gtagattgtg tgatgaagga catggcttag aagtggaaat aaactgcacc 360

cggacccaga ataccaagtg cagatgtaaa ccaaactttt tttgtaactc tactgtatgt 420

gaacactgtg acccttgcac caaatgtgaa catggaatca tcaaggaatg cacactcacc 480

agcaacacca agtgcaaaga ggaaggatcc agatctaact tggggtggct ttgtcttctt 540

cttttgccaa ttccactaat tgtttgggtg aagagaaagg aagtacagaa aacatgcaga 600

aagcacagaa aggaaaacca aggttctcat gaatctccaa ctttaaatcc tgaaacagtg 660

gcaataaatt tatctgatgt tgacttgagt aaatatatca ccactattgc tggagtcatg 720

acactaagtc aagttaaagg ctttgttcga aagaatggtg tcaatgaagc caaaatagat 780

gagatcaaga atgacaatgt ccaagacaca gcagaacaga aagttcaact gcttcgtaat 840

tggcatcaac ttcatggaaa gaaagaagcg tatgacacat tgattaaaga tctcaaaaaa 900

gccaatcttt gtactcttgc agagaaaatt cagactatca tcctcaagga cattactagt 960

gactcagaaa attcaaactt cagaaatgaa atccaaagct tggtctag 1008

<210> 26
<211> 684
<212> DNA
<213> Artificial Sequence

<220>
<223> CDS of Human Fc (from Human Immunoglobulin G)

<400> 26
gacaaaactc acacatgccc accgtgccca gcacctgaac tcctgggggg accgtcagtc 60

ttcctcttcc ccccaaaacc caaggacacc ctcatgatct cccggacccc tgaggtcaca 120

tgcgtggtgg tggacgtgag ccacgaagac cctgaggtca agttcaactg gtacgtggac 180

ggcgtggagg tgcataatgc caagacaaag ccgcgggagg agcagtacaa cagcacgtac 240

cgtgtggtca gcgtcctcac cgtcctgcac caggactggc tgaatggcaa ggagtacaag 300

tgcaaggtct ccaacaaagc cctcccagcc cccatcgaga aaaccatctc caaagccaaa 360

gggcagcccc gagaaccaca ggtgtacacc ctgcccccat cccgggagga gatgaccaag 420

aaccaggtca gcctgacctg cctggtcaaa ggcttctatc ccagcgacat cgccgtggag 480

tgggagagca atgggcagcc ggagaacaac tacaagacca cgcctcccgt gctggactcc 540

gacggctcct tcttcctcta cagcaagctc accgtggaca agagcaggtg gcagcagggg 600

aacgtcttct catgctccgt gatgcatgag gctctgcaca accactacac gcagaagagc 660

ctctccctgt ctccgggtaa atga 684

<210> 27
<211> 127
<212> PRT
<213> Artificial Sequence

<220>
<223> Heavy Chain Variable Region of anti-Influenza B antibody, 2B9

<400> 27
Glu Val Gln Leu Val Glu Ser Gly Gly Gly Leu Val Gln Pro Gly Lys
1 5 10 15

Ser Leu Arg Leu Ser Cys Ala Ala Thr Gly Phe Thr Phe Asp Asp Tyr
20 25 30

Ala Met His Trp Val Arg Gln Ala Pro Gly Lys Gly Leu Glu Trp Val
35 40 45

Ser Ser Leu Asn Trp Lys Gly Asn Ser Val Asp Tyr Ala Asp Ser Val
50 55 60

Arg Gly Arg Phe Thr Met Ser Arg Asp Asn Ala Lys Lys Leu Val Tyr
65 70 75 80

Leu Gln Met Asn Gly Leu Arg Gly Asp Asp Thr Ala Val Tyr Phe Cys
85 90 95

Ala Lys Asp Asn Lys Ala Asp Ala Ser Met Asp Tyr Tyr Tyr His His
100 105 110

Gly Met Asp Val Trp Gly Gln Gly Thr Thr Val Thr Val Ser Ser
115 120 125

<210> 28
<211> 381
<212> DNA
<213> Artificial Sequence

<220>
<223> Nucleic Acid Sequence Encoding Heavy Chain Variable Region of 2B9

<400> 28
gaagtgcagc tggtggagtc tgggggaggc ttggtacagc ctggcaagtc cctgagactc 60

tcctgtgcag ccactggatt cacatttgac gattacgcca tgcactgggt ccgccaagct 120

ccagggaagg gcctggagtg ggtctcaagt cttaattgga agggaaatag tgtagactac 180

gcggactctg tgaggggccg attcaccatg tccagagaca acgccaagaa actagtgtat 240

ctgcaaatga acggtctgag aggtgacgac acggccgtct atttttgtgc aaaagataat 300

aaagcggatg catctatgga ctactactac caccacggta tggacgtctg ggccaaggg 360

accacggtca ccgtctcctc g 381

<210> 29
<211> 121
<212> PRT
<213> Artificial Sequence

<220>
<223> Heavy Chain Variable Region of anti-Influenza A antibody, 4D9

<400> 29
Gln Val Thr Leu Arg Glu Ser Gly Pro Gly Leu Val Lys Pro Ser Glu
1 5 10 15

Thr Leu Ser Leu Thr Cys Thr Ile Ser Gly Ala Ser Ile Asn Thr Asp
20 25 30

Tyr Trp Ser Trp Ile Arg Gln Pro Pro Gly Lys Gly Leu Glu Trp Ile
35 40 45

Gly Tyr Ile Tyr Tyr Arg Gly Arg Thr Asn Tyr Asn Pro Ser Leu Arg
50 55 60

Ser Arg Val Thr Ile Ser Val Asp Thr Ser Lys Asn Gln Phe Ser Leu
65 70 75 80

Gln Met Thr Ser Met Thr Ala Ala Asp Thr Ala Val Tyr Tyr Cys Ala
85 90 95

Arg Asp Val Thr Gly Ile Ser Arg Glu Asn Ala Phe Asp Ile Trp Gly
100 105 110

Gln Gly Thr Leu Val Thr Val Ser Ser
115 120

<210> 30
<211> 363
<212> DNA
<213> Artificial Sequence

<220>
<223> Nucleic Acid Sequence Encoding Heavy Chain Variable Region of anti-Influenza A antibody, 4D9

<400> 30
caggtcacct tgagggagtc gggcccagga ctggtgaagc cttcggagac cctgtccctc 60

acctgcacta tctccggtgc ctccatcaat actgactact ggagctggat ccggcagccc 120

ccagggaagg gactggagtg gattggctat atctattaca gagggcgcac caactacaac 180

ccctccctca ggagccgagt caccatatca gtagacacgt ccaagaatca attctccctg 240

cagatgacgt ctatgaccgc tgctgacacg gccgtatatt actgtgcgag agatgtgact 300

ggcatcagtc gagaaaatgc ttttgatatc tggggccaag gcaccctggt caccgtctcc 360

tcg 363

<210> 31
<211> 107
<212> PRT
<213> Artificial Sequence

<220>
<223> Common Light Chain for 4D9 and 2B9

<400> 31
Ala Ile Arg Met Thr Gln Ser Pro Ser Ser Leu Ser Ala Ser Val Gly
1 5 10 15

Asp Arg Val Thr Ile Thr Cys Arg Ala Ser Gln Ser Ile Ser Gly Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ser Tyr Ser Ile Pro Thr
85 90 95

Thr Phe Gly Gln Gly Thr Arg Leu Glu Ile Lys
100 105

<210> 32
<211> 321
<212> DNA
<213> Artificial Sequence

<220>
<223> Nucleic Acid Sequence Encoding Common Light Chain for 4D9 and 2B9

<400> 32
gccatccgga tgacccagtc tccatcctcc ctgtctgcat ctgtaggaga cagagtcacc 60

atcacttgcc gggcaagtca gagcattagc ggctatttaa attggtatca gcagaaacca 120

gggaaagccc ctaagctctt gatctatgct gcatccagtt tgcagagtgg ggtcccatca 180

aggttcagtg gcagtggatc tgggacagat ttcactctca ccatcagcag tctgcaacct 240

gaagattttg caacttacta ctgtcaacag agctacagta tccccaccac cttcggccaa 300

gggacacgac tggagattaa a 321

<210> 33
<211> 330
<212> PRT
<213> Artificial Sequence

<220>
<223> Human IgG1 Heavy Constant Region

<400> 33
Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys

130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 34
<211> 335
<212> PRT
<213> Artificial Sequence

<220>
<223> Human IgA1 Heavy Constant Region

<400> 34
Ala Ser Pro Thr Ser Pro Lys Val Phe Pro Leu Ser Leu Cys Ser Thr
1 5 10 15

Gln Pro Asp Gly Asn Val Val Ile Ala Cys Leu Val Gln Gly Phe Phe
20 25 30

Pro Gln Glu Pro Leu Ser Val Thr Trp Ser Glu Ser Gly Gln Gly Val
35 40 45

Thr Ala Arg Asn Phe Pro Pro Ser Gln Asp Ala Ser Gly Asp Leu Tyr
50 55 60

Thr Thr Ser Ser Gln Leu Thr Leu Pro Ala Thr Gln Cys Leu Ala Gly
65 70 75 80

Lys Ser Val Thr Cys His Val Lys His Tyr Thr Asn Pro Ser Gln Asp
85 90 95

Val Thr Val Pro Cys Pro Val Pro Ser Thr Pro Pro Thr Pro Ser Pro
100 105 110

Ser Thr Pro Pro Thr Pro Ser Pro Ser Cys Cys His Pro Arg Leu Ser
115 120 125

Leu His Arg Pro Ala Leu Glu Asp Leu Leu Leu Gly Ser Glu Ala Asn
130 135 140

Leu Thr Cys Thr Leu Thr Gly Leu Arg Asp Ala Ser Gly Val Thr Phe
145 150 155 160

Thr Trp Thr Pro Ser Ser Gly Lys Ser Ala Val Gln Gly Pro Pro Glu
165 170 175

Arg Asp Leu Cys Gly Cys Tyr Ser Val Ser Ser Val Leu Pro Gly Cys
180 185 190

Ala Glu Pro Trp Asn His Gly Lys Thr Phe Thr Cys Thr Ala Ala Tyr
195 200 205

Pro Glu Ser Lys Thr Pro Leu Thr Ala Thr Leu Ser Lys Ser Gly Asn
210 215 220

Thr Phe Arg Pro Glu Val His Leu Leu Pro Pro Pro Ser Glu Glu Leu
225 230 235 240

Ala Leu Asn Glu Leu Val Thr Leu Thr Cys Leu Ala Arg Gly Phe Ser
245 250 255

Pro Lys Asp Val Leu Val Arg Trp Leu Gln Gly Ser Gln Glu Leu Pro
260 265 270

Arg Glu Lys Tyr Leu Thr Trp Ala Ser Arg Gln Glu Pro Ser Gln Gly
275 280 285

Thr Thr Thr Phe Ala Val Thr Ser Ile Leu Arg Val Ala Ala Glu Asp
290 295 300

Trp Lys Lys Gly Asp Thr Phe Ser Cys Met Val Gly His Glu Ala Leu
305 310 315 320

Pro Leu Ala Phe Thr Gln Lys Thr Ile Asp Arg Leu Ala Gly Lys
325 330 335

<210> 35
<211> 228
<212> PRT
<213> Artificial Sequence

<220>
<223> Human Immunoglobulin Light chain (Kappa)

<400> 35
Val Leu Met Leu Leu Leu Leu Trp Val Ser Gly Thr Cys Gly Asp Ile
1 5 10 15

Leu Leu Thr Gln Ser Pro Ala Ile Leu Ser Val Ser Pro Gly Glu Arg
20 25 30

Val Ser Phe Ser Cys Arg Ala Ser Gln Phe Val Gly Ser Ser Ile His
35 40 45

Trp Tyr Gln Gln Arg Thr Asn Gly Ser Pro Arg Leu Leu Ile Lys Tyr
50 55 60

Ala Ser Glu Ser Met Ser Gly Ile Pro Ser Arg Phe Ser Gly Ser Gly
65 70 75 80

Ser Gly Thr Asp Phe Thr Leu Ser Ile Asn Thr Val Glu Ser Glu Asp
85 90 95

Ile Ala Asp Tyr Tyr Cys Gln Gln Ser His Ser Trp Pro Phe Thr Phe
100 105 110

Gly Ser Gly Thr Asn Leu Glu Val Lys Arg Thr Val Ala Ala Pro Ser
115 120 125

Val Phe Ile Phe Pro Pro Ser Asp Glu Gln Leu Lys Ser Gly Thr Ala
130 135 140

Ser Val Val Cys Leu Leu Asn Asn Phe Tyr Pro Arg Glu Ala Lys Val
145 150 155 160

Gln Trp Lys Val Asp Asn Ala Leu Gln Ser Gly Asn Ser Gln Glu Ser
165 170 175

Val Thr Glu Gln Asp Ser Lys Asp Ser Thr Tyr Ser Leu Ser Ser Thr
180 185 190

Leu Thr Leu Ser Lys Ala Asp Tyr Glu Lys His Lys Val Tyr Ala Cys
195 200 205

Glu Val Thr His Gln Gly Leu Ser Ser Pro Val Thr Lys Ser Phe Asn
210 215 220

Arg Gly Glu Cys
225

<210> 36
<211> 226
<212> PRT
<213> Artificial Sequence

<220>
<223> Human Immunoglobulin Light chain (Lambda)

<400> 36
Leu Phe Leu Gly Val Leu Ala Tyr Cys Thr Gly Ser Val Ala Ser Tyr
1 5 10 15

Glu Leu Thr Gln Pro Pro Leu Val Ser Val Ser Pro Gly Gln Thr Ala
20 25 30

Ser Ile Thr Cys Ser Gly Asp Lys Leu Gly Asp Asn Tyr Ala Cys Trp
35 40 45

Tyr Gln Leu Lys Pro Gly Gln Ser Pro Val Leu Val Ile Tyr Gln Asp
50 55 60

Asn Arg Arg Pro Ser Gly Ile Pro Glu Arg Ile Ser Gly Ser Asn Ser
65 70 75 80

Gly Asn Thr Ala Thr Leu Thr Ile Ser Gly Thr Gln Ala Met Asp Glu
85 90 95

Ala Glu Tyr Tyr Cys Gln Ala Trp Asp Ser Ser Thr Ser Trp Val Phe
100 105 110

Gly Gly Gly Thr Lys Leu Thr Val Leu Gly Gln Pro Lys Ala Ala Pro
115 120 125

Ser Val Thr Leu Phe Pro Pro Ser Ser Glu Glu Leu Gln Ala Asn Lys
130 135 140

Ala Thr Leu Val Cys Leu Ile Ser Asp Phe Tyr Pro Gly Ala Val Thr
145 150 155 160

Val Ala Trp Lys Ala Asp Ser Ser Pro Val Lys Ala Gly Val Glu Thr
165 170 175

Thr Thr Pro Ser Lys Gln Ser Asn Asn Lys Tyr Ala Ala Ser Ser Tyr
180 185 190

Leu Ser Leu Thr Pro Glu Gln Trp Lys Ser His Arg Ser Tyr Ser Cys

195 200 205

Gln Val Thr His Glu Gly Ser Thr Val Glu Lys Thr Val Ala Pro Thr
210 215 220

Glu Cys
225

<210> 37
<211> 97
<212> PRT
<213> Artificial Sequence

<220>
<223> CH1 domain of mouse IgG1

<400> 37
Ala Lys Thr Thr Pro Pro Ser Val Tyr Pro Leu Ala Pro Gly Ser Ala
1 5 10 15

Ala Gln Thr Asn Ser Met Val Thr Leu Gly Cys Leu Val Lys Gly Tyr
20 25 30

Phe Pro Glu Pro Val Thr Val Thr Trp Asn Ser Gly Ser Leu Ser Ser
35 40 45

Gly Val His Thr Phe Pro Ala Val Leu Gln Ser Asp Leu Tyr Thr Leu
50 55 60

Ser Ser Ser Val Thr Val Pro Ser Ser Pro Arg Pro Ser Glu Thr Val
65 70 75 80

Thr Cys Asn Val Ala His Pro Ala Ser Ser Thr Lys Val Asp Lys Lys
85 90 95

Ile

<210> 38
<211> 97
<212> PRT
<213> Artificial Sequence

<220>
<223> CH1 domain of mouse IgG2ab

<400> 38
Ala Lys Thr Thr Ala Pro Ser Val Tyr Pro Leu Ala Pro Val Cys Gly
1 5 10 15

Gly Thr Thr Gly Ser Ser Val Thr Leu Gly Cys Leu Val Lys Gly Tyr
20 25 30

Phe Pro Glu Pro Val Thr Leu Thr Trp Asn Ser Gly Ser Leu Ser Ser
35 40 45

Gly Val His Thr Phe Pro Ala Leu Leu Gln Ser Gly Leu Tyr Thr Leu
50 55 60

Ser Ser Ser Val Thr Val Thr Ser Asn Thr Trp Pro Ser Gln Thr Ile
65 70 75 80

Thr Cys Asn Val Ala His Pro Ala Ser Ser Thr Lys Val Asp Lys Lys
85 90 95

Ile

<210> 39
<211> 97
<212> PRT
<213> Artificial Sequence

<220>
<223> CH1 domain of mouse IgG2aa

<400> 39
Ala Lys Thr Thr Ala Pro Ser Val Tyr Pro Leu Ala Pro Val Cys Gly
1 5 10 15

Asp Thr Thr Gly Ser Ser Val Thr Leu Gly Cys Leu Val Lys Gly Tyr
20 25 30

Phe Pro Glu Pro Val Thr Leu Thr Trp Asn Ser Gly Ser Leu Ser Ser
35 40 45

Gly Val His Thr Phe Pro Ala Val Leu Gln Ser Asp Leu Tyr Thr Leu
50 55 60

Ser Ser Ser Val Thr Val Thr Ser Ser Thr Trp Pro Ser Gln Ser Ile
65 70 75 80

Thr Cys Asn Val Ala His Pro Ala Ser Ser Thr Lys Val Asp Lys Lys
85 90 95

Ile

<210> 40
<211> 97
<212> PRT

<213> Artificial Sequence

<220>
<223> CH1 domain of mouse IgG2b

<400> 40
Ala Lys Thr Thr Pro Pro Ser Val Tyr Pro Leu Ala Pro Gly Cys Gly
1 5 10 15

Asp Thr Thr Gly Ser Ser Val Thr Ser Gly Cys Leu Val Lys Gly Tyr
20 25 30

Phe Pro Glu Pro Val Thr Val Thr Trp Asn Ser Gly Ser Leu Ser Ser
35 40 45

Ser Val His Thr Phe Pro Ala Leu Leu Gln Ser Gly Leu Tyr Thr Met
50 55 60

Ser Ser Ser Val Thr Val Pro Ser Ser Thr Trp Pro Ser Gln Thr Val
65 70 75 80

Thr Cys Ser Val Ala His Pro Ala Ser Ser Thr Thr Val Asp Lys Lys
85 90 95

Leu

<210> 41
<211> 96
<212> PRT
<213> Artificial Sequence

<220>
<223> CH1 domain of mouse IgG3

<400> 41
Thr Thr Thr Ala Pro Ser Val Tyr Pro Leu Val Pro Gly Cys Ser Asp
1 5 10 15

Thr Ser Gly Ser Ser Val Thr Leu Gly Cys Leu Val Lys Gly Tyr Phe
20 25 30

Pro Glu Pro Val Thr Val Lys Trp Asn Tyr Gly Ala Leu Ser Ser Gly
35 40 45

Val Arg Thr Val Ser Ser Val Leu Gln Ser Gly Phe Tyr Ser Leu Ser
50 55 60

Ser Leu Val Thr Val Pro Ser Ser Thr Trp Pro Ser Gln Thr Val Ile
65 70 75 80

Cys Asn Val Ala His Pro Ala Ser Lys Thr Glu Leu Ile Lys Arg Ile
85 90 95

<210> 42
<211> 97
<212> PRT
<213> Artificial Sequence

<220>
<223> CH1 domain of rat IgG1

<400> 42
Ala Glu Thr Thr Ala Pro Ser Val Tyr Pro Leu Ala Pro Gly Thr Ala
1 5 10 15

Leu Lys Ser Asn Ser Met Val Thr Leu Gly Cys Leu Val Lys Gly Tyr
20 25 30

Phe Pro Glu Pro Val Thr Val Thr Trp Asn Ser Gly Ala Leu Ser Ser
35 40 45

Gly Val His Thr Phe Pro Ala Val Leu Gln Ser Gly Leu Tyr Thr Leu
50 55 60

Thr Ser Ser Val Thr Val Pro Ser Ser Thr Trp Ser Ser Gln Ala Val
65 70 75 80

Thr Cys Asn Val Ala His Pro Ala Ser Ser Thr Lys Val Asp Lys Lys
85 90 95

Ile

<210> 43
<211> 97
<212> PRT
<213> Artificial Sequence

<220>
<223> CH1 domain of rat IgG2a

<400> 43
Ala Glu Thr Thr Ala Pro Ser Val Tyr Pro Leu Ala Pro Gly Thr Ala
1 5 10 15

Leu Lys Ser Asn Ser Met Val Thr Leu Gly Cys Leu Val Lys Gly Tyr
20 25 30

Phe Pro Glu Pro Val Thr Val Thr Trp Asn Ser Gly Ala Leu Ser Ser
35 40 45

Gly Val His Thr Phe Pro Ala Val Leu Gln Ser Gly Leu Tyr Thr Leu
50 55 60

Thr Ser Ser Val Thr Val Pro Ser Ser Thr Trp Pro Ser Gln Thr Val
65 70 75 80

Thr Cys Asn Val Ala His Pro Ala Ser Ser Thr Lys Val Asp Lys Lys
85 90 95

Ile

<210> 44
<211> 95
<212> PRT
<213> Artificial Sequence

<220>
<223> CH1 domain of rat IgG2b

<400> 44
Ala Gln Thr Thr Ala Pro Ser Val Tyr Pro Leu Ala Pro Gly Cys Gly
1 5 10 15

Asp Thr Thr Ser Ser Thr Val Thr Leu Gly Cys Leu Val Lys Gly Tyr
20 25 30

Phe Pro Glu Pro Val Thr Val Thr Trp Asn Ser Gly Ala Leu Ser Ser
35 40 45

Asp Val His Thr Phe Pro Ala Val Leu Gln Ser Gly Leu Tyr Thr Leu
50 55 60

Thr Ser Ser Val Thr Ser Ser Thr Trp Pro Ser Gln Thr Val Thr Cys
65 70 75 80

Asn Val Ala His Pro Ala Ser Ser Thr Lys Val Asp Lys Lys Val
85 90 95

<210> 45
<211> 97
<212> PRT
<213> Artificial Sequence

<220>
<223> CH1 domain of rat IgG2c

<400> 45
Ala Thr Thr Thr Ala Pro Ser Val Tyr Pro Leu Val Pro Gly Cys Ser
1 5 10 15

Gly Thr Ser Gly Ser Leu Val Thr Leu Gly Cys Leu Val Lys Gly Tyr
20 25 30

Phe Pro Glu Pro Val Thr Val Lys Trp Asn Ser Gly Ala Leu Ser Ser
35 40 45

Gly Val His Thr Phe Pro Ala Val Leu Gln Ser Gly Leu Tyr Thr Leu
50 55 60

Ser Ser Ser Val Thr Val Pro Ser Ser Thr Trp Ser Ser Gln Thr Val
65 70 75 80

Thr Cys Ser Val Ala His Pro Ala Thr Lys Ser Asn Leu Ile Lys Arg
85 90 95

Ile

<210> 46
<211> 107
<212> PRT
<213> Artificial Sequence

<220>
<223> CH3 domain of mouse IgG1

<400> 46
Gly Arg Pro Lys Ala Pro Gln Val Tyr Thr Ile Pro Pro Pro Lys Glu
1 5 10 15

Gln Met Ala Lys Asp Lys Val Ser Leu Thr Cys Met Ile Thr Asp Phe
20 25 30

Phe Pro Glu Asp Ile Thr Val Glu Trp Gln Trp Asn Gly Gln Pro Ala
35 40 45

Glu Asn Tyr Lys Asn Thr Gln Pro Ile Met Asn Thr Asn Gly Ser Tyr
50 55 60

Phe Val Tyr Ser Lys Leu Asn Val Gln Lys Ser Asn Trp Glu Ala Gly
65 70 75 80

Asn Thr Phe Thr Cys Ser Val Leu His Glu Gly Leu His Asn His His
85 90 95

Thr Glu Lys Ser Leu Ser His Ser Pro Gly Lys
100 105

<210> 47
<211> 107
<212> PRT
<213> Artificial Sequence

<220>
<223> CH3 domain of mouse IgG2ab

<400> 47
Gly Pro Val Arg Ala Pro Gln Val Tyr Val Leu Pro Pro Pro Ala Glu
1 5 10 15

Glu Met Thr Lys Lys Glu Phe Ser Leu Thr Cys Met Ile Thr Gly Phe
20 25 30

Leu Pro Ala Glu Ile Ala Val Asp Trp Thr Ser Asn Gly Arg Thr Glu
35 40 45

Gln Asn Tyr Lys Asn Thr Ala Thr Val Leu Asp Ser Asp Gly Ser Tyr
50 55 60

Phe Met Tyr Ser Lys Leu Arg Val Gln Lys Ser Thr Trp Glu Arg Gly
65 70 75 80

Ser Leu Phe Ala Cys Ser Val Val His Glu Val Leu His Asn His Leu
85 90 95

Thr Thr Lys Thr Ile Ser Arg Ser Leu Gly Lys
100 105

<210> 48
<211> 107
<212> PRT
<213> Artificial Sequence

<220>
<223> CH3 domain of mouse IgG2aa

<400> 48
Gly Ser Val Arg Ala Pro Gln Val Tyr Val Leu Pro Pro Pro Glu Glu
1 5 10 15

Glu Met Thr Lys Lys Gln Val Thr Leu Thr Cys Met Val Thr Asp Phe
20 25 30

Met Pro Glu Asp Ile Tyr Val Glu Trp Thr Asn Asn Gly Lys Thr Glu
35 40 45

Leu Asn Tyr Lys Asn Thr Glu Pro Val Leu Asp Ser Asp Gly Ser Tyr
50 55 60

Phe Met Tyr Ser Lys Leu Arg Val Glu Lys Lys Asn Trp Val Glu Arg
65 70 75 80

Asn Ser Tyr Ser Cys Ser Val Val His Glu Gly Leu His Asn His His
85 90 95

Thr Thr Lys Ser Phe Ser Arg Thr Pro Gly Lys
100 105

<210> 49
<211> 107
<212> PRT
<213> Artificial Sequence

<220>
<223> CH3 domain of mouse IgG2b

<400> 49
Gly Leu Val Arg Ala Pro Gln Val Tyr Thr Leu Pro Pro Pro Ala Glu
1 5 10 15

Gln Leu Ser Arg Lys Asp Val Ser Leu Thr Cys Leu Val Val Gly Phe
20 25 30

Asn Pro Gly Asp Ile Ser Val Glu Trp Thr Ser Asn Gly His Thr Glu
35 40 45

Glu Asn Tyr Lys Asp Thr Ala Pro Val Leu Asp Ser Asp Gly Ser Tyr
50 55 60

Phe Ile Tyr Ser Lys Leu Asn Met Lys Thr Ser Lys Trp Glu Lys Thr
65 70 75 80

Asp Ser Phe Ser Cys Asn Val Arg His Glu Gly Leu Lys Asn Tyr Tyr
85 90 95

Leu Lys Lys Thr Ile Ser Arg Ser Pro Gly Lys
100 105

<210> 50
<211> 107
<212> PRT
<213> Artificial Sequence

<220>
<223> CH3 domain of mouse IgG3

<400> 50
Gly Arg Ala Gln Thr Pro Gln Val Tyr Thr Ile Pro Pro Pro Arg Glu

1 5 10 15

Gln Met Ser Lys Lys Lys Val Ser Leu Thr Cys Leu Val Thr Asn Phe
20 25 30

Phe Ser Glu Ala Ile Ser Val Glu Trp Glu Arg Asn Gly Glu Leu Glu
35 40 45

Gln Asp Tyr Lys Asn Thr Pro Pro Ile Leu Asp Ser Asp Gly Thr Tyr
50 55 60

Phe Leu Tyr Ser Lys Leu Thr Val Asp Thr Asp Ser Trp Leu Gln Gly
65 70 75 80

Glu Ile Phe Thr Cys Ser Val Val His Glu Ala Leu His Asn His His
85 90 95

Thr Gln Lys Asn Leu Ser Arg Ser Pro Gly Lys
100 105

<210> 51
<211> 107
<212> PRT
<213> Artificial Sequence

<220>
<223> CH3 domain of rat IgG1

<400> 51
Gly Arg Thr Gln Val Pro His Val Tyr Thr Met Ser Pro Thr Lys Glu
1 5 10 15

Glu Met Thr Gln Asn Glu Val Ser Ile Thr Cys Met Val Lys Gly Phe
20 25 30

Tyr Pro Pro Asp Ile Tyr Val Glu Trp Gln Met Asn Gly Gln Pro Gln
35 40 45

Glu Asn Tyr Lys Asn Thr Pro Pro Thr Met Asp Thr Asp Gly Ser Tyr
50 55 60

Phe Leu Tyr Ser Lys Leu Asn Val Lys Lys Glu Lys Trp Gln Gln Gly
65 70 75 80

Asn Thr Phe Thr Cys Ser Val Leu His Glu Gly Leu His Asn His His
85 90 95

Thr Glu Lys Ser Leu Ser His Ser Pro Gly Lys
100 105

<210> 52

<211> 107
<212> PRT
<213> Artificial Sequence

<220>
<223> CH3 domain of rat IgG2a

<400> 52
Gly Thr Pro Arg Gly Pro Gln Val Tyr Thr Met Ala Pro Pro Lys Glu
1 5 10 15

Glu Met Thr Gln Ser Gln Val Ser Ile Thr Cys Met Val Lys Gly Phe
20 25 30

Tyr Pro Pro Asp Ile Tyr Thr Glu Trp Lys Met Asn Gly Gln Pro Gln
35 40 45

Glu Asn Tyr Lys Asn Thr Pro Pro Thr Met Asp Thr Asp Gly Ser Tyr
50 55 60

Phe Leu Tyr Ser Lys Leu Asn Val Lys Lys Glu Thr Trp Gln Gln Gly
65 70 75 80

Asn Thr Phe Thr Cys Ser Val Leu His Glu Gly Leu His Asn His His
85 90 95

Thr Glu Lys Ser Leu Ser His Ser Pro Gly Lys
100 105

<210> 53
<211> 107
<212> PRT
<213> Artificial Sequence

<220>
<223> CH3 domain of rat IgG2b

<400> 53
Gly Leu Val Arg Lys Pro Gln Val Tyr Val Met Gly Pro Pro Thr Glu
1 5 10 15

Gln Leu Thr Glu Gln Thr Val Ser Leu Thr Cys Leu Thr Ser Gly Phe
20 25 30

Leu Pro Asn Asp Ile Gly Val Glu Trp Thr Ser Asn Gly His Ile Glu
35 40 45

Lys Asn Tyr Lys Asn Thr Glu Pro Val Met Asp Ser Asp Gly Ser Phe
50 55 60

Phe Met Tyr Ser Lys Leu Asn Val Glu Arg Ser Arg Trp Asp Ser Arg

65 70 75 80

Ala Pro Phe Val Cys Ser Val Val His Glu Gly Leu His Asn His His
85 90 95

Val Glu Lys Ser Ile Ser Arg Pro Pro Gly Lys
100 105

<210> 54
<211> 107
<212> PRT
<213> Artificial Sequence

<220>
<223> CH3 domain of rat IgG2c

<400> 54
Gly Lys Ala Arg Thr Pro Gln Val Tyr Thr Ile Pro Pro Pro Arg Glu
1 5 10 15

Gln Met Ser Lys Asn Lys Val Ser Leu Thr Cys Met Val Thr Ser Phe
20 25 30

Tyr Pro Ala Ser Ile Ser Val Glu Trp Glu Arg Asn Gly Glu Leu Glu
35 40 45

Gln Asp Tyr Lys Asn Thr Leu Pro Val Leu Asp Ser Asp Glu Ser Tyr
50 55 60

Phe Leu Tyr Ser Lys Leu Ser Val Asp Thr Asp Ser Trp Met Arg Gly
65 70 75 80

Asp Ile Tyr Thr Cys Ser Val Val His Glu Ala Leu His Asn His His
85 90 95

Thr Gln Lys Asn Leu Ser Arg Ser Pro Gly Lys
100 105

## Description

### Technical Field

The present invention relates to a bispecific protein with a high heterodimerization rate and a method of preparing a bispecific protein.

### Background Art

A bispecific antibody (BsAb), which is an antibody having two paratopes capable of recognizing two different types of target antigens, is a generic term for an antibody capable of binding simultaneously to two different target antigens or for an antigen binding fragment thereof. This feature allows for the establishment of a therapeutic strategy which is impossible with conventional monoclonal antibodies. Bispecific antibodies rarely occur naturally and are in most part artificially constructed to simultaneously bind to two different types of biological targets. The double-targeting ability can provide such BsAbs with new applicable fields which have not been managed by monospecific antibodies (MsAbs). When it comes to therapeutic purposes, considerable interest is arising in (1) reliable recruitment of immune cells into the proximity of target cells, (2) inhibition or activation of two distantly separated signaling pathways in target cells to create a synergistic effect, and (3) specific and regulatory delivery of therapeutic substance, medications, toxins, etc. to target cells.

A variety of BsAb-related techniques (45 different formats) has been developed. These techniques are classified into four categories on a structural basis: first, heterologous bispecification of heavy chains by various methods comprising structural complementarity, also known as Knob-into-Hole or simply KiH, electrostatic steering effect, and CH3 domain shuffling (called SEEDbody™); second, various antibody fragment formats such as Diabody™ (Diabody: dimeric antibody fragments) BiTE™ (BiTE: Bi-specific T-cell engagers), and DART™ (DART: dual affinity retargeting bispecific antibody); third, technology using one or more functional domains combined with intact antibodies, such as Modular Antibody™, Zybody™, dAbs™ (dAbs : Single domain antibodies), and DVD-IG™ (DVD-Ig: dual-variable-domain immunoglobulin); and fourth, techniques adopting a full-length IgG-like scheme such as Duobody™ (Fab-Arm Exchange), CrossMab™, Azymetric™, and kI body™ have been developed.

For example, U. S. Patent No. 2013-892198 A to Zymeworks discloses a heteropolymer structure of immunoglobulin chains having mutations in the Fc domain, specifically stating that antibodies of the heteropolymer structure can be constructed by modifying cysteine residues involved in disulfide bonds into charged amino acids to exert an electrical interaction.

There are problems with conventional techniques of constructing bispecific antibodies as follows.

First, there are undesirable combinations of heavy chains. When heavy chains (A and B) originating respectively from two antibodies targeting different epitopes randomly combine with each other, two combinations of the same origins (AA and BB) and one combination of different origins (AB or BA) are formed. Combinations between heavy chains from the same origins act as undesired impurities in constructing bispecific antibodies, decreasing the purity of bispecific antibodies and requiring a process of removing the combinations. Therefore, they may have disadvantageous effects, such as provoking difficulty in the isolation and purification of antibodies, inciting undesired immune responses or signal transduction to cause side effects, etc. Hence, ongoing needs exist for forming only combinations between heavy chains of heterogeneous origins allowing none or up to a considerably restricted level of combinations between heavy chains of homogeneous origin.

Second, although light and heavy chains of homogeneous origins are specifically combined with each other, cases occur in which the light and heavy chains that combine with each other are of heterogeneous origins. A bispecific antibody targets two different kinds of epitopes. Each epitope can be recognized by an antibody in which light and heavy chains of the same origin are combined. Antibodies that are created through combinations of light and heavy chains from heterogeneous origins may recognize new epitopes other than the desired two epitopes, act as an impurity making it difficult to isolate and purify antibodies of interest, and cause a side effect. Therefore, the production of bispecific antibodies requires combinations of correct pairs of light and heavy chains while a non-specific combination of light and heavy chains does not occur or occurs at up to an insignificant level.

In consideration of the above-mentioned problems with the provision of bispecific antibodies, requirements for construction of bispecific antibodies are summarized as follows. A total of ten combinations is possible when bispecific antibodies are constructed with light and heavy chains from two different kinds of antibodies respectively recognizing two different epitopes, as shown in FIG. 1. Of them, the combination marked by a dotted line circle meets the requirements that (1) heavy chains derived from different antibodies are combined with each other and (2) light and heavy chains derived from the same antibodies are coupled to each other. The other nine combinations should be not formed or should be formed at a minimum level.

WO2014142591, which is a previous patent application of the present inventor, introduces "protein in which electrical interaction is introduced within a hydrophobic interaction site and preparation method therefor". Disclosed in the patent application is a protein or an antibody having an electrical interaction introduced in a hydrophobic interaction site thereof wherein the electrical interaction is made by a positive charge and a negative charge on a positively charged substance and a negatively charged substance which are changed from a pair of hydrophobic amino acids selected in the hydrophobic interaction site. Leading to the present invention, intensive and thorough research made by the present inventors resulted in the finding that bispecific antibodies can be formed using electrostatic interaction in a non-hydrophobic interaction site which was not taken into consideration in the previous patent application and can be constructed at high yield even by size-dependent coupling and/or amino acid change (swapping) between coupled pairs.

[Prior Art Document]

[Patent Document]

(Patent Document 1) Korean Patent Number 10-2014-0019385 A (February 14, 2014)

## Disclosure

## Technical Problem

In order to solve the above problems, the present invention provides a bispecific antibody with high purity and a method of preparing the same.

An embodiment provides a dimer comprising a first CH3 domain and a second CH3 domain of an antibody, or Fc regions comprising the CH3 domains, wherein
the first CH3 domain and the second domain are mutated such that at least one selected from among amino acid pairs forming amino acid-amino acid bonds between the first CH3 domain and the second CH3 domain is modified by at least one of the following mutations:

(1) a mutation in which, of at least one amino acid pair between the CH3 domains, one amino acid is substituted with an amino acid having a positive charge and the other is substituted with an amino acid having a negative charge;
(2) a mutation in which amino acids in at least one amino acid pair between the CH3 domains are swapped with each other; and
(3) a mutation in which, of at least one amino acid pair between the CH3 domains, one amino acid is substituted with a large hydrophobic amino acid while the other is substituted with a small hydrophobic amino acid.

The first CH3 domain and the second CH3 domain may be derived from the same or different kinds of antibodies (immunoglobulins).

Another embodiment provides a nucleic acid molecule encoding the modified CH3 domain or a modified Fc region comprising the modified CH3 domain, a recombinant vector carrying the nucleic acid molecule, and a recombinant cell containing the recombinant vector therein.

As used herein the terms "one amino acid" and "the other amino acid" in the expression "of at least one amino acid pair, one amino acid... and the other amino acid..." mean one amino acid and the other amino acid of the two amino acids in each of one or more amino acid pairs, respectively (hereinafter the same definition will be applied).

Another embodiment provides a bispecific protein for targeting two different kinds of targets, the bispecific protein comprising a first CH3 domain or a first Fc region comprising the first CH3 domain and a second CH3 domain or a second Fc region comprising the second CH3 domain, wherein the first CH3 domain and the second CH3 domain are mutated such that at least one selected from amino acid pairs forming amino acid-amino acid bonds between the first CH3 domain and the second CH3 domain is modified by at least one of the following mutations:

(1) a mutation in which, of at least one amino acid pair between the CH3 domains, one amino acid is substituted with an amino acid having a positive charge while the other is substituted with an amino acid having a negative charge;
(2) a mutation in which amino acids in at least one amino acid pair between the CH3 domains are swapped with each other; and
(3) a mutation in which, of at least one amino acid pair between the CH3 domains, one amino acid is substituted with a large hydrophobic amino acid while the other is substituted with a small hydrophobic amino acid.

The amino acid pair that is substituted with amino acids having opposite charges in step (1) may be an amino acid under hydrophobic interaction and/or an amino acid under non-hydrophobic interaction. In one embodiment, at least one of the two amino acids constituting an amino acid pair may be not a hydrophobic amino acid (i.e., a hydrophilic amino acid). The electrostatic interaction that is introduced by step (1) of substitution with amino acids having opposite charges may contribute to improving the formation of a heterodimer between Fc regions.

As used herein, the term "heterodimer" means a fusion body in which two different proteins are coupled to each other and is intended to encompass any bispecific protein in which two proteins targeting respective different targets are coupled to each other (e.g., bispecific antibody), etc.

The amino acid switching (swapping) in step (2) may be conducted in any amino acid pair forming an amino acid-amino acid linkage in the Fc regions or CH3 domains and, for example, in at least one amino acid pair selected from all the amino acid pairs which are each bonded through interaction other than electrostatic interaction, hydrophobic interaction, and amino acid size difference-based interaction. As such, two amino acid residues that interact with each other (e.g., bond themselves to each other) in each amino acid pair (i.e., an amino acid residue of the first CH3 domain and the other amino acid of the second CH3 domain in the interacting amino acid pair) may be exchanged with each other to decrease the possibility of forming a homodimer because the presence of the same amino acid at the counterpart positions for amino acid interaction between the CH3 domains of homogeneous origins makes the bonding therebetween difficult, thereby contributing to improving a heterodimerization rate (for example, when an amino acid pair of S364 and K370 undergoes S364K mutation in the first domain and K370S mutation in the second CH3 domain, there is no interaction between the homogeneous CH3 domains because both amino acids at positions 364 and 370 become lysine (K) in the first CH3 domain and serine (S) in the second CH3 domain, but interaction occurs between the heterogeneous CH3 domain to form a heterodimer only).

The step (3) of substitution with amino acids different in size improves structural engagement suitability between a large amino acid and a small amino acid (that is, a large amino acid is inserted into a spare space established by a small amino acid, thereby increasing bonding efficiency), with the consequent increase of heterodimerization rates. Particularly, an interacting amino acid pair is mutated such that one amino acid is substituted with a large hydrophobic amino acid while the other amino acid is substituted with a small hydrophobic amino acid whereby advantage is taken of the difficulty in making a bond between large amino acids or between small amino acids to minimize a homodimerization rate (large amino acids, if existing respectively in two opposite chains, render the two chains distant from the each other to obstruct dimerization whereas two small amino acids, if existing in two opposite chains, interact with each other at low possibility because of a long distance therebetween and have difficulty in interaction therebetween). On the contrary, a large hydrophobic amino acid in one CH3 domain or Fc and a small hydrophobic amino acid in the other CH3 domain or Fc undergo hydrophobic interaction with each other at a closer distance compared to the pre-mutation amino acids, thus making a condition good for heterodimerization. Therefore, a large and a small amino acid to be substituted in the step of substitution with amino acids different in size may be both selected from among hydrophobic amino acids. For example, the large amino acid may be at least one selected from the group consisting of tryptophan and phenylalanine, which are both hydrophobic. In addition, the small amino acid may be at least one selected from the group consisting of alanine, glycine, and valine, which are all hydrophobic.

The bispecific protein comprising the mutant Fc regions or CH3 domains may be selected from among any type of proteins targeting (e.g., specifically recognizing and/or binding to) two different kinds of targets. For targeting, the bispecific protein comprising the mutant Fc regions or CH3 domains may comprise two targeting domains capable of targeting (specifically recognizing and/or binding to) two different kinds of targets, respectively (for example, a first targeting domain for targeting a first target and a second targeting domain for targeting a second target). The targeting domains may form a covalent or non-covalent bond (linkage) to the mutant Fc regions or CH3 domains, respectively, in a direct or indirect (e.g., via a linker) manner. For example, the bispecific proteins comprising the mutant Fc regions or CH3 domains may be at least one selected from the group consisting of a bispecific antibody, an antigen-binding fragment of a bispecific antibody (e.g., (scFv-Fc)2, etc.), a bispecific antibody analog (e.g., nanobody, peptibody, peptide, aptide, etc.), and a fusion protein of a target-specific binding polypeptide and the mutant Fc region or CH3 domain.

The target-specific binding polypeptide may be any polypeptide that binds specifically to a biological target substance (any compounds present in the body comprising proteins, nucleic acids, and the like) and may be at least one polypeptide selected from, for example, the group consisting of a paratope (e.g., e.g., a CDR or variable region of a heavy chain and/or a light chain), single-chain Fv (scFv), a membrane protein (e.g., various receptors, etc.), a membrane protein ectodomain, and a ligand (e.g., various growth factors, cytokines, etc.). In one embodiment, the fusion protein of a target-specific binding polypeptide and the mutant Fc region or CH3 domain may be at least one selected from the group consisting of a fusion protein of a membrane protein and the mutant Fc region or CH3 domain, a fusion protein of a membrane protein ectodomain and the mutant Fc region or CH3 domain, a fusion protein of a ligand and the mutant Fc region or CH3 domain, and a fusion protein of scFv and the mutant Fc region or CH3 domain.

When the bispecific protein comprising the mutant Fc region or CH3 domain is an antibody, an antigen-binding fragment of an antibody, or an antibody analog, the targeting domain may be a paratope (e.g., e.g., a CDR or variable region of a heavy chain and/or a light chain). For the above-mentioned fusion protein of a target-specific binding polypeptide and the mutant Fc region or CH3 domain, the target-specific binding polypeptide may be at least one selected from the group consisting of a membrane protein (e.g., various receptors), a membrane protein ectodomain, a ligand (e.g.,

various growth factors, cytokines, etc.), and a paratope (e.g., a CDR or variable region of a heavy chain and/or a light chain).

The two different kinds of targets may refer to two different kinds of biological substances (e.g., proteins) or different regions within one biological substance (e.g., one protein). The bispecific protein comprising the mutant Fc region or CH3 domain is characterized by an increase in heterodimerization rate, a decrease in homodimerization, and/or stability, compared to a bispecific protein comprising a non-mutant (wild-type) Fc region or CH3 domain.

Another embodiment provides a bispecific antibody or an antigen-binding fragment thereof, the bispecific antibody comprising a first CH1 domain and a first Cl (light chain constant region) domain derived respectively from the heavy chain and light chain of an antibody recognizing a first epitope and a second CH1 domain and a second CL domain derived respectively from the heavy chain and light chain of an antibody recognizing a second epitope, wherein the CH1 domains and the CL domains are mutated to contain at least one of the following mutations:

a mutation in which, of the two amino acids constituting each pair of one or more first amino acid pairs selected from among amino acid pairs forming respective amino acid-amino acid bonds between the first CH1 domain and the first CL domain, one is substituted with an amino acid having a positive charge and the other is substituted with an amino acid having a negative charge; and

a mutation in which, of the two amino acids constituting each pair of one or more second amino acid pairs selected from among amino acid pairs forming respective amino acid-amino acid bonds between the second CH1 domain and the second CL domain, one is substituted with an amino acid having a positive charge and the other is substituted with an amino acid having a negative charge.

The first epitope and the second epitope may exist in respective different proteins (antibodies) or in different (discriminative) regions of one protein (antigen).

In the bispecific antibody or the antigen-binding fragment thereof according to one embodiment, the amino acids substituted respectively in the first CH1 domain and the second CH1 domain have opposite charges, the amino acids substituted respectively in the first CL domain and the first CH1 domain have opposite charges, and the amino acids substituted respectively in the second CL domain and the second CH1 domain have opposite charges.

In the bispecific antibody or the antigen-binding fragment thereof, for example,

the amino acid, positioned in the first CH1 domain, as a member of at least one first amino acid pair selected from among amino acid pairs forming respective amino acid-amino acid bonds between the first CH1 domain and the first CL domain may be substituted with an amino acid having a positive charge while the other member positioned in the first CL domain may be substituted with an amino acid having a charge different from that of the amino acid substituted in the first CH1 domain, that is, a negative charge, and

the amino acid, positioned in the second CH1 domain, as a member of at least one second amino acid pair selected from among amino acid pairs forming respective amino acid-amino acid bonds between the second CH1 domain and the second CL domain may be substituted with an amino acid having a charge different from that of the amino acid substituted in the first CH1 domain, that is, a negative charge while the other member positioned in the second CL domain may be substituted with an amino acid having a charge different from that of the amino acid substituted in the second CH1 domain, that is, a positive charge.

In the bispecific antibody or the antigen-binding fragment thereof comprising mutant CH1 CL domains, the first and the second amino acid pair to be substituted may be the same or different, the positively charged amino acids substituted in the first and the second amino acid pair may be the same or different, and the negatively charged amino acids substituted in the first and the second amino acid pair may be the same or different. The antigen-binding fragment of the bispecific antibody comprising a mutant CH1 domain and a mutant CL domain may be, for example, a F(ab')2 fragment. The bispecific antibody comprising mutant CH1 and CL domains or the antigen-binding fragment thereof targets the same epitopes as those for a bispecific antibody comprising non-mutant (wild-type) CH1 and CL domains and exhibits higher heavy chain (or heavy chain variable region-CH1)-light chain dimerization rates and/or stability, compared to a bispecific antibody comprising non-mutant (wild-type) CH1 and CL domains.

The bispecific antibody comprising mutant CH1 and CL domains may comprise modified CH3 domains inclusive of a first CH3 domain derived from an antibody recognizing a first epitope and a second CH3 domain derived from an antibody recognizing a second epitope, or an Fc region comprising the modified CH3 domain, wherein the first CH3 domain and the second CH3 domain have at least one of the following mutations:

(1) a mutation in which one member of at least one amino acid pair between the CH3 domains is substituted with an amino acid having a positive charge and the other member is substituted with an amino acid having a negative charge;

(2) a mutation in which the members of at least one amino acid pair between the CH3 domains are switched with each other; and

(3) a mutation in which one member of at least one amino acid pair between the CH3 domains is substituted with a large amino acid (e.g., large hydrophobic amino acids such as tryptophan, phenylalanine, etc.) and the other member is substituted with a small amino acid (e.g., small hydrophobic amino acids such as alanine, glycine, valine, etc.).

A bispecific protein or bispecific antibody comprising the mutant CH1 domain, the mutant CL domain, and the mutant Fc region or modified CH3 domain may exhibit an improvement in heterodimeration rate, dimerization rate between a heavy chain (or heavy chain variable region-CH1) and a light chain, both targeting the same epitope, and/or stability, compared to a bispecific protein or antibody comprising a CH1 domain, a CL domain, and an Fc region or CH3 domain none of which are mutant.

The bispecific antibody comprising mutant CH1 and CL domains or the antigen-binding fragment thereof targets the same epitopes as those for a bispecific antibody comprising non-mutant (wild-type) CH1 and CL domains and exhibits higher heavy chain (or heavy chain variable region-CH1)-light chain dimerization rates and/or stability, compared to a bispecific antibody comprising non-mutant (wild-type) CH1 and CL domains.

Another embodiment provides a method for enhancing heterodimerization of a bispecific protein for targeting different targets, the bispecific protein comprising modified CH3 domains or an Fc region comprising the modified CH3 domains, said method comprising one of the following mutation introducing steps:

(1) substituting one of the two amino acids in at least one amino acid pair selected from amino acid pairs forming amino-amino acid bonds between the CH3 domains with an amino acid having a positive charge and the other with an amino acid having a negative charge;
(2) switching the two amino acids in at least one amino acid pair selected from amino acid pairs forming amino-amino acid bonds between a first CH3 domain and a second CH3 domain with each other; and
(3) substituting one of the two amino acids in at least one amino acid pair selected from amino acid pairs forming amino-amino acid bonds between the first CH3 domain and the second CH3 domain with a large hydrophobic amino acid (for example, tryptophan, phenylalanine, etc.) and the other with a small hydrophobic amino acid (for example, alanine, glycine, valine, etc.).

Another embodiment provides a method for constructing a bispecific antibody or an antigen-binding fragment thereof or for enhancing a dimerization rate between a heavy chain (or heavy chain variable region-CH1) and a light chain, both targeting the same epitope, the method comprising the following CH1 and CL domain mutating steps of:

substituting one of the two amino acid residues constituting at least one selected from amino acid pairs forming amino acid-amino acid bonds between a first CH3 domain derived from the heavy chain and a first CL domain of an antibody recognizing a first epitope with an amino acid having a positive charge and the other with an amino acid having a negative charge; and

substituting one of the two amino acid residues constituting at least one selected from amino acid pairs forming amino acid-amino acid bonds between a second CH3 domain derived from the heavy chain and a second CL domain of an antibody recognizing a second epitope with an amino acid having a positive charge and the other with an amino acid having a negative charge.

The method for constructing a bispecific antibody or an antigen-binding fragment thereof or for enhancing a dimerization rate between a heavy chain (or heavy chain variable region-CH1) and a light chain may comprise, in addition to the CH1 and CL domain mutating steps, at least one of the following CH3 domain mutation steps:

(1) substituting one of the two amino acids in at least one selected from amino acid pairs forming amino-amino acid bonds between the first CH3 domain derived from the heavy chain of an antibody recognizing a first epitope and the second CH3 domain derived from the heavy chain of an antibody recognizing a second epitope with an amino acid having a positive charge and the other with an amino acid having a negative charge;
(2) switching the two amino acids in at least one amino acid pair selected from amino acid pairs forming amino-amino acid bonds between the first CH3 domain and the second CH3 domain with each other; and
(3) substituting one of the two amino acids in at least one amino acid pair selected from amino acid pairs forming amino-amino acid bonds between the first CH3 domain and the second CH3 domain with a large hydrophobic amino acid (for example, tryptophan, phenylalanine, etc.) and the other with a small hydrophobic amino acid (for example, alanine, glycine, valine, etc.).

Another embodiment provides a method for constructing a bispecific antibody or an antigen-binding fragment thereof and for enhancing heterodimerization of a bispecific antibody or an antigen-binding fragment thereof for targeting different targets, the method comprising one of the following mutation introducing steps to introduce at least one mutation into at least one selected from amino acid pairs forming amino acid-amino acid bonds between a first CH3 domain and a second CH3 domain:

(1) substituting one of the two amino acids in at least one selected from amino acid pairs forming amino-amino acid bonds between the first CH3 domain derived from the heavy chain of an antibody recognizing a first epitope and the second CH3 domain derived from the heavy chain of an antibody recognizing a second epitope with an amino acid having a positive charge and the other with an amino acid having a negative charge;
(2) switching the two amino acids in at least one amino acid pair selected from amino acid pairs forming amino-amino acid bonds between the first CH3 domain and the second CH3 domain with each other; and
(3) substituting one of the two amino acids in at least one amino acid pair selected from amino acid pairs forming amino-amino acid bonds between the first CH3 domain and the second CH3 domain with a large hydrophobic amino acid (for example, tryptophan, phenylalanine, etc.) and the other with a small hydrophobic amino acid (for example, alanine, glycine, valine, etc.).

The method for constructing a bispecific antibody or an antigen-binding fragment thereof may further comprise the following CH1 and CL domain mutating steps of:

substituting one of the two amino acid residues constituting at least one selected from amino acid pairs forming amino acid-amino acid bonds between a first CH3 domain derived from the heavy chain and a first CL domain of an antibody recognizing a first epitope with an amino acid having a positive charge and the other with an amino acid having a negative charge; and
substituting one of the two amino acid residues constituting at least one selected from amino acid pairs forming amino acid-amino acid bonds between a second CH3 domain derived from the heavy chain and a second CL domain of an antibody recognizing a second epitope with an amino acid having a positive charge and the other with an amino acid having a negative charge.

The method for constructing a bispecific antibody or an antigen-binding fragment thereof can enhance heterodimerization between CH3 domains or Fc regions derived from antibodies recognizing different epitope as well as between CH1 domains (heavy chains) and CL domains (light chains) derived from antibodies recognizing the same epitope.

**Technical Solution**

Below, a detailed description will be given of the present invention.

The present invention provides a bispecific protein comprising an Fc constant region and/or an Fab constant region and targeting different targets and a construction method therefor, wherein an amino acid mutation is introduced to the Fc constant regions (CH3 domains) linked (fused) respectively to targeting domains different from each other to increase coupling between the Fc constant regions linked to different targeting domains, thereby increasing a heterodimerization rate between the Fc constant regions linked to different targeting domains and decreasing a homodimerization rate between the Fc constant regions linked to the same targeting domain; and/or
an amino acid mutation is introduced to Fab constant regions linked to targeting domains different from each other to increase coupling between the Fab constant regions linked to different targeting domains, thereby a dimerization rate between the same targeting domains and between the Fab constant regions linked thereto,
whereby a bispecific protein having different targeting domains can be produced at high yield.

Amino acid positions in the antibodies (heavy and light chains), CH1 domains, CL domains, Fc regions, and CH3 domains disclosed in the description are all given as numbered according to the EU numbering system [the EU-index set forth in "Kabat et al., Sequences of Proteins of Immunological Interest, 5th Ed. Public Health Service, National Institutes of Health, Bethesda, MD. (1991)"] and differ from numbers accounting for positions of sequences in the sequencing list.

The antibody may be at least one selected from among all kinds of immunoglobulins originating from mammals or birds. For example, the antibody used in the description may be at least one selected from the group consisting of IgG (e.g., IgG type 1 (IgG1), IgG type 2 (IgG2), IgG type 3 (IgG3), and IgG type 4 (IgG4)), IgA (e.g., IgA type 1 (IgAl) and IgA type 2 (IgA2)), IgD, IgE, and IgM. The antibody may be an immunoglobulin derived from mammals such as primates comprising humans, monkeys, etc. and rodents comprising mice, rats, etc. and may be, for example, a human-derived immunoglobulin. In one embodiment, the antibody may be at least one selected from among human IgG1 (constant region; protein: GenBank Accession No. AAC82527.1, gene: GenBank Accession No. J00228.1), human IgG2 (constant region; protein: GenBank Accession No. AAB59393.1, gene: GenBank Accession No. J00230.1), human IgG3 (constant region; protein: GenBank Accession No. P01860, gene: GenBank Accession No. X03604.1), human IgG4 (constant region; protein: GenBank Accession No. AAB59394.1, gene: GenBank Accession No. K01316.1), human IgAl (constant region; protein: GenBank Accession No. AAT74070.1, gene: GenBank Accession No. AY647978.1), human IgA2 (constant region; protein: GenBank Accession No. AAB59396.1, gene: GenBank Accession No. J00221.1), human IgD (constant region; protein: GenBank Accession No. AAA52771.1, AAA52770.1), human IgE (constant region; protein: GenBank Accession No. AAB59395.1, gene: GenBank Accession No. J00222.1), and human IgM (constant region; protein: GenBank Accession No. CAB37838.1, gene: GenBank Accession No. X57086.1). In one embodiment, the antibody may be at least one selected from the group consisting of human-derived IgG1, IgG2, IgG3, and IgG4, but is not limited thereto. The 1$^{st}$ CH3 domain and the 2$^{nd}$ CH3 domain, the 1$^{st}$ CH1 domain and the 1$^{st}$ CL domain, and the 2$^{nd}$ CH1 domain and the 2$^{nd}$ CL domain into all of which a mutation is introduced may each be independently selected from among identical or different immunoglobulin types.

As can be seen in FIG. 33a depicting sequence alignment results of the human IgG1 heavy chain constant region (SEQ ID NO: 33) and the human IgA1 heavy chain constant region (SEQ ID NO: 34) and in FIG. 33b depicting sequence alignment results of the kappa constant region (SEQ ID NO: 35) and lambda constant region (SEQ ID NO: 36) of the human immunoglobulin light chain, the heavy chain constant region and light chain constant region exhibit highly conserved amino acid sequences between subtypes.

In addition, immunoglobulin sequences are highly conserved among species and subtypes from which the sequences are derived. For instance, as shown by the sequence alignment results of heavy chain constant regions among human, mouse, and rat in FIG. 33c (CH1 domain sequence alignment) and FIG. 33d (CH3 domain sequence alignment), the amino acid sequences of heavy chain constant region of immunoglobulins are of high interspecies conservation.

In the description, hence, reference is given to the human IgG1 to designate amino acid positions in the CH1 domains and CH3 domains and to the human kappa constant region to designate amino acid positions in the CL domains. The amino acid positions designated with the human IgG1 and the human kappa constant region serving as references allows corresponding amino acid positions in immunoglobulins of other subtypes and immunoglobulins of species other than humans to be explicitly designated through a typical sequence alignment means (e.g., https://blast.ncbi.nlm.nih.gov/Blast.cgi) (see Table 1).

In addition, amino acid positions in the CH1 domain, CL domain, and CH3 domain, provided in the description, are represented according to the EU numbering system, and with respect to the details thereof, reference may be made to "http://www.imgt.org/IMGTScientificChart/Numbering/Hu_IGHGnber.html (heavy chain constant region)", "http://www.imgt.org/IMGTScientificChart/Numbering/Hu_IGLCnber.html (light chain lambda region)" and "http://www.imgt.org/IMGTScientificChart/Numbering/Hu_IGKCnber.html (light chain kappa region)".

Using the human IgG1 as a reference, the EU numbering system numbers:

(1) the CH1 domain (SEQ ID NO: 1) consecutively, with the first amino acid residue (Ala) given position 118 (i.e., 108 amino acid residues of the CH1 domain of SEQ ID NO: 1 correspond respectively to positions 118 to 215 in IgG1); and

(2) the CH3 domain (SEQ ID NO: 15) consecutively, with the first amino acid residue (Lys) given position 340 (i.e., 108 amino acid residues of the CH3 domain of SEQ ID NO: 15 corresponds respectively to positions 340 to 447 in IgG1) (see, http://www.imgt.org/IMGTScientificChart/Numbering/Hu_IGHGnber.html).

In the description, amino acid positions in the CH1 and CH3 domains and amino acid kinds corresponding thereto are depicted, with the human IgG1 serving as a reference.

Further, according to the EU numbering,

the CL domain (SEQ ID NO: 10) of the human kappa constant region (protein: GenBank Accession No. AAA58989.1 gene: GenBank Accession No. J00241.1) is numbered consecutively, with the first amino acid residue (Val) given position 110 (i.e., 105 amino acid residues of the CL domain of SEQ ID NO: 10 correspond respectively to positions 110 to 214; see http://www.imgt.org/IMGTScientificChart/Numbering/Hu_IGKCnber.html); and

the CL domains (SEQ ID NO: 11 (Lambda1), SEQ ID NO: 12 (Lambda2), SEQ ID NO: 13 (Lambda3), and SEQ ID NO: 14 (Lambda7)) of the human lambda constant region are numbered consecutively, with the first amino acid residue (Lys) given position 110 (for the lambda constant region, positions 169, 201, and 203 are omitted from the serial number established; that is, the 103 amino acid residues of the CL domain of SEQ ID NO: 11 or 12 are numbered from position 110 to position 168, from position 170 to position 200, and from position 203 to position 215; see http://www.imgt.org/IMGTScientificChart/Numbering/Hu_IGLCnber.html). In the description, amino acid positions in the CL domains and amino acid kinds corresponding thereto are depicted, with the human kappa constant region serving as a reference.

The Fab constant region may comprise one heavy chain constant region (i.e., CH1 domain) selected from the group consisting of heavy chain constant regions of Fab fragments of IgG (IgG1, IgG2, IgG3, and IgG 4), IgA (IgA1 and IgA2), IgD, IgE, and IgM and one light chain constant region (i.e. CL domain) selected from the group consisting of the kappa type and lambda types (e.g., lambda type 1, lambda type 2, lambda type 3, and lambda type 7) of immunoglobulin light chains.

By way of example, the CH1 domains of IgG1, IgG2, IgG3, IgG4, IgA1, IgA2, IgD, IgE, and IgM, which are each available as a heavy chain constant region (CH1 domain) of the Fab fragment may comprise the amino acid sequences of SEQ ID NO: 1 (corresponding to positions 118 to 215 according to EU numbering), SEQ ID NO: 2, SEQ ID NO: 3, SEQ ID NO: 4, SEQ ID NO: 5, SEQ ID NO: 6, SEQ ID NO: 7, SEQ ID NO: 8, and SEQ ID NO: 9, respectively. In addition, the CL domains of the kappa type, lambda type 1, lambda type 2, lambda type 3, and lambda type 7 are each available as the light chain constant region (CL domain) and may comprise the amino acid sequences of SEQ ID NO: 10 (corresponding to positions 110 to 214 according to EU numbering), SEQ ID NO: 11, SEQ ID NO: 12, SEQ ID NO: 13, and SEQ ID NO: 14, respectively. In an embodiment, the Fab constant region the CH1 domain (SEQ ID NO: 1) of IgG type 1 and the light chain constant region (CL domain) (SEQ ID NO: 10) of the kappa type. In order to enhance dimerization between molecules targeting the same subject, the amino acid substituted with a negatively charged amino acid or a positively charged amino acid in the CH1 domain may be at least one residue, for example, one, two or more (e.g., two), three or more (e.g., three), or four or more (e.g., four) residues selected from the group consisting of leucine at position 145, lysine at position 147, phenylalanine at position 170, serine at position 183, and valine at position 185 in IgG type 1 (SEQ ID NO: 1) as numbered according to the EU numbering system. In another embodiment, the amino acid substituted with a negatively charged amino acid or a positively charged amino acid in the CH1 domain may be at least one residue selected from the group consisting of amino acids of other IgG subtypes (IgG2, IgG3, and IgG4), IgA1, IgA2, IgD, IgE, and IgM (respectively SEQ ID NOS: 2 to 9) at positions corresponding to leucine at position 145, lysine at position 147, phenylalanine at position 170, serine at position 183, and valine at position 185 on the amino acid sequence of SEQ ID NO: 1.

As used herein, "amino acids at positions corresponding to" can be determined by typical sequence alignment of the amino acid sequence of SEQ ID NO: 1 with target amino acid sequences (i.e., SEQ ID NOS: 2 to 9) without difficulty (hereinafter, the same will be applied).

The amino acid having a positive charge (positively charged amino acid) may be selected from basic amino acids and may be, for example, lysine or arginine. When an amino acid having a positive charge is introduced into the CH1 domain, at least one residue, for example, one, two or more (e.g., two), three or more (e.g., three), or four or more (e.g., four) residues selected from the group consisting of leucine at position 145, lysine at position 147, phenylalanine at position 170, proline at position 171, serine at position 183, and valine at position 185 on the amino acid sequence of SEQ ID NO: 1, and amino acids at positions corresponding thereto on the amino acid sequences of SEQ ID NOS: 2 to 9 may each be independently substituted by a basic amino acid, for example, lysine or arginine.

For example, in order to introduce an amino acid having a positive charge thereinto, the CH1 domain may comprise at least one, two or more (e.g., two), three or more (e.g., three), or four or more (e.g., four) of the following mutations therein (on the amino acid sequence of SEQ ID NO: 1; and also applied to amino acid residues at positions corresponding thereto on the amino acid sequences of SEQ ID NOS: 2 to 9):

substitution of leucine at position 145 with lysine or arginine (e.g., lysine);
substitution of serine at position 183 with lysine or arginine (e.g., lysine);
substitution of lysine at position 147 with arginine;
substitution of phenylalanine at position 170 with lysine or arginine (e.g., lysine);
substitution of proline at position 171 with lysine or arginine (e.g., lysine); and
substitution of valine at position 185 with lysine or arginine (e.g., arginine).

The amino acid having a negative charge may be selected from among acidic amino acid residues and may be, for example, aspartic acid or glutamic acid. Hence, when an amino acid having a negative charge is introduced into the CH1 domain, at least one, for example, one, two or more (e.g., two), three or more (e.g., three), or four or more (e.g., four) residues selected from the group consisting of leucine at position 145, lysine at position 147, phenylalanine at position 170, proline at position 171, serine at position 183, and valine at position 185 on the amino acid sequence of SEQ ID NO: 1, and amino acids at positions corresponding thereto on the amino acid sequences of SEQ ID NOS: 2 to 9 may each be independently substituted by an acidic amino acid, for example, aspartic acid or glutamic acid. For example, in order to introduce an amino acid having a positive charge thereinto, the CH1 domain may comprise at least one, two or more (e.g., two), three or more (e.g., three), or four or more (e.g., four) of the following mutations (on the amino acid sequence of SEQ ID NO: 1; and also applied to amino acid residues at positions corresponding thereto on the amino acid sequences of SEQ ID NOS: 2 to 9):

substitution of leucine at position 145 with aspartic acid or glutamic acid (e.g., glutamic acid);
substitution of lysine at position 147 with aspartic acid or glutamic acid (e.g., aspartic acid);

substitution of serine at position 183 with aspartic acid or glutamic acid (e.g., glutamic acid);
substitution of valine at position 185 with aspartic acid or glutamic acid (e.g., aspartic acid);
substitution of phenylalanine at position 170 with aspartic acid or glutamic acid (e.g., aspartic acid); and
substitution of proline at position 171 with aspartic acid or glutamic acid (e.g., aspartic acid).

In order to enhance dimerization between molecules targeting the same subject, the amino acid substituted with a negatively charged amino acid or a positively charged amino acid in the light chain constant region (CL domain) may be at least one residue, for example, one, two or more (e.g., two), three or more (e.g., three), or four or more (e.g., four) residues selected from the group consisting of serine at position 131, valine at position 133, leucine at position 135, serine at position 162, and threonine at position 180 in in the kappa type (SEQ ID NO: 10). In another embodiment, the amino acid substituted with a negatively charged amino acid or a positively charged amino acid in the CL domain may be at least one residue selected from the group consisting of amino acids of the CL domains of lambda types (lambda type 1, lambda type 2, lambda type 3, and lambda type 7) (respectively SEQ ID NOS: 11 to 14) at positions corresponding to serine at position 131, valine at position 133, leucine at position 135, serine at position 162, and threonine at position 180 on the amino acid sequence of SEQ ID NO: 10.

The amino acid having a positive charge (positively charged amino acid) may be selected from basic amino acids and may be, for example, lysine or arginine. Hence, when an amino acid having a positive charge is introduced into the CL domain, at least one residue, for example, one, two or more (e.g., two), three or more (e.g., three), or four or more (e.g., four) residues selected from the group consisting of serine at position 131, valine at position 133, leucine at position 135, serine at position 162, and threonine at position 180 on the amino acid sequence of SEQ ID NO: 10, and amino acids at positions corresponding thereto on the amino acid sequences of SEQ ID NOS: 11 to 14 may each be independently substituted by a basic amino acid, for example, lysine or arginine.

By way of example, in order to introduce an amino acid having a positive charge thereinto, the CL domain may comprise at least one, for example, one, two or more (e.g., two), three or more (e.g., three), or four or more (e.g., four) of the following mutations (for the amino acid sequence of SEQ ID NO: 10; and also applied to the amino acids at positions corresponding thereto on the amino acid sequences of SEQ ID NOS: 11 to 14):

substitution of serine at position 131 with lysine or arginine (e.g., lysine);
substitution of valine at position 133 with lysine or arginine (e.g., lysine);
substitution of leucine at position 135 with lysine or arginine (e.g., arginine);
substitution of serine at position 162 with lysine or arginine (e.g., lysine); and
substitution of threonine at position 180 with lysine or arginine (e.g., arginine).

In order to further enhance homodimerization, the mutant CH1 domain and/or the mutant CL domain may comprise two or more mutations simultaneously.

For example, lysine at position 147 and valine at position 185 in the CH1 domain may be substituted by an amino acid having a positive or negative charge. By way of example, lysine at position 147 and valine at position 185 in one of the first and the second CH1 domain may be substituted by an amino acid having a positive charge (e.g., lysine or arginine) while lysine at position 147 and valine at position 185 in the other CH1 domain may be substituted with an amino acid having a negative charge (glutamic acid or aspartic acid). In addition, leucine at position 135 and threonine at position 180 in the CL domain may be substituted with an amino acid having a positive or negative charge. By way of example, leucine at position 135 and threonine at position 180 in one of the first and the second CH1 domain may be substituted with an amino acid having a positive charge (e.g., lysine or arginine) while leucine at position 135 and threonine at position 180 in the other CL domain may be substituted with an amino acid having a negative charge (glutamic acid or aspartic acid).

In another embodiment, phenylalanine at position 170 and proline at position 171 in the CH1 domain may be substituted with an amino acid having a positive or negative charge. By way of example, phenylalanine at position 170 and proline at position 171 in one of the first CH1 domain and the second CH1 domain may be substituted with an amino acid having a positive charge (e.g., lysine or arginine) while phenylalanine at position 170 and proline at position 171 in the other CH1 domain may be substituted with an amino acid having a negative charge (glutamic acid or aspartic acid). In addition, substitution with an amino acid having a positive or negative charge may be carried out for leucine at position 135 and serine at position 162 in the CL domain. By way of example, leucine at position 135 and serine at position 162 in one of the first CL domain and the second CL domain may be substituted with an amino acid having a positive charge (e.g., lysine or arginine) while leucine at position 135 and serine at position 162 in the other CL domain may be substituted with an amino acid having a negative charge (e.g., glutamic acid or aspartic acid).

The amino acid having a negative charge may be selected from among acidic amino acids and may be, for example, aspartic acid or glutamic acid. Hence, when an amino acid having a negative charge is introduced into the CL domain, at least one residue, for example, one, two or more (e.g., two), three or more (e.g., three), or four or more (e.g.,

four) residues selected from the group consisting of serine at position 131, valine at position 133, leucine at position 135, serine at position 162, and threonine at position 180 on the amino acid sequence of SEQ ID NO: 10, and amino acids at positions corresponding thereto on the amino acid sequences of SEQ ID NOS: 11 to 14 may each be independently substituted by a basic amino acid, for example, aspartic acid or glutamic acid. By way of example, in order to introduce an amino acid having a positive charge thereinto, the CL domain may comprise at least one, for example, one, two or more (e.g., two), three or more (e.g., three), or four or more (e.g., four) of the following mutations (on the amino acid sequence of SEQ ID NO: 10; and also applied to amino acid residues at positions corresponding thereto on the amino acid sequences of SEQ ID NOS: 11 to 14):

substitution of serine at position 131 with aspartic acid or glutamic acid (e.g., glutamic acid);
substitution of valine at position 133 with aspartic acid or glutamic acid (e.g., glutamic acid);
substitution of leucine at position 135 with aspartic acid or glutamic acid (e.g., aspartic acid);
substitution of serine at position 162 with aspartic acid or glutamic acid (e.g., aspartic acid); and
substitution of threonine at position 180 with aspartic acid or glutamic acid (e.g., aspartic acid).

In one embodiment, a set of two amino acids forming an amino acid pair between the CH1 domain and the CL domain that are to be substituted with a pair of amino acids having opposite charges may be at least one, for example, one, two or more (e.g. two), three or more (e.g., three), or four or more (e.g., four) pairs selected from the group consisting of a pair of leucine at position 145 in the CH1 domain and serine at position 131 in the CL domain, a pair of leucine at position 145 in the CH1 domain and valine at position 133 in the CL domain, a pair of lysine at position 147 in the CH1 domain and threonine at position 180 in the CL domain, a pair of serine at position 183 in the CH1 domain and valine at position 133 in the CL domain, a pair of valine at position 185 in the CH1 domain and leucine at position 135 in the CL domain, a pair of phenylalanine at position 170 in the CH1 domain and leucine at position 135 in the CL domain, and a pair of proline at position 171 in the CH1 domain and serine at position 162 in the CL domain, as numbered on the basis of the amino acid sequences of SEQ ID NO: 1 (CH1 domain) and SEQ ID NO: 10 (CL domain). For example, the amino acid pair between the CH1 and the CL domain to which the mutation is introduced may be one or two or more (e.g., two) pairs selected from the group consisting of a pair of leucine at position 145 in the CH1 domain and serine at position 131 in the CL domain, a pair of leucine at position 145 in the CH1 domain and valine at position 133 in the CL domain, a pair of lysine at position 147 in the CH1 domain and threonine at position 180 in the CL domain, a pair of serine at position 183 in the CH1 domain and valine at position 133 in the CL domain, and a pair of valine at position 185 in the CH1 domain and leucine at position 135 in the CL domain, as numbered on the basis of the amino acid sequences of SEQ ID NO: 1 (CH1 domain) and SEQ ID NO: 10 (CL domain).

The amino acid pairs to which the mutations are introduced between the first CH1 domain and the first CL domain may be the same as or different from those between the second CH1 domain and the second CL domain.

In one embodiment, an amino acid having a positive charge is introduced to the first CH1 domain (with the introduction of an amino acid having a negative charge to the first CL domain) while an amino acid having a negative charge is introduced to the second CH1 domain (with the introduction of an amino acid having a positive charge to the second CL domain).

In order to enhance heterodimerization, at least one, for example, one, two, or three of the following mutations may be introduced to the Fc region of a heavy chain, in detail, the CH3 domain in the Fc region:

(1) mutation in which one amino acid residue of at least one amino acid pair (at least one of the two amino acid residues is not hydrophobic) between CH3 domains is substituted with an amino acid having a positive charge while the other residue is substituted with an amino acid having a negative charge (hereinafter referred to as "electrostatic interaction-induced mutation");
(2) mutation in which amino acid residues in at least one amino acid pair between the CH3 domains are exchanged with each other (hereinafter referred to as "swapping mutation"); and
(3) mutation in which one amino acid residue in at least one amino acid pair between the CH3 domains is substituted with a large amino acid (e.g., a large hydrophobic amino acid such as tryptophan, phenylalanine, etc.) while the other amino acid residue is substituted with a small amino acid (e.g., a small hydrophobic amino acid such as alanine, glycine, valine, etc.) (hereinafter referred to as "size mutation").

As such, the CH3 domain to which the mutations are introduced may be selected from the group consisting of the CH3 domain of human IgG1(SEQ ID NO: 15; corresponding to positions 340 to 447 according to the EU numbering), the CH3 domain of human IgG2 (SEQ ID NO: 16), the CH3 domain of human IgG3(SEQ ID NO: 17), the CH3 domain of human IgG4 (SEQ ID NO: 18), the CH3 domain of human IgA1 (SEQ ID NO: 19), the CH3 domain of human IgA2 (SEQ ID NO: 20), the CH3 domain of human IgD (SEQ ID NO: 21), the CH3 domain of human IgE (SEQ ID NO: 22), and the CH3 domain of human IgM (SEQ ID NO: 23).

The first 1 CH3 domain and the second CH3 domain to which the mutations are introduced may be derived from the same or different immunoglobulin types which may each be independently selected from the group consisting of IgG1, IgG2, IgG3, IgG4, IgA1, IgA2, IgD, IgE, and IgM. In one embodiment, the first CH3 domain and the second CH3 domain may both be the CH3 domain of human IgG1 having the amino acid sequence of SEQ ID NO: 15, but is not limited thereto.

The following amino acid pairs between the first CH3 domain and the second CH3 domain are based on the CH3 domain of human IgG1 having the amino acid of SEQ ID NO: 15, and the basis is true of the amino acid pairs corresponding thereto between CH3 domains of IgG2, IgG3, IgG4, IgA1, IgA2, IgD, IgE, and IgM (respectively, SEQ ID NOS: 16 to 23).

The amino acid pair between the first and second CH3 domains to which one of the mutations is introduced may be at least one, for example, one, two or more (e.g., two), three or more (e.g., three), or four or more (e.g., four) amino acid pairs selected from the group consisting of the amino acid pairs between CH3 domains and amino acid pairs at positions corresponding thereto between CH3 domains of IgG2, IgG3, IgG4, IgA1, IgA2, IgE, and IgM (respectively, SEQ ID NOS: 16 to 23), listed in Table 1, below.

TABLE 1

| Amino acid pair No. | IgG (1-4) | | IgA1 | | IgA2 | | IgE | | IgM | |
|---|---|---|---|---|---|---|---|---|---|---|
| | Chain A | Chain B | Chain A | Chain B | Chain A | Chain B | Chain A | Chain B | Chain A | Chain B |
| 1 | Q347 | K360 | E347 | L360 | E347 | L360 | E444 | R457 | Q347 | K360 |
| 2 | Y349 | S354 | H349 | P354 | H349 | P354 | Y446 | P451 | Y349 | S354 |
| 3 | Y349 | E357 | H349 | E357 | H349 | E357 | Y446 | P454 | Y349 | E357 |
| 4 | Y349 | K360 | H349 | L360 | H349 | L360 | Y446 | R457 | Y349 | K360 |
| 5 | L351 | L351 | L351 | L351 | L351 | L351 | F448 | F448 | L351 | L351 |
| 6 | P352 | P352 | P352 | P352 | P352 | P352 | A449 | A449 | P352 | P352 |
| 7 | S354 | Y349 | P354 | H349 | P354 | H349 | P451 | Y446 | S354 | Y349 |
| 8 | D356 | K439 | E356 | K439 | E356 | K439 | W453 | R539 | D356 | K439 |
| 9 | E357 | Y349 | E357 | H349 | E357 | H349 | P454 | Y446 | E357 | Y349 |
| 10 | E357 | K370 | E357 | R370 | E357 | R370 | P454 | Q467 | E357 | K370 |
| 11 | K360 | Q347 | L360 | E347 | L360 | E347 | R457 | E444 | K360 | Q347 |
| 12 | K360 | Y349 | L360 | H349 | L360 | H349 | R457 | Y446 | K360 | Y349 |
| 13 | S364 | L368 | T364 | L368 | T364 | L368 | T461 | L465 | S364 | L368 |
| 14 | S364 | K370 | T364 | R370 | T364 | R370 | T461 | Q467 | S364 | K370 |
| 15 | T366 | T366 | T366 | T366 | T366 | T366 | A463 | A463 | T366 | T366 |
| 16 | T366 | Y407 | T366 | T407 | T366 | T407 | A463 | F506 | T366 | Y407 |
| 17 | L368 | S364 | L368 | T364 | L368 | T364 | L465 | T461 | L368 | S364 |
| 18 | L368 | K409 | L368 | 1409 | L368 | 1409 | L465 | R508 | L368 | K409 |
| 19 | K370 | E357 | R370 | E357 | R370 | E357 | Q467 | P454 | K370 | E357 |
| 20 | K370 | S364 | R370 | T364 | R370 | T364 | Q467 | T461 | K370 | S364 |
| 21 | K370 | T411 | R370 | R411 | R370 | R411 | Q467 | E510 | K370 | T411 |
| 22 | N390 | S400 | L390 | S400 | L390 | S400 | R489 | K499 | N390 | S400 |
| 23 | K392 | L398 | L392 | Q398 | L392 | Q398 | S491 | K497 | K392 | L398 |
| 24 | T394 | T394 | W394 | W394 | W394 | W394 | T493 | T493 | T394 | T394 |
| 25 | T394 | V397 | W394 | R397 | W394 | R397 | T493 | R496 | T394 | V397 |
| 26 | P395 | P395 | A395 | A395 | A395 | A395 | Q494 | Q494 | P395 | P395 |

(continued)

| Amino acid pair No. | IgG (1-4) | | IgA1 | | IgA2 | | IgE | | IgM | |
|---|---|---|---|---|---|---|---|---|---|---|
| | Chain A | Chain B | Chain A | Chain B | Chain A | Chain B | Chain A | Chain B | Chain A | Chain B |
| 27 | P395 | V397 | A395 | R397 | A395 | R397 | Q494 | R496 | P395 | V397 |
| 28 | V397 | T394 | R397 | W394 | R397 | W394 | R496 | T493 | V397 | T394 |
| 29 | V397 | P395 | R397 | A395 | R397 | A395 | R496 | Q494 | V397 | P395 |
| 30 | L398 | K392 | Q398 | L392 | Q398 | L392 | K497 | S491 | L398 | K392 |
| 31 | S400 | N390 | S400 | K390 | S400 | K390 | K499 | R489 | S400 | N390 |
| 32 | F405 | K409 | A405 | I409 | A405 | I409 | F504 | R508 | F405 | K409 |
| 33 | Y407 | T366 | T407 | T366 | T407 | T366 | F506 | A463 | Y407 | T366 |
| 34 | Y407 | Y407 | T407 | T407 | T407 | T407 | F506 | F506 | Y407 | Y407 |
| 35 | Y407 | K409 | T407 | I409 | T407 | I409 | F506 | R508 | Y407 | K409 |
| 36 | K409 | L368 | 1409 | L368 | 1409 | L368 | R508 | L465 | K409 | L368 |
| 37 | K409 | F405 | I409 | A405 | I409 | A405 | R508 | F504 | K409 | F405 |
| 38 | K409 | Y407 | I409 | T407 | I409 | T407 | R508 | F506 | K409 | Y407 |
| 39 | T411 | K370 | R411 | R370 | R411 | R370 | E510 | Q467 | T411 | K370 |
| 40 | K439 | D356 | K439 | K439 | K439 | K439 | R539 | W453 | K439 | D356 |
| (Chain A: first CH3 domain; Chain B: second CH3 domain) | | | | | | | | | | |

In greater detail, the amino acid pair between the first CH3 domain and the second CH3 domain to which at least one of the following mutations: (1) electrostatic interaction-induced mutation (represented as charge (J) in Table 2 and FIG. 2); (2) swapping mutation (represented as swap (O) in Table 2 and FIG. 2); and (3) size mutation (represented as size (B) in Table 2 and FIG. 2) may be at least one, for example, one, two or more (e.g., two), three or more (e.g., three), or four or more (e.g., four) amino acid pairs selected from the amino acid pairs between CH3 domains of IgG1 (SEQ ID NO: 15) and amino acid pairs at positions corresponding thereto between CH3 domains of IgG2, IgG3 IgG4 IgA1 IgA2 IgD, IgE, and IgM (respectively, SEQ ID NOS: 16 to 23), suggested in Table 2 and FIG. 2:

TABLE 2

| Amino acid pair No. | Charge (J) | | Swap (O) | | Size (B) | |
|---|---|---|---|---|---|---|
| | Chain A | Chain B | Chain A | Chain B | Chain A | Chain B |
| 1 | Q347 | K360 | Q347 | K360 | Q347 | K360 |
| 2 | Y349 | S354 | | | Y349 | S354 |
| 3 | Y349 | E357 | | | Y349 | E357 |
| 4 | Y349 | K360 | | | Y349 | K360 |
| 5 | L351 | L351 | | | L351 | L351 |
| 6 | P352 | P352 | | | P352 | P352 |
| 7 | S354 | Y349 | S354 | Y349 | S354 | Y349 |
| 8 | D356 | K439 | | | D356 | K439 |
| 9 | E357 | Y349 | E357 | Y349 | E357 | Y349 |
| 10 | E357 | K370 | E357 | K370 | E357 | K370 |
| 11 | K360 | Q347 | | | K360 | Q347 |
| 12 | K360 | Y349 | K360 | Y349 | K360 | Y349 |

(continued)

| Amino acid pair No. | Charge (J) | | Swap (O) | | Size (B) | |
|---|---|---|---|---|---|---|
| | Chain A | Chain B | Chain A | Chain B | Chain A | Chain B |
| 13 | S364 | L368 | S364 | L368 | S364 | L368 |
| 14 | S364 | K370 | S364 | K370 | S364 | K370 |
| 15 | T366 | T366 | | | T366 | T366 |
| 16 | T366 | Y407 | | | T366 | Y407 |
| 17 | L368 | S364 | | | L368 | S364 |
| 18 | L368 | K409 | L368 | K409 | L368 | K409 |
| 19 | K370 | E357 | | | K370 | E357 |
| 20 | K370 | S364 | | | K370 | S364 |
| 21 | K370 | T411 | | | K370 | T411 |
| 22 | N390 | S400 | N390 | S400 | N390 | S400 |
| 23 | K392 | L398 | | | K392 | L398 |
| 24 | T394 | T394 | | | T394 | T394 |
| 25 | T394 | V397 | T394 | V397 | T394 | V397 |
| 26 | P395 | P395 | | | P395 | P395 |
| 27 | P395 | V397 | | | P395 | V397 |
| 28 | V397 | T394 | | | V397 | T394 |
| 29 | V397 | P395 | | | V397 | P395 |
| 30 | L398 | K392 | L398 | K392 | L398 | K392 |
| 31 | S400 | N390 | | | S400 | N390 |
| 32 | F405 | K409 | F405 | K409 | F405 | K409 |
| 33 | Y407 | T366 | Y407 | T366 | Y407 | T366 |
| 34 | Y407 | Y407 | | | Y407 | Y407 |
| 35 | Y407 | K409 | | | Y407 | K409 |
| 36 | K409 | L368 | | | K409 | L368 |
| 37 | K409 | F405 | | | K409 | F405 |
| 38 | K409 | Y407 | | | K409 | Y407 |
| 39 | T411 | K370 | T411 | K370 | T411 | K370 |
| 40 | | | | | K439 | D356 |
| (Chain A: first CH3 domain; Chain B: second CH3 domain; the mutations listed in Table 2 are given to positions based on IgG1, but are applied to corresponding positions in the CH3 domains of IgG2, IgG3, IgG4, IgA1, IgA2, IgD, IgE, and IgM). | | | | | | |

As used herein, for example, "Q347" means glutamine at position 347 on the amino acid sequence of SEQ ID NO: 15 in the CH3 domain of human IgG1, and such notation is true of amino acid residues at positions corresponding thereto in CH3 domains of IgG2, IgG3 IgG4, IgA1, IgA2, IgD, IgE, and IgM (respectively, SEQ ID NOS: 16 to 23) (hereinafter, the same definition is applied).

Hereinafter, amino acid pairs between CH3 domains to which the mutations are introduced are numbered on the basis of the amino acid sequence of SEQ ID NO: 15 and unless otherwise described, the numbering is construed to be applied to amino acids at denoted positions in IgG1 as well as at positions corresponding thereto in CH3 domains of other type immunoglobulins (IgG2, IgG3, IgG4, IgA1, IgA2, IgD, IgE, and IgM).

The electrostatic interaction-induced mutation is intended to substitute a positively charged amino acid for one amino acid residue of at least one amino acid pair between Fc regions or CH3 domains (at least one residue of the paired two amino acids is not hydrophobic) and a negatively charged amino acid for the other amino acid residue to introduce electrostatic interaction to a hydrophobic interaction-lacking site, thereby contributing to an increase of electrostatic interaction-induced binding force.

The hydrophobic amino acid may be selected from the group consisting of glycine, alanine, valine, leucine, isoleucine, methionine, proline, phenylalanine, and tryptophan.

The amino acid having a negative charge may be selected from among acidic amino acids and may be, for example, aspartic acid or glutamic acid. The amino acid having a positive charge may be selected from among basic amino acid and may be, for example, lysine or arginine.

The amino acid pair between the first CH3 domain and the second CH3 domain to which an electrostatic interaction-introduced mutation is applicable may be at least one, for example, one, two or more (e.g., two), three or more (e.g., three), or four or more (e.g., four) selected from among amino acid pair numbers 1 to 39 in Table 2 and may be at least one, for example, one, two or more (e.g., two), three or more (e.g., three), or four or more (e.g., four) selected from the group consisting of, for example, a pair of serine at position 364 and leucine at position 368, a pair of threonine at position 394 and threonine at position 394, a pair of glutamic acid at position 357 and lysine at position 370, a pair of glutamic acid at position 357 and tyrosine at position 349, a pair of threonine at position 366 and tyrosine at position 407, and a pair of threonine at position 394 and valine at position 397.

That is, the electrostatic interaction-introduced mutation in the CH3 domain may comprise substitution of an amino acid having a positive charge for one amino acid residue of each of the amino acid pairs, for example, one, two or more (e.g., two), three or more (e.g., three), or four or more (e.g., four) selected from among the amino acid pair number 1 to 39 in Table 2; and an amino acid having a negative charge for the other amino acid residue. By way of example, in each of the amino acid pairs, for example, one or more (e.g., one), two or more (e.g., two), three or more (e.g., three), or four or more (e.g., four) amino acid pairs selected from the group consisting of a pair of serine at position 364 and leucine at position 368, a pair of threonine at position 394 and threonine at position 394, a pair of glutamic acid at position 357 and lysine at position 370, a pair of glutamic acid at position 357 and tyrosine at position 349, a pair of threonine at position 366 and tyrosine at position 407, and a pair of threonine at position 394 and valine at position 397, one amino acid residue is substituted with an amino acid having a positive charge while the other amino acid residue is substituted with an amino acid having a negative charge.

For instance, the electrostatic interaction-introduced mutation in CH3 domains may comprise at least one, for example, one, two or more (e.g., two), three or more (e.g., three), or four or more (e.g., four) of the following mutations:

substitution of serine at position 364 with an amino acid having a positive charge and leucine at position 368 with an amino acid having a negative charge;
substitution of threonine at position 394 with an amino acid having a positive charge and threonine at position 394 with an amino acid having a negative charge;
substitution of glutamic acid at position 357 with an amino acid having a positive charge and lysine at position 370 with an amino acid having a negative charge;
substitution of glutamic acid at position 357 with an amino acid having a positive charge and tyrosine at position 349 with an amino acid having a negative charge;
substitution of threonine at position 366 with an amino acid having a positive charge and tyrosine at position 407 with an amino acid having a negative charge;
substitution of threonine at position 394 with an amino acid having a positive charge and valine at position 397 with an amino acid having a negative charge; and
substitution of tyrosine at position 349 with an amino acid having a positive charge and glutamic acid at position 357 with an amino acid having a negative charge.

Such an electrostatic interaction introduction in CH3 domains may achieve a heterodimerization rate of 60% or higher, 65% or higher, 70% or higher, 73% or higher, 75% or higher, 78% or higher, 80% or higher, 85% or higher, 90% or higher, 95% or higher, or 100%.

The swapping mutation means a mutation in which two amino acid residues constituting an amino acid pair are exchanged (swapped) with each other.

As illustrated in Table 2 and FIG. 2, the amino acid pair between the first CH3 domain and the second CH3 domain to which the swapping mutation is applicable may be at least one, for example, one, two or more (e.g., two), three or more (e.g., three), or four or more (e.g., four) selected from the group consisting of a pair of glutamine at position 354 and lysine at position 360, a pair of serine at position 357 and tyrosine at position 349, a pair of glutamic acid at position 357 and lysine at position 370, a pair of lysine at position 360 and tyrosine at position 349, a pair of serine at position 364 and leucine at position 368, a pair of serine at position 364 and lysine at position 370, a pair of leucine at position 368 and lysine at position 409, a pair of asparagine at position 390 and serine at position 400, a pair of threonine at position 394 and valine at position 397, a pair of leucine at position 398 and lysine at position 392, a pair of phenylalanine at position 405 and lysine at position 409, a pair of tyrosine at position 407 and threonine at position 366, and a pair of threonine at position 411 and lysine at position 370 and particularly from the group consisting of a pair of serine at position 364 and lysine at position 370, a pair of tyrosine at position 407 and threonine at position 366, a pair of glutamic acid at position 357 and lysine at position 370, a pair of phenylalanine at position 405 and lysine at position 409, and a pair of serine at position 357 and tyrosine at position 349.

That is, the swapping mutation in CH3 domains may comprise substitution in which exchange (swapping) is made between two paired amino acid residues in each of at least one, for example, one, two or more (e.g., two), three or more (e.g., three), or four or more (e.g., four) amino acid pairs selected from the group consisting of a pair of glutamine at position 354 and lysine at position 360, a pair of serine at position 357 and tyrosine at position 349, a pair of glutamic acid at position 357 and lysine at position 370, a pair of lysine at position 360 and tyrosine at position 349, a pair of serine at position 364 and leucine at position 368, a pair of serine at position 364 and lysine at position 370, a pair of leucine at position 368 and lysine at position 409, a pair of asparagine at position 390 and serine at position 400, a pair of threonine at position 394 and valine at position 397, a pair of leucine at position 398 and lysine at position 392, a pair of phenylalanine at position 405 and lysine at position 409, a pair of tyrosine at position 407 and threonine at position 366, and a pair of threonine at position 411 and lysine at position 370; and for example, from the group consisting of a pair of serine at position 364 and lysine at position 370, a pair of tyrosine at position 407 and threonine at position 366, a pair of glutamic acid at position 357 and lysine at position 370, a pair of phenylalanine at position 405 and lysine at position 409, and a pair of serine at position 357 and tyrosine at position 349.

For example, the swapping mutation in CH3 domains may comprise at least one, for example, one, two or more (e.g., two), three or more (e.g., three), or four or more (e.g., four) of the following mutations:

substitution of serine at position 364 with lysine and lysine at position 370 with serine;
substitution of phenylalanine at position 405 with lysine and lysine at position 409 with phenylalanine;
substitution of tyrosine at position 407 with threonine and threonine at position 366 with tyrosine;
substitution of glutamic acid at position 357 with lysine and lysine at position 370 with glutamic acid; and
substitution of serine at position 357 with tyrosine and tyrosine at position 349 with serine.

Such a swapping mutation in CH3 domains may achieve a heterodimerization rate of 60% or higher, 65% or higher, 70% or higher, 73% or higher, 75% or higher, 78% or higher, 80% or higher, 85% or higher, 90% or higher, 95% or higher, or 100%.

The size mutation means a mutation in which, of the two paired amino acids in at least one amino acid pair between CH3 domains, one residue is substituted with a large hydrophobic amino acid (e.g., tryptophan, phenylalanine, etc.) and the other is substituted with a small hydrophobic amino acid (e.g., alanine, glycine, valine, etc.) so that the large amino acid is fitted to the space secured by the small amino acid, thereby contributing to heterodimerization.

The large amino acid may comprise a cyclic residue and may be selected from the group consisting of tryptophan and phenylalanine, and particularly tryptophan. The small amino acid may be selected from the group consisting of alanine, glycine, and valine and may be, for example, alanine.

The amino acid pair between the first CH3 domain and the second CH3 domain to which the size mutation is applicable may be at least one, for example, one, two or more (e.g., two), three or more (e.g., three), or four or more (e.g., four) selected from among amino acid pair numbers 1 to 40 in Table 2 and may be, for example, a pair of lysine at position 409 and tyrosine at position 407, a pair of lysine at position 409 and phenylalanine at position 405, or a combination thereof.

That is, the size mutation may comprise substitution in which, of two paired amino acid residues in each of at least one, for example, one, two or more (e.g., two), three or more (e.g., three), or four or more (e.g., four) amino acid pairs selected from among amino acid pair numbers 1 to 40 in Table 2, one is substituted with a large hydrophobic amino acid (e.g., tryptophan, phenylalanine, etc.), for example, tryptophan while the other is substituted with a small hydrophobic amino acid (e.g., alanine, glycine, valine, etc.), for example, alanine. By way of example, in a pair of lysine at position 409 and tyrosine at position 407 or a pair of lysine at position 409 and phenylalanine at position 405, or in each of them, one residue may be substituted with a large hydrophobic amino acid, for example, phenylalanine or tryptophan while the other residue may be substituted with a small hydrophobic amino acid, for example, alanine, glycine, or valine.

For example, the size mutation in CH3 domains may comprise at least one of the following mutations:

substitution of lysine at position 409 with tryptophan and tyrosine at position 407 with alanine; and
substitution of lysine at position 409 with tryptophan and phenylalanine at position 405 with alanine.

The modified CH3 domains may comprise at least one, for example, one or two of the three mutations described above, that is, electrostatic interaction-introduced mutation, swapping mutation, and size mutation.

In order to exert the most advantageous effect on dimerization, the modified CH3 domains may comprise at least one, for example, one, two, or three mutations selected from the group consisting of substitution of one residue in a pair of serine at position 364 and leucine at position 368 with an amino acid having a positive charge and the other with an amino acid having a negative charge (electrostatic interaction-introduced mutation), exchange of the two residues of a pair of serine at position 364 and lysine at position 370 with each other (swapping mutation), and exchange of the two residues of a pair of phenylalanine at position 405 and lysine at position 409 with each other (swapping mutation).

For example, the modified CH3 domains may comprise one, two, or three of the following mutations:

(a) substitution of serine at position 364 with an amino acid having a positive charge and leucine at position 368 with an amino acid having a negative charge;
(b) substitution of serine at position 364 with lysine and lysine at position 370 with serine; and
(c) substitution of phenylalanine at position 405 with lysine and lysine at position 409 with phenylalanine.

In order to decrease monomerization rates, but increase dimerization rates, an additional amino acid modification may be introduced after the three selected mutations ((a)-(c)). As such, the amino acid to which the additional amino acid mutation can be introduced may be serine at position 364, phenylalanine at position 405, and/or lysine at position 409. For example, in a pair of serine at position 364 and leucine at position 368 which is to undergo an electrostatic interaction-induced mutation, when leucine at position 368 may be substituted with an amino acid having a negative charge (aspartic acid or glutamic acid, e.g., aspartic acid), serine at position 364 may be substituted with an amino acid having a positive charge (lysine or arginine) (S364K or S364R; electrostatic interaction-introduced mutation) or with asparagine (S364N). In addition, in a pair of lysine at position 370 and serine at position 364, which is to undergo a swapping mutation, lysine at position 370 may be substituted with serine and serine at position 364 may be substituted with lysine (S364K; swapping mutation) or with arginine or asparagine (S364R or S364N).

Further, in a pair of lysine at position 409 and phenylalanine at position 405, which is to undergo swapping mutation, lysine at position 409 may be substituted with phenylalanine (for swapping mutation) or with tryptophan and phenylalanine at position 405 may be substituted with lysine (F405K; for swapping mutation) or with arginine, glutamine, or asparagine (F405R, F405Q, or F405N).

In one embodiment, the modified CH3 domains may comprise at least one of the following mutations:

substitution of serine at position 364 with lysine and leucine at position 368 with aspartic acid;
substitution of serine at position 364 with lysine and lysine at position 370 with serine;
substitution of phenylalanine at position 405 with lysine and lysine at position 409 with phenylalanine;
substitution of phenylalanine at position 405 with arginine and lysine at position 409 with phenylalanine;
substitution of phenylalanine at position 405 with lysine and lysine at position 409 with tryptophan; and
substitution of phenylalanine at position 405 with arginine and lysine at position 409 with tryptophan.

In one embodiment, the modified CH3 domains may comprise at least one of the following double mutations:

substitution of serine at position 364 in the first CH3 domain with lysine and leucine at position 368 in the second CH3 domain with aspartic acid, and substitution of lysine at position 409 in the first CH3 domain with phenylalanine and phenylalanine at position 405 in the second CH3 domain with lysine;
substitution of serine at position 364 in the first CH3 domain with lysine and leucine at position 368 in the second CH3 domain with aspartic acid, and substitution of lysine at position 409 in the first CH3 domain with phenylalanine and phenylalanine at position 405 in the second CH3 domain with arginine;
substitution of serine at position 364 in the first CH3 domain with lysine and lysine at position 370 in the second CH3 domain with serine, and substitution of lysine at position 409 in the first CH3 domain with phenylalanine and phenylalanine at position 405 in the second CH3 domain with lysine;
substitution of serine at position 364 in the first CH3 domain with lysine and lysine at position 370 in the second CH3 domain with serine, and substitution of lysine at position 409 in the first CH3 domain with phenylalanine and phenylalanine at position 405 in the second CH3 domain with arginine;
substitution of serine at position 364 in the first CH3 domain with lysine and leucine at position 368 in the second CH3 domain with aspartic acid, and substitution of lysine at position 409 in the first CH3 domain with tryptophan and phenylalanine at position 405 in the second CH3 domain with lysine;
substitution of serine at position 364 in the first CH3 domain with lysine and leucine at position 368 in the second CH3 domain with aspartic acid, and substitution of lysine at position 409 in the first CH3 domain with tryptophan and phenylalanine at position 405 in the second CH3 domain with arginine;
substitution of serine at position 364 in the first CH3 domain with lysine and lysine at position 370 in the second CH3 domain with serine, and substitution of lysine at position 409 in the first CH3 domain with tryptophan and phenylalanine at position 405 in the second CH3 domain with lysine; or
substitution of serine at position 364 in the first CH3 domain with lysine and lysine at position 370 in the second CH3 domain with serine, and substitution of lysine at position 409 in the first CH3 domain with tryptophan and phenylalanine at position 405 in the second CH3 domain with arginine.

Such a double mutation in the CH3 domain may result in a dimerization rate of 70% or higher, 75% or higher, 80% or higher, 85% or higher, 90% or higher, 92% or higher, 93% or higher, 94% or higher, 95% or higher, or 96% or higher.
Another aspect of the present invention provides an anti-influenza B antibody comprising a heavy chain variable region composed of the amino acid sequence of SEQ ID NO: 27 and a light chain variable region composed of the amino acid sequence of SEQ ID NO: 31, or an antigen-binding fragment thereof. Another aspect of the present invention provides an anti-influenza A antibody comprising a heavy chain variable region composed of the amino acid sequence of SEQ ID NO: 29 and a light chain variable region composed of the amino acid sequence of SEQ ID NO: 31, or an antigen-binding fragment thereof.
Another aspect of the present invention provides an anti-influenza A/anti-influenza B bispecific antibody comprising an anti-influenza B antibody comprising a heavy chain variable region composed of the amino acid sequence of SEQ ID NO: 27 and a light chain variable region composed of the amino acid sequence of SEQ ID NO: 31, and an anti-influenza A antibody comprising a heavy chain variable region composed of the amino acid sequence of SEQ ID NO: 29 and a light chain variable region composed of the amino acid sequence of SEQ ID NO: 31, or an antigen-binding fragment thereof. The anti-influenza A/anti-influenza B bispecific antibody may comprise (1) the modified CH3 domain (as mentioned above, a mutation pair of CH3-CH3 introduced); (2) the mutant CH1 domain and the mutant CL domain (as mentioned above, a mutation pair of CH1-CL introduced); or (3) both the modified CH3 domain, and the mutant CH1 domain and the mutant CL domain.
The bispecific protein or bispecific antibody of the present invention is a bispecific matter constructed according to the **C**orrelated and **H**armonious **I**nterfacial **M**utation between **P**rotein **S**ubunits (hereinafter referred to as "Chimps").
As used herein, the term "antibody" refers to a class of structurally related glycoproteins consisting of two pairs of polypeptide chains, one pair of light (L) low molecular weight chains and one pair of heavy (H) chains, all four inter-connected by disulfide bonds. The structure of antibodies has been well characterized (see, for instance, [Fundamental Immunology Ch. 7 (Paul, W., 2nd ed. Raven Press, N. Y. (1989)]). In brief, each heavy chain typically is comprised of a heavy chain variable region (abbreviated herein as VH) and a heavy chain constant region. The heavy chain constant region typically is comprised of three domains, CH1, CH2, and CH3. The heavy chains are inter-connected via disulfide bonds in the so-called "hinge region". Each light chain typically is comprised of a light chain variable region (abbreviated herein as VL) and a light chain constant region. The light chain constant region typically is comprised of one domain, CL. Typically, the numbering of amino acid residues in the constant region is performed according to the EU-index as described in the document [Kabat et al., Sequences of Proteins of Immunological Interest, 5th Ed. Public Health Service, National Institutes of Health, Bethesda, Md. (1991)]. The VH and VL regions may be further subdivided into regions of hypervariability (or hypervariable regions which may be hypervariable in sequence and/or form of structurally defined loops), also termed complementarity determining regions (CDRs), interspersed with regions that are more conserved, termed framework regions (FRs). Each VH and VL is typically composed of three CDRs and four FRs, arranged from amino-terminus to carboxy-terminus in the following order: FR1, CDR1, FR2, CDR2, FR3, CDR3, FR4 (see also document [Chothia and Lesk J. Mol. Biol. 196, 901 917 (1987)]).
As used herein, the term "Fab-arm" refers to one heavy chain-light chain pair.
The term "Fc region", as used herein, refers to an antibody region comprising a CH2 domain and a CH3 domain and may further comprise the hinge region, optionally.
As used herein, the term "bispecific antibody" refers to an antibody having specificities for at least two different epitope, typically non-overlapping epitopes.
The term "full-length antibody", as used herein, refers to an antibody which contains all heavy and light chain constant and variable domains corresponding to those that are normally found in an antibody of that isotype. In an embodiment, a full-length antibody comprises two full-length heavy chains and two full-length light chains. As used herein, "isotype" refers to the antibody class (for instance IgG1, IgG2, IgG3, IgG4, IgD, IgA, IgE, or IgM) that is encoded by heavy chain constant region genes.
The term "antigen-binding fragment" is intended to mean a portion of an antibody containing variable domains of the antibody and may be selected from among Fab, F(ab')2, scFv, (scFv)2, scFv-Fc, (scFv-Fc), etc., but is not limited thereto.
The term "epitope" means a protein determinant capable of specifically binding to an antibody. Epitopes usually consist of surface groupings of molecules such as amino acids or sugar side chains and usually have specific three dimensional structural characteristics as well as specific charge characteristics.
The term "bispecific antibody" is generic to the antibody that recognizes and/or binds to two different antigens or two different (non-overlapping) epitopes on one antigen. In an embodiment, the bispecific antibody may comprise one antigen-binding site directed against a tumor cell antigen and another antigen-binding site directed against a cytotoxic trigger molecule. By way of example, the bispecific antibody may be selected from the group consisting of an anti-FcγRI/anti-CD15 antibody, an anti-p185HER2/anti-FcγRIII (CD16) antibody, an anti-CD3/anti-malignant B-cell (1D10) antibody, an anti-CD 3/anti-p185HER2 antibody, an anti-CD3/anti-p97 antibody, an anti-CD3/anti-renal cell carcinoma antibody, an anti-CD3/anti-OVCAR-3 antibody, an anti-CD3/anti-L-D1 (anti-colon cancer) antibody, an anti-CD3/anti-melanocyte stimulating hormone analog antibody, an anti-EGF receptor/anti-CD3 antibody, an anti-CD3/anti-CAMA1 antibody, an anti-CD3/anti-CD19 antibody, an anti-CD3/MoV18 antibody, an anti-neural cell adhesion molecule (NCAM)/anti-CD3 antibody, an anti-folate binding protein (FBP)/anti-CD3 antibody, an anti-pan carcinoma associated antigen (AMOC-31)/anti-CD3 antibody, but are not limited thereto. In another embodiment, bispecific antibodies with one antigen-binding site which binds specifically to a tumor antigen and another antigen-binding site which binds to a toxin may comprise, but is not limited to, an anti-saporin/anti-Id-1 antibody, an anti-CD22/anti-saporin antibody, an anti-CD7/anti-saporin antibody, an anti-CD38/anti-saporin antibody, an anti-CEA/anti-ricin A chain antibody, an anti-interferon-a (IFN-α)/anti-hybridoma idiotype antibody, and an anti-CEA/anti-vinca alkaloid antibody. In another embodiment, the bispecific antibody may be selected from among antibodies for converting enzyme activated prodrugs such as anti-CD30/anti-alkaline phosphatase (which catalyzes conversion of mitomycin phosphate prodrug to mitomycin alcohol), but is not limited thereto. In another embodiment, the bispecific antibody may be selected from among those that can be used as fibrinolytic agents such as anti-fibrin/anti-tissue plasminogen activator (tPA), anti-fibrin/anti-urokinase-type plasminogen activator (uPA), etc., but is not limited thereto. In another embodiment, the bispecific antibody may be selected from among those for targeting immune complexes to cell surface receptors such as anti-low density lipoprotein (LDL)/anti-Fc receptor (e.g. FcγRI, FcγRII or FcγRIII), but is not limited thereto. In another embodiment, the bispecific antibody may be selected from those for use in therapy of infectious diseases (e.g., viral infection diseases) such as anti-influenza A/anti-influenza B, anti-CD3/anti-herpes simplex virus (HSV), anti-T-cell receptor:CD3 complex/anti-influenza, anti-FcγR/anti-HIV, etc., but is not limited thereto. In another embodiment, the bispecific antibody may be selected from those for tumor detection in vitro or in vivo such as anti-CEA/anti-EOTUBE, anti-CEA/anti-DPTA, anti-p185 HER2/anti-hapten, etc., but is not limited thereto. In another embodiment, the bispecific antibody may be selected from those for use as diagnostic tools such as anti-rabbit IgG/anti-ferritin, anti-horse radish peroxidase (HRP)/anti-hormone, anti-somatostatin/anti-substance P, anti-HRP/anti-FITC, anti-CEA/anti-β-galactosidase, etc. In another embodiment, examples of the bispecific antibody comprise, but are not limited to, an antibody inclusive of a first antigen-binding site directed against CD30 and an antigen-binding site directed against erbB2; an antibody inclusive of a first antigen-binding site directed against CD30 and a second antigen-binding site directed against Pseudomonas exotoxin (PE); an antibody inclusive of a first antigen-binding site directed against CD30 and a second antigen-binding site directed against streptavidin.

According to another embodiment, the targeting domain of the bispecific protein may comprise at least one target specific binding polypeptide selected from the group consisting of various membrane proteins, for example, various receptors (e.g., receptor tyrosine kinase (RTKs), etc.), ectodomains (extracellular domains) of the receptors, and various ligands (e.g., various growth factors, cytokines, etc.). Examples of the receptor comprise, but are not limited to, tumor necrosis factor receptor (TNFR) (e.g., TNFR1, TNFR2, etc.), epidermal growth factor receptor (EGFR) (e.g., Her1(epidermal growth factor receptor 1), Her2 (human epidermal growth factor receptor 2), Her3 (human epidermal growth factor receptor 3), etc.), angiopoietin receptor (e.g., Tie1, Tie2, etc.), transforming growth factor receptor (e.g, TGFbR1, TGFbR2, TGFbR3, TGFaR1, etc.), bone morphogenetic protein receptor (e.g, BMPR1b), interleukin receptor (e.g., interleukin 12 receptor subunit beta 1 (IL-12R-b1)), IL-4Ra, IL-12A, IL-4, IL-1R1L, IL-17RA, IL-17A, IL-12R-b2, IL-13Ra1, IL-12B, IL-13, IL-1RAP, IL-17RC, IL-17F, etc.), integrin (e.g., integrin alpha 4 (ITGA4), integrin subunit alpha 2b (ITGA2B), ITGB1, ITGB3, etc.), interferon receptor (e.g., interferon-alpha/beta receptor 1 (IFNAR1), IFNAR2, IFNGR, etc.), Fas (tumor necrosis factor receptor superfamily member 6; TNFRSF6), VEGF receptor (e.g., Flt1 (fms related tyrosine kinase 1), etc.), hepatocyte growth factor receptor (e.g., Met, etc.), and Interferon gamma receptor (IFNGR). The ligand may be at least one selected from the group consisting of tumor necrosis factor (TNF), epidermal growth factor (EGF), vascular endothelial cell growth factor (VEGF-A, VEGF-B, VEGF-C, VEGF-D, etc.), angiopoietin (e.g., Ang1, Ang2, etc.), transforming growth factor (TGF), hepatocyte growth factor (HGF), bone morphogenetic protein (e.g., BMP2, BMP7, etc.), interleukin, and interferon, but is not limited thereto.

The term "host cell", as used herein, is intended to refer to a cell into which an expression vector has been introduced, e.g. an expression vector encoding an antibody of the invention. Recombinant host cells comprise, for example, transfectomas, such as CHO cells, HEK293 cells, NS/0 cells, and lymphocytic cells.

CL domains, CH1 domains, and Fc regions (e.g., CH3 domains) in the antibodies of the present invention may be obtained from any antibody such as IgG1, IgG2, IgG3, or IgG4 subtype, IgA, IgE, IgD, or IgM. The antibodies may be derived from mammals comprising primates such as humans, monkeys, etc. and rodents such as mice, rats, etc. Because antibodies derived from mammals exhibit high sequence homology and structural homology among species, an explanation given of the CL domain, CH1 domain, and CH3 domain in the description is generally applicable to antibodies derived from mammals. In one embodiment, the CL domain, CH1 domain, and CH3 domain may be derived from IgG (e.g., IgG1), but is not limited thereto. As mentioned above, the Fc region in the antibodies described in the present invention comprise two different heavy chains (e.g., different in the sequence of variable domain). Of the two different heavy chains, at least one undergoes an amino acid mutation to increase a possibility of stably forming a heterodimer between the two different heavy chains, but decrease a possibility of stably forming a homodimer between two identical heavy chains.

Bispecific proteins, bispecific antibodies, and antigen-binding fragments thereof provided in the description can be constructed using any means and, for example, by a chemical synthesis or recombinant method. The proteins, the antibodies, and the fragments may be non-naturally occurring.

Mutation sites necessary for dimerization of Fc in the present invention are depicted in FIG. 2. Targets amount to 39 sites for electrostatic interaction-introduced mutation and 14 sites for swapping mutation, and 40 sites for size mutation. In FIG. 2, amino acid residues are expressed as capital letters according to a typical method. The numbering of amino acid residues in the constant region is performed according to the EU-index as described in the document [Kabat et al., Sequences of Proteins of Immunological Interest, 5th Ed. Public Health Service, National Institutes of Health, Bethesda, MD. (1991)].

Another aspect of the present invention provides a pharmaceutical composition comprising the aforementioned bispecific protein or bispecific antibody and optionally a pharmaceutically acceptable carrier. Another aspect provides a use of the aforementioned bispecific protein or bispecific antibody in preparing a pharmaceutical composition. Another aspect provides a method for preparing a pharmaceutical composition comprising the aforementioned bispecific protein or bispecific antibody.

An antibody and a composition comprising the same (e.g., pharmaceutical composition) can be applied to diagnosis and treatment, and as such, can be contained in a therapeutic or diagnostic kit.

As used herein, "pharmaceutically acceptable carrier" comprises any and all physiologically compatible solvents, dispersion media, coatings, antibacterial and antifungal agents, isotonicity agents, absorption delaying agents, typical vehicles used for preparation of other drugs, excipients, and additives. Preferably, the carrier may be suitable for intravenous, intramuscular, subcutaneous, parenteral, intraspinal, or epidermal administration (e.g., by injection or infusion).

A composition of the present invention can be administered by a variety of methods known in the art. As will be appreciated by a person skilled in the art, the route and/or mode of administration will vary depending upon the desired results. To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents comprise saline and aqueous buffer solutions. Pharmaceutical carriers comprise sterile aqueous solutions or dispersions and sterile powders for the extemporaneous preparation of sterile injectable solutions or dispersion. The use of such media and agents for pharmaceutically active substances is known in the art.

The phrases "parenteral administration" and "administered parenterally", as used herein, means modes of administration other than enteral and topical administration, usually by injection, and comprises intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion.

These compositions may also contain adjuvants such as preservatives, wetting agents, emulsifying agents and dispersing agents. Prevention of presence of microorganisms may be ensured both by sterilization procedures, *supra*, and by the inclusion of various antibacterial and antifungal agents, for example, paraben, chlorobutanol, phenol, sorbic acid, and the like. It may also be desirable to comprise isotonic agents, such as sugars, sodium chloride, and the like into the compositions. In addition, prolonged absorption of the injectable pharmaceutical form may be brought about by the inclusion of agents which delay absorption such as aluminum monostearate and gelatin. Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors comprising the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The administration subject may be selected from among mammals comprising primates such as humans, monkeys, etc., rodents such as mice, rats, etc., and the like, isolates therefrom comprising cells, tissues, and body fluid (e.g., blood, etc.), and cultured products thereof..

**Advantageous Effects**

The present invention provide a highly pure heterodimeric protein as a Chimps protein (e.g., antibody), which is significantly free of contaminants such as homodimers or monomers, and a construction technique therefor. Another advantage of the present invention is to increase the purity of the bispecific antibody and to introduce a minimal number of mutations to a natural antibody, thereby causing no significant structural changes of natural antibodies and reducing the risk of inducing the antibody to undergo functional loss or abnormality and/or to elicit immune rejection.

**Description of Drawings**

FIG. 1 is a schematic diagram showing only one perfect heterodimeric bispecific antibody (dotted line circle) among a variety of antibody forms possible for construction of bispecific antibodies (10 in total). A and B represent respective heavy chains different from each other and a and b represent respective light chain different from each other.

FIG. 2 shows residue positions of antibodies used in electrostatic interaction, swapping, and size methods for inducing heterodimerization in Fc constant regions according to one embodiment, and residue positions related thereto.

FIG. 3 is an SDS-PAGE profile showing heterodimerization results from electrostatic interaction-induced mutations listed in Table 4.

FIG. 4 is an SDS-PAGE profile showing heterodimerization results from swapping-associated mutations listed in Table 5.

FIG. 5 is an SDS-PAGE profile showing heterodimerization results from size-associated mutations listed in Table 6.

FIG. 6a shows comparison of 12 mutations that allow outstanding heterodimerization among the single mutations liisted in Table 7.

FIG. 6b shows heterodimerization results after S364K of two key mutations was substituted with other amino acids according to one embodiment.

FIG. 6c shows heterodimerization results after F405K of two key mutations was substituted with other amino acids according to one embodiment.

FIG. 6d shows heterodimerization results after additional mutations were introduced to the three selected key-lock mutation pairs S364K-L368D, S364K-K370S, and F405K-K409F according to one embodiment, wherein x-axis accounts for molecular weights (kD).

FIG. 7 shows heterodimerization efficiency of the single mutations S364K-L368D, S364K-K370S, and F405K-K409F in comparison with conventional techniques (KiH, CPC, and AzS controls). When amino acid residues corresponding to each key are changed to other different amino acids, K is best for S364 and K and R (arginine) show almost the same effect for F405. Positions at which single mutations are made on A and B chains are indicated and heterodimerization quantity is numerically expressed.

FIG. 8 shows heterodimization efficiencies of a total of four double mutation pairs in comparison with conventional techniques (KiH, CPC, AzS controls), wherein the four double mutations are obtained by selecting two double mutation pairs from combinations of the three mutation pairs S364K-L368D, S364K-K370S, and F405K-K409F and mutating F405 into two types K and R for each pair.

FIGS. 9a to 9c are SDS-PAGE profiles after L368, K370, and K409 corresponding to lock mutations in double mutation pairs are substituted with other amino acids as indicated in Table 6 in order to identify better effects when amino acid residues corresponding to lock mutations are changed to other mutations.

FIG. 10 shows positions of electrostatic interaction-associated mutations, size-associated mutations, and swap-associated mutations in Fab of antibodies.

FIG. 11 is a schematic diagram of a competitive pairing (CPP) assay procedure.

FIG. 12 shows pairing modes established through the process of FIG. 11 between heavy and light chains and visualized on SDS-PAGE after 4D9 and 2B9 having conventional mutation pairs C1, DuetMab, and V23 between heavy and light chains were cloned and co-expressed.

FIG. 13 is an SDS-PAGE profile showing pairing modes when an electrostatic interaction-associated mutation between heavy and light chains is established by substituting K(lysine) for target amino acids on A chain (2B9 heavy chain) and D (aspartic acid) for target amino acids on B chain (4D9 heavy chain).

FIG. 14 shows pairing modes of the mutation at position 30 resulting from substituting heavy chain L145 and light chain V133 with K and D, respectively, which exhibit relatively high pairing accuracy among the list of mutation pairs as the 4D9 and 2B9 antibodies are compared on SDS-PAGE.

FIGS. 15 and 16 show pairing modes after mutation at position 29 S131D and/or S131K is introduced to the light chain of the antibody in which heavy chain L145 is substituted with E or D and light chain V133 is substituted with R, as analyzed by SDS-PAGE.

FIG. 17 shows pairing modes of mutation pairs at position 48 (heavy chain S183 and light chain V133 were substituted with K(R) and D(E), respectively), as analyzed by SDS-PAGE.

FIG. 18 shows pairing modes analyzed by SDS-PAGE after the mutation pair c29c30c48FΦ29f30f48 shown in Table 19 and variations thereof are introduced.

FIG. 19 shows pairing modes analyzed by SDS-PAGE after the mutation pairs of Table 20 are introduced.

FIG. 20 shows pairing modes analyzed by SDS-PAGE after the mutation pairs of Table 21 are introduced.

FIG. 21 shows paring ratios of chains after combinations of the mutation pairs at position 34 and 51 selected from among the heavy and light chain mutation pairs are introduced according to one embodiment.

FIG. 22 shows paring ratios of chains after c34Φf51 mutation pair selected from among the heavy and light chain mutation pairs is introduced according to one embodiment.

FIG. 23 shows paring ratios of chains after c40Φf44 mutation pair selected from among the heavy and light chain mutation pairs is introduced according to one embodiment.

FIG. 24 is a schematic diagram of a bispecific antibody in which the heavy and light chains are mutated according to one embodiment.

FIG. 25 is a graph showing thermal stability of A chain and B chain for the heavy chain and a chain and b chain for the light chain in antibodies constructed according to one embodiment.

FIG. 26 is a cleavage map of pcDNA3.

FIG. 27 is a graph showing hydrophobic interaction chromatography (HIC) results of the bispecific antibodies Trabev and Adabev constructed according to one embodiment, each having c'29c'30c48Φf'29f'30f'48 mutation pair and AWBB mutation pair introduced thereto.

FIG. 28 is a graph showing size exclusion chromatography (SEC) analysis results of the bispecific antibody Trabev, constructed according to one embodiment, having c'29c'30c48Φf'29f'30f'48 mutation pair and AWBB mutation pair introduced thereto.

FIG. 29 is a graph showing size exclusion chromatography (SEC) analysis results of the bispecific antibodies Trabev and Adabev constructed according to one embodiment, each having c'29c'30c48Φf'29f'30f'48 mutation pair and AWBB mutation pair introduced thereto.

FIG. 30 shows dimerization modes of the bispecific antibodies Trabev and Adabev constructed according to one embodiment, each having c'29c'30c48Φf'29f'30f'48 mutation pair and AWBB mutation pair introduced thereto.

FIG. 31 is a graph showing affinity for antigens (Her2 and VEGF) of the bispecific antibody Trabev, constructed according to one embodiment, having c'29c'30c48Φ29f'30f'48 mutation pair and AWBB mutation pair introduced thereto.

FIG. 32 is a graph showing affinity for antigens (TNF-alpha and VEGF) of the bispecific antibody Adabev, constructed according to one embodiment, having c'29c'30c48Φf'29f'30f'48 mutation pair and AWBB mutation pair introduced thereto.

FIG. 33a shows sequence alignment between the human IgG1 heavy chain constant region and the human IgAl heavy chain constant region (human IgG1 heavy chain constant region: SEQ ID NO: 33; human IgAl heavy chain constant region: SEQ ID NO: 34).

FIG. 33b shows sequence alignment between the kappa constant region and lambda constant region of human immunoglobulin light chain (kappa constant region of human immunoglobulin light chain: SEQ ID NO: 35; and lambda constant region of human immunoglobulin light chain: SEQ ID NO: 36).

FIGS. 33c and 33d show sequence alignment of heavy chain constant regions among human, mouse, and rat IgG subtypes (CH1 domain sequences in FIG. 33c and CH3 domain sequences in FIG. 33d) (Human IgG1: SEQ ID NO: 1, Human IgG2: SEQ ID NO: 2, Human IgG3: SEQ ID NO: 3, Human IgG4: SEQ ID NO: 4, Mouse IgG1: SEQ ID NO: 37, Mouse IgG2a$^{b}$: SEQ ID NO: 38, Mouse IgG2a$^{a}$: SEQ ID NO: 39, Mouse IgG2b: SEQ ID NO: 40, Mouse IgG3: SEQ ID NO: 41, Rat IgG1: SEQ ID NO: 42, Rat IgG2a: SEQ ID NO: 43, Rat IgG2b: SEQ ID NO: 44, Rat IgG2c: SEQ ID NO: 45 in FIG. 33c (CH1 domain); and Human IgG1: SEQ ID NO: 15, Human IgG2: SEQ ID NO: 16, Human IgG3: SEQ ID NO: 17, Human IgG4: SEQ ID NO: 18, Mouse IgG1: SEQ ID NO: 46, Mouse IgG2a$^{b}$: SEQ ID NO: 47, Mouse IgG2a$^{a}$: SEQ ID NO: 48, Mouse IgG2b: SEQ ID NO: 49, Mouse IgG3: SEQ ID NO: 50, Rat IgG1: SEQ ID NO: 51, Rat IgG2a: SEQ ID NO: 52, Rat IgG2b: SEQ ID NO: 53, and Rat IgG2c: SEQ ID NO: 54 in FIG. 33d (CH3 domain)).

FIG. 34 shows paring levels between light and heavy chains in antibodies, constructed according to one embodiment, having c34Φf51 mutation pair introduced thereto.

FIG. 35 is a graph showing a HIC result of the bispecific antibody Trabev, constructed according to one embodiment, having c34Φf51 mutation pair and AWBB mutation pair.

FIG. 36 shows graphs of HIC results of the bispecific antibody Adabev, constructed according to one embodiment, having c34Φf51 mutation pair and AWBB mutation pair.

FIG. 37 shows dimerization modes of the bispecific antibodies Trabev and Adabev, constructed according to one embodiment, each having c34Φf51 mutation pair and AWBB mutation pair introduced thereto.

FIG. 38 shows pairing levels between light and heavy chains in an antibody, constructed according to one embodiment, having c40Φf44 mutation pair introduced thereto.

FIG. 39 shows graphs of HIC results of the bispecific antibody Trabev, constructed according to one embodiment, having c40Φf44 mutation pair and AWBB mutation pair.

FIG. 40 shows graphs of HIC results of the bispecific antibody Adabev, constructed according to one embodiment, having c40Φf44 mutation pair and AWBB mutation pair.

FIG. 41 shows dimerization modes of the bispecific antibodies Trabev and Adabev, constructed according to one embodiment, each having c40Φf44 mutation pair and AWBB mutation pair introduced thereto.

FIG. 42 is an SDS-PAGE profile showing homodimerization levels upon single transfection of Enb-Fc and Fas-Fc, separately, according to a Comparative Example.

## Mode for Invention

Hereinafter, the present invention will be described in further detail with reference to examples. It is to be understood, however, that these examples are for illustrative purposes only and are not to be construed to limit the scope of the present invention.

For the practice of the present invention, all samples were prepared after the following procedure.

Protein Expression

1. A target gene was cloned to an expression vector (pcDNA3 (Invitrogen).
2. HEK293E cells (ATCC) were cultured in high-glucose DMEM (Dulbecco's modified Eagle's medium) supplemented with 5 % FBS (fetal bovine serum) in a humidified, $CO_2$ incubator.
3. A prepared plasmid DNA was introduced by transient transfection into HEK393E cells grown to full confluency. Before transfection, the cells were washed with PBS (phosphate-buffered saline) and, followed by exchanging the culture medium with serum-free high-glucose DMEM.
4. After incubation for one week, a conditioned medium was used to harvest the proteins which were then filtered. Fc-fusion proteins and antibodies were isolated by protein A chromatography.
5. Isolated proteins were quantitatively determined as analyzed at 280nm.

## EXAMPLE 1: Selection of Mutation for Heterodimerization of Two Fc Regions

Mutation positions for heterodimerization of two Fc regions (CH3 domain; SEQ ID NO: 15) (on the basis of IgG1) are depicted in FIG. 2. Section was made of 39 positions for electrostatic interaction-associated mutation (electrostatic interaction-introduced mutation) (represented by "Charge J"), 14 positions for swapping-associated mutation (swapping mutation) (represented by "Swap O"), and 40 positions for size-associated mutation (size mutation) (represented by "Size B"). Finally selected positions in CH3 domains and mutated amino acids thereat are summarized in Table 3. Each of the mutation pairs was applied two Fc regions (respectively represented by A chain and B chain) derived from different antibodies, followed by cloning for co-expression. For electrostatic interaction-associated mutation, mutations were carried out to substitute the corresponding amino acids on A chain with K (lysine) representative of amino acids having a positive charge and on B chain with D (aspartic acid) representative of amino acids having a negative charge. Swapping-associated mutation was carried out to exchange the corresponding amino acids on A chain and B chain with each other. For size-associated mutation, the corresponding amino acids were substituted with W (tryptophan) on A chain and with the small size amino acid A (alanine) on B chain.

TABLE 3

| Amino acid pair No. | Charge (J) | | Swap (O) | | Size (B) | |
|---|---|---|---|---|---|---|
| | Chain A | Chain B | Chain A | Chain B | Chain A | Chain B |
| 1 | Q347K | K360D | Q347K | K360Q | Q347W | K360A |
| 2 | Y349K | S354D | | | Y349W | S354A |

(continued)

| Amino acid pair No. | Charge (J) | | Swap (O) | | Size (B) | |
|---|---|---|---|---|---|---|
| | Chain A | Chain B | Chain A | Chain B | Chain A | Chain B |
| 3 | Y349K | E357 | | | Y349W | E357A |
| 4 | Y349K | K360D | | | Y349W | K360D |
| 5 | L351K | L351D | | | L351W | L351A |
| 6 | P352K | P352D | | | P352W | P352A |
| 7 | S354K | Y349D | S354Y | Y349S | S354W | Y349A |
| 8 | D356K | K439D | | | D356W | K439A |
| 9 | E357K | Y349D | E357Y | Y349E | E357W | Y349A |
| 10 | E357K | K370D | E357K | K370E | E357W | K370A |
| 11 | K360K | Q347D | | | K360W | Q347A |
| 12 | K360K | Y349D | K360Y | Y349K | K360W | Y349A |
| 13 | S364K | L368D | S364L | L368S | S364W | L368A |
| 14 | S364K | K370D | S364K | K370S | S364W | K370A |
| 15 | T366K | T366D | | | T366W | T366A |
| 16 | T366K | Y407D | | | T366W | Y407A |
| 17 | L368K | S364D | | | L368W | S364A |
| 18 | L368K | K409D | L368K | K409L | L368W | K409A |
| 19 | K370 | E357D | | | K370W | E357A |
| 20 | K370 | S364D | | | K370W | S364A |
| 21 | K370 | T411D | | | K370W | T411A |
| 22 | N390K | S400D | N390S | S400N | N390W | S400A |
| 23 | K392 | L398D | | | K392W | L398A |
| 24 | T394K | T394D | | | T394W | T394A |
| 25 | T394K | V397D | T394V | V397T | T394W | V397A |
| 26 | P395K | P395D | | | P395W | P395A |
| 27 | P395K | V397D | | | P395W | V397A |
| 28 | V397K | T394D | | | V397W | T394A |
| 29 | V397K | P395D | | | V397W | P395A |
| 30 | L398K | K392D | L398K | K392L | L398W | K392A |
| 31 | S400K | N390D | | | S400W | N390A |
| 32 | F405K | K409D | F405K | K409F | F405W | K409A |
| 33 | Y407K | T366D | Y407T | T366Y | Y407W | T366A |
| 34 | Y407K | Y407D | | | Y407W | Y407A |
| 35 | Y407K | K409D | | | Y407W | K409A |
| 36 | K409 | L368D | | | K409W | L368A |
| 37 | K409 | F405D | | | K409W | F405A |
| 38 | K409 | Y407D | | | K409W | Y407A |
| 39 | T411K | K370D | T411K | K370T | T411W | K370A |

(continued)

| Amino acid pair No. | Charge (J) | | Swap (O) | | Size (B) | |
|---|---|---|---|---|---|---|
| | Chain A | Chain B | Chain A | Chain B | Chain A | Chain B |
| 40 | | | | | K439W | D356A |

In order to easily identify homodimers or heterodimers of Fc-fusion proteins through SDS-PAGE, fusion was made of the ectodomain (coding sequence of region 1-771 of SEQ ID NO: 24) of TNF-alpha receptor (TNFRSF1B: NP_001057.1 (coding gene: CDS of NM_001066.2; SEQ ID NO: 24)) to Fc (coding gene: SEQ ID NO: 26) on one chain (chain A: Enbrel) and the ectodomain (coding sequence of region 1-519 of SEQ ID NO: 25) of Fas receptor (NP_000034.1 (coding gene: CDS of NM_000043.5; SEQ ID NO: 25)) to Fc (coding gene: SEQ ID NO: 26) on the other chain (chain B: Fas), using pcDNA3 vector (see FIG. 26; Invitrogen) as a backbone. The monomer of chain A has a size of 53 kD while the monomer of chain B has a size of about 44 kD. Because chains A and B, each having an Fc-ectodomain fusion, were different in size from each other, two homodimers (AA and BB) and one heterodimer (AB) could be easily discriminated on SDS-PAGE.

Percentages (%) of dimerization between chains A and between chains B (homodimerization) are expressed as $S_{AA}$ and $S_{BB}$, respectively whereas $S_{AB}$ accounts for percentages (%) of dimerization between chains A and B (heterodimerization).

When modes of dimerization were observed by SDS-PAGE, ratios (%) of homodimerization (AA and BB) and heterodimerization (AB) between chains A and B having the mutations of Table 3 introduced thereinto were compared with those between chains A and B that had not been mutated (represented by WT). Results are given Table 4 and FIG. 3 for the electrostatic interaction-induced mutation, Table 5 and FIG. 4 for the size mutation, and Table 6 and FIG. 5 for the swapping-associated mutation.

TABLE 4

| A | WT | **Y349K** | Y349K | S354K | E356K | **E357K** | **E357K** | **S364K** | **T366K** | **T394K** | **T394K** | T411K |
|---|---|---|---|---|---|---|---|---|---|---|---|---|
| B | WT | **E357D** | K360D | Y349D | K439D | **Y349D** | **K370D** | **L368D** | **Y407D** | **T394D** | **V397D** | K370D |
| $S_{AA}$ | 28 | **11** | 5 | 27 | 10 | **15** | **12** | **0** | **0** | **11** | **10** | 15 |
| $S_{AB}$ | 46 | **70** | 60 | 63 | 64 | **75** | **78** | **100** | **75** | **79** | **73** | 67 |
| $S_{BB}$ | 26 | **19** | 35 | 10 | 26 | **10** | **10** | **0** | **25** | **10** | **17** | 18 |

TABLE 5

| A | **E357Y** | **E357K** | S364L | **S364K** | **F405K** | **Y407T** | T411K |
|---|---|---|---|---|---|---|---|
| B | **Y349E** | **K370E** | L368S | **K370S** | **K409F** | **T366Y** | K370T |
| $S_{AA}$ | **8** | **4** | 8 | **0** | **0** | **10** | 13 |
| $S_{AB}$ | **71** | **80** | 61 | **90** | **70** | **83** | 60 |
| $S_{BB}$ | **21** | **16** | 31 | **10** | **30** | **7** | 27 |

TABLE 6

| A | WT | K409W | **K409W** |
|---|---|---|---|
| B | WT | F405A | **Y407A** |
| $S_{AA}$ | 22 | 3 | **2** |
| $S_{AB}$ | 56 | 60 | **85** |
| $S_{BB}$ | 23 | 37 | **13** |

In Tables 4 to 6, mutations accounting for a heterodimerization rate (%) of 70 % or higher are expressed in bold. As is understood from data of Tables 4 to 6, the tested mutation pairs exhibited a heterodimerization rate of 60 % or higher.

Through the results, selection was made of 12 mutation pairs that gave higher rates to heterodimerization than homodimerization, with the heterodimerization rate being 70 % or higher (electrostatic interaction-introduced mutation: Y349K-E357D, E357K-Y349D, E357K-K370D, S364K-L368D, T366K-Y407D, T394K-T394D, T394K-V397D; and swapping mutation: E357Y-Y349E, E357K-K370E, S364K-K370S, F405K-K409F, Y407T-T366Y) (expressed in bold in Tables 4 and 5).

Each of the 12 amino acid residue mutations contained in the selected 12 amino acid pairs was introduced into the Fas-Fc fusion protein to express mutant Fas-Fc fusion proteins that contained single mutations at the amino acid positions, respectively. In the same condition, homodimer and monomer survival rates ($S_{sm}$; 1=100%) were compared, and the results are depicted in Table 7 and FIG. 6a.

TABLE 7

| **SM** | $S_{SM}$ |
|---|---|
| E357Y | 0.03 |
| Y349D | 0.03 |
| K370D | 0.06 |
| L368D | 0.06 |
| **S364K** | **0.77** |
| K370S | 0.05 |
| S400N | 0.05 |
| T394D | 0.11 |
| **F405K** | **0.67** |
| K409F | 0.05 |
| T366A | 0.09 |
| T366Y | 0.46 |

In Table 7 and FIG. 6a, two mutations S364K and F405K, which were low in homodimerization rate but high in monomer survival rate (50 % or higher), were selected.

It was postulated that the two selected mutations acted as "key" mutations while the CH3 domain sites interacting therewith on the other chain were "lock" mutations. As combinations therebetween, three key-lock pairs S364K-L368D, S364K-K370S, and F405K-K409F were selected. These mutation pairs were introduced into chain A (key mutation) and chain B (lock mutation) to make three different single mutation pairs (see TABLE 8).

TABLE 8

| Mutation type | Sample | Key Single Mutation | Lock Single Mutation |
|---|---|---|---|
| Introduction of electrostatic interaction | J13 | S364K | L368D |
| Swapping | O14 | S364K | K370S |
| Swapping | O32 | F405K | K409F |

The amino acids at respective key mutation positions were substituted with different amino acid residues and tested for the single mutation-induced heterodimerization effect, as described above, so as to identify mutation types effective for heterodimerization at the positions. For S364, substitution with K (lysine) was observed to bring about the highest heterodimerization effect. An outstanding effect was detected upon substitution with N (asparagine) and R (arginine) (see FIG. 6b). F405 exhibited excellent similar heterodimerization effects upon substitution with K and R and outstanding heterodimerization effects upon substitution with N and Q (glutamine) (see FIG. 6c).

The additional mutations S364N, S364R, F405R, F405N, and F405Q, which were identified in FIGS. 6b and 6c, were applied to the three selected key-lock mutation pairs S364K-L368D, S364K-K370S, and F405K-K409F, to introduce the lock mutations to chain A (TNFR2-Fc) and the key mutations to chain B (Fas-Fc), followed by analyzing heterodimerization effects on SDS-PAGE.

The results thus obtained are depicted in Table 9 and FIG. 6d:

TABLE 9

| Sample | A (TNFR2) | B (Fas) | $S_{AA}$ | $S_{AB}$ | $S_{BB}$ |
|---|---|---|---|---|---|
| | Key Single Mutation | Lock Single Mutation | | | |
| J13(K:D) | S364K | L368D | 8 | 92 | 0 |
| J13(N:D) | S364N | L368D | 8 | 82 | 9 |
| J13(R:D) | S364R | L368D | 20 | 79 | 1 |
| O14(K:S) | S364K | K370S | 25 | 65 | 9 |
| O14(N:S) | S364N | K370S | 13 | 84 | 3 |
| O14(R:S) | S364R | K370S | 23 | 76 | 1 |
| O32(K:F) | F405K | K409F | 0 | 75 | 25 |
| O32(N:F) | F405N | K409F | 21 | 62 | 17 |
| O32(Q:F) | F405Q | K409F | 5 | 68 | 27 |
| O32(R:F) | F405R | K409F | 5 | 89 | 6 |
| ($S_{AA}$: AA homodimerization rate (%); $S_{AB}$: AB heterodimerization rate (%); $S_{BB}$: BB homodimerization rate (%)) | | | | | |

**EXAMPLE 2: Test for Heterodimerization of Fc Region by Single Mutation**

The three key-lock mutation pairs S364K-L368D, S364K-K370S, and F405K-K409F, which were selected in Example 1, and the mutation pair F405R-K409F, which resulted from substituting F405 with R instead of K, were tested for heterodimerization on SDS-PAGE. The heterodimerization effects were compared with those obtained with the conventional heterodimer Fc mutation pairs KiH, CPC, and AzS used as controls.

SDS-PAGE data in this Example and all the following Examples were obtained by quantitating the band intensities with the aid of GelQuant.NET Software.

The results are depicted in Table 10 and FIG. 7:

TABLE 10

| **Sample** | **Chain A (eTNFR2)** | **Chain B (eFas)** | $S_{AB}$ | $S_A$ | $S_B$ | $S_M$ | $T_m$ |
|---|---|---|---|---|---|---|---|
| J13 | S364K | L368D | 0.93 | 0.86 | 0.10 | 0.48 | 66.8 |
| O14 | S364K | K370S | 0.87 | 0.86 | 0.07 | 0.47 | 67.6 |
| O32 | F405K | K409F | 0.72 | 0.91 | 0.08 | 0.49 | 65.2 |
| O32' | F405R | K409F | 0.72 | 0.91 | 0.08 | 0.49 | 65.2 |
| KiH | T366S/L368A/Y407V | T366W | 0.90 | 0.68 | 0.64 | 0.66 | 67.4 |
| CPC | K392D/K409D | E356K/D399K | 0.74 | 0.85 | 0.30 | 0.58 | 66.4 |
| AzS | T350V/T366L/K392L/T394W | T350V/L351Y/F 405A/Y407V | 0.84 | 0.84 | 0.64 | 0.74 | 69.2 |
| ($S_{AB}$: AB heterodimerization rate (%); $S_A$; AA homodimerization rate (%); $S_B$: BB homodimerization rate (%); $S_M$: monomer survival rate (%)) | | | | | | | |

Tm was measured as follows:

Reagent: Invitrogen 4461146 "Protein Thermal Shift™" Dye Kit

Instrument: Chromo4-PTC200 (MJ Research)

Reaction mixture: 20 $\mu\ell$ in total

| | |
|---|---|
| Protein | 10μl |
| DW | 3 μl |
| Protein Thermal Shift™ Buffer | 5 μl |
| 1/100 diluted Protein Thermal Shift™ Dye | 2 μl |

Protocol:

1. Incubate at 50.0°Cfor 30sec;

2. Melting Curve from 50.0°Cto 90.0°C read every 0.2°C hold for 2 sec;

3. Incubate at 90.0°Cfor 2 min

4. Incubate at 10.0 forever

5. End

As shown in Table 10 and FIG. 7, when chain A and chain B were each separately expressed, chain A retaining the key did not form a homodimer, but is expressed as a monomer. A sample resulting from co-expression was observed to contain no homodimers, but mostly heterodimers (see FIG. 7).

**EXAMPLE 3: Heterodimerization of Fc Region by Double Mutation**

Less plausibility of homodimerization might result from existence of the key mutations on both of chains A and B than on either of the chains. Two double mutation pairs were made from combinations of the three mutation pairs selected in Example 1. For each double mutation pair, F405 was mutated into two types K and R. Thus, a total of four double mutation pairs were obtained. These found double mutations pairs were analyzed for heterodimerization on SDS-PAGE and the dimerization effects were compared with those of the controls KiH, CPC, and AzS.

The results are given in Table 11 and FIG. 8:

TABLE 11

| DMP | Chain A (eTNFR2) | Chain B (eFas) | $S_{AB}$ | $S_A$ | $S_B$ | $S_M$ | $T_m$ |
|---|---|---|---|---|---|---|---|
| J13/O32 | **S364K**/K409F | L368D/**F405K** | 0.96 | 1.00 | 0.74 | 0.87 | 58.2 |
| J13/O32' | **S364K**/K409F | L368D/**F405R** | 0.95 | 1.00 | 0.73 | 0.87 | 61.5 |
| O14PO32 | **S364K**/K409F | K370S/**F405K** | 0.95 | 1.00 | 0.72 | 0.86 | 64.1 |
| O14/O32' | **S364K**/K409F | K370S/**F405R** | 0.93 | 1.00 | 0.71 | 0.86 | 64.4 |
| KiH | T366S/L368A/Y407V | T366W | 0.91 | 0.64 | 0.61 | 0.63 | 67.4 |
| CPC | K392D/K409D | E356K/D399K | 0.73 | 0.81 | 0.30 | 0.58 | 66.4 |
| AzS | T350V/T366L/K392L/T394W | T350V/L351Y/F405A/Y407V | 0.84 | 0.82 | 0.62 | 0.72 | 69.2 |

As shown in Table 11 and FIG. 8, the key mutations present in both chains allowed the homodimerization of the chains for none of the four mutation pairs upon separate expression. All of the proteins in a sample resulting from co-expression were observed to be heterodimers (See FIG. 8). In addition, higher thermal stability was measured in a double-mutation pair having F405R introduced thereto than F405K introduced thereto (Table 11).

In order to examine whether a better effect was obtained when amino acids corresponding to lock mutation were substituted with other residues, L368, K370, and K409, which were the lock in the double mutation pairs, were substituted with other amino acid residues. Mutation combinations obtained by variously mutating L368, K370, and K409 are summarized in Table 12. The mutation combinations were tested for heterodimerization on SDS-PAGE (NR: 8% SDS-PAGE gel; Sample: 24ul Loading), and the results are depicted in FIGS. 9a to 9c.

TABLE 12

| Lock variants | | | | | | | | |
|---|---|---|---|---|---|---|---|---|
| **X** | **Chain A** | **Chain B** | **Y** | **Chain A** | **Chain B** | **Z** | **Chain A** | **Chain B** |
| UA | **S364K**/K409F | L368**A**/**F405R** | XA | **S364K**/K409F | K370**A**/**F405R** | ZA | **S364K**/K409**A** | K370S/**F405R** |
| UC | **S364K**/K409F | L368**C**/**F405R** | XC | **S364K**/K409F | K370**C**/**F405R** | ZC | **S364K**/K409C | K370S/**F405R** |
| UD | **S364K**/K409F | L368**D**/**F405R** | XD | **S364K**/K409F | K370**D**/**F405R** | ZD | **S364K**/K409**D** | K370S/**F405R** |
| UE | **S364K**/K409F | L368**E**/**F405R** | XE | **S364K**/K409F | K370**E**/**F405R** | ZE | **S364K**/K409**E** | K370S/**F405 R** |
| UF | **S364K**/K409F | L368**F**/**F405R** | XF | **S364K**/K409F | K370**F**/**F405R** | ZF | **S364K**/K409**F** | K370S/**F405R** |
| UG | **S364K**/K409F | L368**G**/**F405R** | XG | **S364K**/K409F | K370**G**/**F405R** | ZG | **S364K**/K409**G** | K370S/**F405R** |
| UH | **S364K**/K409F | L368**H**/**F405R** | XH | **S364K**/K409F | K370**H**/**F405R** | ZH | **S364K**/K409**H** | K370S/**F405R** |
| UI | **S364K**/K409F | L368**I**/**F 405R** | XI | **S364K**/K409F | K370**I**/**F405R** | ZI | **S364K**/K409**I** | K370S/**F405R** |
| UK | **S364K**/K409F | L368**K**/**F405R** | XK | **S364K**/K409F | K370/**F405R** | ZK | **S364K**/K409**K** | K370S/**F405R** |
| UL | **S364K**/K409F | L368/**F405R** | XL | **S364K**/K409F | K370**L**/**F405R** | ZL | **S364K**/K409**L** | K370S/**F405R** |
| UM | **S364K**/K409F | L368**M**/**F405R** | XM | **S364K**/K409F | K370**M**/**F405R** | ZM | **S364K**/K409**M** | K370S/**F405R** |
| UN | **S364K**/K409F | L368N/ **F405R** | XN | **S364K**/K409F | K370**N**/**F405R** | ZN | **S364K**/K409**N** | K370S/**F405R** |
| UQ | **S364K**/K409F | L368**Q**/**F405R** | XQ | **S364K**/K409F | K370**Q**/**F405R** | ZQ | **S364K**/K409**Q** | K370S/**F405R** |
| UR | **S364K**/K409F | L368**R**/**F405R** | XR | **S364K**/K409F | K370**R**/**F405R** | ZR | **S364K**/K409**R** | K370S/**F405R** |
| US | **S364K**/K409F | L368**S**/**F 405R** | XS | **S364K**/K409F | K370**S**/**F405R** | ZS | **S364K**/K409**S** | K370S/**F405R** |
| UT | **S364K**/K409F | L368**T**/**F405R** | XT | **S364K**/K409F | K370**T**/**F405R** | ZT | **S364K**/K409**T** | K370S/**F405R** |
| UV | **S364K**/K409F | L368V/**F405R** | XV | **S364K**/K409F | K370**V**/**F405R** | ZV | **S364K**/K409**V** | K370S/**F405R** |
| UW | **S364K**/K409F | L368W/**F405R** | XW | **S364K**/K409F | K370**W**/**F405R** | ZW | **S364K**/K409**W** | K370S/**F405R** |
| UY | **S364K**/K409F | L368**Y**/**F405R** | XY | **S364K**/K409F | K370**Y**/**F405R** | ZY | **S364K**/K409**Y** | K370S/**F405R** |

The combinations were measured for thermal stability (see Example 2) and the results are given in Table 13.

TABLE 13

| | Chain A | Chain B | Tm |
|---|---|---|---|
| UC | S364K/K409F | L368C/F405R | 63.8 |
| UD | S364K/K409F | L368D/F405R | 60.8 |
| UL | S364K/K409F | L368/F405R | 63.0 |
| UW | S364K/K409F | L368W/F405R | 61.2 |
| UY | S364K/K409F | L368Y/F405R | 61.2 |
| **ZF** | **S364K/K409F** | **K370S/F405R** | **65.6** |
| ZH | S364K/K409H | K370S/F405R | 64.4 |
| ZI | S364K/K409I | K370S/F405R | 62.4 |
| ZN | S364K/K409N | K370S/F405R | 61.0 |
| ZR | S364K/K409R | K370S/F405R | 62.8 |
| ZT | S364K/K409T | K370S/F405R | 65.4 |
| ZV | S364K/K409V | K370S/F405R | 66.0 |
| **ZW** | **S364K/K409W** | **K370S/F405R** | **67.0** |
| ZY | S364K/K409Y | K370S/F405R | 63.8 |

Analysis showed higher thermal stability of K409W (tryptophan) than K409F.

Based on the data obtained above, mutations were made of S364K and K409W into chain A and K370S and F405R into chain B to produce a double mutation pair, termed AWBB mutation pair, for use in the following Fc heterodimerization test of CH3 domain.

**EXAMPLE 4: Selection of Mutation for Heavy Chain and Light Chain in Antibody Fab**

To select mutations in heavy and light chains of antibodies, electrostatic interaction-associated mutations, size-associated mutations, and swapping-associated mutations were carried out. Positions of interaction at heavy and light chains are depicted in FIG. 10.

In order to easily identify mutations in heavy and light chains, antibodies having the same light chain were cloned. 4D9 antibody (anti-Influenza A antibody) and 2B9 antibody (anti-Influenza B antibody), which are both anti-influenza antibodies, have the same light chain (common light chain: CLC). Because they have the same light chain, SDS-PAGE analysis allows interaction between heavy and light chains to be easily understood. Although identical in the amino acid sequence of light chain, the two antibodies 4D9 and 2B9 are different from each other with respect to the sequence and size of the heavy chain (the heavy chain of 2B9 has more amino acid residues by six than that of 4D9 and a size (50130.62 Daltons) greater than that of 4D9 (49499.98 Daltons): they can be clearly discriminated on SDS-PAGE). Thus, size analysis on SDS-PAGE makes it possible to understand which of the two chains interacts with the light chain.

Amino acid sequences and coding nucleic acid sequences thereof in the heavy chain variable regions and light chain variable regions of the two antibodies 4D9 and 2B9 are listed in Table 14, below.

TABLE 14

| | Amino Acid Sequence | Nucleic Acid Sequence |
|---|---|---|
| 2B9 Heay Chain Variable Region | EVQLVESGGGLVQPGKSLRLSC<br>AATGFTFDDYAMHWVRQAPG<br>KGLEWVSSLNWKGNSVDYAD<br>SVRGRFTMSRDNAKKLVYLQM<br>NGLRGDDTAVYFCAKDNKAD | GAAGTGCAGCTGGTGGAGTCTGGGGGAG<br>GCTTGGTACAGCCTGGCAAGTCCCTGAG<br>ACTCTCCTGTGCAGCCACTGGATTCACAT<br>TTGACGATTACGCCATGCACTGGGTCCGC<br>CAAGCTCCAGGGAAGGGCCTGGAGTGGG |

(continued)

| | Amino Acid Sequence | Nucleic Acid Sequence |
|---|---|---|
| | ASMDYYYHHGMDVWGQGTT<br>VTVSS (SEQ ID NO: 27) | TCTCAAGTCTTAATTGGAAGGGAAATAGT<br>GTAGACTACGCGGACTCTGTGAGGGGCC<br>GATTCACCATGTCCAGAGACAACGCCAA<br>GAAACTAGTGTATCTGCAAATGAACGGT<br>CTGAGAGGTGACGACACGGCCGTCTATTT<br>TTGTGCAAAAGATAATAAAGCGGATGCA<br>TCTATGGACTACTACTACCACCACGGTAT<br>GGACGTCTGGGGCCAAGGGACCACGGTC<br>ACCGTCTCCTCG (SEQ ID NO: 28) |
| 4D9 Heay Chain Variable Region | QVTLRESGPGLVKPSETLSLTCT<br>ISGASINTDYWSWIRQPPGKGLE<br>WIGYIYYRGRTNYNPSLRSRVTI<br>SVDTSKNQFSL<br>QMTSMTAADTAVYYCARDVT<br>GISRENAFDIWGQGTLVTVSS<br>(SEQ ID NO: 29) | CAGGTCACCTTGAGGGAGTCGGGCCCAG<br>GACTGGTGAAGCCTTCGGAGACCCTGTCC<br>CTCACCTGCACTATCTCCGGTGCCTCCAT<br>CAATACTGACTACTGGAGCTGGATCCGG<br>CAGCCCCCAGGGAAGGGACTGGAGTGGA<br>TTGGCTATATCTATTACAGAGGGCGCACC<br>AACTACAACCCCTCCCTCAGGAGCCGAG<br>TCACCATATCAGTAGACACGTCCAAGAA<br>TCAATTCTCCCTG<br>CAGATGACGTCTATGACCGCTGCTGACAC<br>GGCCGTATATTACTGTGCGAGAGATGTG<br>ACTGGCATCAGTCGAGAAAATGCTTTTGA<br>TATCTGGGGCCAAGGCACCCTGGTCACC<br>GTCTCCTCG (SEQ ID NO: 30) |
| Light Chain (CLC) Varuable Region | AIRMTQSPSSLSASVGDRVTITC<br>RASQSISGYLNWYQQKPGKAP<br>KLLIYAASSLQSGVPSRFSGSGS<br>GTDFTLTISSLQPEDFATYYCQQ<br>SYSIPTTFGQGTRLEIK (SEQ ID<br>NO: 31) | GCCATCCGGATGACCCAGTCTCCATCCTC<br>CCTGTCTGCATCTGTAGGAGACAGAGTCA<br>CCATCACTTGCCGGGCAAGTCAGAGCATT<br>AGCGGCTATTTAAATTGGTATCAGCAGA<br>AACCAGGGAAAGCCCCTAAGCTCTTGAT<br>CTATGCTGCATCCAGTTTGCAGAGTGGGG<br>TCCCATCAAGGTTCAGTGGCAGTGGATCT<br>GGGACAGATTTCACTCTCACCATCAGCAG<br>TCTGCAACCTGAAGATTTTGCAACTTACT<br>ACTGTCAACAGAGCTACAGTATCCCCACC<br>ACCTTCGGCCAAGGGACACGACTGGAGA<br>TTAAA (SEQ ID NO: 32) |

The two antibodies 4D9 and 2B9 employ the constant region of IgG1 as a heavy chain constant region and the kappa constant region as a light chain constant region.

Examination was made to see whether a light chain having a mutation introduced thereinto pairs with only a heavy chain having a mutation interacting with the mutation of the light chain. In this regard, a light chain, a heavy chain pairing with the light chain, and a heavy chain forming a mispair with the light chain were co-expressed to afford antibodies. SDS-PAGE analysis of the antibodies in a reducing condition can identify the extent to which the pairings are accurately formed. For convenience, 4D9 heavy chain is referred to as A chain and a light chain pairing therewith as a chain, and 2B9 heavy chain is referred to as B chain and a light chain pairing therewith as b chain (see the following illustrations).

| | | | |
|---|---|---|---|
| A heavy(+) | 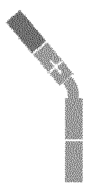 | B(-) | 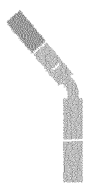 |

(continued)

| | | | | | |
|---|---|---|---|---|---|
| a light(-) | 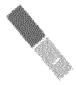 | | b(+) | 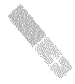 | |
| B heavy(-) | 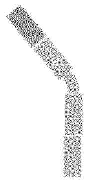 | | A(+) | 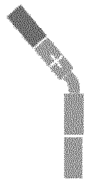 | |
| Product | 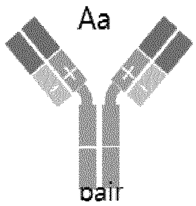 | 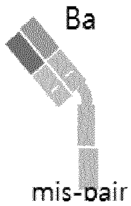 | Product | 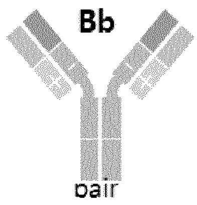 | 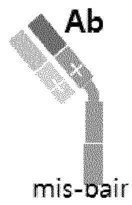 |

First, 4D9 and 2B9 having conventional mutation pairs between heavy and light chains, already known to be effective, were cloned and co-expressed. Pairing modes established through competition between the resulting mutant chains, that is, two heavy chains and one light chain were examined on SDS-PAGE. (Competitive Pairing (CPP) Assay). The process of performing a competitive pairing (CPP) assay is schematically illustrated in FIG. 11, and the result thus obtained is depicted in FIG. 12. As shown in FIG. 12, all the already known mutation pairs undergo normal pairing and abnormal mispairing, concurrently.

For electrostatic interaction-associated mutations between heavy and light chains, antibodies in which various mutation pairs were introduced with the substitution of corresponding amino acids in B chain (2B9 heavy chain) with K (lysine) and in A chain (4D9 heavy chain) with D (aspartic acid) were subjected to CPP assay on SDS-PAGE (see FIG. 11).

As a result, seven candidate mutation pairs that were identified to undergo relatively accurate pairing were screened in the electrostatic interaction-associated mutation group. CPP assay results on SDS-PAGE of the antibodies into which the seven screened mutation pairs were introduced are given in Table 15 and depicted in FIG. 13.

TABLE 15

| No | SOP (Symmetric Orthogonal Pairs) | A (4D9) | B (2B9) | a | b | $a^{CPP}$ | $b^{CPP}$ | $S^{CPP}$ |
|---|---|---|---|---|---|---|---|---|
| c | cΦf | L145 | L145 | S131 | S131 | | | |
| 1 | c29Φf29 | L145D | L145K | S131K | S131D | 58 | 50 | 54 |
| 2 | c30Φf30 | L145D | L145K | V133K | V133D | 50 | 79 | 65 |
| 3 | c34Φf34 | K147D | K147K | T180K | T180D | 49 | 87 | 68 |
| 4 | c40Φf40 | F170D | F170K | L135K | L135D | 59 | 61 | 60 |
| 5 | c44Φf44 | P171D | P171K | S162K | S162D | 59 | 51 | 55 |
| 6 | c48Φf48 | S183D | S183K | V133K | V133D | 62 | 80 | 72 |
| 7 | c51Φf51 | V185D | V185K | L135K | L135D | 67 | 73 | 70 |

$$\text{(CPP Score } (S^{CPP}) = \tfrac{1}{2}(a^{CPP} + b^{CPP})$$

$$a^{CPP} = 100(C_{aA})/(C_{aA}+C_{aB}) = 100(C_A)/(C_A+C_B)$$

$$b^{CPP} = 100(C_{bB})/(C_{bB}+C_{bA}) = 100(C_B)/(C_A+C_B))$$

Comparison on SDS-PAGE found some mutation pairs that underwent relatively accurate pairing in the list of mutation pairs using the 4D9 (A chain) and 2B9 (B chain) antibodies (expressed in bold in Table 15). For further study, mutation at position 30 (expressed as c30Φf30) was modified as in Table 16.

TABLE 16

| Mutation c30Φf30 | | | |
|---|---|---|---|
| | Heavy Chain | Light Chain | Paring Accuracy |
| Aa (4D9) | L145D | V133R | 75% |
| Bb (2B9) | L145R | V133D | 60% |

The mutation at position 30 resulted from substitution heavy chain L145 and light chain V133 with K and D, respectively. As is understood from the data of Table 16, the effect (pairing accuracy: Aa pairing or Bb pairing ratio) was observed to be good (Aa pairing accuracy 75%, Bb pairing accuracy: 60%) for the variations in which heavy chain L145 and light chain V133 were substituted with R and D, respectively (see FIG. 14).

Addition of mutation S131D on the light chain (termed mutation at position 29) to the mutation at position 30 or variations thereof (heavy chain L145 and light chain V133 were substituted with K or R, and D or E, respectively) improved the accuracy of pairing (see Table 17 and FIG. 15 (c29c$^{R}$30Φf$^{R}$30 result; Aa pairing accuracy: 80% and Bb pairing accuracy: 70%) and FIG. 16 (c$^{R}$29c$^{RE}$30Φf$^{R}$29f$^{RE}$30 result; Aa pairing accuracy: 90% and Bb pairing accuracy: 80%)).

TABLE 17

| | Heavy Chain | Light Chain | Paring Accuracy |
|---|---|---|---|
| **c29c$^{R}$30Φf$^{R}$30** | | | |
| Aa (4D9) | L145D | V133R | 80% |
| Bb (2B9) | L145R | S131D/V133D | 70% |
| **c$^{R}$29c$^{RE}$30Φf$^{R}$29f$^{RE}$30** | | | |
| Aa (4D9) | L145E | S131K/V133R | 90% |
| Bb (2B9) | L145R | S131D/V133E | 80% |

As can be seen in FIGS. 14 and 15, outstanding pairing accuracy was detected in the cases where heavy chain L145 and light chain V133 were substituted with R and D, respectively and where substitution of light chains S131 and V133 were respectively made by K and R, or D and E as well as in mutation at position 30 wherein heavy chain L145 and light chain V133 were substituted with K and D, respectively.

In addition, a mutation pair in which heavy chain S183 and light chain V133 were respectively substituted with K (or R) and D (or E) (termed mutation at position 48) was also observed to be effective (Table 18 and FIG. 17 (Aa pairing accuracy (low bands): 45%, Bb **pairing accuracy** (upper bands): 95%).

TABLE 18

| LC | V133K (a) | V133D (b) |
|---|---|---|
| 2B9 Heavy Chain (B) | S183K | |
| 4D9 Heavy Chain (A) | S183D | |
| mg/L | 2.6 | 2.4 |

Antibodies containing a combination of the aforementioned mutation at position 29, mutation at position 30, and mutation at position 48 (c29c30c48FΦ29f30f48) or variations thereof were constructed (see Table 19) and then tested for pairing accuracy. The results are depicted in FIG. 18 (lower bands: Aa pairing; and upper bands: Bb paring):

TABLE 19

| | Heavy Chain | Light Chain | Paring Accuracy |
|---|---|---|---|
| **c$^{R}$29c$^{RE}$30c48Φf$^{R}$29f$^{RE}$30f48** | | | |
| Aa (4D9) | L145E/S183D | S131K/V133R | 100% |

(continued)

| | Heavy Chain | Light Chain | Paring Accuracy |
|---|---|---|---|
| **$c^{R}29c^{RE}30c48\Phi f^{R}29f^{RE}30f48$** | | | |
| Bb (2B9) | L145R/S183K | S131D/V133E | 90% |
| **Mutation 1** | | | |
| Aa (4D9) | L145D/S183D | S131K/V133K | 85% |
| Bb (2B9) | L145K/S183K | S131D/V133D | 90% |
| **Mutation 2** | | | |
| Aa (4D9) | L145D/S183D | S131D/V133K | 95% |
| Bb (2B9) | L145K/S183K | S131K/V133D | 95% |

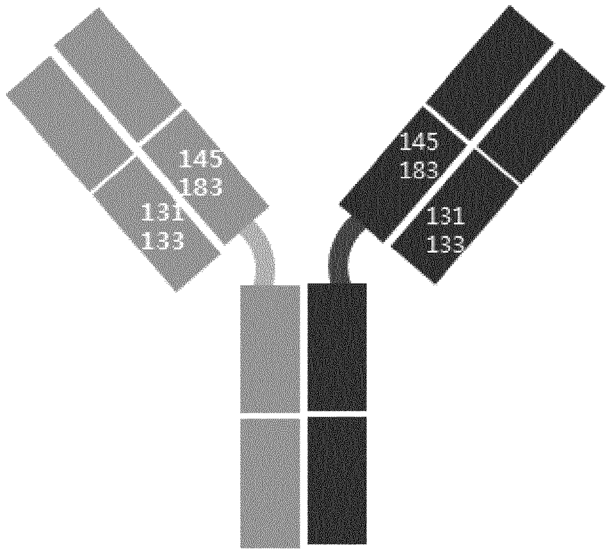


In addition, antibodies containing a variant mutation pair of at least one of mutation at position 29, mutation at position 30, and mutation at position 48 (see Table 20) were examined for pairing accuracy and the results are depicted in FIG. 19 (lower bands: Aa pairing; and upper bands: Bb paring):

TABLE 20

| No. | 1 | | 2 | | 3 | | 4 | | 5 | |
|---|---|---|---|---|---|---|---|---|---|---|
| **C** | **c30Φf30** | | **$c^R$30Φ$f^R$30** | | **c9$c^R$30Φ$f^R$30** | | **$c^R$29$c^{RE}$30Φ$f^R$29$f^{RE}$30** | | **$c^R$29$c^{RE}$30f48Φ$f^R$29$f^{RE}$30f48** | |
| LC | V133K (a) | V133D (b) | V133R | V133D | V133R | S131D/V133D | S131K/V133R | S131D/V133E | S131K/V133R | S131D/V133E |
| 4D9 (A) | L145D | | L145D | | L145D | | L145E | | L145E/S183D | |
| 2B9 (B) | L145K | | L145R | | L145R | | L145R | | L145R/S183K | |
| Aa | 79% | | 67% | | 80% | | 90% | | 100% | |
| Bb | 50% | | 62% | | 70% | | 80% | | 90% | |
| (Aa: Aa pairing accuracy; Bb: Bb pairing accuracy) | | | | | | | | | | |

Further, combinations of mutation at position 29, mutation at position 30, and mutation at position 48 were subjected to swapping between D and E to search for combinations that allowed for relative accurate pairing (Table 21 and FIG. 20).

TABLE 21

| Code | A | B | a | b | Aa | Bb |
|---|---|---|---|---|---|---|
| 0 | L145K/S183K | L145D/S183D | S131D/V133D | S131K/V133K | 65% | 100% |
| ab | L145K/S183K | L145E/S183E | S131D/V133D | S131K/V133K | 95% | 95% |
| gh | L145K/S183K | L145D/S183D | S131E/V133E | S131K/V133K | 100% | 100% |
| abgh | L145K/S183K | L145E/S183E | S131E/V133E | S131K/V133K | 100% | 100% |
| (Aa: Aa pairing accuracy; Bb: Bb pairing accuracy) | | | | | | |

As can be seen in Table 21 and FIG. 20, all the tested mutation pairs ab, gh, and abgh exhibited a pairing accuracy of 65% or higher, or 95% or higher. Of them, abgh was selected and termed c'29c'30c48Φf'29f'30f'48 mutation pair.

Chains into which combinations of the mutation pairs at position 34 and 51 selected from among the mutation pairs identified in Table 15 were introduced were tested for pairing and the results are given in Table 22 and FIG. 21.

TABLE 22

| LC | L135K/T180K (a) | L135D/T180D (b) |
|---|---|---|
| 4D9 Heavy Chain (A) | K147D/V185D | |
| 2B9 Heavy Chain (B) | K147/V185K | |
| Aa (%) | 95% | |
| Bb (%) | 62% | |

In addition, mutation at position 34 and mutation at position 51 of Table 15 were subjected to change from K to R and from D to E, followed by a pairing test to detect combinations having improved pairing accuracy. As a result, a combination in which L135 and T180 on the light chain were each substituted with E was improved in pairing accuracy and termed c34Φf51 mutation pair (see Table 23 and FIG. 22).

TABLE 23

| LC | L135K/T180K (a) | L135E/T180E (b) |
|---|---|---|
| 4D9 Heavy Chain (A) | K147D/V185D | |
| 2B9 Heavy Chain (B) | K147/V185K | |
| Aa(%) | 95% | |
| Bb(%) | 86% | |

Of various mutation pairs found in Table 15, a combination of mutation at position 40 and mutation at position 44 was also obverted to improve in pairing accuracy.

In addition, all the mutation pairs were subjected to swapping between K and R and between D and E to search for a combination that allowed for the most accurate pairing. The combination was a mutation pair in which light chain L135 was substituted with R and E, and was termed c40Φf44 mutation pair (see Table 24 and FIG. 23):

TABLE 24

| LC | L135R/S 162K (a) | L135E/S162D (b) |
|---|---|---|
| 4D9 Heavy Chain (A) | F170D/P171D | |
| 2B9 Heavy Chain (B) | F170K/P171K | |

(continued)

| LC | L135R/S 162K (a) | L135E/S162D (b) |
|---|---|---|
| Aa(%) | 95% | |
| Bb(%) | 84% | |

**EXAMPLE 5: Bispecific Antibody Formation by Coupling between Heavy Chains and between Heavy Chain and Light Chain**

**5.1. Bispecific antibody having c'29c'30c48Φf'29f'30f'48 mutation pair introduced thereto**

**5.1.1. Test of coupling between heavy and light chain using 4D9/2B9 antibody**

Antibodies were constructed by coupling heavy and light chains containing the c'29c'30c48Φf'29f'30f'48 mutation pair (see Table 25):

TABLE 25

| Antibody | Heavy Chain Constant Region (HC: CH1) | Light Chain Constant Region (LC) |
|---|---|---|
| 4D9 (Aa) | L145E/S183E | S131K/V133K |
| 2B9 (Bb) | L145K/S183K | S131E/V133E |

In order to examine the accuracy of pairing among A chain, B chain, a chain, and b chain in the bispecific antibodies, individual heavy chains and light chains were co-expressed in all possible combinations of normal pairs and abnormal mispairs thereamong. Expression levels measured by SDS-PAGE are given in Table 26, below. Thermal stability (Tm) of the combinations was measured and the results are given in Table 27 and FIG. 25:

TABLE 26

| HC | **A** | | B | |
|---|---|---|---|---|
| LC | a | b | a | b |
| Expression Amount | 89.8mg/l | 18.8 mg/l | 24.5 mg/l | 68.5 mg/l |

TABLE 27

| **Pairs** | **Tm** |
|---|---|
| 4D9 WT | 69.9 ±2.00 |
| 2B9 WT | 69.1 ±1.50 |
| **Aa** | **70.3 ±1.70** |
| Ab | 58.5 ±11.96 |
| **Bb** | **68.7 ±0.92** |
| Ba | 58.6 ±0.53 |

Thermal stability (Tm) was measured with reference to the method explained in Example 2.

As shown in Tables 26 and 27 and FIG. 25, the bispecific antibodies having c'29c'30c48Φf'29f'30f'48 mutation pair introduced thereto were found to be higher in expression level and thermal stability for normal pairing (Aa/Bb) than for abnormal pairing (Ab/Ba).

**5.1.2. Trastuzumab/Bevacizumab bispecific antibody or Adalimumab/Bevacizumab bispecific antibody**

Trastuzumab (Herceptin®; Roche), Bevacizumab (Avastin™; Roche), and Adalimumab (Humira®; AbbVie) were purchased and subjected to amino acid sequencing (the Korea Basic Science Institute, Korea). cDNAs corresponding to the amino acid sequences were synthesized and used to construct bispecific antibodies to which the c'29c'30c48Φf'29f'30f'48 mutation pair and the AWBB mutation pair of CH3 domain selected in Example 3 were introduced in the combinations shown in Table 28, below (pcDNA3 vector (see FIG. 26) used).

TABLE 28

| Antibody | Heavy Chain Constant Region (HC) | | Light Chain Constant Region (LC) |
|---|---|---|---|
| | CH1 CH3 | | |
| Trabev | | | |
| Trastuzumab (Aa) | L145E/S183E | S364K/K409W | S131K/V133K |
| Bevacizumab (Bb) | L145K/S183K | K370S/F405R | S131E/V133E |
| Adabev | | | |
| Adalimumab (Aa) | L145E/S183E | S364K/K409W | S131K/V133K |
| Bevacizumab (Bb) | L145K/S183K | K370S/F405R | S131E/V133E |

The bispecific antibodies Trabev and Adabev were subjected to hydrophobic interaction chromatography (HIC) in the following condition and the results are depicted in FIG. 27 (y-axis: Value (mAU); and x-axis: time (min)):

Instrument: HPLC-U3000

Column: MAbPac Hic-20

Flow rate: 0.2mL/min

Detection: UV, 280nm

Mobile Phase: 0.10 M Ammonium acetate, pH 7.0

Passage of the obtained protein through the HIC column formed peaks at different time points depending on the hydrophobicity of the protein, whereby the construction of accurate bispecific antibodies could be accounted for. As can be seen in FIG. 27, peaks for the heterodimerized bispecific antibodies (Trabev and Adabev) are distinctively observed between peaks for two different homodimer antibodies (Trastuzumab and Bevacizumab, or Adalimumab and Bevacizumab), indicating the fine formation of bispecific antibodies, each composed of halves from two different antibodies.

In addition, the bispecific antibodies Trabev and Adabev thus obtained were subjected to size exclusion chromatography (SEC) analysis, and the results are given in Table 29 and FIGS. 28 and 29 (x-axis: time (min)):

Instrument: HPLC-U3000

Column: size-exclusion chromatography T SKgel G3000SWXL Tosoh Bioscience

Flow rate: 1.0mL/min

Detection: UV, 280nm

Mobile Phase: 25mM Tris-HCl (pH 8.5), 150 mM NaCl

TABLE 29

| WT (Monospecific Antibody) | | BsAb (Bispecific Antibody) | |
|---|---|---|---|
| 1A.Trastuzumab | 8.007min. | 1" Trabev | 7.847 min. |
| 1B.Bevacizumab | 7.743 min. | 2" Adabev | 7.833 min. |

(continued)

| WT (Monospecific Antibody) | BsAb (Bispecific Antibody) |
|---|---|
| 2A.Adalimumab 7.987 min. | |

In the SEC analysis, peaks are detected according to protein size and can elucidate protein aggregation. As shown in Table 29 and FIGS. 28 and 29, peaks for the bispecific antibodies are present between peaks of the two corresponding antibodies on the time axis, indicating the fine formation of the bispecific antibodies.

Further, heterodimization modes of the bispecific antibodies Trabev and Adabev on SDS-PAGE are depicted in FIG. 30. As can be seen in FIG. 30, the bispecific antibodies Trabev and Adabev, which are heterodimers, were detected as single bands at intermediate sizes between Trastuzumab and Bevacizumab and between Adalimumab and Bevacizumab, respectively. These results imply that the bispecific antibodies are not homodimers, but are constructed only as a result of normal pairing.

Through ELISA, tests were conducted to examine whether the BsAbs bind effectively to respective antigens (Trastuzumab: Her2), Bevacizumab (VEGF), and Adalimumab (TNF-alpha).

Affinity for antigen was measured as follows:

<u>Reagent</u>

Detection antibody: goat anti-human kappa-HRP (southern biotech, 2060-05)
TMB single solution (LIFE TECHNOLOGY, 002023)

<u>Instrument</u>

Emax precision microplate reader (Molecular devices)

<u>Protocol</u>

Coating buffer: Carbonate buffer pH 9.6
Blocking buffer: protein-free(TBS) blocking buffer (Thermo scientific)
Wash buffer: 0.05%(w/v) Tween20 in TBS, pH7.4 (TBST)
Diluent: 0.05%(w/v) Tween20 in TBS, pH7.4
Stop buffer: 1N Hydrochloric acid solution (HCl)

<u>Protocol</u>

Coating: dilute antigen in coating buffer, load 100 ul of dilution to each well, and incubate at 4 overnight (Her2, VEGF: 50ng/well, TNF-alpha: 100ng/well);
Washing 3 times with washing buffer;
Blocking: load 300 ul of blocking buffer, incubate at room temperature (RT) for 1 hour;
Washing 3 times with washing buffer;
Binding: load antibodies at an aliquot of 100ng/well, and incubate at RT for 1 hr;
Washing 3 times with washing buffer;
Detection Antibody: dilute goat anti-human kappa-HRP in TBST at a ratio of 1:4000 and incubate at RT for 1 hr;
Washing 3 times with washing buffer
Detection: load 100 ul of TMB solution per well, incubate at RT for 3 min in the dark;
Stop solution: load 100 ul of 1N HCl per well;
Reading: read at optical density 450nm
The results thus obtained are given in Table 30 and FIG. 31 (for Trabev) and in Table 31 and FIG. 32 (for Adabev):

TABLE 30

| Antigen | Her2 | | | VEGF | | |
|---|---|---|---|---|---|---|
| Anti-body | Trastuzumab | Bevacizumab | **Trabev** | Trastuzumab | Bevacizumab | **Trabev** |
| O.D | 2.50 | 0.05 | 2.54 | 0.65 | 2.89 | 2.04 |
| | 2.96 | 0.05 | 2.60 | 0.70 | 2.88 | 2.25 |
| | 2.58 | 0.04 | 2.49 | 0.60 | 3.12 | 2.36 |
| Mean | 2.68 | 0.05 | 2.54 | 0.65 | 2.96 | 2.22 |
| blank | 0.05 | | | 0.05 | | |

TABLE 31

| Antigen | TNF-alpha | | | VEGF | | |
|---|---|---|---|---|---|---|
| Antibody | Adalimumab | Bevacizumab | **Adabev** | Adalimumab | Bevacizumab | **Adabev** |
| O.D | 1.24 | 0.23 | 0.93 | 0.07 | 2.24 | 2.37 |
| | 1.26 | 0.22 | 1.16 | 0.06 | 2.27 | 2.28 |
| | 1.19 | 0.27 | 1.09 | 0.07 | 2.60 | 2.47 |
| Mean | 1.23 | 0.24 | 1.06 | 0.07 | 2.37 | 2.37 |
| blank | 0.06 | | | 0.06 | | |

As shown in Table 29 and FIG. 31, the bispecific antibody Trabev was found to bind to both Her2 and VEGF, which are antigens of Trastuzumab and Bevacizumab, respectively. Also, data in Table 30 and FIG. 32 proved that the bispecific antibody Adabev binds to TNF-alpha and VEGF, which are antigens of Adalimumab and Bevacizumab, respectively. These results confirmed that the two bispecific antibodies normally exerting desired functions were successfully constructed.

**5.2. Bispecific antibody having c34Φf51 mutation pair introduced thereto**

With reference to Example 5.1.1, antibodies having c34Φf51 mutation pair (see Table 23) introduced to 4D9 (Aa) and 2B9 (Bb) thereof were constructed. For each heavy chain, a and b light chains were co-expressed, and inter-light/heavy chain pairing ratios were measured on SDS-PAGE (conducted for heavy chains A and B, each). Tm was measured as in Example 2, and the results are given in Table 32, below and FIG. 34.

TABLE 32

| | **Test** 1 | | **Test 2** | |
|---|---|---|---|---|
| **HC** | **A (K147D/V185D)** | | **B (K147/V185K)** | |
| **LC** | **a (L135K/T180K)** | **b (L135E/T180E)** | **b (L135E/T180E)** | **a (L135K/T180K)** |
| *q* | 90% | 10% | 89% | 11% |
| Tm | 65.8 | N/A | 65.8 | N/A |
| (q: light chain/heavy chain paring ratio (%); N/A: not available) | | | | |

As shown in Table 32 and FIG. 34, normal heavy chain/light chain pairs (Aa and Bb) were formed at a ratio of as high as 90% and 89%, respectively, and exhibited high thermal stability.

In addition, bispecific antibodies Trabev (Aa: Trastuzumab; Bb: Bevacizumab) and Adabev (Aa: Adalimumab; Bb: Bevacizumab) to each of which the c34Φf51 mutation pair and the CH3 domain AWBB mutation pair (Aa: S364K/K409W; Bb: K370S/F405R) selected in Example 3 were introduced were constructed using Trastuzumab (Herceptin®; Roche), Bevacizumab (Avastin™; Roche), and Adalimumab (Humira®; AbbVie) in a manner similar to the construction procedure for bispecific antibodies of Example 5.1.2.

The constructed bispecific antibodies Trabev and Adabev, each having the c34Φf51 mutation pair and AWBB mutation pair introduced thereinto were analyzed using hydrophobic interaction chromatography (HIC) with reference to the method of Example 5.1.2.

The resulting analysis data are given in Table 33 and FIG. 35 (for Trabev) and in Table 34 and FIG. 36 (for Adabev).

TABLE 33

| # Set1 | Antibody | Time (min.) |
|---|---|---|
| Aa | Trastuzumab | 23.41 |
| Bb | Bevacizumab | 38.05 |
| **1AB** | **Trabev** | **29.13** |

TABLE 34

| # Set2 | Antibody | Time (min.) |
|---|---|---|
| Aa | Adalimumab | 22.07 |
| Bb | Bevacizumab | 38.05 |
| **2AB** | **Adabev** | **30.21** |

As can be seen in Table 33 and FIG. 35 and in Table 34 and FIG. 36, the peak of each of the heterodimeric bispecific antibodies (Trabev and Adabev) is distinctively observed between the peaks of two corresponding monospecific antibodies (Trastuzumab and Bevacizumab, or Adalimumab and Bevacizumab). The result implies the fine formation of bispecific antibodies having the c34Φf51 mutation pair and AWBB mutation pair introduced thereto, each composed of halves from two different antibodies.

Further, heterodimization modes of the bispecific antibodies having the c34Φf51 mutation pair and AWBB mutation pair introduced thereto are depicted in FIG. 37 (SDS-PAGE gel 6%, Non-Reducing condition). As can be seen in FIG. 37, concurrent introduction of the c34Φf51 mutation pair and AWBB mutation pair into already known antibodies was found to construct pure heterodimers as analyzed by SDS-PAGE.

### 5.3. Bispecific antibody having c40Φf44 mutation pair introduced thereto

With reference to Example 5.1.1, antibodies having c40Φf44 mutation pair (see Table 24) introduced to 4D9 (Aa) and 2B9 (Bb) thereof were constructed. For each heavy chain, a and b light chains were co-expressed, and inter-light/heavy chain pairing ratios were measured on SDS-PAGE (conducted for heavy chains A and B, each). Tm was measured as in Example 2, and the results are given in Table 35, below and FIG. 38.

TABLE 35

| **HC** | **A (F170D/P171D)** | | **B (F170K/P171K)** | |
|---|---|---|---|---|
| **LC** | **a (L135R/S162K)** | **b (L135E/S162D)** | **b (L135E/S162D)** | **a (L135R/S162K)** |
| *q* | 99% | 1% | 99% | 1% |
| Tm | 65.0 | N/A | 63.4 | N/A |
| (q: light chain/heavy chain pairing ratio (%); N/A: not available) | | | | |

As shown in Table 35 and FIG. 38, normal heavy chain/light chain pairs (Aa and Bb) were both formed at a ratio of as high as 99%, and exhibited high thermal stability.

In addition, bispecific antibodies Trabev (Aa: Trastuzumab; Bb: Bevacizumab) and Adabev (Aa: Adalimumab; Bb: Bevacizumab) to each of which the c40Φf44 mutation pair and the AWBB mutation pair (A: S364K/K409W; B: K370S/F405R) were introduced were constructed using Trastuzumab (Herceptin®; Roche), Bevacizumab (Avastin™;

Roche), and Adalimumab (Humira®; AbbVie) in a manner similar to the construction procedure for bispecific antibodies of Example 5.1.2.

The constructed bispecific antibodies Trabev and Adabev, each having the c40Φf44 mutation pair and AWBB mutation pair introduced thereinto, were analyzed using hydrophobic interaction chromatography (HIC) with reference to the method of Example 5.1.2.

The resulting analysis data are given in Table 36 and FIG. 39 (for Trabev) and in Table 37 and FIG. 40 (for Adabev).

TABLE 36

| # Set1 | Antibody | Time (min.) |
|---|---|---|
| Aa | Trastuzumab | 23.41 |
| Bb | Bevacizumab | 38.05 |
| **1AB** | **Trabev** | **26.11** |

TABLE 37

| # Set2 | Antibody | Time (min.) |
|---|---|---|
| Aa | Adalimumab | 22.07 |
| Bb | Bevacizumab | 38.05 |
| **2AB** | **Adabev** | **31.97** |

As can be seen in Table 36 and FIG. 39 and in Table 37 and FIG. 40, the peak of each of the heterodimeric bispecific antibodies (Trabev and Adabev) is distinctively observed between the peaks of two corresponding monospecific antibodies (Trastuzumab and Bevacizumab, or Adalimumab and Bevacizumab). The result implies the fine formation of bispecific antibodies having the c40Φf44 mutation pair and AWBB mutation pair introduced thereto, each composed of halves from two different antibodies. In addition, only one peak distinctively appearing between the two monospecific antibodies indicates that only one kind of normal pairing was made without mispairs between two heavy chains and between heavy and light chains.

Further, heterodimization modes of the bispecific antibodies having the c40Φf44 mutation pair and AWBB mutation pair introduced thereto are depicted in FIG. 41 (SDS-PAGE gel 6%, Non-Reducing condition). As can be seen in FIG. 41, concurrent introduction of the c40Φf44 mutation pair and AWBB mutation pair into already known antibodies was found to construct pure heterodimers as analyzed by SDS-PAGE.

## COMPARATIVE EXAMPLE 1

Heterodimerization rates were examined for cases where the mutations of CH3 domain proposed herein, which were different from the mutations suggested in Examples 1 to 3 although being identical amino acid pairs, were introduced.

For comparison, TNFRSF1B-Fc fusion protein (Enbrel; Enb) and Fas-Fc fusion protein (Fas) to which mutations of Table 38 were introduced were constructed with reference to Example 1 (expressed as BEAT-A and BEAT-B).

TABLE 38

| | Enb | Fas |
|---|---|---|
| AW/BB | AW (S364K/K409W) | BB (K370S/F405R) |
| BEAT-A | S364K/K409W | K370T/F405A |
| BEAT-B | S364T/K409R | K370T/F405S |

TNFRSF1B-Fc fusion protein (A chain) and Fas-Fc fusion protein having CH3 domain mutations selected in Example 3, which are representative of CH3 domain mutations proposed in the description (e.g., A chain having S364K and K409W introduced thereto and B chain having K370S and F405R introduced thereto) (expressed as AW/BB in Table 38) were prepared.

When Enb-Fc and Fas-Fc which had the mutation pairs listed in Table 38 were subjected to single transfection, homodimization modes were observed on SDS-PAGE. The results are given in FIG. 42.

As can be seen in FIG. 42, AW/BB exhibited high percentages of monomers whereas higher percentages of homodimers than monomers were detected in BEAT-A and BEAT-B. On the basis of the result, BEAT-A and BEAT-B are more likely to form homodimers than AW/BB when there is a difference in expression level between A chain and B chain. If a large quantity of homodimers is produced when heavy and light chains are combined so as to construct bispecific antibodies, an accurate heterodimer is difficult to separate from the homodimers because there are almost no differences in physical properties between the heterodimer and the homodimers. Accordingly, minimalizing homodimerization is very important for constructing a bispecific antibody of high purity. A combination of mutations that permits heterodimerization as little as possible is advantageous for the construction of bispecific antibodies.

Taken together, the data show that BEAT-A and BEAT-B have low heterodimerization potentials due to high homodimizerization rates compared to AW/BB and make it difficult to isolate accurate heterodimers.

## Claims

1. A bispecific protein for targeting two different kinds of targets, the bispecific protein comprising a first CH3 domain or a first Fc region comprising the first CH3 domain and a second CH3 domain or a second Fc region comprising the second CH3 domain, wherein the first CH3 domain and the second CH3 domain are mutated such that at least one selected from among amino acid pairs forming respective amino acid-amino acid bonds between the first CH3 domain and the second CH3 domain is modified by at least one of the following mutations:

(1) a mutation in which the two amino acids in at least one amino acid pair between the CH3 domains are swapped with each other (swapping mutation);
(2) a mutation in which, of at least one amino acid pair between the CH3 domains, one amino acid is substituted with an amino acid having a positive charge while the other is substituted with an amino acid having a negative charge, at least one of the two amino acid residues in the amino acid pair not being hydrophobic (electrostatic interaction-introduced mutation); and
(3) a mutation in which, of at least one amino acid pair between the CH3 domains, one amino acid is substituted with a large hydrophobic amino acid while the other is substituted with a small hydrophobic amino acid (size mutation), wherein the large amino acid is selected from the group consisting of tryptophan and phenylalanine and the small amino acid is selected from the group consisting of alanine, glycine, and valine.

2. The bispecific protein of claim 1, wherein the first CH3 domain and the second CH3 domain are each independently derived from an immunoglobulin selected from the group consisting of IgG1, IgG2, IgG3, IgG4, IgA1, IgA2, IgE, and IgM.

3. The bispecific protein of claim 1, wherein the amino acid pair forming an amino acid-amino acid bond is at least one selected from amino acid numbers 1 to 40 listed in the following table according to Eu numbering in IgG1-derived CH3 domains or from the group consisting of amino acid pairs at positions corresponding thereto in CH3 domains of IgG2, IgG3, IgG4, IgA1, IgA2, IgE, and IgM:

| Amino acid pair No. | IgG (1-4) | | IgA1 | | IgA2 | | IgE | | IgM | |
|---|---|---|---|---|---|---|---|---|---|---|
| | Chain A | Chain B | Chain A | Chain B | Chain A | Chain B | Chain A | Chain B | Chain A | Chain B |
| 1 | Q347 | K360 | E347 | L360 | E347 | L360 | E444 | R457 | Q347 | K360 |
| 2 | Y349 | S354 | H349 | P354 | H349 | P354 | Y446 | P451 | Y349 | S354 |
| 3 | Y349 | E357 | H349 | E357 | H349 | E357 | Y446 | P454 | Y349 | E357 |
| 4 | Y349 | K360 | H349 | L360 | H349 | L360 | Y446 | R457 | Y349 | K360 |
| 5 | L351 | L351 | L351 | L351 | L351 | L351 | F448 | F448 | L351 | L351 |
| 6 | P352 | P352 | P352 | P352 | P352 | P352 | A449 | A449 | P352 | P352 |
| 7 | S354 | Y349 | P354 | H349 | P354 | H349 | P451 | Y446 | S354 | Y349 |
| 8 | D356 | K439 | E356 | K439 | E356 | K439 | W453 | R539 | D356 | K439 |
| 9 | E357 | Y349 | E357 | H349 | E357 | H349 | P454 | Y446 | E357 | Y349 |
| 10 | E357 | K370 | E357 | R370 | E357 | R370 | P454 | Q467 | E357 | K370 |
| 11 | K360 | Q347 | L360 | E347 | L360 | E347 | R457 | E444 | K360 | Q347 |
| 12 | K360 | Y349 | L360 | H349 | L360 | H349 | R457 | Y446 | K360 | Y349 |
| 13 | S364 | L368 | T364 | L368 | T364 | L368 | T461 | L465 | S364 | L368 |
| 14 | S364 | K370 | T364 | R370 | T364 | R370 | T461 | Q467 | S364 | K370 |
| 15 | T366 | T366 | T366 | T366 | T366 | T366 | A463 | A463 | T366 | T366 |
| 16 | T366 | Y407 | T366 | T407 | T366 | T407 | A463 | F506 | T366 | Y407 |
| 17 | L368 | S364 | L368 | T364 | L368 | T364 | L465 | T461 | L368 | S364 |
| 18 | L368 | K409 | L368 | 1409 | L368 | 1409 | L465 | R508 | L368 | K409 |
| 19 | K370 | E357 | R370 | E357 | R370 | E357 | Q467 | P454 | K370 | E357 |
| 20 | K370 | S364 | R370 | T364 | R370 | T364 | Q467 | T461 | K370 | S364 |
| 21 | K370 | T411 | R370 | R411 | R370 | R411 | Q467 | E510 | K370 | T411 |
| 22 | N390 | S400 | L390 | S400 | L390 | S400 | R489 | K499 | N390 | S400 |
| 23 | K392 | L398 | L392 | Q398 | L392 | Q398 | S491 | K497 | K392 | L398 |
| 24 | T394 | T394 | W394 | W394 | W394 | W394 | T493 | T493 | T394 | T394 |
| 25 | T394 | V397 | W394 | R397 | W394 | R397 | T493 | R496 | T394 | V397 |
| 26 | P395 | P395 | A395 | A395 | A395 | A395 | Q494 | Q494 | P395 | P395 |
| 27 | P395 | V397 | A395 | R397 | A395 | R397 | Q494 | R496 | P395 | V397 |
| 28 | V397 | T394 | R397 | W394 | R397 | W394 | R496 | T493 | V397 | T394 |

(continued)

| Amino acid pair No. | IgG (1-4) | | IgA1 | | IgA2 | | IgE | | IgM | |
|---|---|---|---|---|---|---|---|---|---|---|
| | Chain A | Chain B | Chain A | Chain B | Chain A | Chain B | Chain A | Chain B | Chain A | Chain B |
| 29 | V397 | P395 | R397 | A395 | R397 | A395 | R496 | Q494 | V397 | P395 |
| 30 | L398 | K392 | Q398 | L392 | Q398 | L392 | K497 | S491 | L398 | K392 |
| 31 | S400 | N390 | S400 | K390 | S400 | K390 | K499 | R489 | S400 | N390 |
| 32 | F405 | K409 | A405 | I409 | A405 | I409 | F504 | R508 | F405 | K409 |
| 33 | Y407 | T366 | T407 | T366 | T407 | T366 | F506 | A463 | Y407 | T366 |
| 34 | Y407 | Y407 | T407 | T407 | T407 | T407 | F506 | F506 | Y407 | Y407 |
| 35 | Y407 | K409 | T407 | I409 | T407 | I409 | F506 | R508 | Y407 | K409 |
| 36 | K409 | L368 | 1409 | L368 | 1409 | L368 | R508 | L465 | K409 | L368 |
| 37 | K409 | F405 | I409 | A405 | I409 | A405 | R508 | F504 | K409 | F405 |
| 38 | K409 | Y407 | I409 | T407 | I409 | T407 | R508 | F506 | K409 | Y407 |
| 39 | T411 | K370 | R411 | R370 | R411 | R370 | E510 | Q467 | T411 | K370 |
| 40 | K439 | D356 | K439 | K439 | K439 | K439 | R539 | W453 | K439 | D356 |

**4.** The bispecific protein of claim 1, wherein the electrostatic interaction-introduced mutation is a mutation in which, of the two amino acids constituting: at least one amino acid pair selected from the group consisting of serine at position 364 and leucine at position 368, threonine at position 394 and threonine at position 394, glutamic acid at position 357 and lysine at position 370, glutamic acid at position 357 and tyrosine at position 349, threonine at position 366 and tyrosine at position 407, and threonine at position 394 and valine at position 397 in an IgG1 CH3 domain (EU numbering); or an amino acid pair at a position corresponding to the at least one amino acid pair in CH3 domains of IgG2, IgG3, IgG4, IgA1, IgA2, IgE, and IgM,
one is substituted with an amino acid having a positive charge and the other is substituted with an amino acid having a negative charge.

**5.** The bispecific protein of claim 4, wherein the amino acid having a negative charge is aspartic acid or glutamic acid and the amino acid having a positive charge is lysine or arginine.

**6.** The bispecific protein of claim 4, wherein the electrostatic interaction-introduced mutation comprises at least one of the following mutations on the basis of IgG1 (EU numbering), or at least one of corresponding mutations in the CH3 domains of IgG2, IgG3, IgG4, IgA1, IgA2, IgE, and IgM:

substitution of serine at position 364 with an amino acid having a positive charge and leucine at position 368 with an amino acid having a negative charge;
substitution of threonine at position 394 with an amino acid having a positive charge and threonine at position 394 with an amino acid having a negative charge;
substitution of glutamic acid at position 357 with an amino acid having a positive charge and lysine at position 370 with an amino acid having a negative charge;
substitution of glutamic acid at position 357 with an amino acid having a positive charge and tyrosine at position 349 with an amino acid having a negative charge;
substitution of threonine at position 366 with an amino acid having a positive charge and tyrosine at position 407 with an amino acid having a negative charge;
substitution of threonine at position 394 with an amino acid having a positive charge and valine at position 397 with an amino acid having a negative charge; and
substitution of tyrosine at position 349 with an amino acid having a positive charge and glutamic acid at position 357 with an amino acid having a negative charge.

**7.** The bispecific protein of claim 6, wherein the amino acid having a negative charge is aspartic acid or glutamic acid and the amino acid having a positive charge is lysine or arginine.

**8.** The bispecific protein of claim 1, wherein the swapping mutation is substitution in which exchange is made between two paired amino acid residues in:

at least one amino acid pair selected from the group consisting of a pair of serine at position 364 and lysine at position 370, a pair of phenylalanine at position 405 and lysine at position 409, a pair of glutamine at position 354 and lysine at position 360, a pair of serine at position 357 and tyrosine at position 349, a pair of glutamic acid at position 357 and lysine at position 370, a pair of lysine at position 360 and tyrosine at position 349, a pair of serine at position 364 and leucine at position 368, a pair of leucine at position 368 and lysine at position 409, a pair of asparagine at position 390 and serine at position 400, a pair of threonine at position 394 and valine at position 397, a pair of leucine at position 398 and lysine at position 392, a pair of tyrosine at position 407 and threonine at position 366, and a pair of threonine at position 411 and lysine at position 370 on the basis of CH3 domain IgG1 (EU numbering); or
an amino acid pair at a position corresponding to the at least one amino acid pair in CH3 domains of IgG2, IgG3, IgG4, IgA1, IgA2, IgE, and IgM.

**9.** The bispecific protein of claim 8, wherein the swapping mutation comprises at least one of the following mutations on the basis of the CH3 domain of IgG1 (EU numbering) or a mutation corresponding to the at least one mutation in CH3 domains of IgG2, IgG3, IgG4, IgA1, IgA2, IgE, and IgM:

substitution of serine at position 364 with lysine and lysine at position 370 with serine;
substitution of phenylalanine at position 405 with lysine and lysine at position 409 with phenylalanine;
substitution of tyrosine at position 407 with threonine and threonine at position 366 with tyrosine;
substitution of glutamic acid at position 357 with lysine and lysine at position 370 with glutamic acid; and
substitution of serine at position 357 with tyrosine and tyrosine at position 349 with serine.

**10.** The bispecific protein of claim 1, wherein the size mutation comprises substitution in which, of two paired amino acid residues in: at least one amino acid pair selected from the group consisting of a pair of lysine at position 409 and tyrosine at position 407, a pair of lysine at position 409 and phenylalanine at position 405, and a combination thereof on the basis of CH3 domain of IgG1 (EU numbering); or an amino acid pair at a position corresponding to the at least one amino acid pair in CH3 domains of IgG2, IgG3, IgG4, IgA1, IgA2, IgE, and IgM,
one is substituted with a large hydrophobic amino acid while the other is substituted with a small hydrophobic amino acid wherein the large hydrophobic amino acid is selected from the group consisting of tryptophan and phenylalanine and the small hydrophobic amino acid is selected from the group consisting of alanine, glycine, and valine.

**11.** The bispecific protein of claim 10, wherein the large amino acid is tryptophan and the small amino acid is alanine.

**12.** The bispecific protein of claim 10, wherein the size mutation comprises: at least one mutation selected from among the following mutations on the basis of the CH3 domain of IgG1 (EU numbering); and a mutation corresponding the at least one mutation in CH3 domains of IgG2, IgG3, IgG4, IgA1, IgA2, IgE, and IgM:

substitution of lysine at position 409 with tryptophan and tyrosine at position 407 with alanine; and
substitution of lysine at position 409 with tryptophan and phenylalanine at position 405 with alanine.

**13.** The bispecific protein of claim 1, comprising at least two of the electrostatic interaction-introduced mutation, the swapping mutation, and the size mutation.

**14.** The bispecific protein of claim 1, comprising:

at least one mutation selected from the group consisting of substitution of one of serine at position 364 and leucine at position 368 with an amino acid having a positive charge and the other with an amino acid having a negative charge, exchange of serine at position 364 with lysine at position 370, and exchange phenylalanine at position 405 with lysine at position 409, on the basis of the CH3 domain of IgG1 (EU numbering); or
a mutation corresponding to the at least one mutation in CH3 domains of IgG2, IgG3, IgG4, IgA1, IgA2, IgE, and IgM.

**15.** The bispecific protein of claim 14, comprising at least one of the following mutations on the basis of the CH3 of IgG1, or a mutation corresponding to the at least one mutation in CH3 domains of IgG2, IgG3 IgG4 IgA1 IgA2 IgD, IgE, and IgM:

(a) substitution serine at position 364 with lysine or arginine, and leucine at position 368 with aspartic acid or glutamic acid;
(b) substitution of serine at position 364 with lysine, and lysine at position 370 with serine; and
(c) substitution of phenylalanine at position 405 with lysine, and lysine at position 409 with phenylalanine.

**16.** A bispecific protein for targeting two different kinds of targets, the bispecific protein comprising at least one of the following mutations on the basis of CH3 domain of IgG1 (EU numbering), or a mutation corresponding to the at least one mutation in CH3 domains of IgG2, IgG3, IgG4, IgA1, IgA2, IgE, and IgM:

substitution of lysine at position 409 with phenylalanine or tryptophan, and phenylalanine at position 405 with lysine, arginine, glutamine, or asparagine;
substitution of leucine at position 368 with aspartic acid, glutamic acid, or for example, aspartic acid), and serine at position 364 with lysine, arginine, or asparagine; and
substitution of lysine at position 370 with serine, and serine at position 364 with lysine, arginine, or asparagine.

**17.** The bispecific protein of claim 16, comprising at least one of the following mutations on the basis of the CH3 domain of IgG1 (EU numbering), or a mutation corresponding to the at least one mutation in CH3 domains of IgG2, IgG3, IgG4, IgA1, IgA2, IgE, and IgM:

substitution of phenylalanine at position 405 with arginine, and lysine at position 409 with tryptophan;
substitution of serine at position 364 with lysine, and leucine at position 368 with aspartic acid;
substitution of serine at position 364 with lysine, and lysine at position 370 with serine;
substitution of phenylalanine at position 405 with lysine, and lysine at position 409 with phenylalanine;
substitution of phenylalanine at position 405 with arginine, and lysine at position 409 with phenylalanine; and
substitution of phenylalanine at position 405 with lysine, and lysine at position 409 with tryptophan.

**18.** The bispecific protein of claim 17, comprising at least one of the following mutations on the basis of the CH3 domain of IgG1 (EU numbering), or a mutation corresponding to the at least one mutation in CH3 domains of IgG2, IgG3, IgG4, IgA1, IgA2, IgE, and IgM:

substitution of serine at position 364 in the first CH3 domain with lysine and leucine at position 368 in the second CH3 domain with aspartic acid, and substitution of lysine at position 409 in the first CH3 domain with phenylalanine and phenylalanine at position 405 in the second CH3 domain with lysine;
substitution of serine at position 364 in the first CH3 domain with lysine and leucine at position 368 in the second CH3 domain with aspartic acid, and substitution of lysine at position 409 in the first CH3 domain with phenylalanine and phenylalanine at position 405 in the second CH3 domain with arginine;
substitution of serine at position 364 in the first CH3 domain with lysine and lysine at position 370 in the second CH3 domain with serine, and substitution of lysine at position 409 in the first CH3 domain with phenylalanine and phenylalanine at position 405 in the second CH3 domain with lysine;
substitution of serine at position 364 in the first CH3 domain with lysine and lysine at position 370 in the second CH3 domain with serine, and substitution of lysine at position 409 in the first CH3 domain with phenylalanine and phenylalanine at position 405 in the second CH3 domain with arginine;
substitution of serine at position 364 in the first CH3 domain with lysine and leucine at position 368 in the second CH3 domain with aspartic acid, and substitution of lysine at position 409 in the first CH3 domain with tryptophan and phenylalanine at position 405 in the second CH3 domain with lysine;
substitution of serine at position 364 in the first CH3 domain with lysine and leucine at position 368 in the second CH3 domain with aspartic acid, and substitution of lysine at position 409 in the first CH3 domain with tryptophan and phenylalanine at position 405 in the second CH3 domain with arginine;
substitution of serine at position 364 in the first CH3 domain with lysine and lysine at position 370 in the second CH3 domain with serine, and substitution of lysine at position 409 in the first CH3 domain with tryptophan and phenylalanine at position 405 in the second CH3 domain with lysine; or
substitution of serine at position 364 in the first CH3 domain with lysine and lysine at position 370 in the second CH3 domain with serine, and substitution of lysine at position 409 in the first CH3 domain with tryptophan and phenylalanine at position 405 in the second CH3 domain with arginine.

**19.** The bispecific protein of one of claims 1 to 18, wherein bispecific protein is at least one selected from the group consisting of a bispecific antibody; an antigen-binding fragment of a bispecific antibody, a bispecific antibody analog, and a fusion protein of a target-specific binding polypeptide and an Fc region or CH3 domain.

**20.** The bispecific protein of claim 19, wherein the target-specific binding polypeptide is selected from the group consisting of a receptor, an ectodomain, and a ligand.

**21.** A bispecific antibody or an antigen-binding fragment thereof, the bispecific antibody comprising a first CH1 domain and a first CL (light chain constant region) domain derived respectively from the heavy chain and light chain of an antibody recognizing a first epitope and a second CH1 domain and a second CL domain derived respectively from the heavy chain and light chain of an antibody recognizing a second epitope, wherein the CH1 domains and the CL domains are mutated to contain at least one of the following mutations:

a mutation in which, of the two amino acids constituting each pair of one or more first amino acid pairs selected from among amino acid pairs forming respective amino acid-amino acid bonds between the first CH1 domain and the first CL domain, one is substituted with an amino acid having a positive charge and the other is substituted with an amino acid having a negative charge; and
a mutation in which, of the two amino acids constituting each pair of one or more second amino acid pairs selected from among amino acid pairs forming respective amino acid-amino acid bonds between the second CH1 domain and the second CL domain, one is substituted with an amino acid having a positive charge and the other is substituted with an amino acid having a negative charge.

**22.** The bispecific antibody or the antigen-binding fragment of claim 21, wherein:

the amino acids substituted respectively in the first CH1 domain and the second CH1 domain have opposite charges,
the amino acids substituted respectively in the first CL domain and the first CH1 domain have opposite charges, and
the amino acids substituted respectively in the second CL domain and the second CH1 domain have opposite charges.

**23.** The bispecific antibody or antigen-binding fragment of claim 21, wherein the amino acid having a positive charge is lysine or arginine, the amino acid having a negative charge is aspartic acid or glutamic acid, and the amino acid to be substituted with an amino acid having a positive or negative charge in the CH1 domain is at least one of the following amino acids on the basis of the CH1 domain of IgG1 (EU numbering), or an amino acid at a position corresponding to the at least one amino acid in CH1 domains of IgG2, IgG3, IgG4, IgA1, IgA2, IgE, and IgM:

leucine at position 145,
serine at position 183,
lysine at position 147,
phenylalanine at position 170,
proline at position 171, and
valine at position 185, and
the amino acid to be substituted with an amino acid having a positive or negative charge in the CL domain is at least one of the following amino acids on the basis of the CL domain of kappa type (EU numbering), or an amino acid at a position corresponding to the at least one amino acid in the CL domain of lambda type:

serine at position 131,
valine at position 133,
leucine at position 135,
serine at position 162, and
threonine at position 180.

**24.** The bispecific antibody or antigen-binding fragment of claim 21, wherein
the amino acid having a positive charge is lysine or arginine,
the amino acid having a negative charge is aspartic acid or glutamic acid, and
a set of two amino acids forming an amino acid pair between the CH1 domain and the CL domain is at least one of the following amino acid pairs on the basis of the CH1 domain of IgG1 and the CL domain of kappa type (EU numbering), or an amino acid pair at a position corresponding to the at least one amino acid pair in CH1 domains and CL domain of lambda type of IgG2, IgG3, IgG4, IgA1, IgA2, IgE, and IgM:

a pair of leucine at position 145 in the CH1 domain and serine at position 131 in the CL domain,

a pair of leucine at position 145 in the CH1 domain and valine at position 133 in the CL domain,
a pair of serine at position 183 in the CH1 domain and valine at position 133 in the CL domain,
a pair of lysine at position 147 in the CH1 domain and threonine at position 180 in the CL domain,
a pair of valine at position 185 in the CH1 domain and leucine at position 135 in the CL domain,
a pair of phenylalanine at position 170 in the CH1 domain and leucine at position 135 in the CL domain, and
a pair of proline at position 171 in the CH1 domain and serine at position 162 in the CL domain.

**25.** The bispecific antibody or antigen-binding fragment of one of claims 21 to 24, wherein the bispecific antibody comprises modified CH3 domains or a mutant Fc region inclusive of the modified CH3 domains, the modified CH3 domains comprising a first CH3 domain and a second CH3 domain and being mutated such that at least one selected from amino acid pairs forming amino acid-amino acid bonds between the first CH3 domain derived from the heavy chain of an antibody recognizing a first epitope and the second CH3 domain derived from the heavy chain of an antibody recognizing a second epitope has at least one of the following mutations:

a mutation in which amino acids in at least one amino acid pair between the CH3 domains are switched with each other (swapping mutation);
a mutation in which, of at least one amino acid pair between the CH3 domains, one amino acid is substituted with an amino acid having a positive charge while the other is substituted with an amino acid having a negative charge, at least one of the two amino acid residues in the amino acid pair not being hydrophobic (electrostatic interaction-introduced mutation); and
a mutation in which, of at least one amino acid pair between the CH3 domains, one amino acid is substituted with a large hydrophobic amino acid while the other is substituted with a small hydrophobic amino acid (size mutation), wherein the large amino acid is selected from the group consisting of tryptophan and phenylalanine and the small amino acid is selected from the group consisting of alanine, glycine, and valine.

**26.** The bispecific antibody or antigen-binding fragment of claim 25, wherein the first CH3 domain and the second CH3 domain are each independently derived from an immunoglobulin selected from the group consisting of IgG1, IgG2, IgG3, IgG4, IgA1, IgA2, IgE, and IgM.

**27.** The bispecific antibody or antigen-binding fragment of claim 25, wherein the amino acid pair forming an amino acid-amino acid bond is at least one selected from amino acid numbers 1 to 40 listed in the following table according to Eu numbering in IgG1-derived CH3 domains or from the group consisting of amino acid pairs at positions corresponding thereto in CH3 domains of IgG2, IgG3, IgG4, IgA1, IgA2, IgE, and IgM:

| Amino acid pair No. | IgG (1-4) | | IgA1 | | IgA2 | | IgE | | IgM | |
|---|---|---|---|---|---|---|---|---|---|---|
| | Chain A | Chain B | Chain A | Chain B | Chain A | Chain B | Chain A | Chain B | Chain A | Chain B |
| 1 | Q347 | K360 | E347 | L360 | E347 | L360 | E444 | R457 | Q347 | K360 |
| 2 | Y349 | S354 | H349 | P354 | H349 | P354 | Y446 | P451 | Y349 | S354 |
| 3 | Y349 | E357 | H349 | E357 | H349 | E357 | Y446 | P454 | Y349 | E357 |
| 4 | Y349 | K360 | H349 | L360 | H349 | L360 | Y446 | R457 | Y349 | K360 |
| 5 | L351 | L351 | L351 | L351 | L351 | L351 | F448 | F448 | L351 | L351 |
| 6 | P352 | P352 | P352 | P352 | P352 | P352 | A449 | A449 | P352 | P352 |
| 7 | S354 | Y349 | P354 | H349 | P354 | H349 | P451 | Y446 | S354 | Y349 |
| 8 | D356 | K439 | E356 | K439 | E356 | K439 | W453 | R539 | D356 | K439 |
| 9 | E357 | Y349 | E357 | H349 | E357 | H349 | P454 | Y446 | E357 | Y349 |
| 10 | E357 | K370 | E357 | R370 | E357 | R370 | P454 | Q467 | E357 | K370 |
| 11 | K360 | Q347 | L360 | E347 | L360 | E347 | R457 | E444 | K360 | Q347 |
| 12 | K360 | Y349 | L360 | H349 | L360 | H349 | R457 | Y446 | K360 | Y349 |
| 13 | S364 | L368 | T364 | L368 | T364 | L368 | T461 | L465 | S364 | L368 |

(continued)

| Amino acid pair No. | IgG (1-4) | | IgA1 | | IgA2 | | IgE | | IgM | |
|---|---|---|---|---|---|---|---|---|---|---|
| | Chain A | Chain B | Chain A | Chain B | Chain A | Chain B | Chain A | Chain B | Chain A | Chain B |
| 14 | S364 | K370 | T364 | R370 | T364 | R370 | T461 | Q467 | S364 | K370 |
| 15 | T366 | T366 | T366 | T366 | T366 | T366 | A463 | A463 | T366 | T366 |
| 16 | T366 | Y407 | T366 | T407 | T366 | T407 | A463 | F506 | T366 | Y407 |
| 17 | L368 | S364 | L368 | T364 | L368 | T364 | L465 | T461 | L368 | S364 |
| 18 | L368 | K409 | L368 | 1409 | L368 | 1409 | L465 | R508 | L368 | K409 |
| 19 | K370 | E357 | R370 | E357 | R370 | E357 | Q467 | P454 | K370 | E357 |
| 20 | K370 | S364 | R370 | T364 | R370 | T364 | Q467 | T461 | K370 | S364 |
| 21 | K370 | T411 | R370 | R411 | R370 | R411 | Q467 | E510 | K370 | T411 |
| 22 | N390 | S400 | L390 | S400 | L390 | S400 | R489 | K499 | N390 | S400 |
| 23 | K392 | L398 | L392 | Q398 | L392 | Q398 | S491 | K497 | K392 | L398 |
| 24 | T394 | T394 | W394 | W394 | W394 | W394 | T493 | T493 | T394 | T394 |
| 25 | T394 | V397 | W394 | R397 | W394 | R397 | T493 | R496 | T394 | V397 |
| 26 | P395 | P395 | A395 | A395 | A395 | A395 | Q494 | Q494 | P395 | P395 |
| 27 | P395 | V397 | A395 | R397 | A395 | R397 | Q494 | R496 | P395 | V397 |
| 28 | V397 | T394 | R397 | W394 | R397 | W394 | R496 | T493 | V397 | T394 |
| 29 | V397 | P395 | R397 | A395 | R397 | A395 | R496 | Q494 | V397 | P395 |
| 30 | L398 | K392 | Q398 | L392 | Q398 | L392 | K497 | S491 | L398 | K392 |
| 31 | S400 | N390 | S400 | K390 | S400 | K390 | K499 | R489 | S400 | N390 |
| 32 | F405 | K409 | A405 | I409 | A405 | I409 | F504 | R508 | F405 | K409 |
| 33 | Y407 | T366 | T407 | T366 | T407 | T366 | F506 | A463 | Y407 | T366 |
| 34 | Y407 | Y407 | T407 | T407 | T407 | T407 | F506 | F506 | Y407 | Y407 |
| 35 | Y407 | K409 | T407 | I409 | T407 | I409 | F506 | R508 | Y407 | K409 |
| 36 | K409 | L368 | 1409 | L368 | 1409 | L368 | R508 | L465 | K409 | L368 |
| 37 | K409 | F405 | I409 | A405 | I409 | A405 | R508 | F504 | K409 | F405 |
| 38 | K409 | Y407 | I409 | T407 | I409 | T407 | R508 | F506 | K409 | Y407 |
| 39 | T411 | K370 | R411 | R370 | R411 | R370 | E510 | Q467 | T411 | K370 |
| 40 | K439 | D356 | K439 | K439 | K439 | K439 | R539 | W453 | K439 | D356 |

**28.** The bispecific antibody or antigen-binding fragment of claim 25, wherein the electrostatic interaction-introduced mutation is a mutation in which, of the two amino acids constituting: at least one amino acid pair selected from the group consisting of serine at position 364 and leucine at position 368, threonine at position 394 and threonine at position 394, glutamic acid at position 357 and lysine at position 370, glutamic acid at position 357 and tyrosine at position 349, threonine at position 366 and tyrosine at position 407, and threonine at position 394 and valine at position 397 in an IgG1 CH3 domain (EU numbering); or an amino acid pair at a position corresponding to the at least one amino acid pair in CH3 domains of IgG2, IgG3, IgG4, IgA1, IgA2, IgE, and IgM,
one is substituted with an amino acid having a positive charge and the other is substituted with an amino acid having a negative charge.

**29.** The bispecific antibody or antigen-binding fragment of claim 28, wherein the amino acid having a negative charge is aspartic acid or glutamic acid and the amino acid having a positive charge is lysine or arginine.

**30.** The bispecific antibody or antigen-binding fragment of claim 28, wherein the electrostatic interaction-introduced mutation comprises at least one of the following mutations on the basis of IgG1 (EU numbering), or at least one of corresponding mutations in the CH3 domains of IgG2, IgG3, IgG4, IgA1, IgA2, IgE, and IgM:

substitution of serine at position 364 with an amino acid having a positive charge and leucine at position 368 with an amino acid having a negative charge;
substitution of threonine at position 394 with an amino acid having a positive charge and threonine at position 394 with an amino acid having a negative charge;
substitution of glutamic acid at position 357 with an amino acid having a positive charge and lysine at position 370 with an amino acid having a negative charge;
substitution of glutamic acid at position 357 with an amino acid having a positive charge and tyrosine at position 349 with an amino acid having a negative charge;
substitution of threonine at position 366 with an amino acid having a positive charge and tyrosine at position 407 with an amino acid having a negative charge;
substitution of threonine at position 394 with an amino acid having a positive charge and valine at position 397 with an amino acid having a negative charge; and
substitution of tyrosine at position 349 with an amino acid having a positive charge and glutamic acid at position 357 with an amino acid having a negative charge.

**31.** The bispecific antibody or antigen-binding fragment of claim 30, wherein the amino acid having a negative charge is aspartic acid or glutamic acid and the amino acid having a positive charge is lysine or arginine.

**32.** The bispecific antibody or antigen-binding fragment of claim 25, wherein the swapping mutation is substitution in which exchange is made between two paired amino acid residues in:

at least one amino acid pair selected from the group consisting of a pair of glutamine at position 354 and lysine at position 360, a pair of serine at position 357 and tyrosine at position 349, a pair of glutamic acid at position 357 and lysine at position 370, a pair of lysine at position 360 and tyrosine at position 349, a pair of serine at position 364 and leucine at position 368, a pair of serine at position 364 and lysine at position 370, a pair of leucine at position 368 and lysine at position 409, a pair of asparagine at position 390 and serine at position 400, a pair of threonine at position 394 and valine at position 397, a pair of leucine at position 398 and lysine at position 392, a pair of phenylalanine at position 405 and lysine at position 409, a pair of tyrosine at position 407 and threonine at position 366, and a pair of threonine at position 411 and lysine at position 370 on the basis of CH3 domain IgG1 (EU numbering); or
an amino acid pair at a position corresponding to the at least one amino acid pair in CH3 domains of IgG2, IgG3, IgG4, IgA1, IgA2, IgE, and IgM.

**33.** The bispecific antibody or antigen-binding fragment of claim 32, wherein the swapping mutation comprises at least one of the following mutations on the basis of the CH3 domain of IgG1 (EU numbering) or a mutation corresponding to the at least one mutation in CH3 domains of IgG2, IgG3, IgG4, IgA1, IgA2, IgE, and IgM:

substitution of serine at position 364 with lysine and lysine at position 370 with serine;
substitution of phenylalanine at position 405 with lysine and lysine at position 409 with phenylalanine;
substitution of tyrosine at position 407 with threonine and threonine at position 366 with tyrosine;
substitution of glutamic acid at position 357 with lysine and lysine at position 370 with glutamic acid; and
substitution of serine at position 357 with tyrosine and tyrosine at position 349 with serine.

**34.** The bispecific antibody or antigen-binding fragment of claim 25, wherein the size mutation comprises substitution in which, of two paired amino acid residues in: at least one amino acid pair selected from the group consisting of a pair of lysine at position 409 and tyrosine at position 407, a pair of lysine at position 409 and phenylalanine at position 405, and a combination thereof on the basis of CH3 domain of IgG1 (EU numbering); or an amino acid pair at a position corresponding to the at least one amino acid pair in CH3 domains of IgG2, IgG3, IgG4, IgA1, IgA2, IgE, and IgM,
one is substituted with a large hydrophobic amino acid while the other is substituted with a small hydrophobic amino acid wherein the large hydrophobic amino acid is selected from the group consisting of tryptophan and phenylalanine and the small hydrophobic amino acid is selected from the group consisting of alanine, glycine, and valine.

**35.** The bispecific antibody or antigen-binding fragment of claim 34, wherein the large amino acid is tryptophan and the small amino acid is alanine.

**36.** The bispecific antibody or antigen-binding fragment of claim 34, wherein the size mutation comprises: at least one mutation selected from among the following mutations on the basis of the CH3 domain of IgG1 (EU numbering); and a mutation corresponding the at least one mutation in CH3 domains of IgG2, IgG3, IgG4, IgA1, IgA2, IgE, and IgM:

substitution of lysine at position 409 with tryptophan and tyrosine at position 407 with alanine; and
substitution of lysine at position 409 with tryptophan and phenylalanine at position 405 with alanine.

**37.** The bispecific antibody or antigen-binding fragment of claim 25, wherein the bispecific antibody comprises at least two of the electrostatic interaction-introduced mutation, the swapping mutation, and the size mutation.

**38.** The bispecific antibody or antigen-binding fragment of claim 25, wherein the bispecific antibody comprises:

at least one mutation selected from the group consisting of substitution of one of serine at position 364 and leucine at position 368 with an amino acid having a positive charge and the other with an amino acid having a negative charge, exchange of serine at position 364 with lysine at position 370, and exchange phenylalanine at position 405 with lysine at position 409, on the basis of the CH3 domain of IgG1 (EU numbering); or
a mutation corresponding to the at least one mutation in CH3 domains of IgG2, IgG3, IgG4, IgA1, IgA2, IgE, and IgM.

**39.** The bispecific antibody or antigen-binding fragment of claim 38, wherein the bispecific antibody comprises at least one of the following mutations on the basis of the CH3 domain of IgG1, or a mutation corresponding to the at least one mutation in CH3 domains of IgG2, IgG3 IgG4 IgA1 IgA2 IgD, IgE, and IgM:

(a) substitution of serine at position 364 with lysine or arginine, and leucine at position 368 with aspartic acid or glutamic acid;
(b) substitution of serine at position 364 with lysine, and lysine at position 370 with serine; and
(c) substitution of phenylalanine at position 405 with lysine, and lysine at position 409 with phenylalanine.

**40.** The bispecific antibody or antigen-binding fragment of claim 25, wherein the bispecific protein comprises at least one of the following mutations on the basis of CH3 domain of IgG1 (EU numbering), or a mutation corresponding to the at least one mutation in CH3 domains of IgG2, IgG3, IgG4, IgA1, IgA2, IgE, and IgM:

substitution of lysine at position 409 with phenylalanine or tryptophan, and phenylalanine at position 405 with lysine, arginine, glutamine, or asparagine;
substitution of leucine at position 368 with aspartic acid, glutamic acid, or aspartic acid, and serine at position 364 with lysine, arginine, or asparagine; and
substitution of lysine at position 370 with serine, and serine at position 364 with lysine, arginine, or asparagine.

**41.** The bispecific antibody or antigen-binding fragment of claim 40, wherein the bispecific antibody comprises at least one of the following mutations on the basis of the CH3 domain of IgG1 (EU numbering), or a mutation corresponding to the at least one mutation in CH3 domains of IgG2, IgG3, IgG4, IgA1, IgA2, IgE, and IgM:

substitution of phenylalanine at position 405 with arginine, and lysine at position 409 with tryptophan;
substitution of serine at position 364 with lysine, and leucine at position 368 with aspartic acid;
substitution of serine at position 364 with lysine, and lysine at position 370 with serine;
substitution of phenylalanine at position 405 with lysine, and lysine at position 409 with phenylalanine;
substitution of phenylalanine at position 405 with arginine, and lysine at position 409 with phenylalanine; and
substitution of phenylalanine at position 405 with lysine, and lysine at position 409 with tryptophan.

**42.** The bispecific antibody or antigen-binding fragment of claim 41, wherein the bispecific antibody comprises at least one of the following mutations on the basis of the CH3 domain of IgG1 (EU numbering), or a mutation corresponding to the at least one mutation in CH3 domains of IgG2, IgG3, IgG4, IgA1, IgA2, IgE, and IgM:

substitution of serine at position 364 in the first CH3 domain with lysine and lysine at position 370 in the second CH3 domain with serine, and substitution of lysine at position 409 in the first CH3 domain with tryptophan and phenylalanine at position 405 in the second CH3 domain with arginine;

substitution of serine at position 364 in the first CH3 domain with lysine and leucine at position 368 in the second CH3 domain with aspartic acid, and substitution of lysine at position 409 in the first CH3 domain with phenylalanine and phenylalanine at position 405 in the second CH3 domain with lysine;
substitution of serine at position 364 in the first CH3 domain with lysine and leucine at position 368 in the second CH3 domain with aspartic acid, and substitution of lysine in the first CH3 domain at position 409 with phenylalanine and phenylalanine at position 405 in the second CH3 domain with arginine;
substitution of serine at position 364 in the first CH3 domain with lysine and lysine at position 370 in the second CH3 domain with serine, and substitution of lysine at position 409 in the first CH3 domain with phenylalanine and phenylalanine at position 405 in the second CH3 domain with lysine;
substitution of serine at position 364 in the first CH3 domain with lysine and lysine at position 370 in the second CH3 domain with serine, and substitution of lysine at position 409 in the first CH3 domain with phenylalanine and phenylalanine at position 405 in the second CH3 domain with arginine;
substitution of serine at position 364 in the first CH3 domain with lysine and leucine at position 368 in the second CH3 domain with aspartic acid, and substitution of lysine at position 409 in the first CH3 domain with tryptophan and phenylalanine at position 405 in the second CH3 domain with lysine;
substitution of serine at position 364 in the first CH3 domain with lysine and leucine at position 368 in the second CH3 domain with aspartic acid, and substitution of lysine at position 409 in the first CH3 domain with tryptophan and phenylalanine at position 405 in the second CH3 domain with arginine; or
substitution of serine at position 364 in the first CH3 domain with lysine and lysine at position 370 in the second CH3 domain with serine, and substitution of lysine at position 409 in the first CH3 domain with tryptophan and phenylalanine at position 405 in the second CH3 domain with lysine.

**43.** A method for constructing a bispecific protein for targeting different targets, the method comprising one of the following mutation introducing steps to introduce at least one mutation into at least one selected from amino acid pairs forming amino acid-amino acid bonds between a first CH3 domain and a second CH3 domain:

switching the two amino acids in at least one amino acid pair selected from amino acid pairs forming amino-amino acid bonds between a first CH3 domain and a second CH3 domain with each other;
substituting one of the two amino acids in at least one amino acid pair selected from amino acid pairs forming amino-amino acid bonds between the CH3 domains with an amino acid having a positive charge and the other with an amino acid having a negative charge; and
substituting one of the two amino acids in at least one amino acid pair selected from amino acid pairs forming amino-amino acid bonds between the CH3 domains with a large hydrophobic amino acid and the other with a small hydrophobic amino acid, wherein the large amino acid is selected from the group consisting of tryptophan and phenylalanine and the small amino acid is selected from the group consisting of alanine, glycine, and valine.

**44.** A method for constructing a bispecific antibody or an antigen-binding fragment thereof, comprising the following CH1 and CL domain mutating steps of:

substituting one of the two amino acid residues constituting at least one selected from amino acid pairs forming amino acid-amino acid bonds between a first CH1 domain derived from the heavy chain and a first CL domain of an antibody recognizing a first epitope with an amino acid having a positive charge and the other with an amino acid having a negative charge; and
substituting one of the two amino acid residues constituting at least one selected from amino acid pairs forming amino acid-amino acid bonds between a second CH1 domain derived from the heavy chain and a second CL domain of an antibody recognizing a second epitope with an amino acid having a positive charge and the other with an amino acid having a negative charge.

**45.** The method of claim 44, further comprising at least one of the following CH3 domain mutating steps:

switching the two amino acids in at least one amino acid pair selected from amino acid pairs forming amino-amino acid bonds between a first CH3 domain and a second CH3 domain with each other;
substituting one of the two amino acids in at least one selected from amino acid pairs forming amino-amino acid bonds between the CH3 domains with an amino acid having a positive charge and the other with an amino acid having a negative charge; and
substituting one of the two amino acid residues constituting at least one selected from amino acid pairs forming amino acid-amino acid bonds between a first CH3 domain derived from the heavy chain of an antibody recognizing a first epitope and a second CH3 domain derived from the heavy chain of an antibody recognizing a second epitope with an amino acid having a positive charge and the other with an amino acid having a negative charge; and
substituting one of the two amino acids in at least one amino acid pair selected from amino acid pairs forming amino-amino acid bonds between the first CH3 domain and the second CH3 domain with a large hydrophobic amino acid and the other with a small hydrophobic amino acid, wherein the large amino acid is selected from the group consisting of tryptophan and phenylalanine and the small amino acid is selected from the group consisting of alanine, glycine, and valine.

**46.** An anti-influenza B antibody or an antigen-binding fragment thereof, the antibody comprising the heavy chain variable region of SEQ ID NO: 27 and the light chain variable region of SEQ ID NO: 31.

**47.** An anti-influenza A antibody or an antigen-binding fragment thereof, the antibody comprising the heavy chain variable region of SEQ ID NO: 29 and the light chain variable region of SEQ ID NO: 31.

**48.** An anti-influenza A/anti-influenza B bispecific antibody or an antigen-binding fragment thereof, the bispecific antibody comprising an anti-influenza B antibody comprising the heavy chain variable region of SEQ ID NO: 27 and the light chain variable region of SEQ ID NO: 31; and an anti-influenza A antibody comprising the heavy chain variable region of SEQ ID NO: 29 and the light chain variable region of SEQ ID NO: 31.

**49.** The bispecific antibody or antigen-binding fragment of one of claims 21 to 24, wherein
the antibody recognizing a first epitope is an an anti-influenza B antibody comprising the heavy chain variable region of SEQ ID NO: 27 and the light chain variable region of SEQ ID NO: 31; and
the antibody recognizing a second epitope is an anti-influenza A antibody comprising the heavy chain variable region of SEQ ID NO: 29 and the light chain variable region of SEQ ID NO: 31.

**50.** The bispecific antibody or antigen-binding fragment of claim 25, wherein
the antibody recognizing a first epitope is an an anti-influenza B antibody comprising the heavy chain variable region of SEQ ID NO: 27 and the light chain variable region of SEQ ID NO: 31; and
the antibody recognizing a second epitope is an anti-influenza A antibody comprising the heavy chain variable region of SEQ ID NO: 29 and the light chain variable region of SEQ ID NO: 31.

FIG. 1

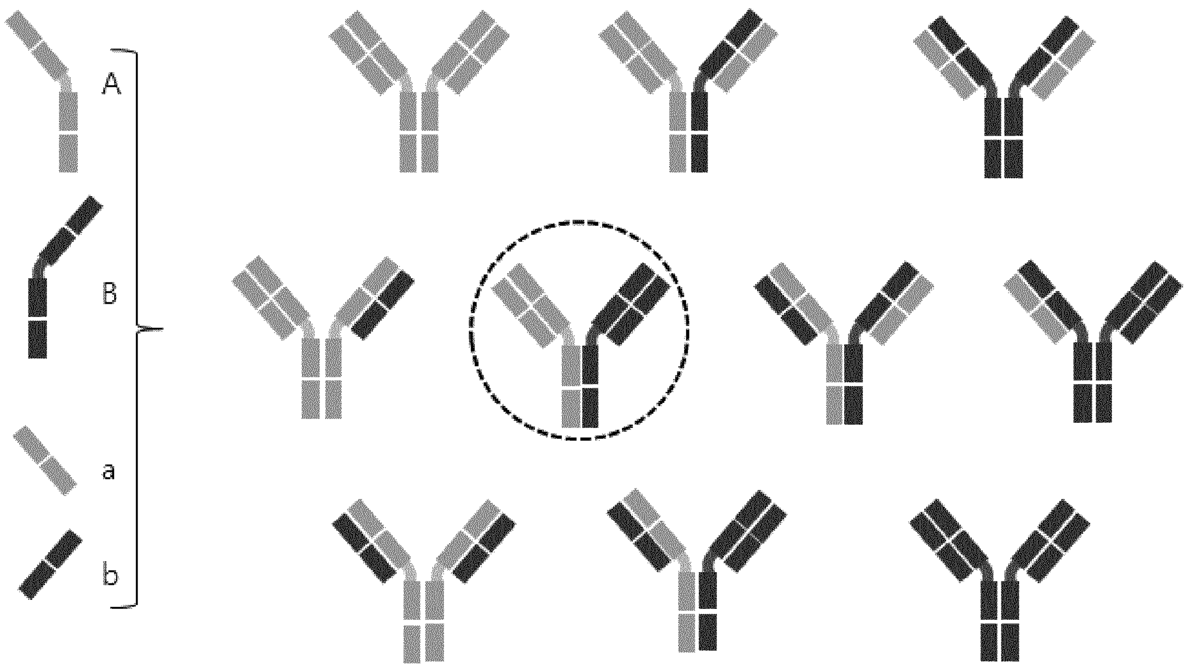

FIG. 2

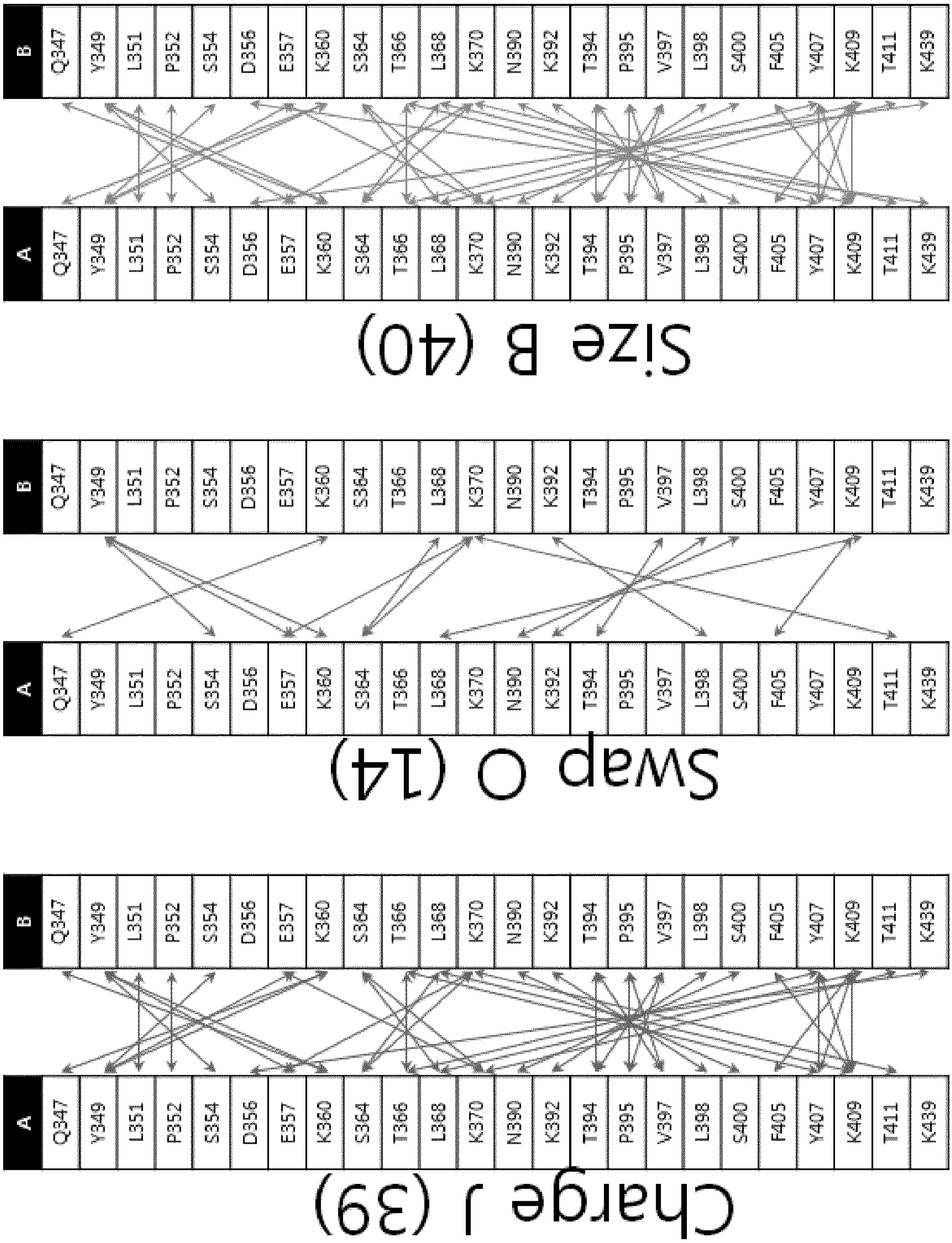

FIG. 3

| A | WT | Y349K | Y349K | S354K | E356K | E357K | E357K | S364K | T366K | T394K | T394K | T411K |
|---|---|---|---|---|---|---|---|---|---|---|---|---|
| B | WT | E357D | K360D | Y349D | K439D | Y349D | K370D | L368D | Y407D | T394D | V397D | K370D |
| $S_{AA}$ | 28 | **11** | 5 | 27 | 10 | **15** | **12** | **0** | **0** | **11** | **10** | 15 |
| $S_{AB}$ | 46 | **70** | 60 | 63 | 64 | **75** | **78** | **100** | **75** | **79** | **73** | 67 |
| $S_{BB}$ | 26 | **19** | 35 | 10 | 26 | **10** | **10** | **0** | **25** | **10** | **17** | 18 |

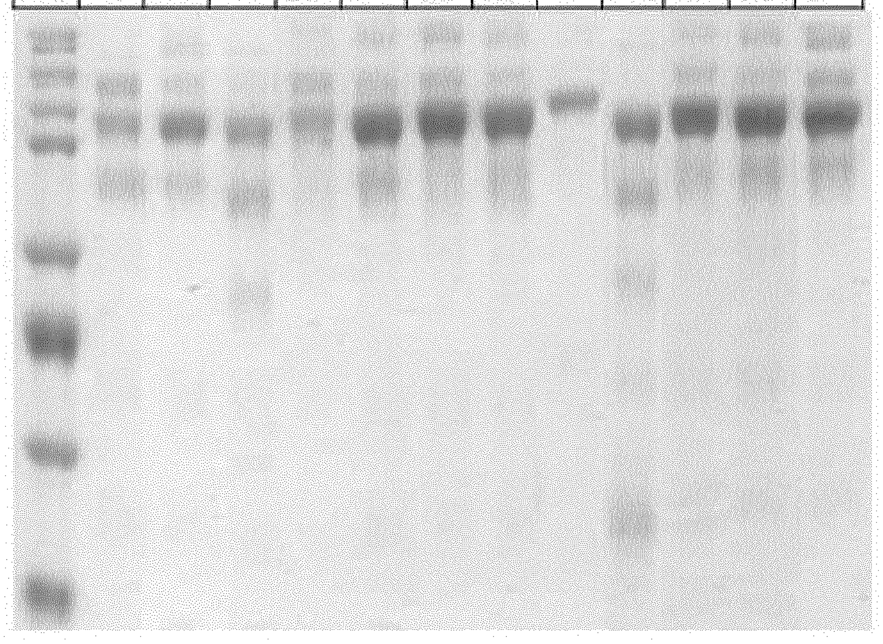

FIG. 4

| | | | | | | | |
|---|---|---|---|---|---|---|---|
| A | E357Y | E357K | S364L | S364K | F405K | Y407T | T411K |
| B | Y349E | K370E | L368S | K370S | K409F | T366Y | K370T |
| $S_{AA}$ | 8 | 4 | 8 | 0 | 0 | 10 | 13 |
| $S_{AB}$ | 71 | 80 | 61 | 90 | 70 | 83 | 60 |
| $S_{BB}$ | 21 | 16 | 31 | 10 | 30 | 7 | 27 |

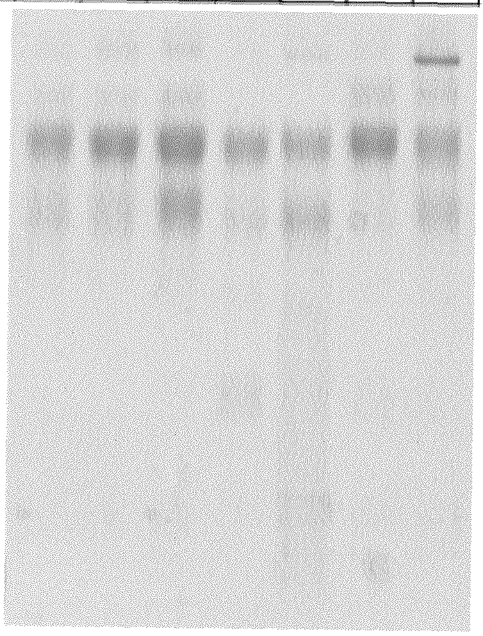

FIG. 5

| | | | |
|---|---|---|---|
| A | WT | K409W | K409W |
| B | WT | F405A | Y407A |
| $S_{AA}$ | 22 | 3 | 2 |
| $S_{AB}$ | 56 | 60 | 85 |
| $S_{BB}$ | 23 | 37 | 13 |

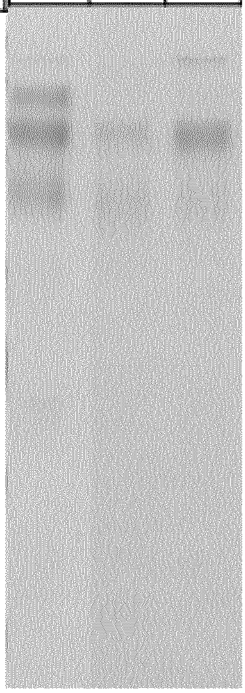

FIG. 6a

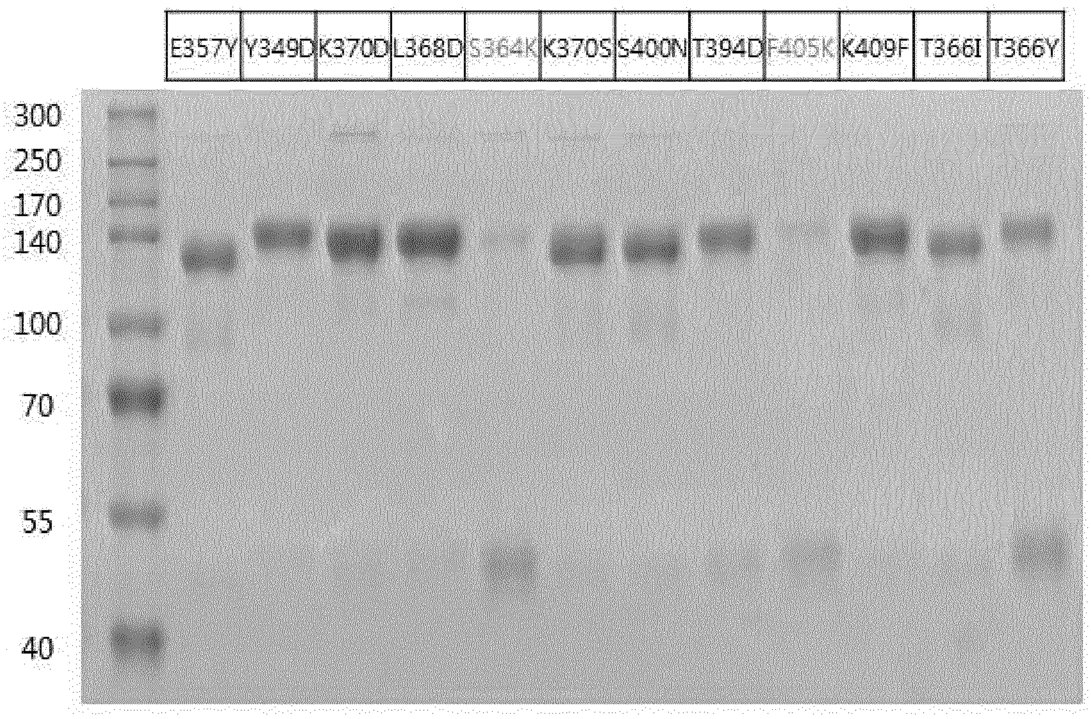
E357Y
Y349D
K370D
L368D
S364K
K370S
S400N
T394D
F405K
K409F
T366I
T366Y
300
250
170
140
100
70
55
40

FIG. 6b

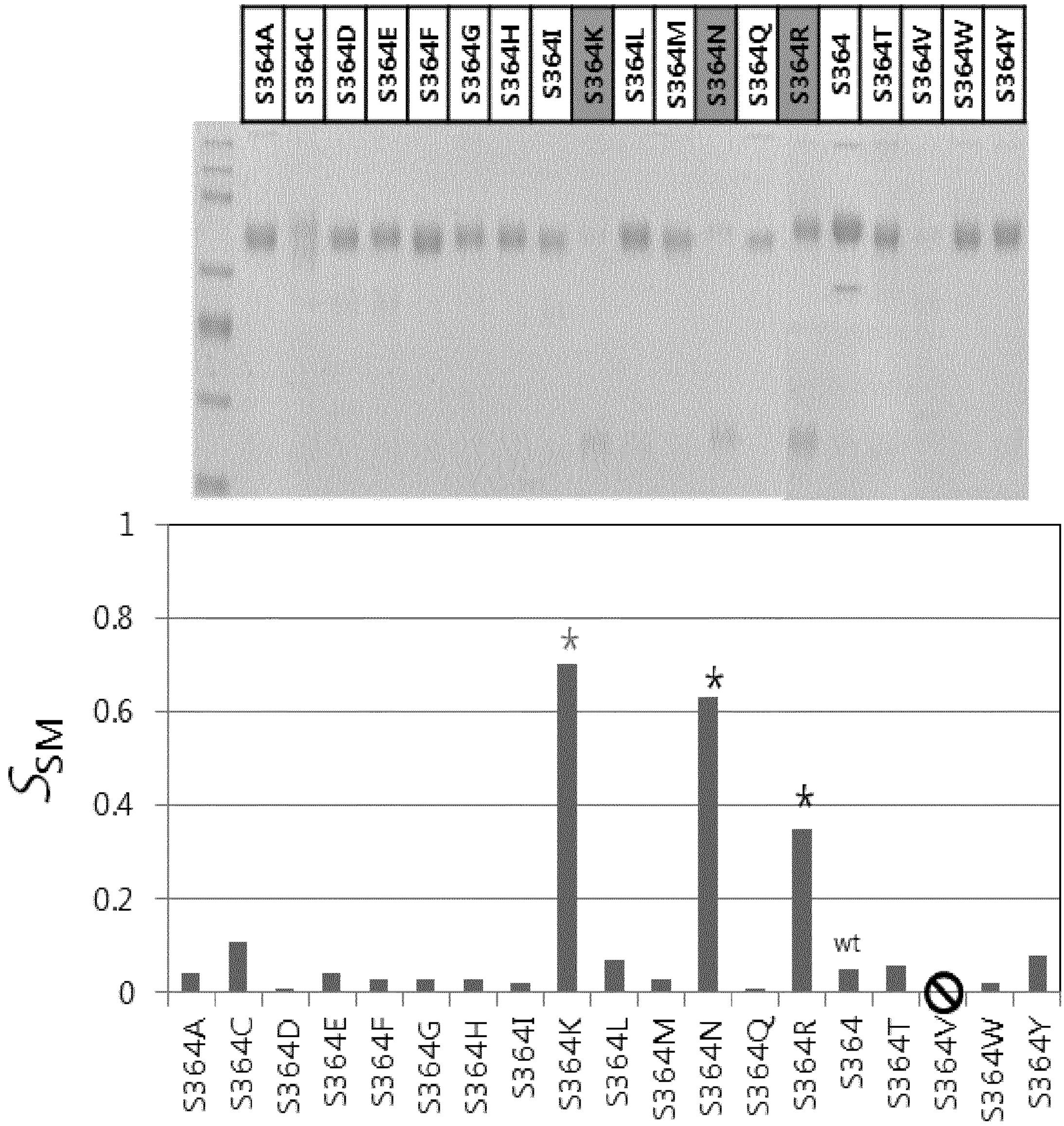
S364A
S364C
S364D
S364E
S364F
S364G
S364H
S364I
S364K
S364L
S364M
S364N
S364Q
S364R
S364
S364T
S364V
S364W
S364Y
1
0.8
0.6
0.4
0.2
0
$S_{SM}$
*
*
*
wt

FIG. 6c

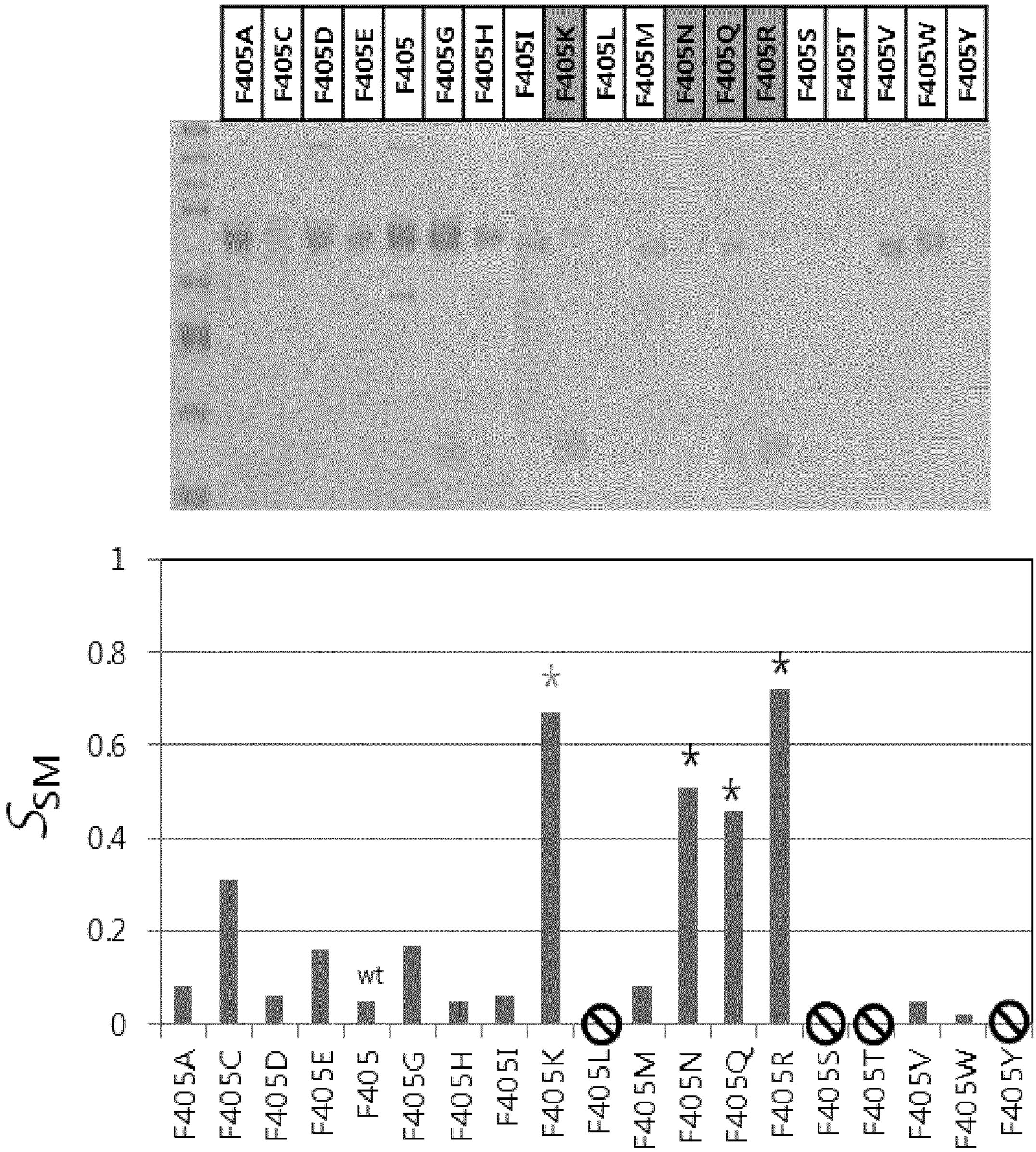
F405A
F405C
F405D
F405E
F405
F405G
F405H
F405I
F405K
F405L
F405M
F405N
F405Q
F405R
F405S
F405T
F405V
F405W
F405Y
1
0.8
0.6
0.4
0.2
0
$S_{SM}$
wt

FIG. 6d

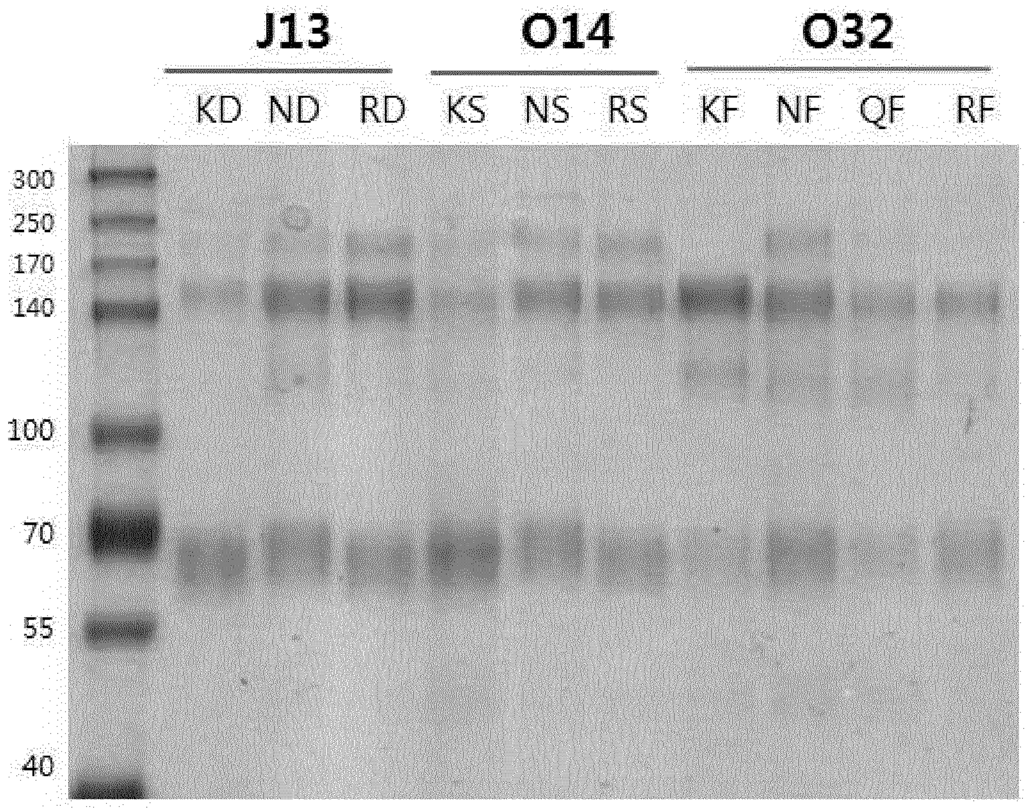
J13
O14
O32
KD
ND
RD
KS
NS
RS
KF
NF
QF
RF
300
250
170
140
100
70
55
40

FIG. 7

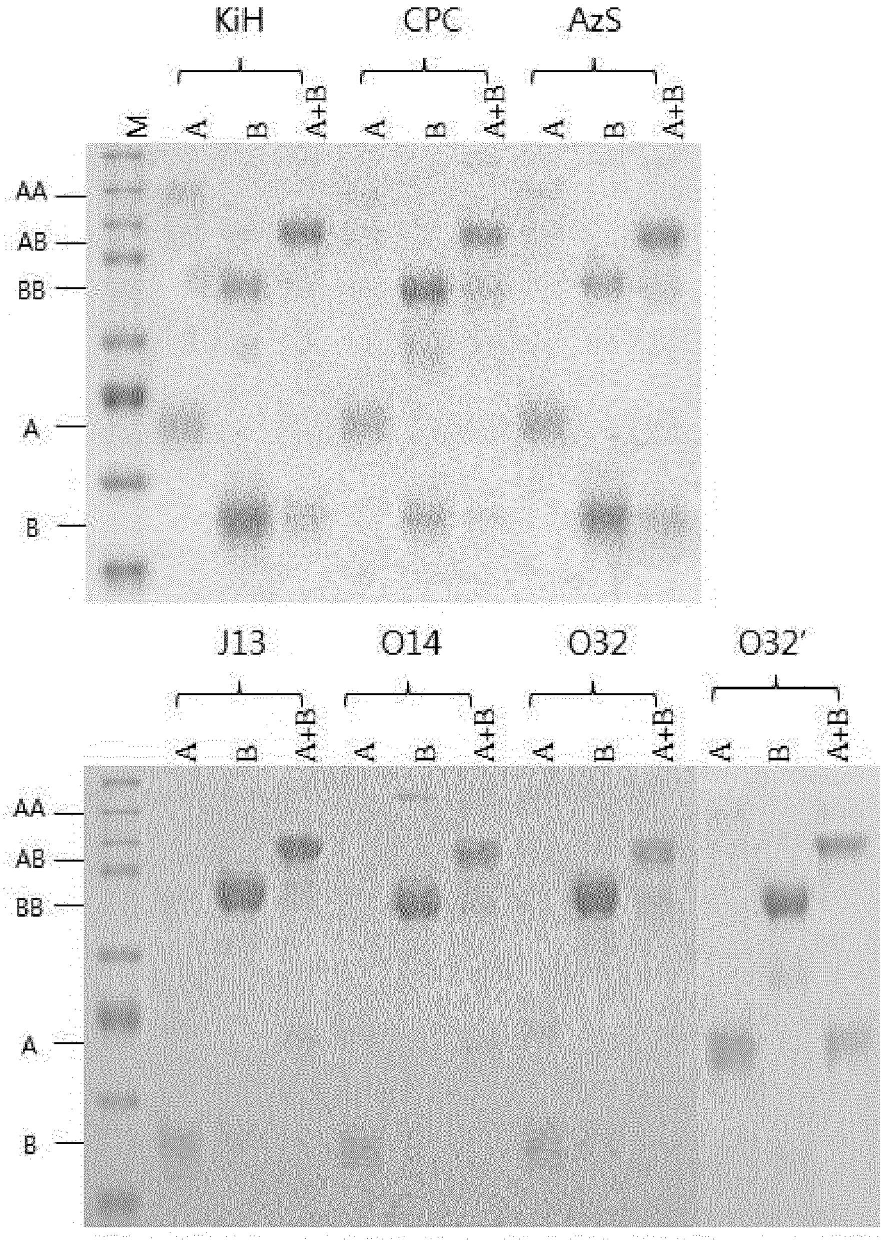

FIG. 8

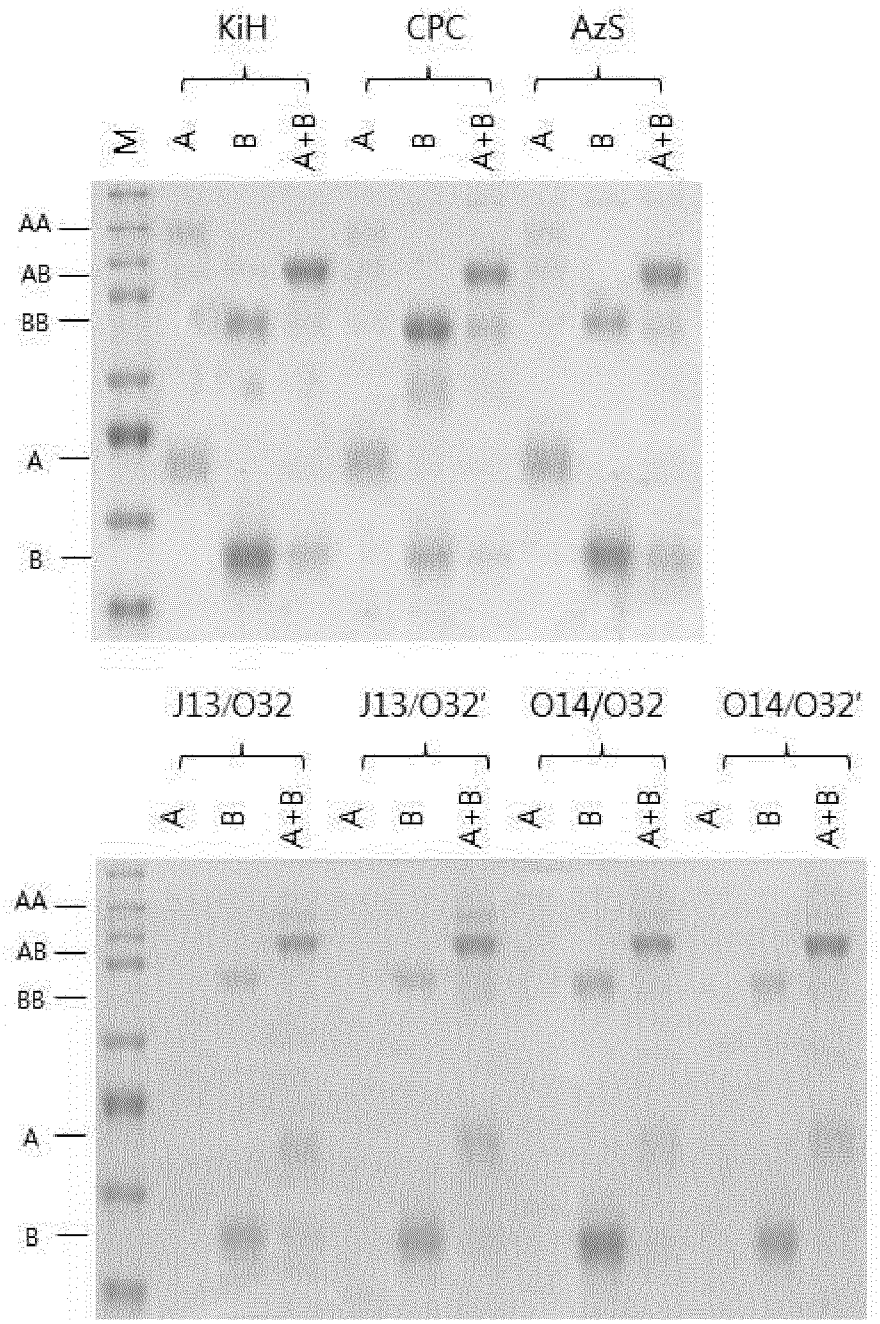

FIG. 9a

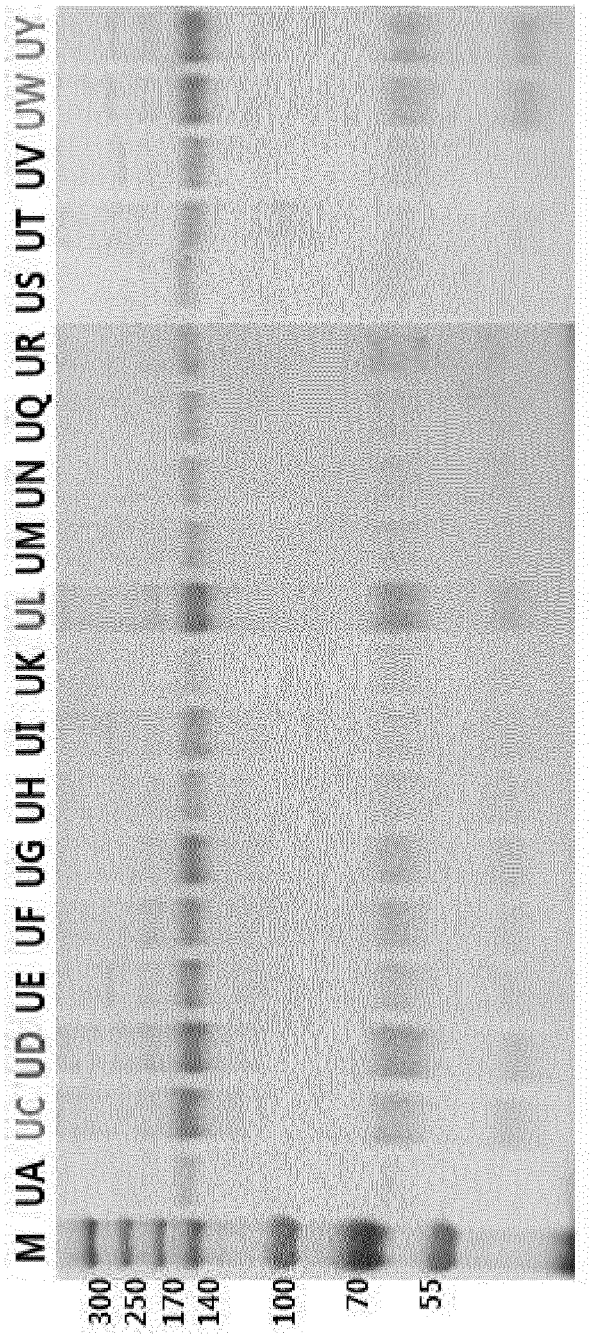
M
UA
UC
UD
UE
UF
UG
UH
UI
UK
UL
UM
UN
UQ
UR
US
UT
UV
UW
UY
300
250
170
140
100
70
55

FIG. 9b

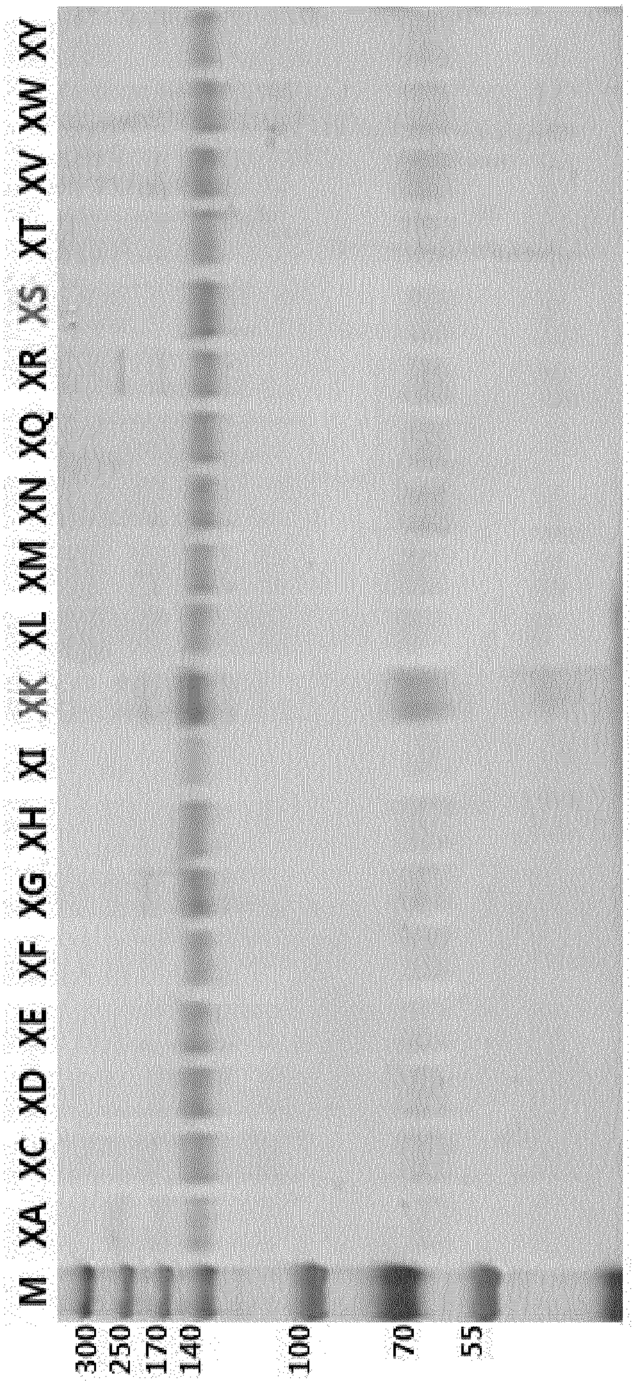
M
XA
XC
XD
XE
XF
XG
XH
XI
XK
XL
XM
XN
XQ
XR
XS
XT
XV
XW
XY
300
250
170
140
100
70
55

FIG. 9c

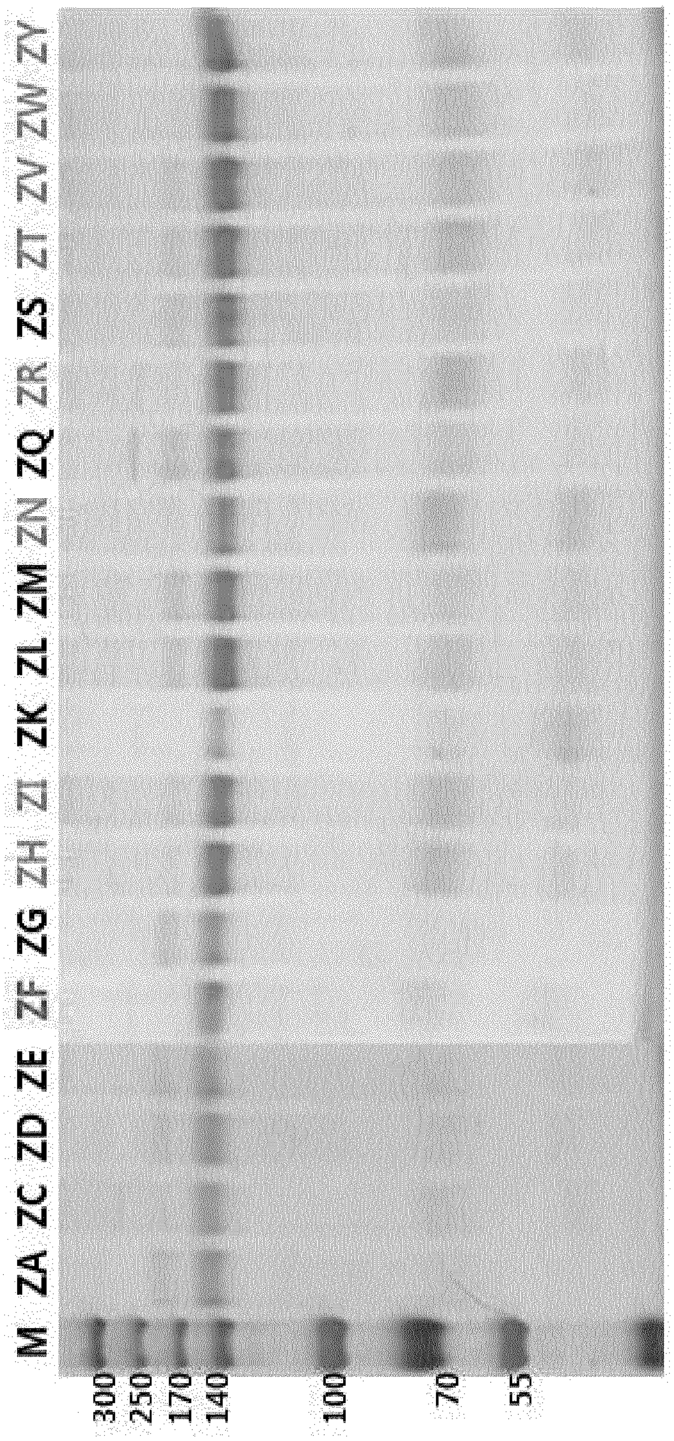
M
ZA
ZC
ZD
ZE
ZF
ZG
ZH
ZI
ZK
ZL
ZM
ZN
ZQ
ZR
ZS
ZT
ZV
ZW
ZY
300
250
170
140
100
70
55

FIG. 10

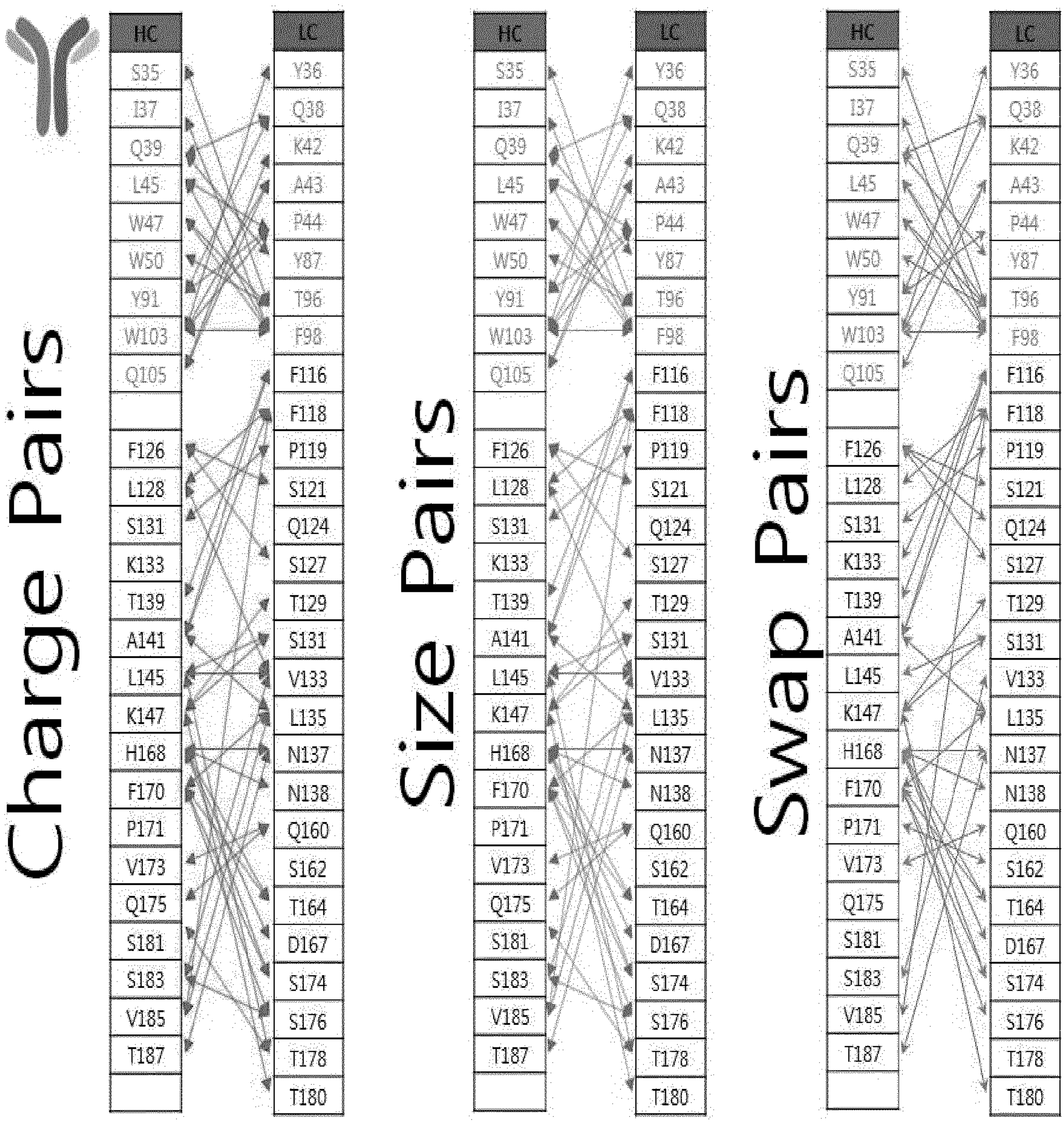
Charge Pairs
Size Pairs
Swap Pairs
HC
S35 I37 Q39 L45 W47 W50 Y91 W103 Q105 F126 L128 S131 K133 T139 A141 L145 K147 H168 F170 P171 V173 Q175 S181 S183 V185 T187
LC
Y36 Q38 K42 A43 P44 Y87 T96 F98 F116 F118 P119 S121 Q124 S127 T129 S131 V133 L135 N137 N138 Q160 S162 T164 D167 S174 S176 T178 T180

FIG. 11

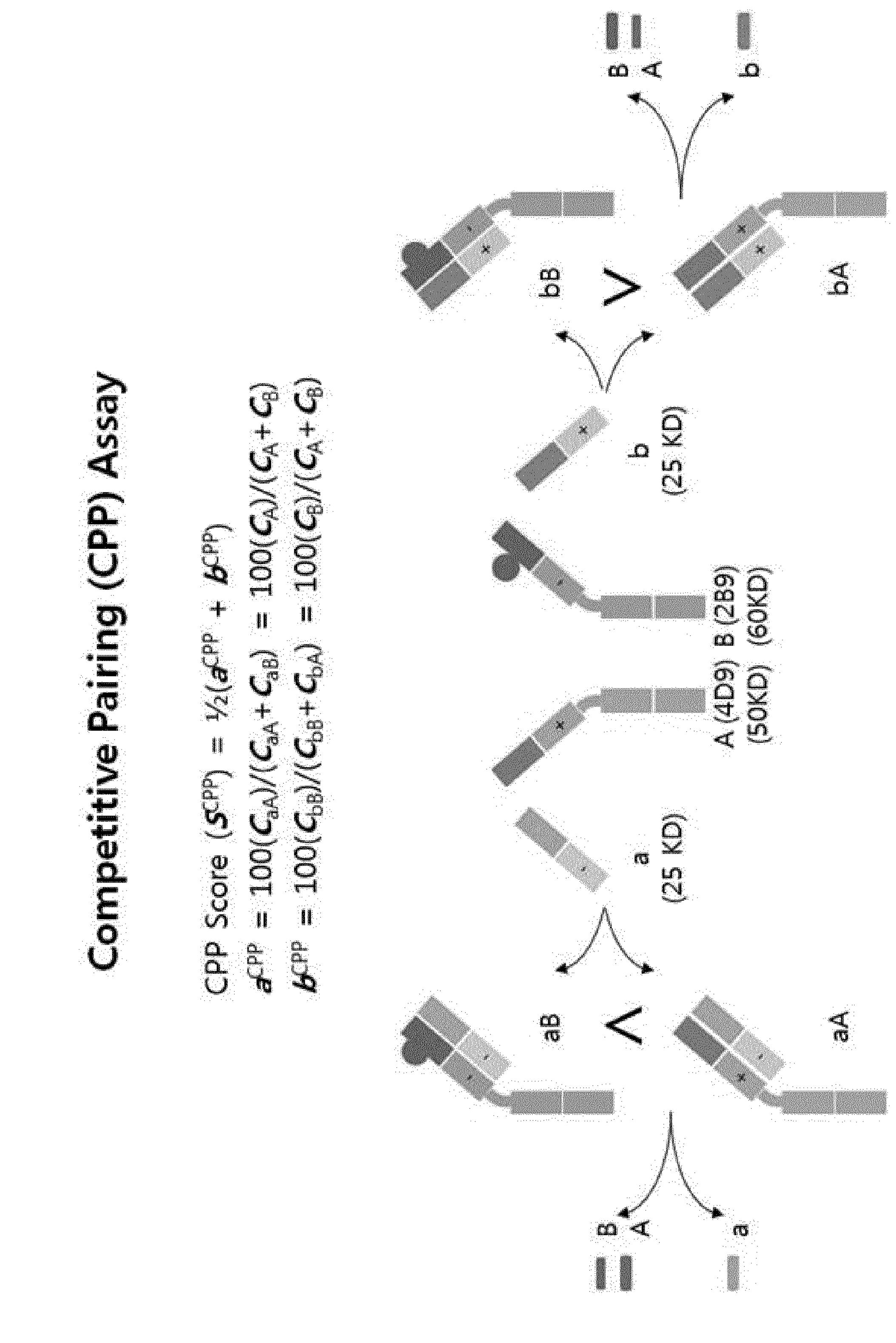
Competitive Pairing (CPP) Assay
CPP Score ($S^{CPP}$) = ½($a^{CPP}$ + $b^{CPP}$)
$a^{CPP}$ = 100($C_{aA}$)/($C_{aA}$+$C_{aB}$) = 100($C_A$)/($C_A$+$C_B$)
$b^{CPP}$ = 100($C_{bB}$)/($C_{bB}$+$C_{bA}$) = 100($C_B$)/($C_A$+$C_B$)
aB
aA
a
(25 KD)
A (4D9)
(50KD)
B (2B9)
(60KD)
b
(25 KD)
bB
bA
B
A
a
b

FIG. 12

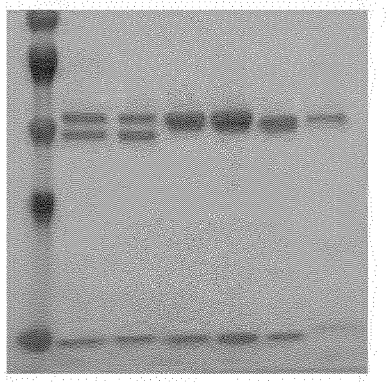


- CPP Score ($S^{CPP}$) = $\frac{1}{2}(a^{CPP} + b^{CPP})$

$$a^{CPP} = 100(C_{aA})/(C_{aA}+C_{aB}) = 100(C_A)/(C_A+C_B)$$

$$b^{CPP} = 100(C_{bB})/(C_{bB}+C_{bA}) = 100(C_B)/(C_A+C_B)$$

$$S^{CPP} = \begin{cases} 100 & \text{for positively productive pairing} \\ 0 & \text{for nonproductive or random pairing} \\ -100 & \text{for negatively productive pairing} \end{cases}$$

| | a | A (4D9) | B (2B9) | b | $a^{CPP}$ | $b^{CPP}$ | $S^{CPP}$ |
|---|---|---|---|---|---|---|---|
| C1 | A1R/Q38D/ L135Y/S176W | Q39K/S62E/ H172A/F174G | Q39Y/S62K | A1D/Q38R | 0 | 0 | 0 |
| C2 | S121C/C214V | F126C/C220V | - | - | -100 | 100 | 0 |
| C3 | Q38D/A43D/ S176K | Q39K/Q105K/ S183D | Q39D/Q105D/ S183K | Q38K/A43K/ S176D | -100 | 100 | 0 |

FIG. 13

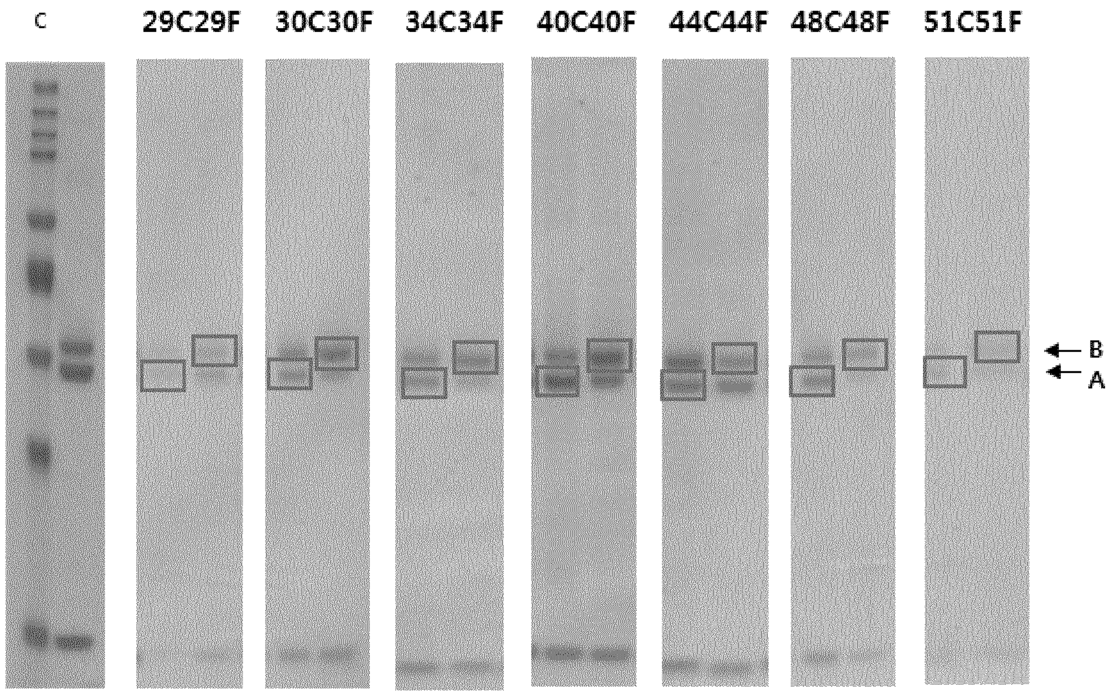

FIG. 14

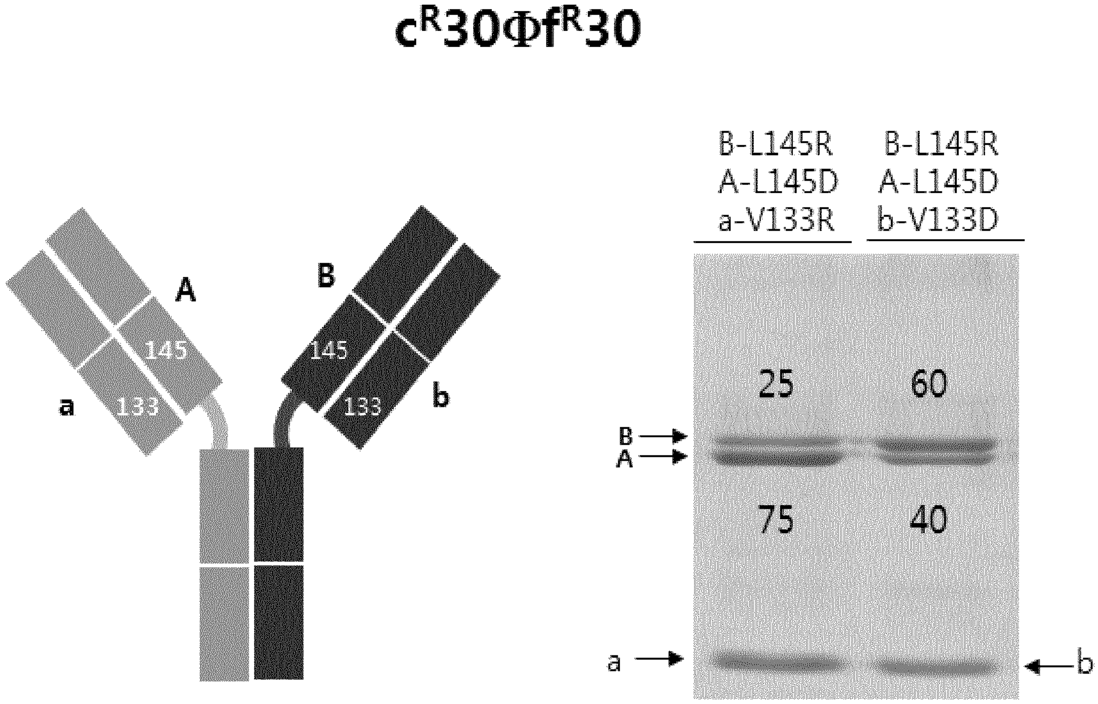
c^R30Φf^R30
A
B
145
145
a
133
133
b
B-L145R
A-L145D
a-V133R
B-L145R
A-L145D
b-V133D
25
60
B
A
75
40
a
b

FIG. 15

## $c29c^{R}30\Phi f^{R}30$

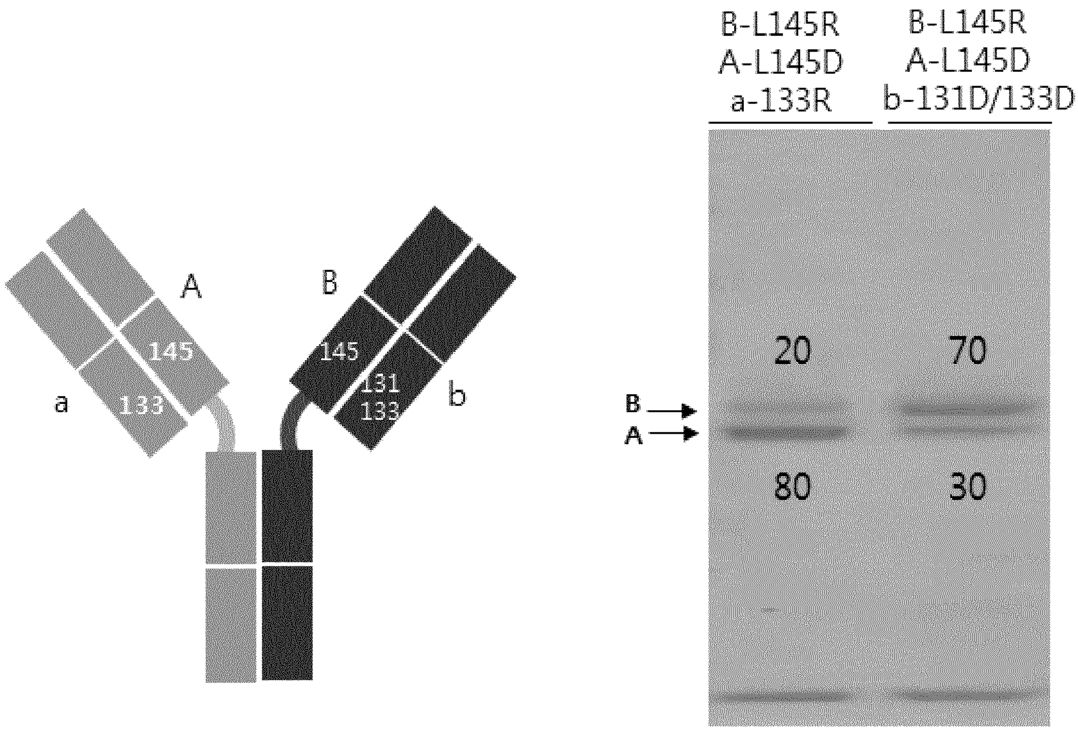

FIG. 16

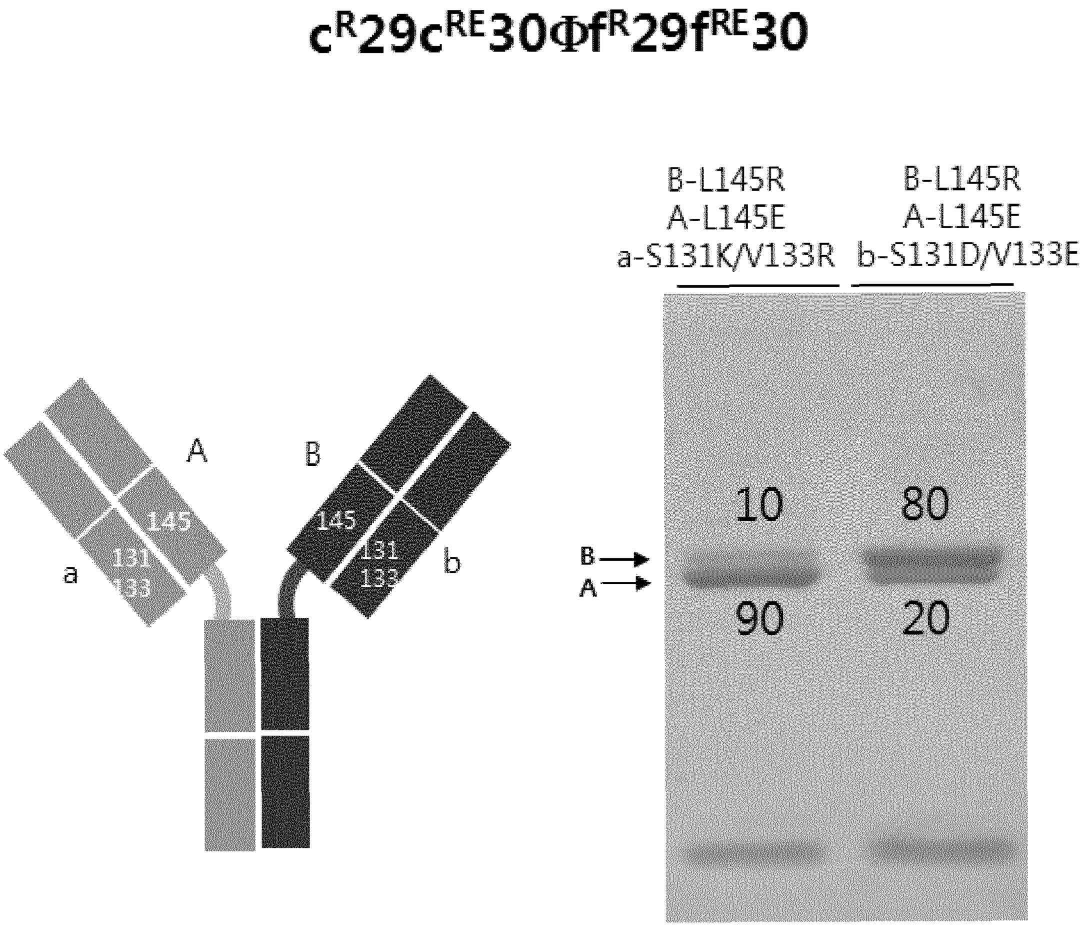
$c^R29c^{RE}30\Phi f^R29f^{RE}30$
A
B
a
b
145
131
133
145
131
133
B-L145R
A-L145E
a-S131K/V133R
B-L145R
A-L145E
b-S131D/V133E
10
80
90
20
B
A

FIG. 17

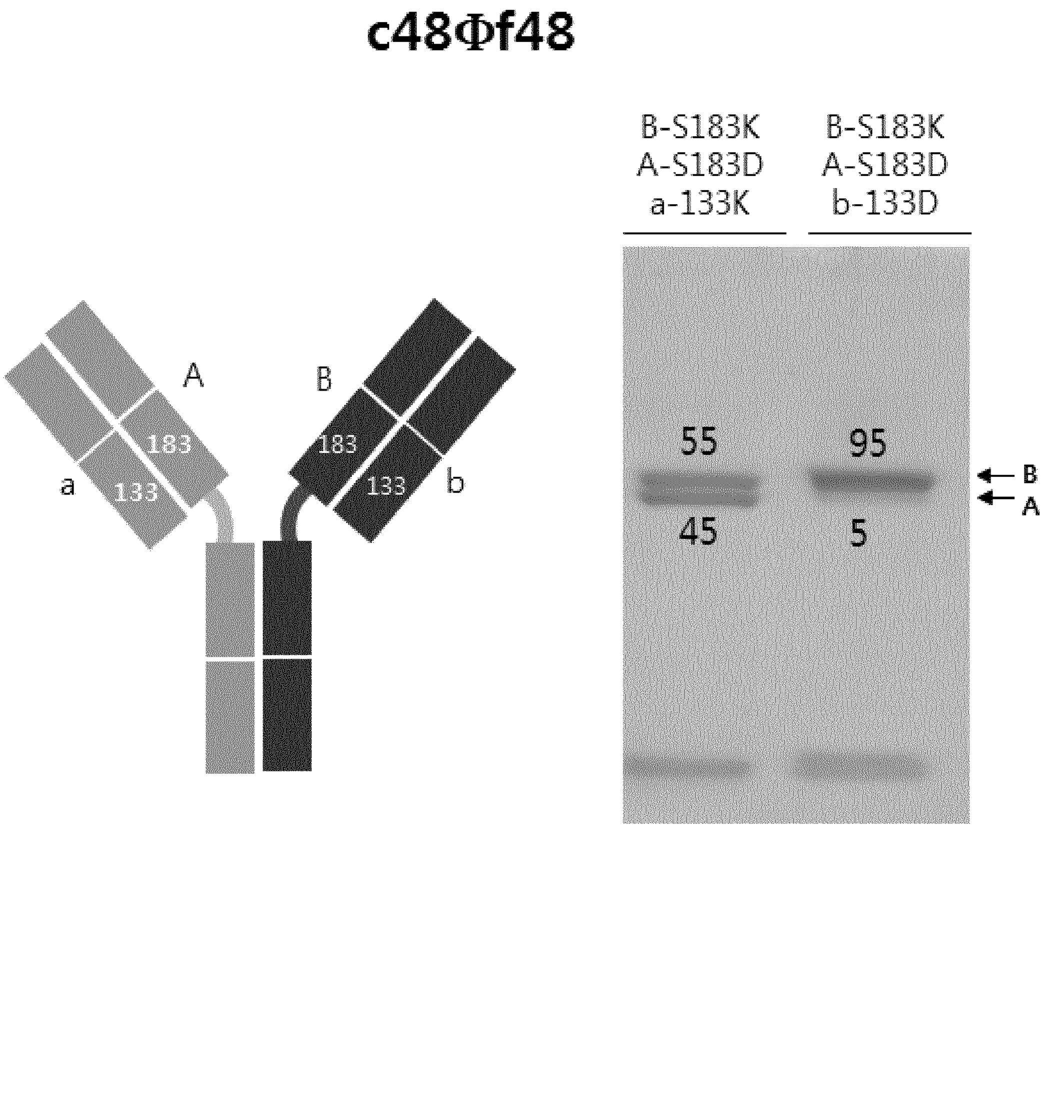
c48Φf48
A
B
a
b
183
133
183
133
B-S183K
A-S183D
a-133K
B-S183K
A-S183D
b-133D
55
45
95
5
B
A

FIG. 18

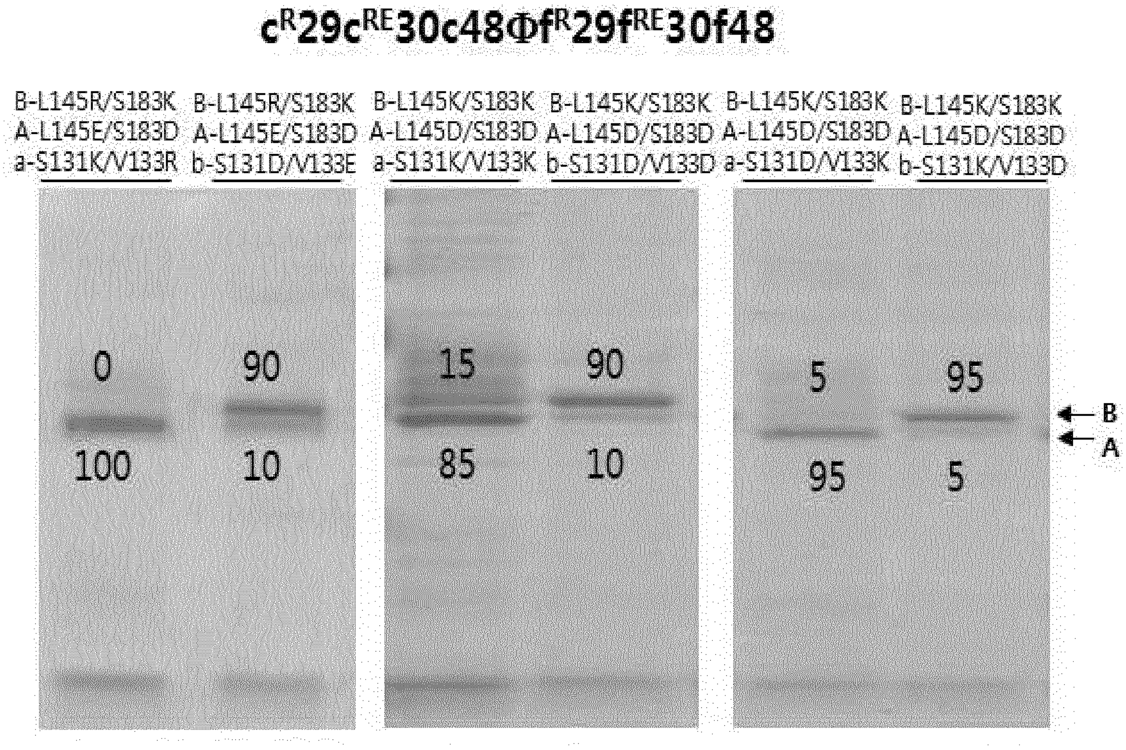

$c^R29c^{RE}30c48\Phi f^R29f^{RE}30f48$
B-L145R/S183K
A-L145E/S183D
a-S131K/V133R
B-L145R/S183K
A-L145E/S183D
b-S131D/V133E
B-L145K/S183K
A-L145D/S183D
a-S131K/V133K
B-L145K/S183K
A-L145D/S183D
b-S131D/V133D
B-L145K/S183K
A-L145D/S183D
a-S131D/V133K
B-L145K/S183K
A-L145D/S183D
b-S131K/V133D
0
90
15
90
5
95
100
10
85
10
95
5
B
A

FIG. 19

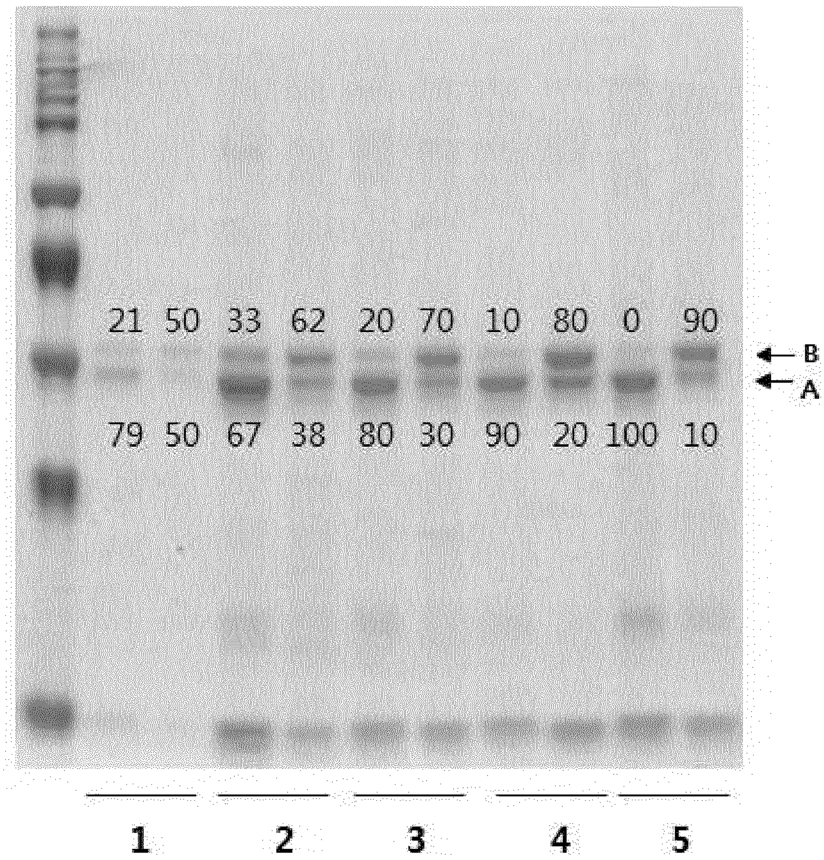

FIG.20

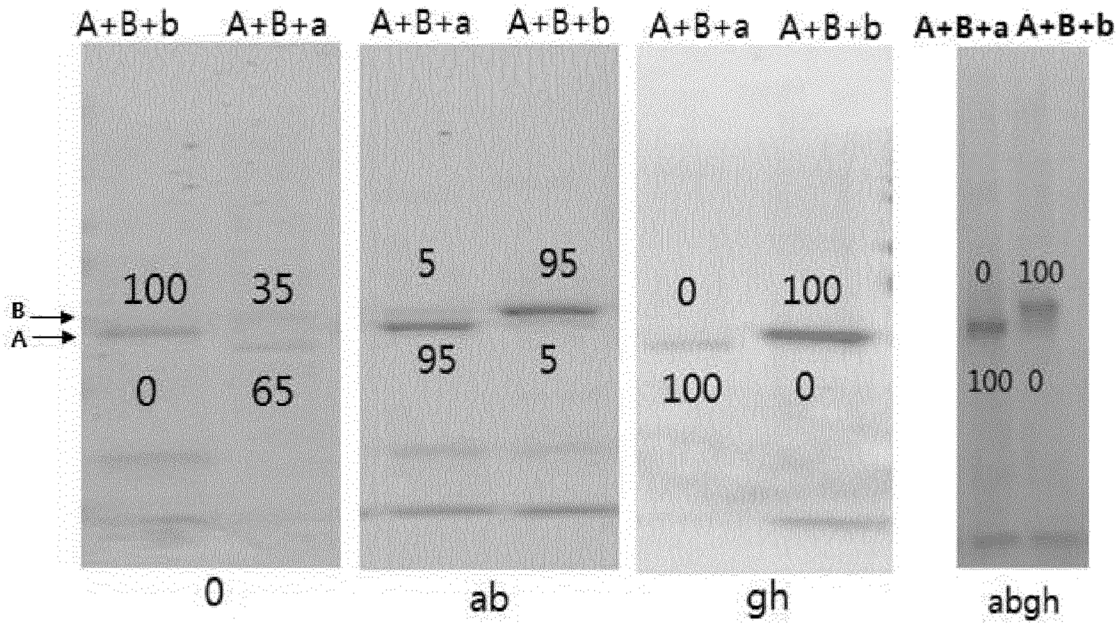
A+B+b
A+B+a
A+B+a
A+B+b
A+B+a
A+B+b
A+B+a
A+B+b
100
35
5
95
0
100
0
100
B
A
0
65
95
5
100
0
100
0
0
ab
gh
abgh

FIG. 21

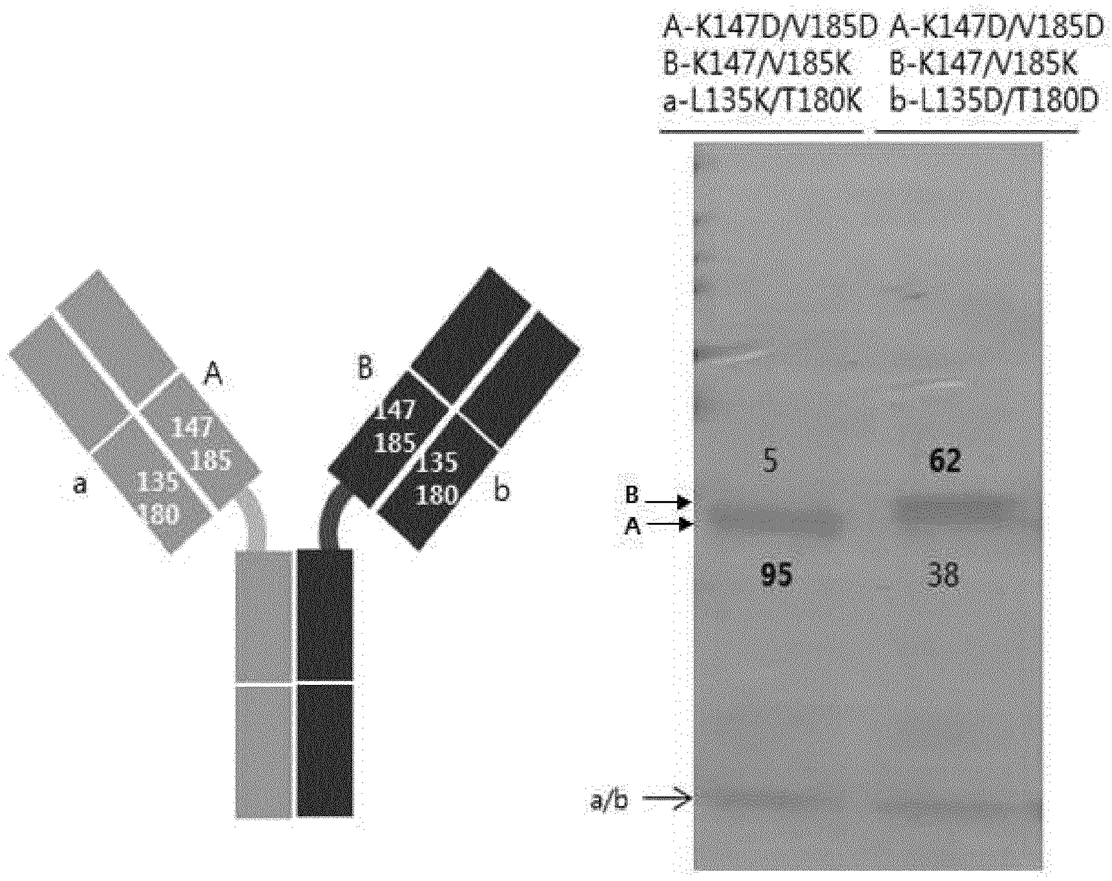
A
B
147
185
147
185
a
135
180
135
180
b
A-K147D/V185D
B-K147/V185K
a-L135K/T180K
A-K147D/V185D
B-K147/V185K
b-L135D/T180D
B
A
5
62
95
38
a/b

FIG. 22

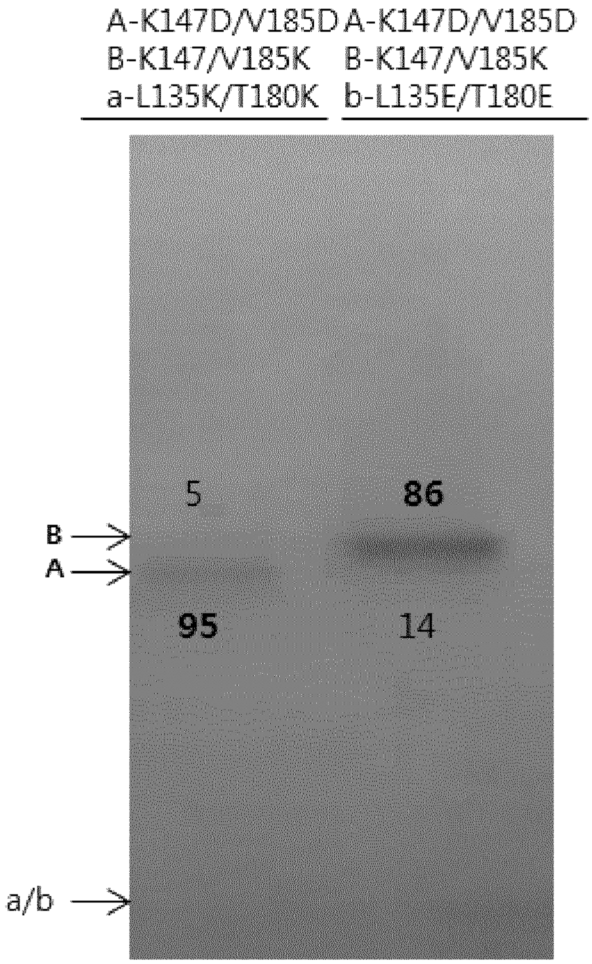
A-K147D/V185D
B-K147/V185K
a-L135K/T180K
A-K147D/V185D
B-K147/V185K
b-L135E/T180E
5
86
B
A
95
14
a/b

FIG. 23

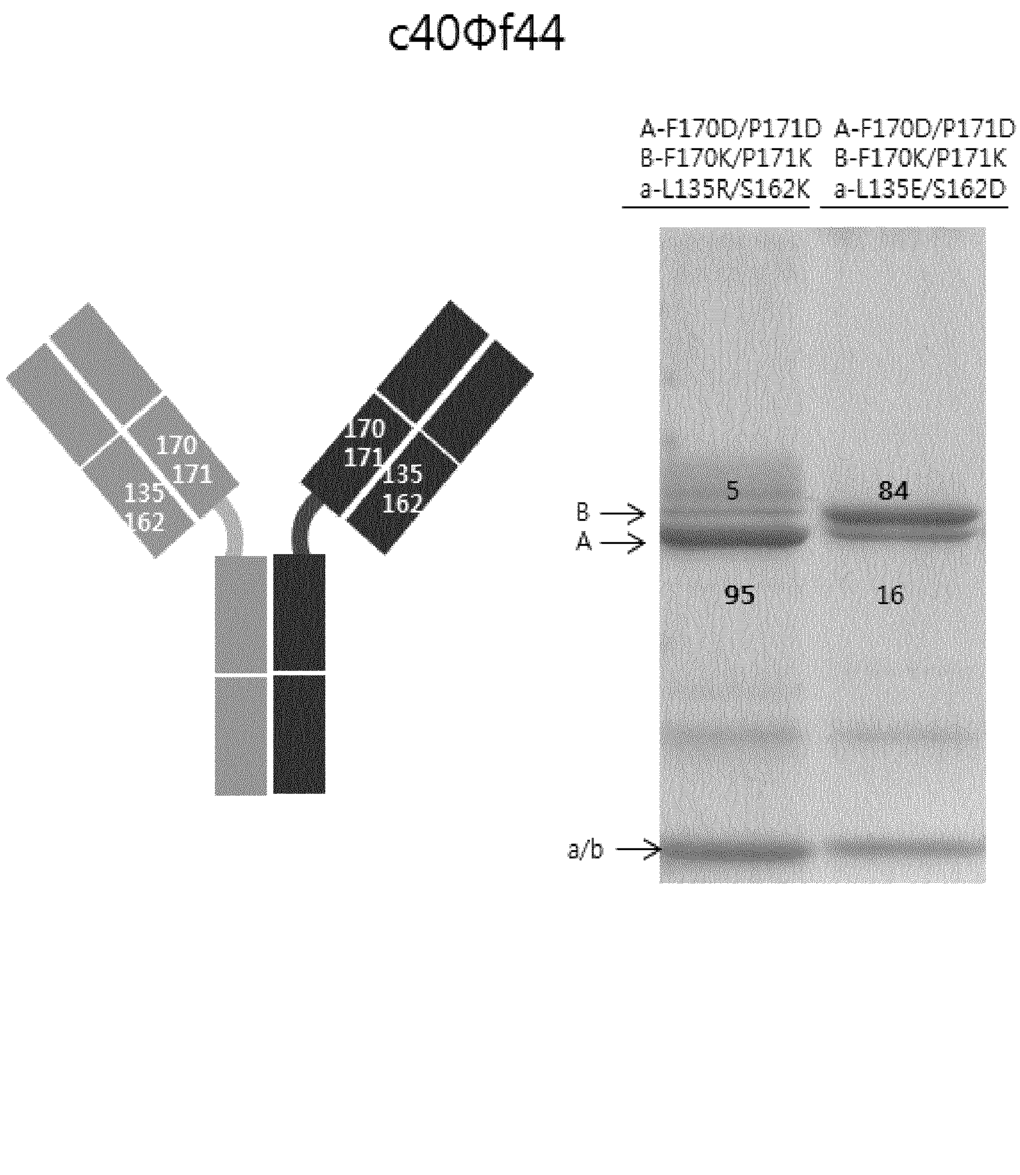
c40Φf44
170
171
135
162
170
171
135
162
A-F170D/P171D
B-F170K/P171K
a-L135R/S162K
A-F170D/P171D
B-F170K/P171K
a-L135E/S162D
5
84
B
A
95
16
a/b

FIG. 24

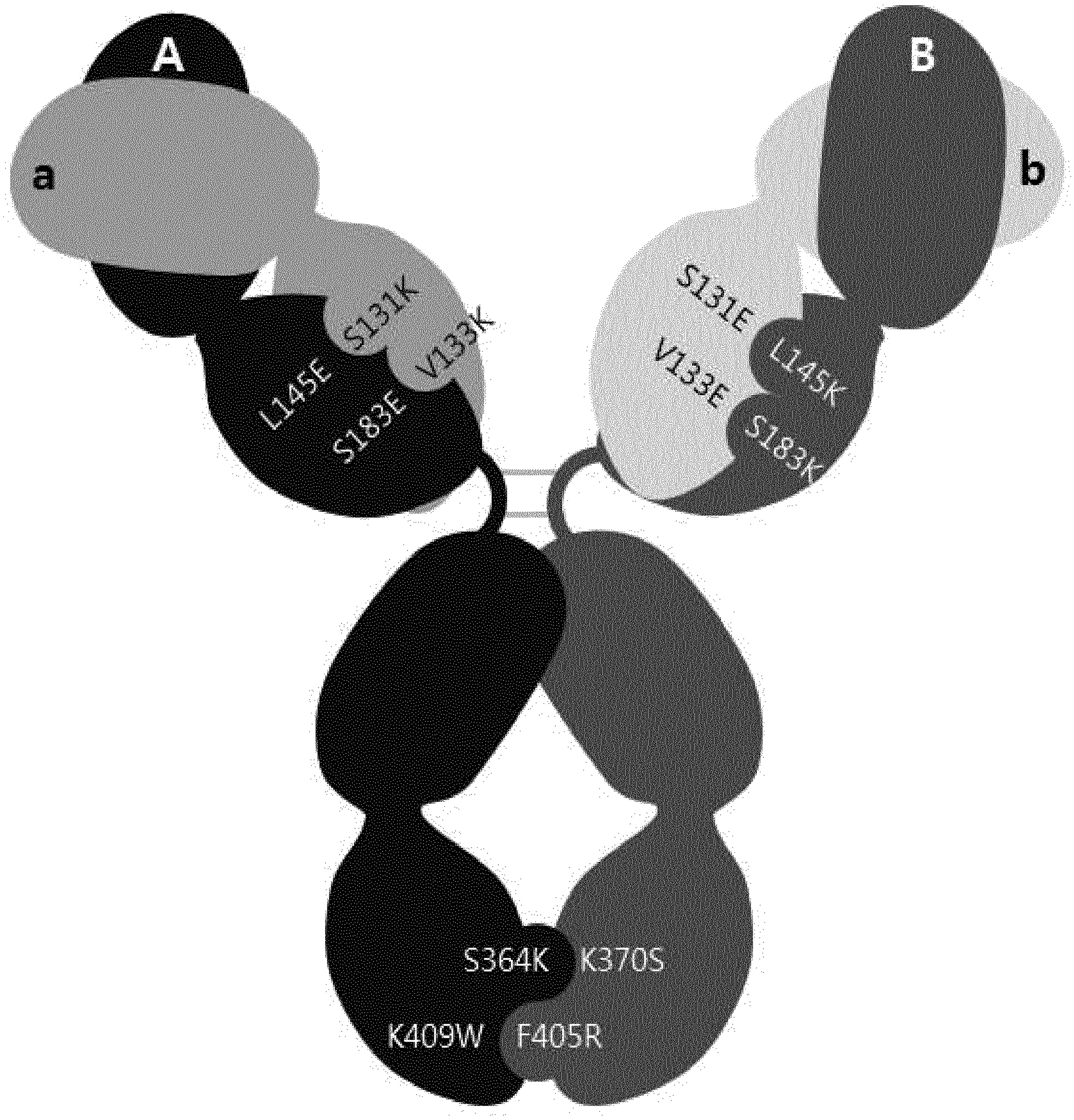
A
B
a
b
S131K
V133K
L145E
S183E
S131E
V133E
L145K
S183K
S364K
K370S
K409W
F405R

FIG. 25

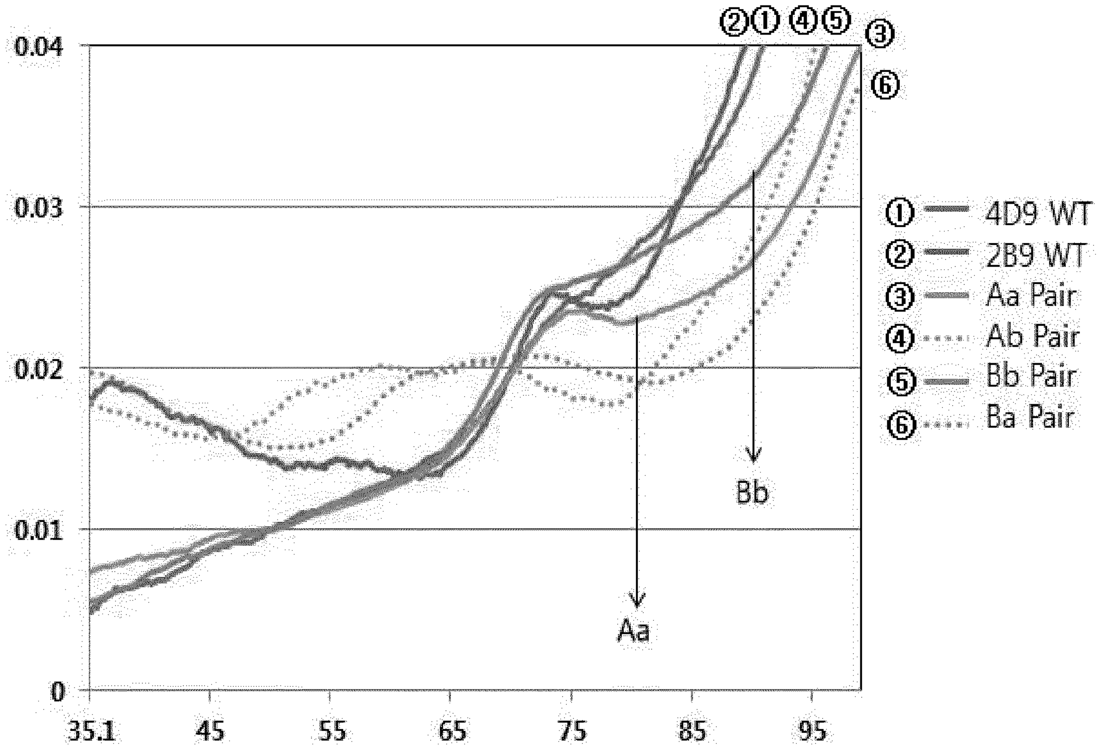
②①④⑤
③
⑥
0.04
0.03
0.02
0.01
0
35.1
45
55
65
75
85
95
Bb
Aa
① 4D9 WT
② 2B9 WT
③ Aa Pair
④ Ab Pair
⑤ Bb Pair
⑥ Ba Pair

FIG. 26

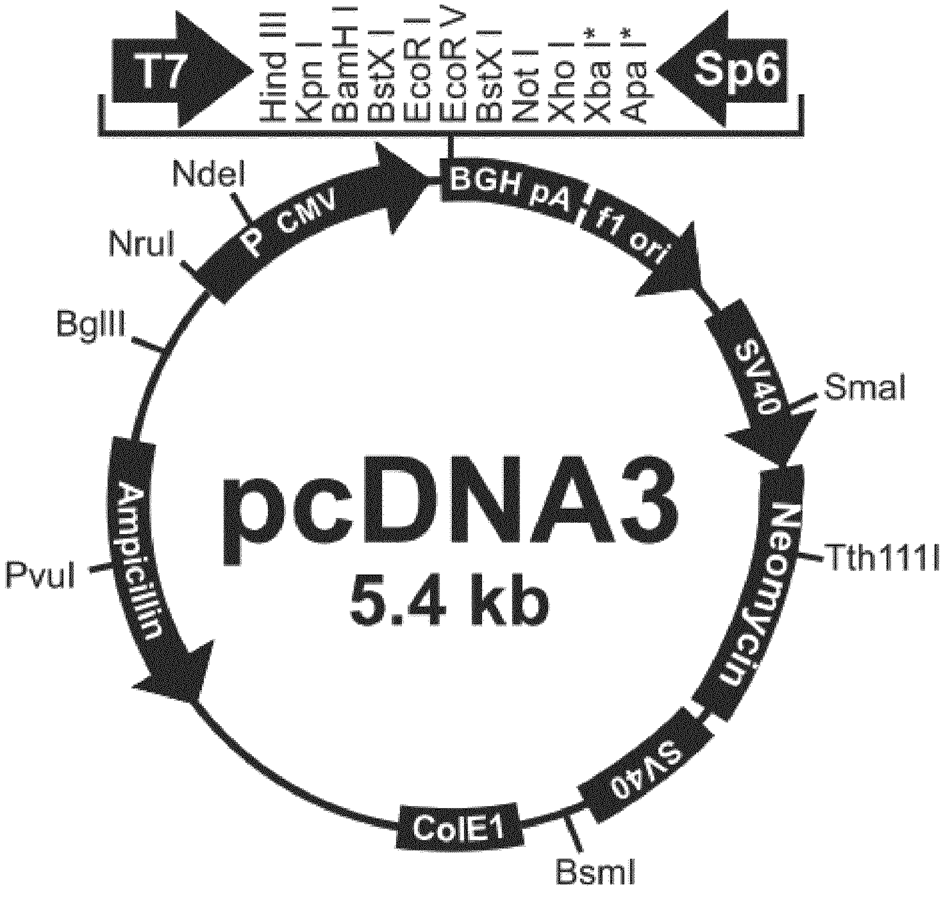
T7
Hind III
Kpn I
BamH I
BstX I
EcoR I
EcoR V
BstX I
Not I
Xho I
Xba I*
Apa I*
Sp6
NdeI
NruI
BglII
P CMV
BGH pA
f1 ori
SV40
SmaI
pcDNA3
5.4 kb
Neomycin
Tth111I
Ampicillin
PvuI
SV40
ColE1
BsmI

FIG. 27

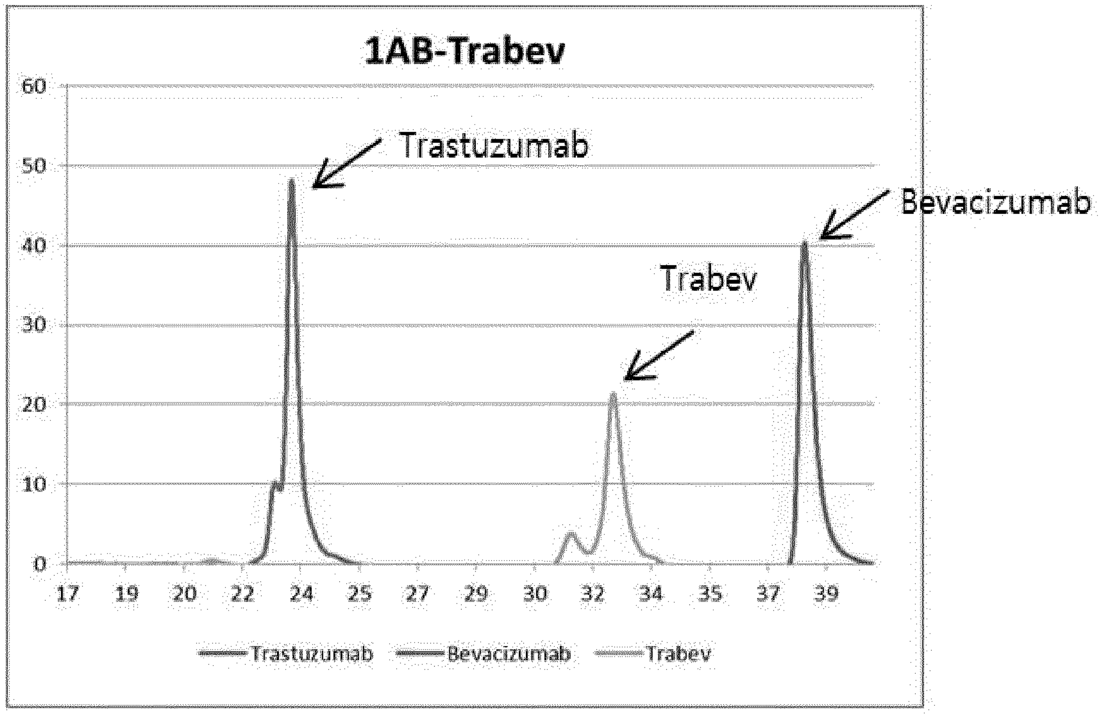
1AB-Trabev
60
50
40
30
20
10
0
17 19 20 22 24 25 27 29 30 32 34 35 37 39
Trastuzumab
Bevacizumab
Trabev
Trastuzumab
Bevacizumab
Trabev

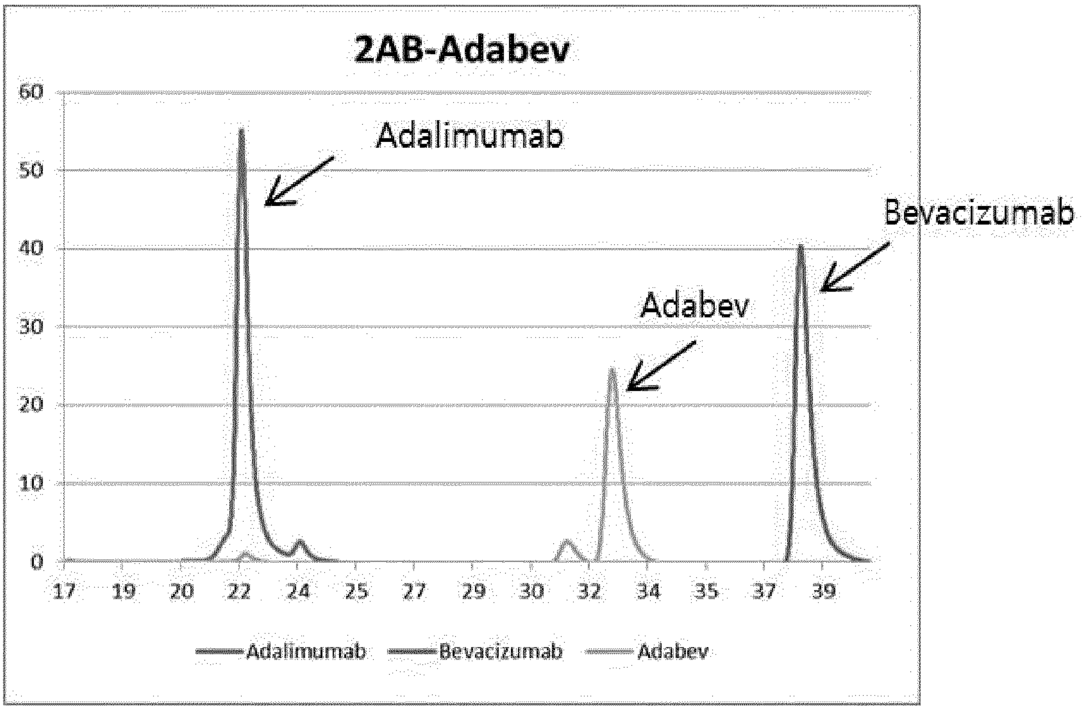
2AB-Adabev
60
50
40
30
20
10
0
17 19 20 22 24 25 27 29 30 32 34 35 37 39
Adalimumab
Bevacizumab
Adabev
Adalimumab
Bevacizumab
Adabev

FIG. 28

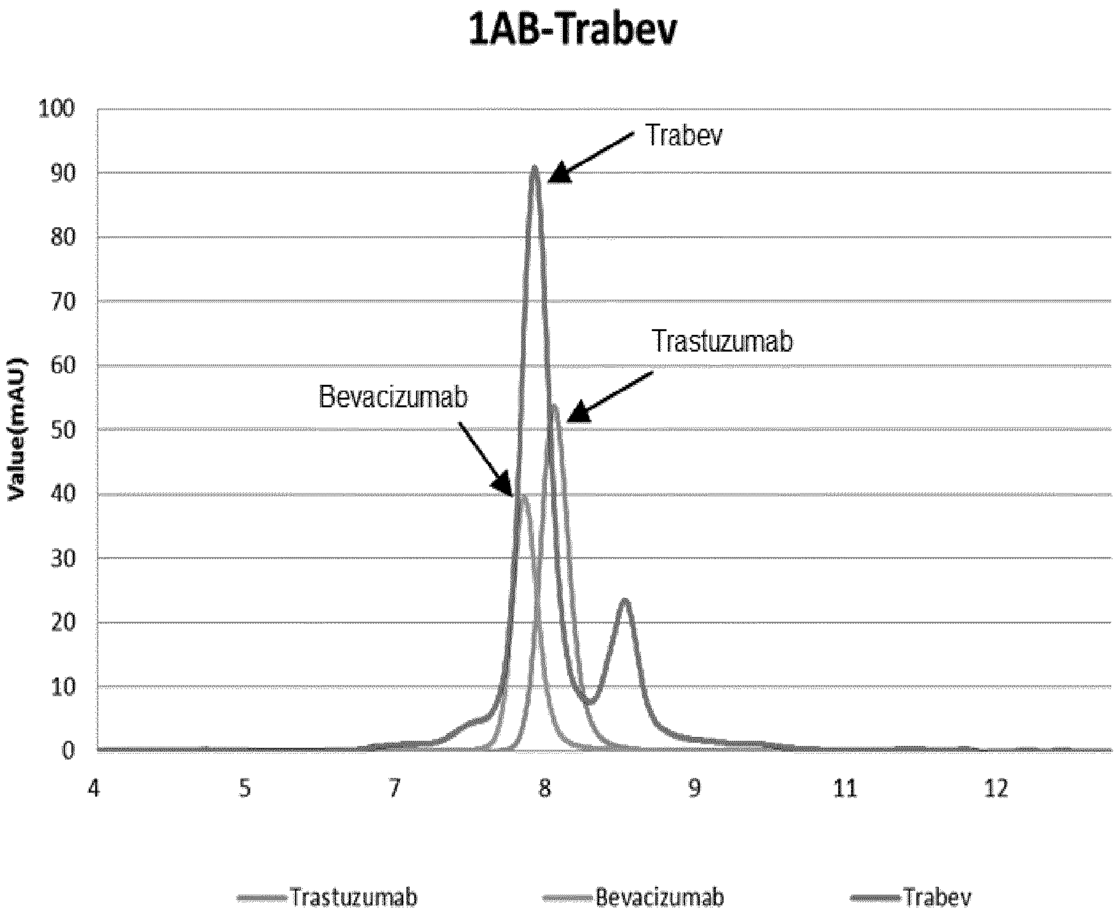
1AB-Trabev
Value(mAU)
100
90
80
70
60
50
40
30
20
10
0
4
5
7
8
9
11
12
Trabev
Trastuzumab
Bevacizumab
Trastuzumab
Bevacizumab
Trabev

FIG. 29

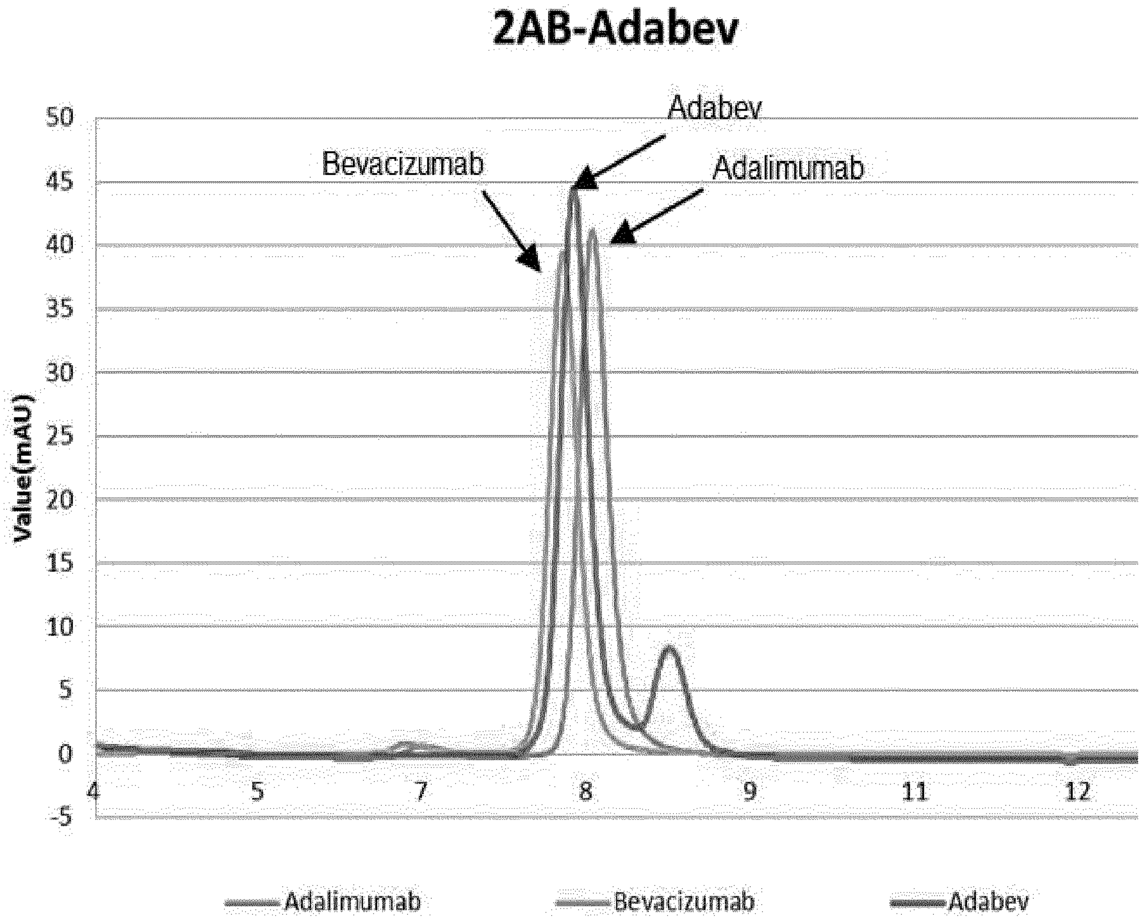
2AB-Adabev
Adabev
Bevacizumab
Adalimumab
Value(mAU)
50
45
40
35
30
25
20
15
10
5
0
-5
4
5
7
8
9
11
12
Adalimumab
Bevacizumab
Adabev

FIG. 30

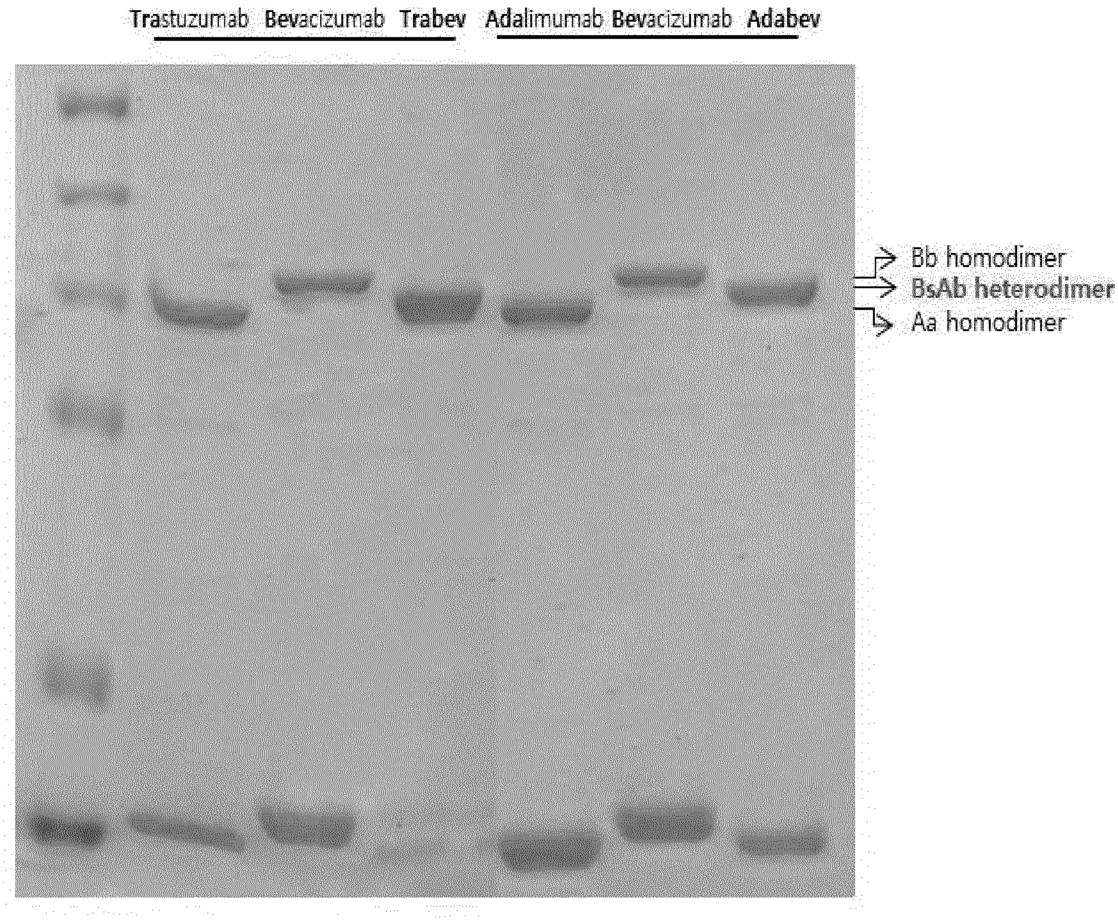
Trastuzumab
Bevacizumab
Trabev
Adalimumab
Bevacizumab
Adabev
Bb homodimer
BsAb heterodimer
Aa homodimer

FIG. 31

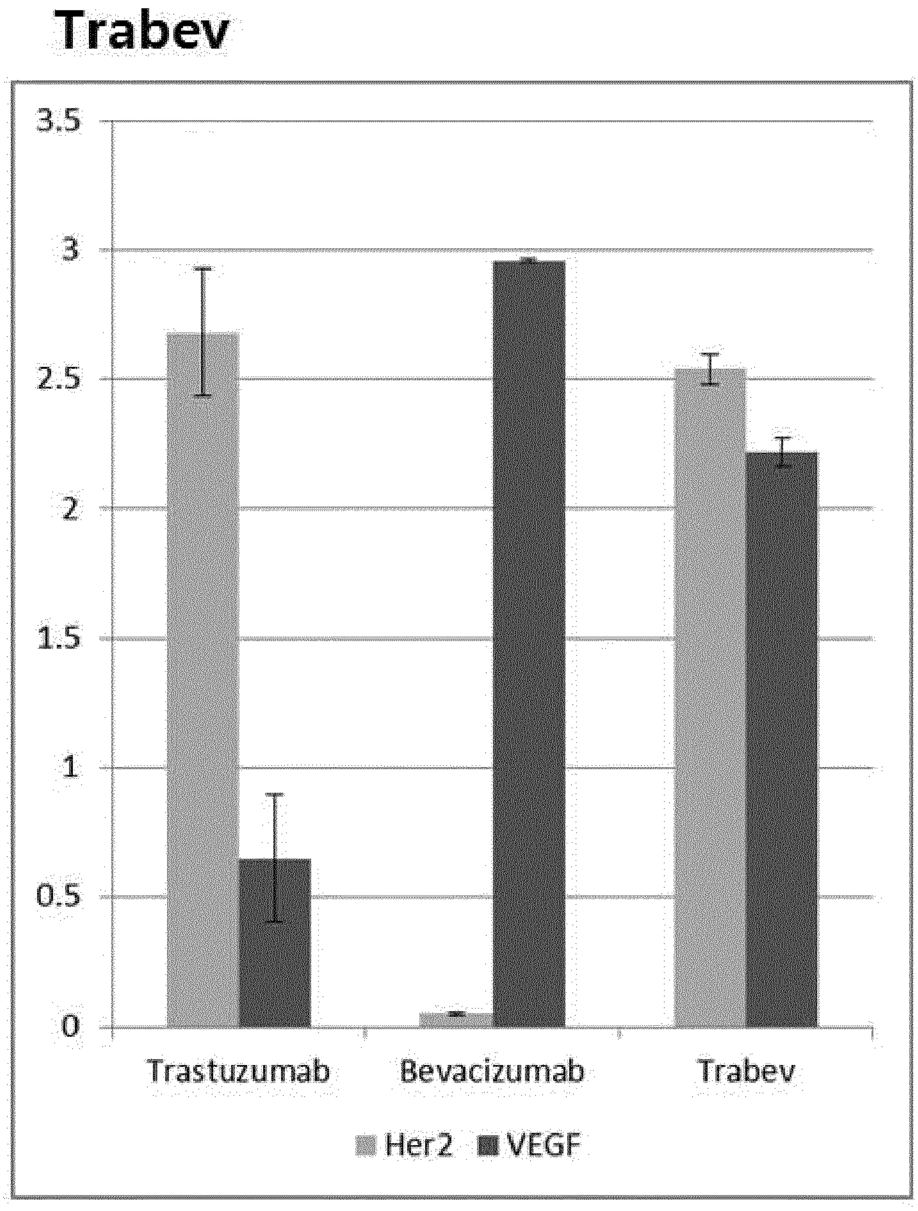
Trabev
3.5
3
2.5
2
1.5
1
0.5
0
Trastuzumab
Bevacizumab
Trabev
Her2
VEGF

FIG. 32

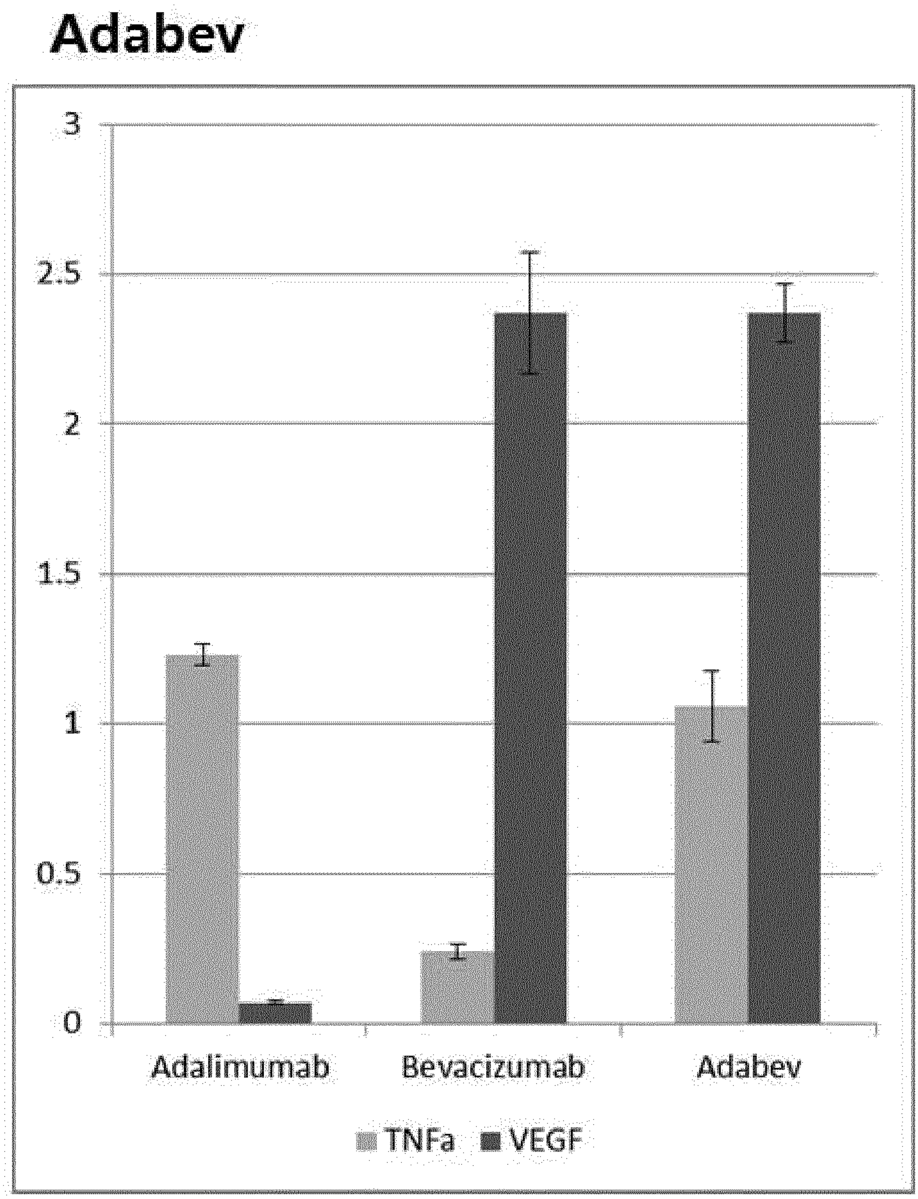
Adabev
3
2.5
2
1.5
1
0.5
0
Adalimumab
Bevacizumab
Adabev
TNFa
VEGF

FIG. 33a

Query : Human IgG1 Heavy Constant

Subject : Human IgA1 Heavy Constant

| Score | Expect | Method | Identities | Positives | Gaps |
|---|---|---|---|---|---|
| 125 bits(315) | 7e-38 | Compositional matrix adjust. | 108/341(32%) | 159/341(46%) | 17/341(4%) |

```
Query  1     ASTKGPSVFPLAPSSKSTSGGTAALGCLVKDYFP-EPVTVSWNSGALTSGVHTFPAVLQS  59
             AS   P VFPL+  S +   G   + CLV+ +FP EP++V+W+          FP    +
Sbjct  1     ASPTSPKVFPLSLCS-TQPDGNVVIACLVQGFFPQEPLSVTWSESGQGVTARNFPPSQDA  59

Query  60    SG-LYSLSSVVTVPSSS-LGTQTYICNVNH--KPSNTKVDKKVEPKSCDKTHTCPPCPAP  115
             SG LY+ SS +T+P++  L  ++  C+V H   PS         P +        P
Sbjct  60    SGDLYTTSSQLTLPATQCLAGKSVTCHVKHYTNPSQDVTVPCPVPSTPPTPSPSTPPTPS  119

Query  116   ELLGGPSVFLFPPKPKDTLMISRTPEVTCVVVDVSHEDPEVKFNWYVDGVEVHNAKTKPR  175
                  P + L  P  +D L+ S    +TC +  +  +   V F W     +  +A   P
Sbjct  120   PSCCHPRLSLHRPALEDLLLGSEA-NLTCTLTGL-RDASGVTFTWTPSSGK--SAVQGPP  175

Query  176   EEQYNSTYRVVSVLTVLHQDWLNGKEYKCKVSNKALPAPIEKTISKAKGQPREPQVYTLP  235
             E      Y V SVL    + W +GK + C  +      P+  T+SK+ G    P+V+ LP
Sbjct  176   ERDLCGCYSVSSVLPGCAEPWNHGKTFTCTAAYPESKTPLTATLSKS-GNTFRPEVHLLP  234

Query  236   PSRDELTKNQ-VSLTCLVKGFYPSDIAVEWESNGQ--PENNYKTTPPVLD-SDG--SFFL  289
             P  +EL  N+ V+LTCL +GF P D+ V W    Q  P   Y T     + S G  +F +
Sbjct  235   PPSEELALNELVTLTCLARGFSPKDVLVRWLQGSQELPREKYLTWASRQEPSQGTTTFAV  294

Query  290   YSKLTVDKSRWQQGNVFSCSVMHEALHNHYTQKSLSLSPGK  330
              S L V    W++G+ FSC V HEAL   +TQK++    GK
Sbjct  295   TSILRVAAEDWKKGDTFSCMVGHEALPLAFTQKTIDRLAGK  335
```

K409 S364 K370 F405

FIG. 33b

Query : Human Immunoglobulin Light chain (Kappa)

Subject : Human Immunoglobulin Light chain (Lambda)

| Score | Expect | Method | Identities | Positives | Gaps |
|---|---|---|---|---|---|
| 151 bits(382) | 2e-50 | Compositional matrix adjust. | 90/233(39%) | 138/233(59%) | 12/233(5%) |

```
Query  7    VLMLLLLWVSGTCGDILLTQSPAILSVSPGERVSFSCRASQFVGSSIHWYQQRTNGSPRL  66
            + + +L + +G+      LTQ P ++SVSPG+  S +C    +    WYQ +    SP L
Sbjct  6    LFLGVLAYCTGSVASYELTQ-PPLVSVSPGQTASITCSGDKLGDNYACWYQLKPGQSPVL  64

Query  67   LIKYASESMSGIPSRFSGSGSGTDFTLSINTVESEDIADYYCQ---QSHSWPFTFGSGTN  123
            +I   +    SGIP R SGS SG   TL+I+  ++ D A+YYCQ    S SW   FG GT
Sbjct  65   VIYQDNRRPSGIPERISGSNSGNTATLTISGTQAMDEAEYYCQAWDSSTSW--VFGGGTK  122

Query  124  LEV-KRTVAAPSVFIFPPSDEQLKSGTASVVCLLNNFYPREAKVQWKVDNA-LQSGNSQE  181
            L V  +   AAPSV +FPPS E+L++   A++VCL+++FYP     V WK D++ +++G   E
Sbjct  123  LTVLGQPKAAPSVTLFPPSSEELQANKATLVCLISDFYPGAVTVAWKADSSPVKAG--VE  180

Query  182  SVTEQDSKDSTYSLSSTLTLSKADYEKHKVYACEVTHQGLSSPVTKSFNRGEC  234
            + T     ++ Y+ SS L+L+    ++ H+ Y+C+VTH+G  S V K+    EC
Sbjct  181  TTTPSKQSNNKYAASSYLSLTPEQWKSHRSYSCQVTHEG--STVEKTVAPTEC  231
```

S131 & V133

FIG. 33c

$C_H1$ Sequences

L145 and S183 are boxed in the aligned sequences.

| | | |
|---|---|---|
| Human | IgG1 | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKV |
| Human | IgG2 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTV |
| Human | IgG3 | ASTKGPSVFPLAPCSRSTSGGTAALGCLVKDYFPEPVTVSWNSGALTSGVHTFPAVLQSSGLYSLSSVVTVPSSSLGTQTYTCNVNHKPSNTKVDKRV |
| Human | IgG4 | ASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRV |
| Mouse | IgG1 | AKTTPPSVYPLAPGSAAQTNSMVTLGCLVKGYFPEPVTVTWNSGSLSSGVHTFPAVLQS_DLYTLSSSVTVPSSPRPSETVTCNVAHPASSTKVDKKI |
| Mouse | IgG2a[b] | AKTTAPSVYPLAPVCGGTTGSSVTLGCLVKGYFPEPVTLTWNSGSLSSGVHTFPALLQS_GLYTLSSSVTVTSNTWPSQTITCNVAHPASSTKVDKKI |
| Mouse | IgG2a[a] | AKTTAPSVYPLAPVCGDTTGSSVTLGCLVKGYFPEPVTLTWNSGSLSSGVHTFPAVLQS_DLYTLSSSVTVTSSTWPSQSITCNVAHPASSTKVDKKI |
| Mouse | IgG2b | AKTTPPSVYPLAPGCGDTTGSSVTSGCLVKGYFPEPVTVTWNSGSLSSSVHTFPALLQS_GLYTMSSSVTVPSSTWPSQTVTCSVAHPASSTTVDKKL |
| Mouse | IgG3 | _TTTAPSVYPLVPGCSDTSGSSVTLGCLVKGYFPEPVTVKWNYGALSSGVRTVSSVLQS_GFYSLSSLVTVPSSTWPSQTVICNVAHPASKTELIKRI |
| Rat | IgG1 | AETTAPSVYPLAPGTALKSNSMVTLGCLVKGYFPEPVTVTWNSGALSSGVHTFPAVLQS_GLYTLTSSVTVPSSTWSSQAVTCNVAHPASSTKVDKKI |
| Rat | IgG2a | AETTAPSVYPLAPGTALKSNSMVTLGCLVKGYFPEPVTVTWNSGALSSGVHTFPAVLQS_GLYTLTSSVTVPSSTWPSQTVTCNVAHPASSTKVDKKI |
| Rat | IgG2b | AQTTAPSVYPLAPGCGDTTSSTVTLGCLVKGYFPEPVTVTWNSGALSSDVHTFPAVLQS_GLYTLTSSVT__SSTWPSQTVTCNVAHPASSTKVDKKV |
| Rat | IgG2c | ATTTAPSVYPLVPGCSGTSGSLVTLGCLVKGYFPEPVTVKWNSGALSSGVHTFPAVLQS GLYTLSSSVTVPSSTWSSQTVTCSVAHPATKSNLIKRI |

FIG. 33d

S364 K370 F405 K409

$C_H3$ Sequences

| | | |
|---|---|---|
| Human | IgG1 | GQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| Human | IgG2 | GQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |
| Human | IgG3 | GQPREPQVYTLPPSREEMTKNQVSLTCLVKGFYPSDIAVEWESSGQPENNYNTTPPMLDSDGSFFLYSKLTVDKSRWQQGNIFSCSVMHEALHNRFTQKSLSLSPGK |
| Human | IgG4 | GQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK |
| Mouse | IgG1 | GRPKAPQVYTIPPPKEQMAKDKVSLTCMITDFFPEDITVEWQWNGQPAENYKNTQPIMNTNGSYFVYSKLNVQKSNWEAGNTFTCSVLHEGLHNHHTEKSLSHSPGK |
| Mouse | IgG2a[b] | GPVRAPQVYVLPPPAEEMTKKEFSLTCMITGFLPAEIAVDWTSNGRTEQNYKNTATVLDSDGSYFMYSKLRVQKSTWERGSLFACSVVHEVLHNHLTTKTISRSLGK |
| Mouse | IgG2a[a] | GSVRAPQVYVLPPPEEEMTKKQVTLTCMVTDFMPEDIYVEWTNNGKTELNYKNTEPVLDSDGSYFMYSKLRVEKKNWVERNSYSCSVVHEGLHNHHTTKSFSRTPGK |
| Mouse | IgG2b | GLVRAPQVYTLPPPAEQLSRKDVSLTCLVVGFNPGDISVEWTSNGHTEENYKDTAPVLDSDGSYFIYSKLNMKTSKWEKTDSFSCNVRHEGLKNYYLKKTISRSPGK |
| Mouse | IgG3 | GRAQTPQVYTIPPPREQMSKKKVSLTCLVTNFFSEAISVEWERNGELEQDYKNTPPILDSDGTYFLYSKLTVDTDSWLQGEIFTCSVVHEALHNHHTQKNLSRSPGK |
| Rat | IgG1 | GRTQVPHVYTMSPTKEEMTQNEVSITCMVKGFYPPDIYVEWQMNGQPQENYKNTPPTMDTDGSYFLYSKLNVKKEKWQQGNTFTCSVLHEGLHNHHTEKSLSHSPGK |
| Rat | IgG2a | GTPRGPQVYTMAPPKEEMTQSQVSITCMVKGFYPPDIYTEWKMNGQPQENYKNTPPTMDTDGSYFLYSKLNVKKETWQQGNTFTCSVLHEGLHNHHTEKSLSHSPGK |
| Rat | IgG2b | GLVRKPQVYVMGPPTEQLTEQTVSLTCLTSGFLPNDIGVEWTSNGHIEKNYKNTEPVMDSDGSFFMYSKLNVERSRWDSRAPFVCSVVHEGLHNHHVEKSISRPPGK |
| Rat | IgG2c | GKARTPQVYTIPPPREQMSKNKVSLTCMVTSFYPASISVEWERNGELEQDYKNTLPVLDSDESYFLYSKLSVDTDSWMRGDIYTCSVVHEALHNHHTQKNLSRSPGK |

FIG. 34

c34Φf51

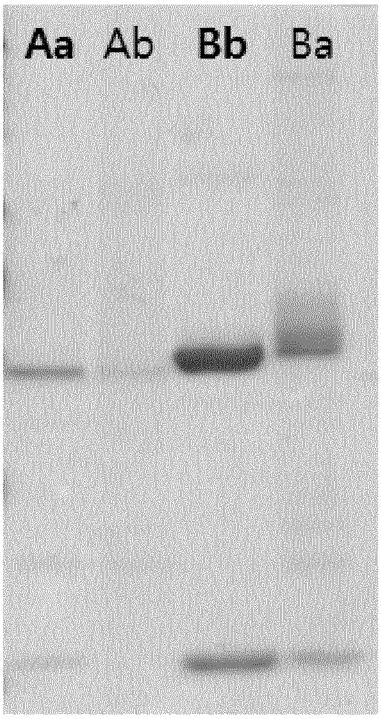

FIG. 35

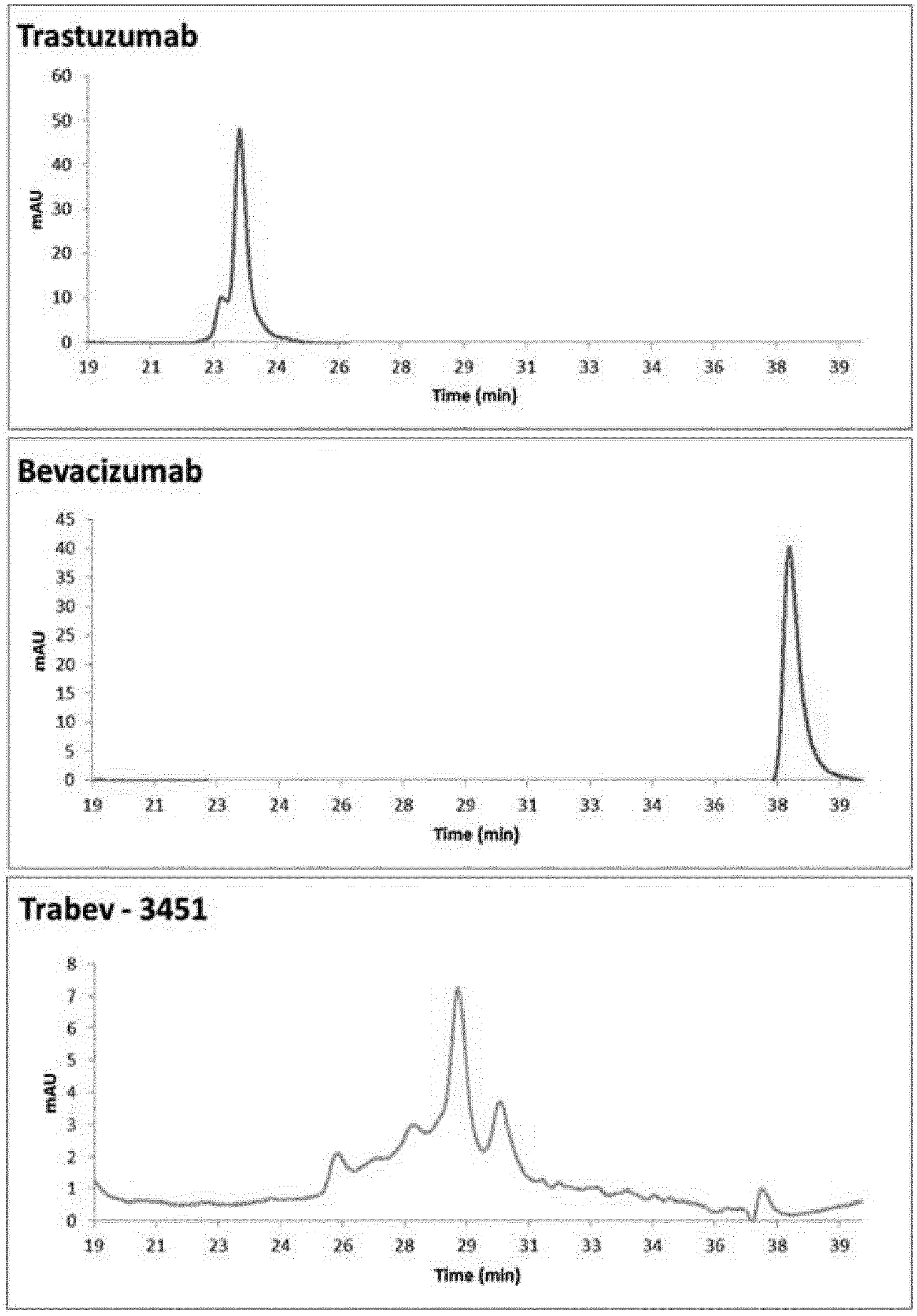
Trastuzumab
60
50
40
30
20
10
0
mAU
19
21
23
24
26
28
29
31
33
34
36
38
39
Time (min)
Bevacizumab
45
40
35
30
25
20
15
10
5
0
mAU
19
21
23
24
26
28
29
31
33
34
36
38
39
Time (min)
Trabev - 3451
8
7
6
5
4
3
2
1
0
mAU
19
21
23
24
26
28
29
31
33
34
36
38
39
Time (min)

FIG. 36

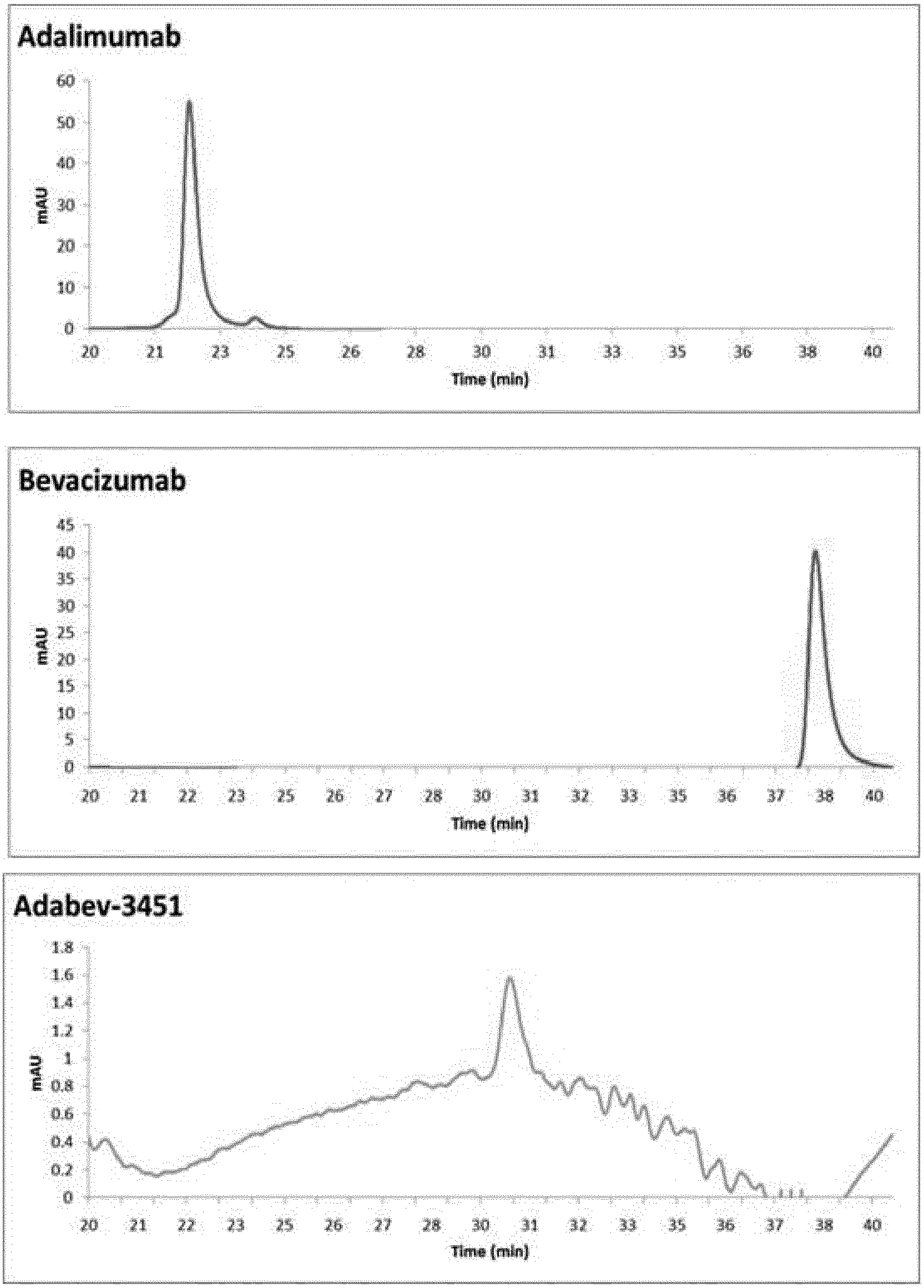
Adalimumab
mAU
60
50
40
30
20
10
0
20 21 23 25 26 28 30 31 33 35 36 38 40
Time (min)
Bevacizumab
mAU
45
40
35
30
25
20
15
10
5
0
20 21 22 23 25 26 27 28 30 31 32 33 35 36 37 38 40
Time (min)
Adabev-3451
mAU
1.8
1.6
1.4
1.2
1
0.8
0.6
0.4
0.2
0
20 21 22 23 25 26 27 28 30 31 32 33 35 36 37 38 40
Time (min)

FIG. 37

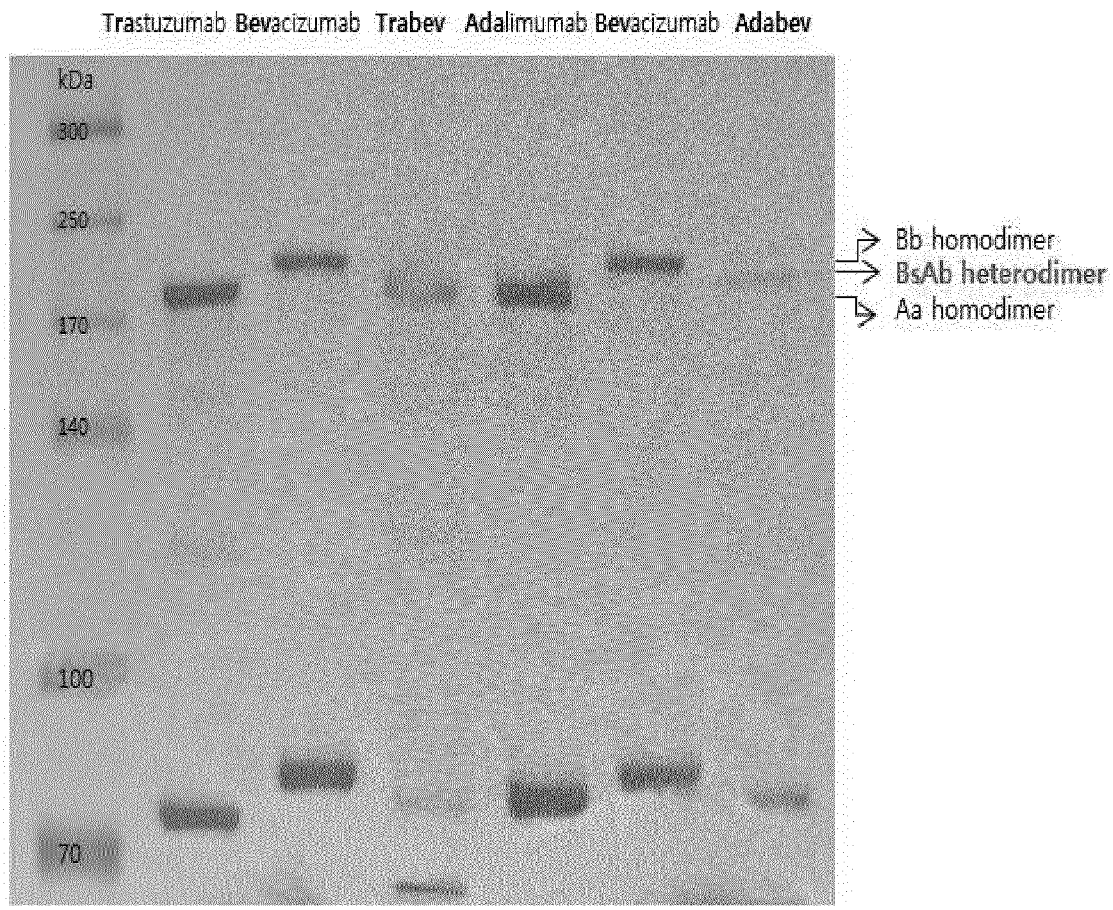
Trastuzumab
Bevacizumab
Trabev
Adalimumab
Bevacizumab
Adabev
kDa
300
250
170
140
100
70
Bb homodimer
BsAb heterodimer
Aa homodimer

FIG. 38

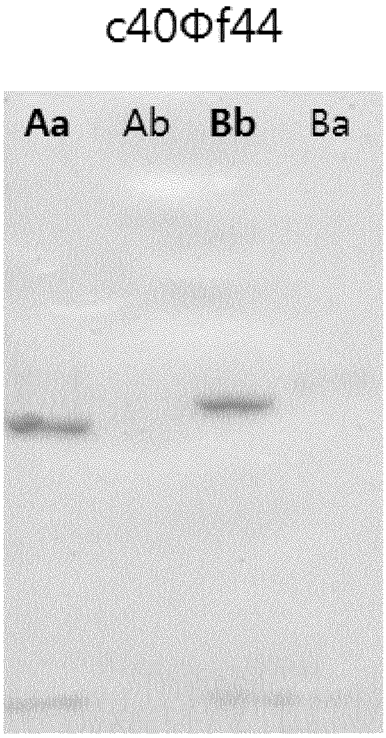

FIG. 39

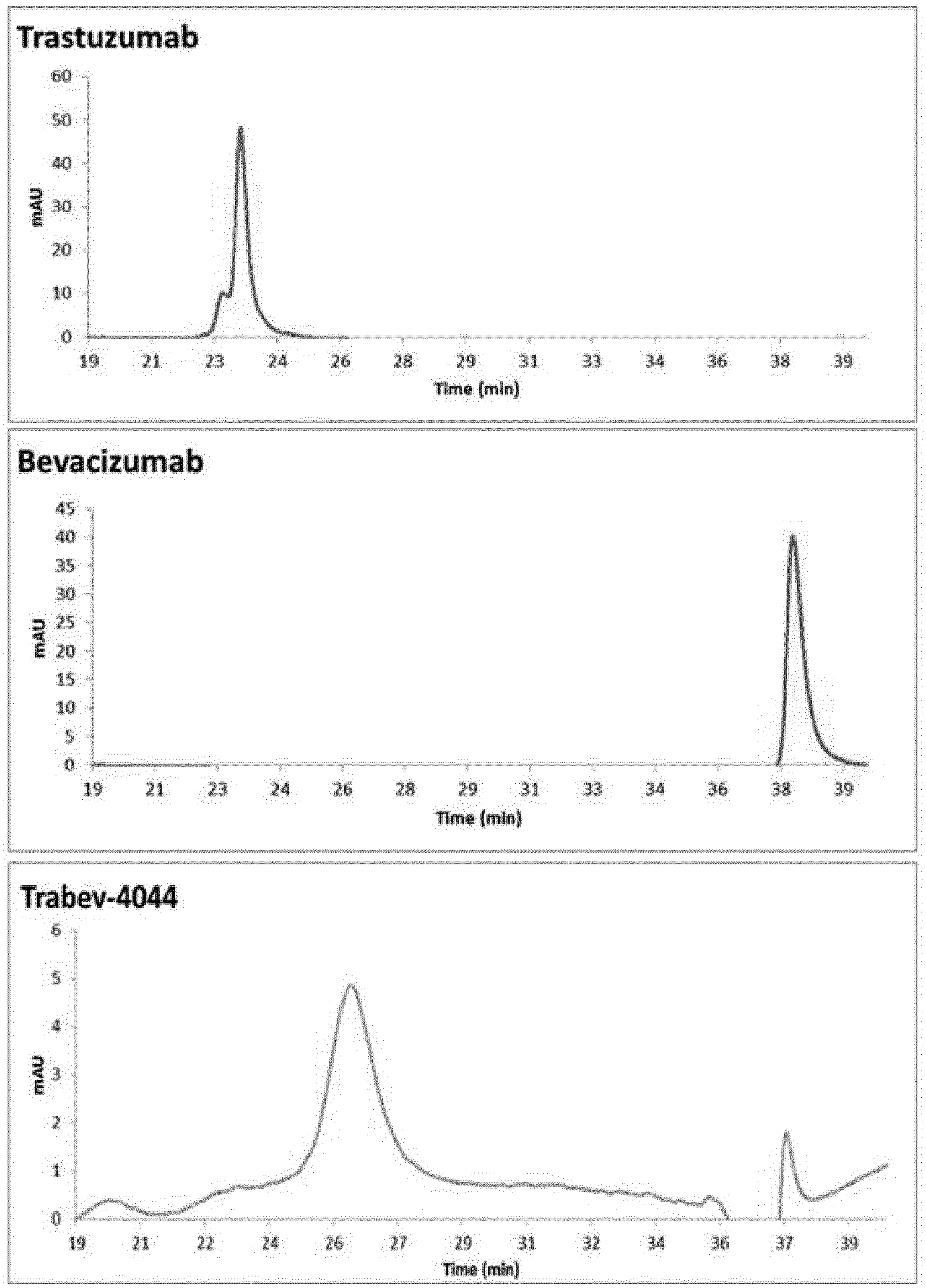
Trastuzumab
mAU
60
50
40
30
20
10
0
19
21
23
24
26
28
29
31
33
34
36
38
39
Time (min)
Bevacizumab
mAU
45
40
35
30
25
20
15
10
5
0
19
21
23
24
26
28
29
31
33
34
36
38
39
Time (min)
Trabev-4044
mAU
6
5
4
3
2
1
0
19
21
22
24
26
27
29
31
32
34
36
37
39
Time (min)

FIG. 40

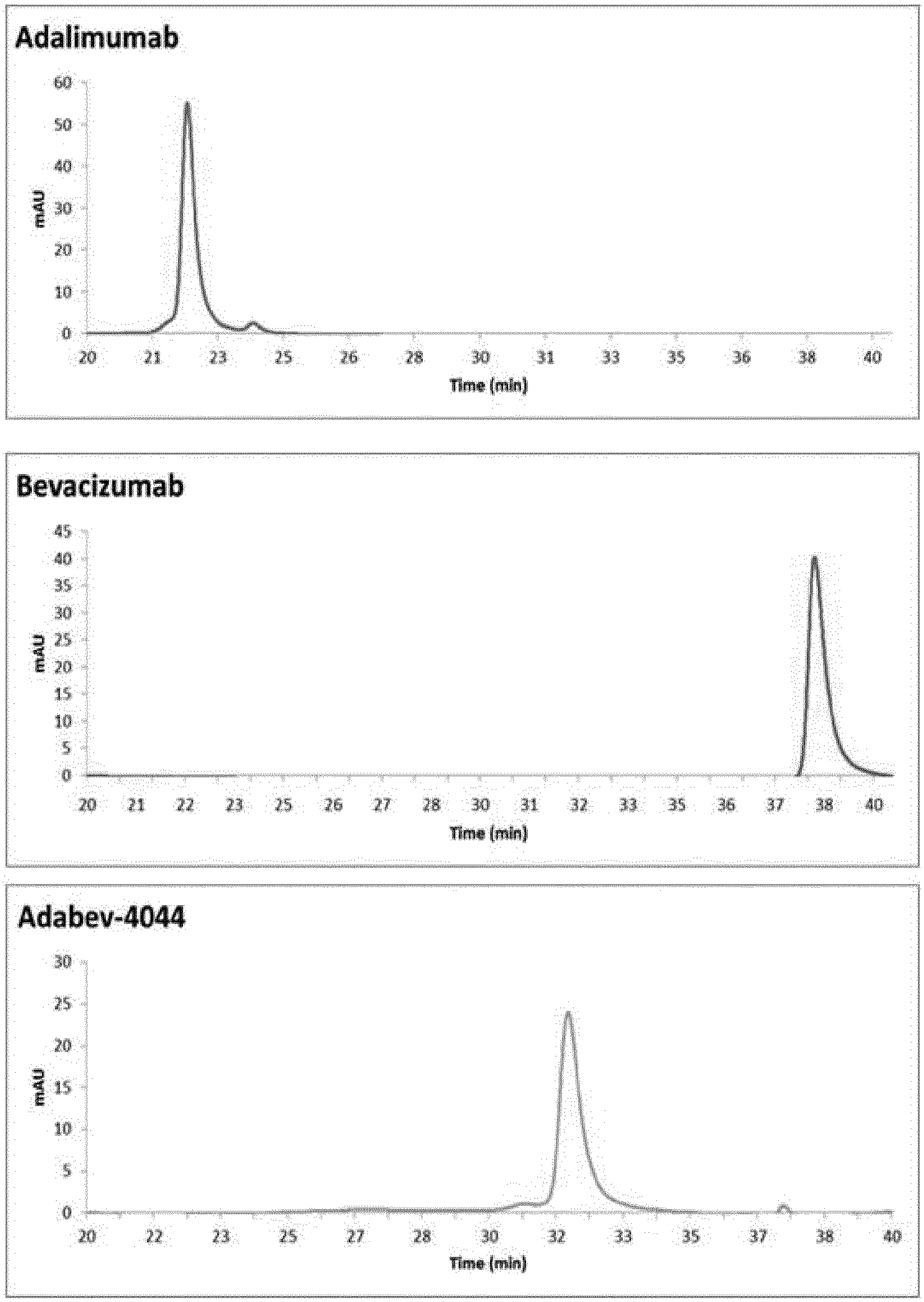
Adalimumab
60
50
40
30
20
10
0
mAU
20 21 23 25 26 28 30 31 33 35 36 38 40
Time (min)
Bevacizumab
45
40
35
30
25
20
15
10
5
0
mAU
20 21 22 23 25 26 27 28 30 31 32 33 35 36 37 38 40
Time (min)
Adabev-4044
30
25
20
15
10
5
0
mAU
20 22 23 25 27 28 30 32 33 35 37 38 40
Time (min)

FIG. 41

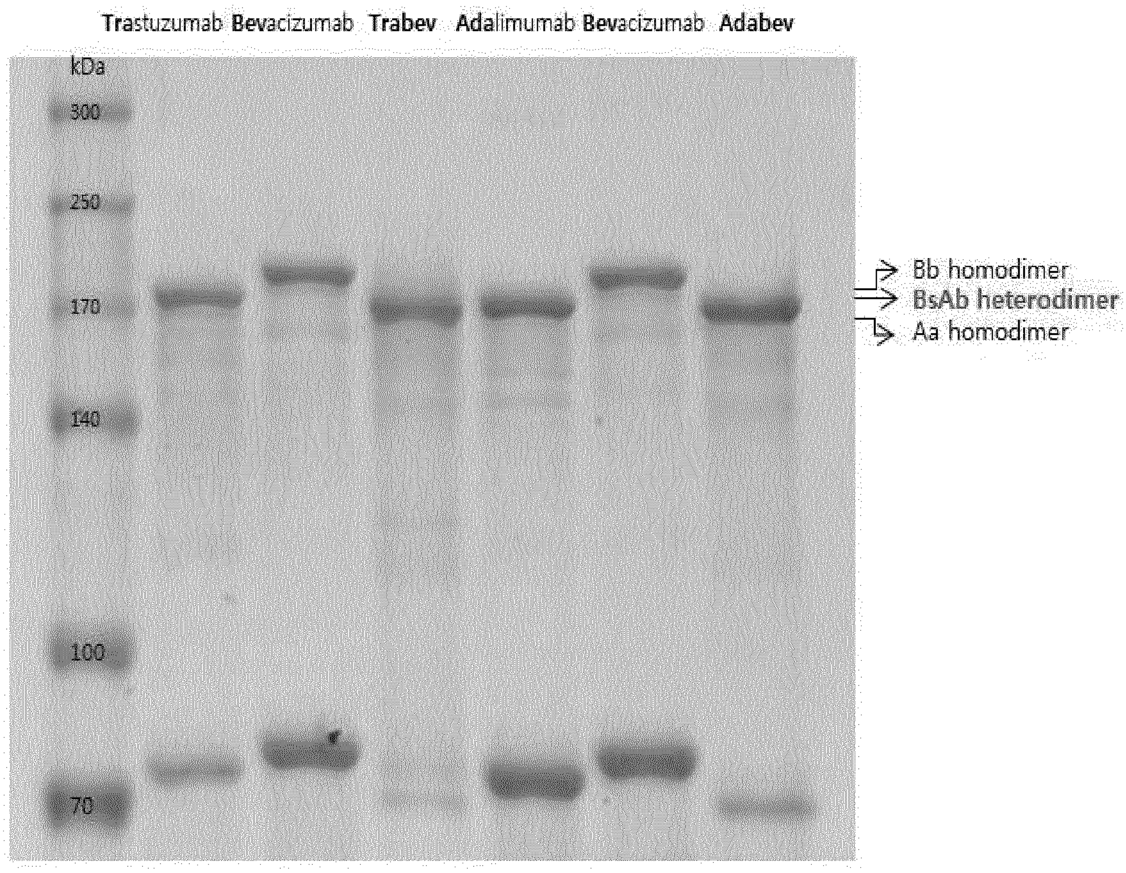
Trastuzumab
Bevacizumab
Trabev
Adalimumab
Bevacizumab
Adabev
kDa
300
250
170
140
100
70
Bb homodimer
BsAb heterodimer
Aa homodimer

FIG. 42

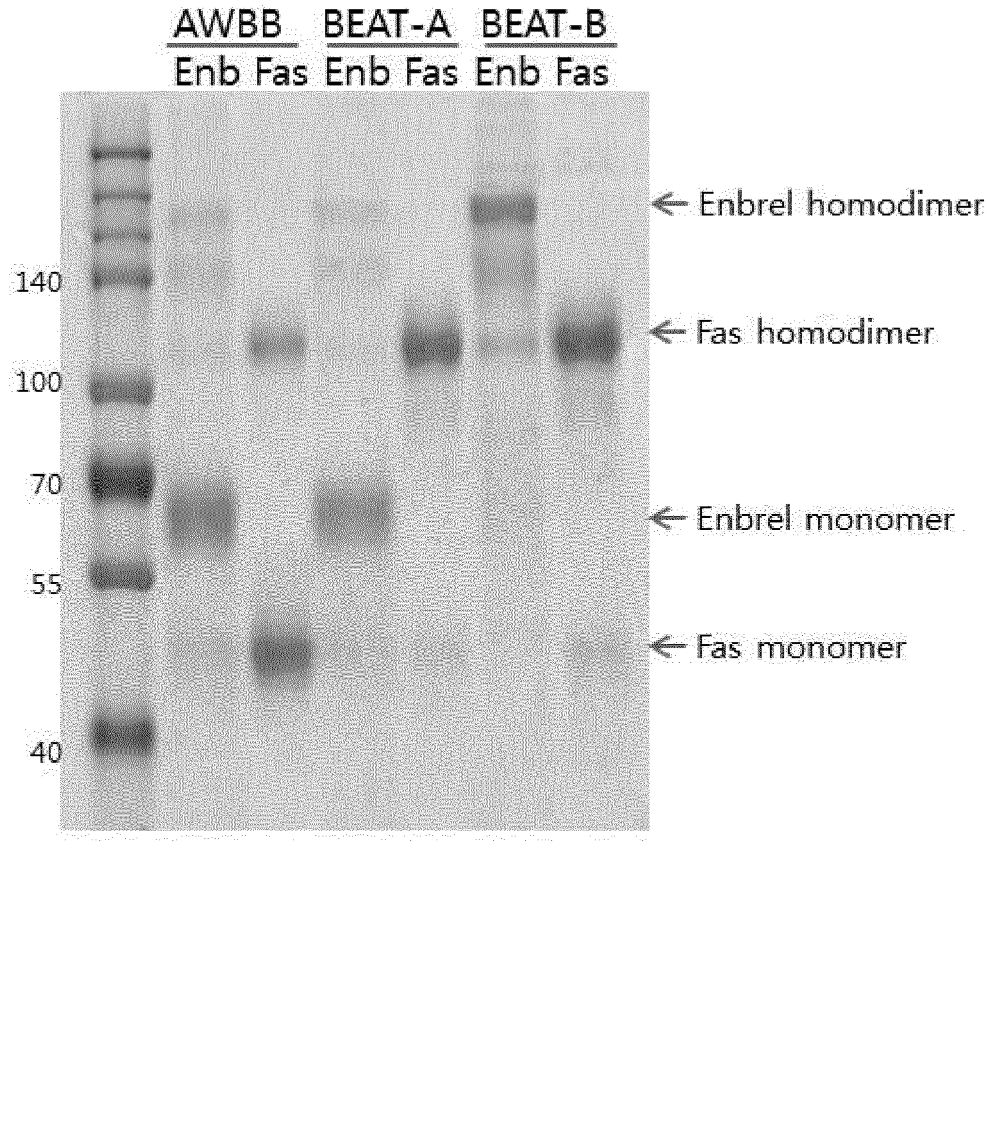
AWBB
BEAT-A
BEAT-B
Enb Fas Enb Fas Enb Fas
140
100
70
55
40
Enbrel homodimer
Fas homodimer
Enbrel monomer
Fas monomer

## INTERNATIONAL SEARCH REPORT

International application No.
PCT/KR2017/007791

A. CLASSIFICATION OF SUBJECT MATTER

*C07K 16/46(2006.01)i*

According to International Patent Classification (IPC) or to both national classification and IPC

B. FIELDS SEARCHED

Minimum documentation searched (classification system followed by classification symbols)
C07K 16/46; A61K 39/395; C07K 19/00; C07K 16/18; C07K 16/00; C12N 15/13; C12P 21/06

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: dual-specificity protein, antibody, swapping modification, CH3, CH1

C. DOCUMENTS CONSIDERED TO BE RELEVANT

| Category* | Citation of document, with indication, where appropriate, of the relevant passages | Relevant to claim No. |
|---|---|---|
| X | WO 2014-142591 A1 (IBENTRUS, INC.) 18 September 2014<br>See claims 4, 13-16, 19, 23. | 1-3,19-27,43-45 |
| Y | | 4-7,10-13,16-17,28-31,34-37,40-41 |
| A | | 8-9,14-15,18,32-33,38-39,42,46-50 |
| Y | WO 2014-084607 A1 (AJOU UNIVERSITY INDUSTRY-ACADEMIC COOPERATION FOUNDATION) 05 June 2014<br>See claims 1-2, 13-17, 26. | 4-7,10-13,16-17,28-31,34-37,40-41 |
| X | KR 10-2014-0084249 A (CHUGAI SEIYAKU KABUSHIKIKAISHA) 04 July 2014<br>See abstract; paragraphs [0256]-[0260], [0274]-[0275]; and claims 1, 3. | 1-7,21-24 |
| A | US 2010-0286374 A1 (KANNAN, Gunasekaran et al.) 11 November 2010<br>See claims 1-34. | 1-50 |
| A | FAN, Gaowei et al.," Bispecific Antibodies and Their Applications", Journal of Hematology & Oncology, 2015, vol. 8, Article No. 130, internal pages 1-14<br>See page 5. | 1-50 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 NOVEMBER 2017 (01.11.2017) | **01 NOVEMBER 2017 (01.11.2017)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea | |
| Facsimile No. +82-42-481-8578 | Telephone No. |

Form PCT/ISA/210 (second sheet) (January 2015)

INTERNATIONAL SEARCH REPORT
Information on patent family members

International application No.
PCT/KR2017/007791

| Patent document cited in search report | Publication date | Patent family member | Publication date |
|---|---|---|---|
| WO 2014-142591 A1 | 18/09/2014 | AU 2014-230291 A1 | 05/11/2015 |
| | | CA 2918328 A1 | 18/09/2014 |
| | | CN 105229036 A | 06/01/2016 |
| | | EP 2975061 A1 | 20/01/2016 |
| | | EP 2975061 A4 | 01/03/2017 |
| | | KR 10-2016-0012151 A | 02/02/2016 |
| | | US 2016-0152726 A1 | 02/06/2016 |
| WO 2014-084607 A1 | 05/06/2014 | AU 2013-352812 A1 | 16/07/2015 |
| | | CA 2892623 A1 | 05/06/2014 |
| | | CN 104955953 A | 30/09/2015 |
| | | EA 201591038 A1 | 30/11/2015 |
| | | EP 2927321 A1 | 07/10/2015 |
| | | EP 2927321 A4 | 15/06/2016 |
| | | JP 2016-506377 A | 03/03/2016 |
| | | KR 10-1522954 B1 | 27/05/2015 |
| | | KR 10-2014-0067944 A | 05/06/2014 |
| | | US 2015-0307628 A1 | 29/10/2015 |
| KR 10-2014-0084249 A | 04/07/2014 | BR 112014010257 A2 | 18/04/2017 |
| | | CA 2853230 A1 | 10/05/2013 |
| | | CN 104011207 A | 27/08/2014 |
| | | EP 2787078 A1 | 08/10/2014 |
| | | EP 2787078 A4 | 17/06/2015 |
| | | MX 2014005045 A | 22/08/2014 |
| | | RU 2014-122028 A | 10/12/2015 |
| | | US 2014-0370020 A1 | 18/12/2014 |
| | | WO 2013-065708 A1 | 10/05/2013 |
| US 2010-0286374 A1 | 11/11/2010 | AU 2009-204501 A1 | 16/07/2009 |
| | | AU 2009-204501 B2 | 12/02/2015 |
| | | CA 2709847 A1 | 16/07/2009 |
| | | CY 1117162 T1 | 05/04/2017 |
| | | DK 2235064 T3 | 11/01/2016 |
| | | EP 2235064 A1 | 06/10/2010 |
| | | EP 2235064 B1 | 25/11/2015 |
| | | EP 3061770 A1 | 31/08/2016 |
| | | ES 2563027 T3 | 10/03/2016 |
| | | HU E028536 T2 | 28/12/2016 |
| | | JP 2011-508604 A | 17/03/2011 |
| | | JP 2016-093175 A | 26/05/2016 |
| | | JP 6157046 B2 | 05/07/2017 |
| | | PT 2235064 E | 01/03/2016 |
| | | SI 2235064 T1 | 29/04/2016 |
| | | US 2014-0024111 A1 | 23/01/2014 |
| | | US 8592562 B2 | 26/11/2013 |
| | | WO 2009-089004 A1 | 16/07/2009 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2013892198 A, Zymeworks
- WO 2014142591 A
- KR 1020140019385 A


### Non-patent literature cited in the description


- **KABAT et al.** Sequences of Proteins of Immunological Interest. Public Health Service, National Institutes of Health, 1991
- **PAUL, W.** Fundamental Immunology. Raven Press, 1989
- **CHOTHIA ; LESK.** *J. Mol. Biol.,* 1987, vol. 196, 901-917